# EUROPEAN PATENT APPLICATION

(11) **EP 4 796 542 A1**
(43) Date of publication of application: **26.08.2026**
(21) Application number: 25160026.8
(22) Date of filing: 25.02.2025
(51) Int. Cl.: C07D 471/04, C07D 487/04, A01N 43/90, A01P 5/00, A01P 7/04

(54) **HETEROBICYCLIC COMPOUNDS FOR THE CONTROL OF INVERTEBRATE PESTS**

(71) Applicant: BASF Agricultural Solutions Deutschland GmbH, 67117 Limburgerhof (DE)
(72) Inventor: SCHISSLER, Christoph, 67056 Ludwigshafen am Rhein (DE); POHLMAN, Matthias, 67056 Ludwigshafen am Rhein (DE); CHAUDHURI, Rupsha, 67056 Ludwigshafen am Rhein (DE); WAKEHAM, Matthew Charles Linford, 67056 Ludwigshafen am Rhein (DE)
(74) Representative: Maiwald GmbH

(57) **Abstract**

The present invention is in the field of agricultural or veterinary applications and relates to compounds of formula (I), as described herein, that have pesticidal activity and uses thereof as well as methods of use.

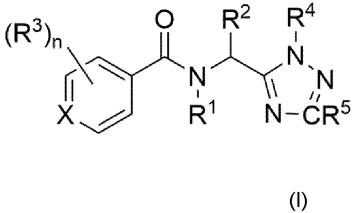

## Description

### Field of the invention

The present invention is in the field of agricultural or veterinary applications and relates to compounds that have pesticidal activity and uses thereof as well as methods of use, as described herein.

### Background of the invention

Invertebrate pests and in particular insects, arachnids and nematodes destroy growing and harvested crops and attack wooden dwelling and commercial structures, thereby causing large economic loss to the food supply and to property. Accordingly, agents for combating invertebrate pests are widely used in this field.

Due to the ability of target pests to develop resistance to pesticidally-active agents, there is an ongoing need to identify further compounds, which are suitable for combating invertebrate pests such as insects, arachnids and nematodes. Furthermore, there is a need for new compounds having a high pesticidal activity and showing a broad activity spectrum against a large number of different invertebrate pests, especially against difficult to control insects, arachnids and nematodes.

International patent publications WO2017/192385, WO2019/170626, WO2019/197468, WO2019/206799, WO2021/165195 and WO2021/224323 describe structurally related compounds which are mentioned to be useful for combating invertebrate pests.

Nevertheless, there remains a need for highly effective and versatile agents for combating invertebrate pests. It is therefore an object of the invention to provide additional compounds having a good pesticidal activity and showing a broad activity spectrum against a large number of different invertebrate pests, especially against difficult to control pests, such as insects.

It has been found that these objects can be achieved by compounds of formula I as depicted and defined herein below, and by their N-oxides, stereoisomers, tautomers, and agriculturally or veterinarily acceptable salts thereof.

### Summary of the invention

It has been found that these objects can be achieved by heterobicyclic compounds of formula I, as depicted and defined below, including their stereoisomers, their salts, in particular their agriculturally or veterinarily acceptable salts, their tautomers and their N-oxides.

In a first aspect, the present invention relates to the compounds of formula I, 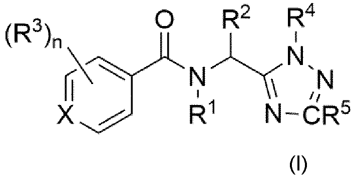 wherein
- R¹: is H, OR^{10a}, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, wherein the alkyl, alkoxy, alkenyl, alkynyl, and cycloalkyl moieties are unsubstituted or substituted with R¹¹; C(=N-R¹³)R¹², or C(O)R^{11a};
- R²: is H, C₁-C₃-alkyl, C₂-C₃-alkenyl, C₃-C₆-cycloalkyl, or C₂-C₃-alkynyl, wherein the alkyl, alkenyl, alkynyl and cycloalkyl moieties are unsubstituted or substituted with halogen;
- R³: is halogen, CN, NO₂, SF₅; C₁-C₆-alkyl, C₁-C₆-alkoxy, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, alkoxy, heterocyclyl and cycloalkyl moieties are unsubstituted or substituted with R¹¹; OR^{10a}, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a}; or
two R³ bound to two adjacent C-atoms can form a 4-, 5-, or 6-membered ring, which may contain one or two heteroatoms selected from N, O, and S as ring members, wherein the ring is unsubstituted or substituted with halogen, CN, C₁-C₃-alkyl, and/or C₁-C₃-haloalkyl;
- R⁴: is a heterocyclic ring which is selected from the group consisting of 8-, 9- or 10-membered saturated or partially unsaturated bicyclic heterocyclyl, and 8- 9- or 10- membered bicyclic hetaryl rings, wherein the heterocyclyl moieties are unsubstituted or substituted by 1, 2, 3 or 4 substituents and the hetaryl moieties are unsubstituted or substituted by 1, 2 or 3 substituents, wherein the substituents are independently selected from the group consisting of halogen, =O (oxo), -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C,-Cₑ-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, S(O)ₘ-R¹⁴, OR^{10a}, NR¹²R¹³, C(O)NR¹²R¹³, C(O)OR¹⁰, C(O)R¹⁴, - C(=NOC₁-C₄-alkyl)H, -C(=NOC₁-C₄-alkyl)-C₁-C₄-alkyl or -C(=NOC₁-C₄-alkyl)-C₃-C₆-cycloalkyl, wherein alkyl, alkenyl, alkynyl, cycloalkyl, and alkoxy are unsubstituted or substituted with R¹¹;
- R⁵: is H, halogen, CN, NH₂, NO₂, CONH₂, C₁-C₆-alkyl, or C₃-C₆-cycloalkyl, C₃-C₆-cycloalkyl-C₁-C₆-alkyl, C₁-C₆-alkyl-C₃-C₆-cycloalkyl, C₁-C₆-alkoxy, CH(C₁-C₆-alkoxy)₂, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, OR^{10a}, NR¹²R¹³, C(O)NR¹²R¹³, C(O)OR¹⁰, C(O)R¹⁴, S(O)ₘ-R¹⁴, -C(=NOC₁-C₄-alkyl)H, or -C(=NOC₁-C₄-alkyl)-C₁-C₄-alkyl, wherein alkyl, alkenyl, alkynyl, cycloalkyl and alkoxy are unsubstituted or substituted with R¹¹;
- R¹⁰: is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
- R^{10a}: is H, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
- R¹¹: is halogen, CN, NO₂, NR¹²R¹³, C(O)NH₂, C(S)NH₂, C(O)OH, OR¹⁰, Si(CH₃)₃; 3- to 6-membered heterocyclyl, 5- or 6-membered hetaryl, or phenyl, wherein the heterocyclyl, hetaryl and phenyl moieties are unsubstituted or substituted with halogen, C₁-C₃-haloalkyl, and/or CN;
- R^{11a}: is NR¹²R¹³, C(O)NH₂, C(S)NH₂, C(O)OH, OR¹⁰, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, wherein the cycloalkyl moiety is unsubstituted or substituted with 1 or 2 halogen; or 3- to 6-membered heterocyclyl, wherein the heterocyclyl moiety is unsubstituted or substituted with halogen, C₁-C₃-haloalkyl, and/or CN;
- R¹², R¹³: are independently from each other H, C₁-C₄-alkyl, C₁-C₄-alkoxy, C₁-C₄-haloalkoxy, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, wherein the phenyl, heterocyclyl and hetaryl moieties are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, wherein the phenyl, heterocyclyl and hetaryl moieties are unsubstituted or substituted with halogen, C₁-C₃-haloalkyl, and/or CN; or
R¹² and R¹³ together with the atom(s) to which they are bound, form a 3-, 4-, 5-, 6-, or 7-membered saturated, partially or fully unsaturated heterocycle, which heterocycle may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R^{12a}, R^{13a}: are independently from each other C₁-C₄-alkyl, C₁-C₄-haloalkyl, C₃-C₆-cycloalkyl, unsubstituted or substituted with halogen, C₁-C₃-haloalkyl, and/or CN; or R^{12a} and R^{13a} together with the atom to which they are bound, form a 3-, 4-, 5-, 6-, or 7-membered saturated, partially or fully unsaturated heterocycle, wherein the heterocycle moiety may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R¹⁴: is H, C₁-C₆-alkyl, C₃-C₆-cycloalkyl, wherein the alkyl and cycloalkyl moieties are unsubstituted or partially or fully substituted with R¹¹; or 3- to 6-membered heterocyclyl, 5- or 6-membered hetaryl, or phenyl, wherein the phenyl, heterocyclyl and hetaryl moieties are unsubstituted or substituted with R^{a};
- R^{a}: is halogen, CN, NO₂, OH, C₁-C₄-alkyl, C₁-C₄-alkoxy, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl;
- m: is 0, 1, or 2;
- n: is 0, 1, 2, or 3;
- X: is N, CH, or CR³;
and the N-oxides, stereoisomers, tautomers, and agriculturally or veterinarily acceptable salts thereof.

In various embodiments, R¹ is H, methyl or CH₂-cyclopropyl.

In various embodiments, R² is H or C₁-C₃-alkyl, preferably methyl.

In various embodiments, X is CR³, preferably CH.

In various embodiments, n is 2 and the two R³ groups are in positions 3 and 5 of the phenyl ring, i.e. in meta positions relatively to its attachment point to the remainder of the structure.

In various embodiments, at least one R³ group in (R³)ₙ is selected from R^{3d}, wherein
each R^{3d} is independently C₁-C₆-alkyl, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, wherein alkyl, alkenyl, alkynyl and cycloalkyl are unsubstituted or substituted with halogen, CN, NO₂, OH, NR¹²R¹³, C(O)NR¹²R¹³, C(O)OR¹⁰, C(O)R¹⁴, OS(O)₂-R¹⁴ and S(O)ₘ-R¹⁴ wherein m is 1 or 2.

In various embodiments, R⁴ is selected from any one of: 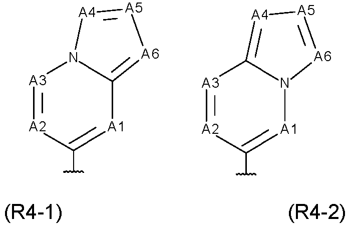 wherein
A1, A2 and A3 are independently CR^{4a} or N, provided not more than two of A1, A2 and A3 are N and at least one of A1 and A2 is N;
A4, A5 and A6 are independently CR^{4a} or N, provided not more than two of A4, A5 and A6 are N;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, - N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl;
with the provisos that
not more than four of A1, A2, A3, A4, A5 and A6 are N;
if in R4-1 A3 and A4 are N, A2 and A5 are not N;
if in R4-1 A2 and A3 are N, A4 is not N;
if in R4-1 A4 and A5 are N, A3 and A6 are not N;
if in R4-2 A1 and A6 are N, A5 is not N;
if in R4-2 A5 and A6 are N, A1 and A4 are not N,
wherein preferably one of A1 and A2 is N and the other is CH or CCH₃.

In various other embodiments, R⁴ is selected from any one of the following groups 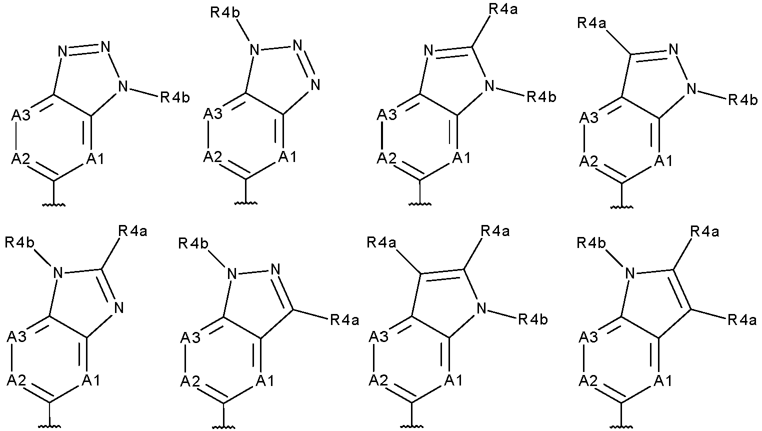 wherein
A1, A2 and A3 are independently CR^{4a} or N, provided not more than two of A1, A2 and A3 are N and at least one of A1 and A2 is N;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, - N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; and
R^{4b} is selected from H, halogen, -OH, -CN, -COOH, -CONH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, - CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl.

In still other embodiments, R⁴ is selected from any one of the following 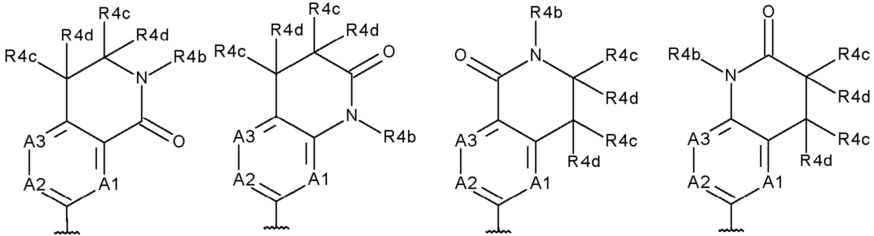 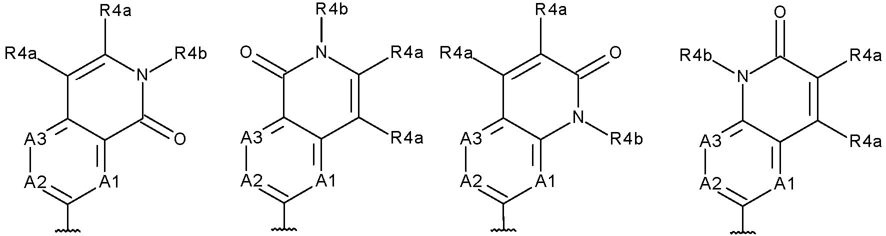 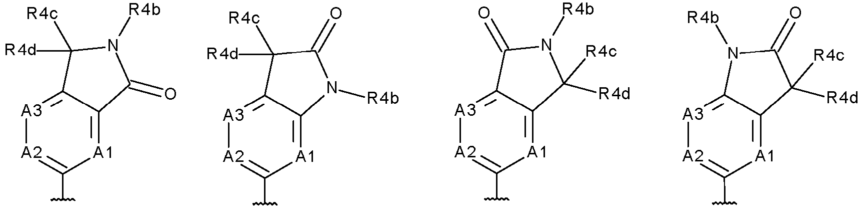 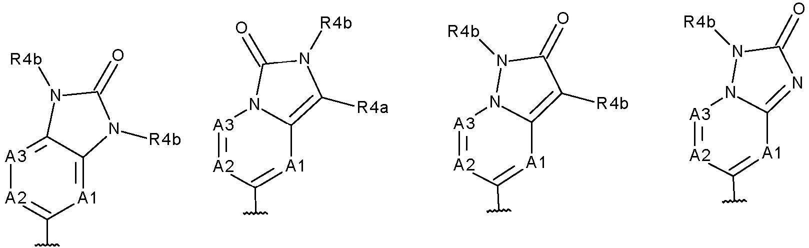 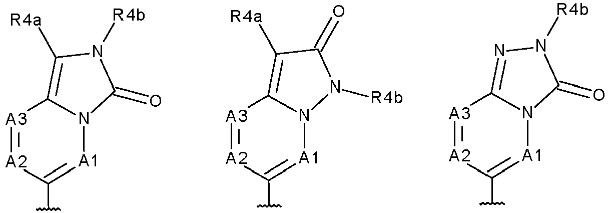 wherein
A1, A2 and A3 are independently CR^{4a} or N, provided not more than two of A1, A2 and A3 are N and at least one of A1 and A2 is N;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, - N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl;
R^{4b} is selected from H, halogen, -OH, -CN, -COOH, -CONH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, - CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; and
R^{4c} and R^{4d} are independently from each other selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, or
R^{4c} and R^{4d} together with the carbon atom to which they are bound, form a 3-, 4-, 5-, or 6-membered saturated or unsaturated carbocyclic ring.

In still other embodiments, R⁴ is selected from any one of the following:

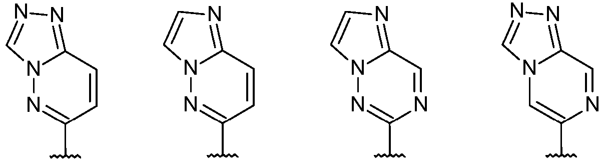

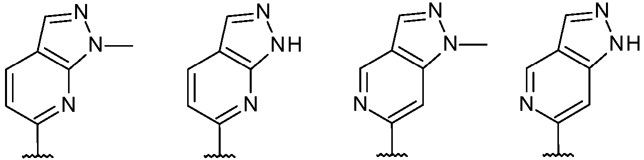

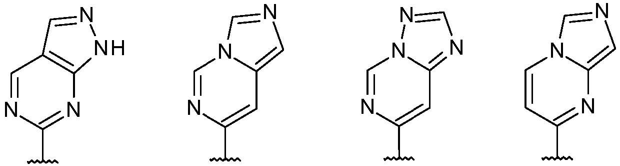

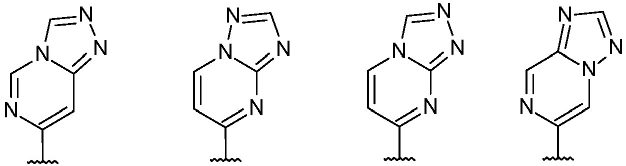

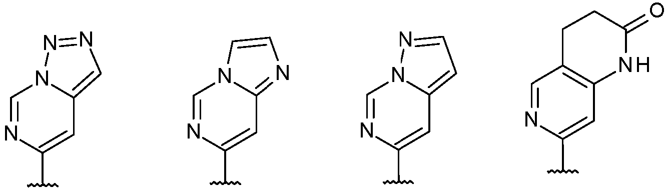

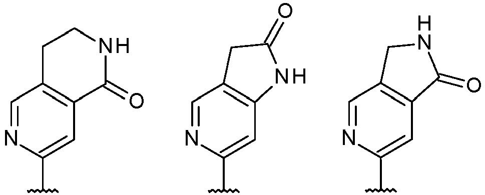

In various embodiments, the compound is any one of the following compounds:
(1) *N*-[1-[2-([1,2,4]triazolo[4,3-b]pyridazin-6-yl)-1,2,4-triazol-3-yl]ethyl]-3,5-bis(trifluoromethyl)benzamide;
(2) *N*-[1-(2-imidazo[2,1-f][1,2,4]triazin-2-yl-1,2,4-triazol-3-yl)ethyl]-3,5-bis(trifluoromethyl)benzamide;
(3) 3-chloro-*N*-[1-[2-([1,2,4]triazolo[4,3-b]pyridazin-6-yl)-1,2,4-triazol-3-yl]ethyl]-5-(trifluoromethyl)benzamide;
(4) 3-chloro-*N*-[1-(2-imidazo[2,1-f][1,2,4]triazin-2-yl-1,2,4-triazol-3-yl)ethyl]-5-(trifluoromethyl)benzamide;
(5) *N*-[1-[2-(4-methylsulfanylimidazo[2,1-f][1,2,4]triazin-2-yl)-1,2,4-triazol-3-yl]ethyl]-3,5-bis(trifluoromethyl)benzamide;
(6) *N*-[1-(2-imidazo[2,1-f][1,2,4]triazin-2-yl-1,2,4-triazol-3-yl)ethyl]-3,5-bis(trifluoromethyl)benzamide;
(7) 3-chloro-*N*-[1-[2-(8-methyl-[1,2,4]triazolo[4,3-b]pyridazin-6-yl)-1,2,4-triazol-3-yl]ethyl]-5-(trifluoromethyl)benzamide;
(8) *N*-[1-[2-(8-methyl-[1,2,4]triazolo[4,3-b]pyridazin-6-yl)-1,2,4-triazol-3-yl]ethyl]-3,5-bis(trifluoromethyl)benzamide;
(9) 3-chloro-*N*-[1-[2-([1,2,4]triazolo[4,3-a]pyrazin-6-yl)-1,2,4-triazol-3-yl]ethyl]-5-(trifluoromethyl)benzamide;
(10) 3-chloro-*N*-methyl-*N*-[1-[2-([1,2,4]triazolo[4,3-b]pyridazin-6-yl)-1,2,4-triazol-3-yl]ethyl]-5-(trifluoromethyl)benzamide;
(11) *N*-[1-[2-([1,2,4]triazolo[4,3-a]pyrazin-6-yl)-1,2,4-triazol-3-yl]ethyl]-3,5-bis(trifluoromethyl)benzamide;
(12) *N*-methyl-*N*-[1-[2-([1,2,4]triazolo[4,3-b]pyridazin-6-yl)-1,2,4-triazol-3-yl]ethyl]-3,5-bis(trifluoromethyl)benzamide;
(13) 3-chloro-*N*-[1-[2-([1,2,4]triazolo[4,3-b]pyridazin-6-yl)-1,2,4-triazol-3-yl]ethyl]-5-(trifluoromethylsulfonyl)benzamide;
(14) [3-chloro-5-[1-[2-([1,2,4]triazolo[4,3-b]pyridazin-6-yl)-1,2,4-triazol-3-yl]ethylcarbamoyl]phenyl] trifluoromethanesulfonate;
(15) 3-chloro-*N*-[1-[2-([1,2,4]triazolo[4,3-b]pyridazin-6-yl)-1,2,4-triazol-3-yl]ethyl]-5-(trifluoromethylsulfinyl)benzamide;
(16) 3-chloro-5-methylsulfonyl-*N*-[1-[2-([1,2,4]triazolo[4,3-b]pyridazin-6-yl)-1,2,4-triazol-3-yl]ethyl]benzamide;
(17) 3-bromo-5-(1-cyano-1-methyl-ethyl)-*N*-[1-[2-([1,2,4]triazolo[4,3-b]pyridazin-6-yl)-1,2,4-triazol-3-yl]ethyl]benzamide;
(18) 3-(1-cyanocyclopropyl)-5-(difluoromethoxy)-*N*-[1-[2-([1,2,4]triazolo[4,3-b]pyridazin-6-yl)-1,2,4-triazol-3-yl]ethyl]benzamide;
(19) 3-chloro-5-(4-fluorophenyl)sulfonyl-*N*-[1-[2-([1,2,4]triazolo[4,3-b]pyridazin-6-yl)-1,2,4-triazol-3-yl]ethyl] benzamide;
(20) *N*-[1-[2-(2-oxo-3,4-dihydro-1*H*-1,6-naphthyridin-7-yl)-1,2,4-triazol-3-yl]ethyl]-3,5-bis(trifluoromethyl)benzamide;
(21) *N*-[1-[2-(2-oxo-1,3-dihydropyrrolo[3,2-c]pyridin-6-yl)-1,2,4-triazol-3-yl]ethyl]-3,5-bis(trifluoromethyl)benzamide;
(22) *N-[1-[2-(1-oxo-2,3-dihydropyrrolo[3,4-c]pyridin-6-yl)-1,2,4-triazol-3-yl]ethyl]-3,5-*bis(trifluoromethyl)benzamide;
(23) *N*-[1-[2-(5-oxo-7,8-dihydro-6*H*-2,6-naphthyridin-3-yl)-1,2,4-triazol-3-yl]ethyl]-3,5-bis(trifluoromethyl)benzamide;
(24) *N*-methyl-*N*-[1-[2-(1-methylpyrazolo[3,4-b]pyridin-6-yl)-1,2,4-triazol-3-yl]ethyl]-3,5-bis(trifluoromethyl)benzamide;
(25) *N*-methyl-*N*-[1-[2-(1*H*-pyrazolo[3,4-b]pyridin-6-yl)-1,2,4-triazol-3-yl]ethyl]-3,5-bis(trifluoromethyl)benzamide;
(26) *N*-[1-[2-(1*H*-pyrazolo[3,4-b]pyridin-6-yl)-1,2,4-triazol-3-yl]ethyl]-3,5-bis(trifluoromethyl)benzamide;
(27) *N*-[1-(2-imidazo[1,5-c]pyrimidin-7-yl-1,2,4-triazol-3-yl)ethyl]-3,5-bis(trifluoromethyl)benzamide;
(28) *N*-[1-[2-([1,2,4]triazolo[1,5-c]pyrimidin-7-yl)-1,2,4-triazol-3-yl]ethyl]-3,5-bis(trifluoromethyl)benzamide;
(29) *N*-[1-[2-(3-amino-[1,2,4]triazolo[4,3-b]pyridazin-6-yl)-1,2,4-triazol-3-yl]ethyl]-3,5-bis(trifluoromethyl)benzamide;
(30) 3-chloro-*N*-[1-[2-([1,2,4]triazolo[1,5-c]pyrimidin-7-yl)-1,2,4-triazol-3-yl]ethyl]-5-(trifluoromethyl)benzamide;
(31) *N*-[1-[2-(3-amino-[1,2,4]triazolo[4,3-b]pyridazin-6-yl)-1,2,4-triazol-3-yl]ethyl]-N-methyl-3,5-bis(trifluoromethyl)benzamide;
(32) *N*-methyl-*N*-[1-[2-(1-methylpyrazolo[4,5-c]pyridin-6-yl)-1,2,4-triazol-3-yl]ethyl]-3,5-bis(trifluoromethyl)benzamide;
(33) *N-*[1-[2-(1*H*-pyrazolo[4,5-c]pyridin-6-yl)-1,2,4-triazol-3-yl]ethyl]-3,5-bis(trifluoromethyl)benzamide;
(34) *N*-[1-(2-imidazo[1,5-a]pyrimidin-2-yl-1,2,4-triazol-3-yl)ethyl]-3,5-bis(trifluoromethyl)benzamide;
(35) *N*-[1-[2-(1*H*-pyrazolo[5,4-d]pyrimidin-6-yl)-1,2,4-triazol-3-yl]ethyl]-3,5-bis(trifluoromethyl)benzamide;
(36) *N*-methyl-*N*-[1-[2-(1*H*-pyrazolo[4,5-c]pyridin-6-yl)-1,2,4-triazol-3-yl]ethyl]-3,5-bis(trifluoromethyl)benzamide;
(37) *N*-[1-[2-(3-amino-[1,2,4]triazolo[4,3-a]pyrazin-6-yl)-1,2,4-triazol-3-yl]ethyl]-3,5-bis(trifluoromethyl)benzamide;
(38) *N*-[1-[2-(3-amino-[1,2,4]triazolo[4,3-a]pyrazin-6-yl)-1,2,4-triazol-3-yl]ethyl]-N-methyl-3,5-bis(trifluoromethyl)benzamide;
(39) 6-[5-[(1*S*)-1-[[3,5-bis(trifluoromethyl)benzoyl]amino]ethyl]-1,2,4-triazol-1-yl]-N,N-dimethyl-[1,2,4]triazolo[4,3-b]pyridazine-8-carboxamide;
(40) 6-[5-[(1*S*)-1-[[3,5-bis(trifluoromethyl)benzoyl]-methyl-amino]ethyl]-1,2,4-triazol-1-yl]-[1,2,4]triazolo[4,3-b]pyridazine-8-carboxylic acid;
(41) ethyl 6-[5-[(1*S*)-1-[[3,5-bis(trifluoromethyl)benzoyl]amino]ethyl]-1,2,4-triazol-1-yl]-[1,2,4]triazolo[4,3-a]pyrazine-3-carboxylate;
(42) 6-[5-[(1*S*)-1-[[3,5-bis(trifluoromethyl)benzoyl]amino]ethyl]-1,2,4-triazol-1-yl]-[1,2,4]triazolo[4,3-b]pyridazine-8-carboxamide;
(43) *N*-methyl-*N*-[(1*S*)-1-[2-(8-methyl-[1,2,4]triazolo[4,3-b]pyridazin-6-yl)-1,2,4-triazol-3-yl]ethyl]-3,5-bis(trifluoromethyl)benzamide;
(44) 6-[5-[(1*S*)-1-[[3,5-bis(trifluoromethyl)benzoyl]-methyl-amino]ethyl]-1,2,4-triazol-1-yl]-[1,2,4]triazolo[4,3-b]pyridazine-8-carboxamide;
(45) 6-[5-[(1*S*)-1-[[3,5-bis(trifluoromethyl)benzoyl]amino]ethyl]-1,2,4-triazol-1-yl]-[1,2,4]triazolo[4,3-a]pyrazine-3-carboxamide;
(46) 3-(1-cyanocyclopropyl)-5-(difluoromethoxy)-N-[1-[2-([1,2,4]triazolo[1,5-c]pyrimidin-7-yl)-1,2,4-triazol-3-yl]ethyl]benzamide;
(47) 3-chloro-N-[1-[2-([1,2,4]triazolo[1,5-c]pyrimidin-7-yl)-1,2,4-triazol-3-yl]ethyl]-5-(trifluoromethylsulfonyl)benzamide;
(48) 6-[5-[(1S)-1-[[3,5-bis(trifluoromethyl)benzoyl]amino]ethyl]-1,2,4-triazol-1-yl]imidazo[1,2-b]pyridazine-8-carboxamide;
(49) N-[1-[2-(2-oxo-1,3,3a,7a-tetrahydroimidazo[4,5-c]pyridin-6-yl)-1,2,4-triazol-3-yl]ethyl]-3,5-bis(trifluoromethyl)benzamide;
(50) N-[1-(2-imidazo[1,2-c]pyrimidin-7-yl-1,2,4-triazol-3-yl)ethyl]-3,5-bis(trifluoromethyl)benzamide;
(51) 3-chloro-N-[1-[2-([1,2,4]triazolo[1,5-c]pyrimidin-7-yl)-1,2,4-triazol-3-yl]ethyl]-5-(trifluoromethylsulfinyl)benzamide;
(52) 3-chloro-N-[1-[2-([1,2,4]triazolo[1,5-c]pyrimidin-7-yl)-1,2,4-triazol-3-yl]ethyl]-5-(trifluoromethoxy)benzamide;
(53) 3-bromo-5-(1-cyano-1-methyl-ethyl)-N-[1-[2-([1,2,4]triazolo[1,5-c]pyrimidin-7-yl)-1,2,4-triazol-3-yl]ethyl]benzamide;
(54) [3-chloro-5-[1-[2-([1,2,4]triazolo[1,5-c]pyrimidin-7-yl)-1,2,4-triazol-3-yl]ethylcarbamoyl]phenyl] trifluoromethanesulfonate;
(55) 3-(1-cyanocyclopropyl)-N-[1-[2-([1,2,4]triazolo[1,5-c]pyrimidin-7-yl)-1,2,4-triazol-3-yl]ethyl]-5-(trifluoromethoxy)benzamide;
(56) N-[1-(2-pyrazolo[1,5-c]pyrimidin-5-yl-1,2,4-triazol-3-yl)ethyl]-3,5-bis(trifluoromethyl)benzamide;
(57) N-[1-[2-(3,3-dimethyl-2-oxo-3a,7a-dihydro-1H-pyrrolo[3,2-c]pyridin-6-yl)-1,2,4-triazol-3-yl]ethyl]-3,5-bis(trifluoromethyl)benzamide;
(58) N-[1-[2-(triazolo[1,5-c]pyrimidin-5-yl)-1,2,4-triazol-3-yl]ethyl]-3,5-bis(trifluoromethyl)benzamide;
(59) N-[1-[2-(3,3-dimethyl-2-oxo-1,4,4a,8a-tetrahydro-1,6-naphthyridin-7-yl)-1,2,4-triazol-3-yl]ethyl]-3,5-bis(trifluoromethyl)benzamide;
(60) N-[1-[2-(2-ethyl-[1,2,4]triazolo[1,5-c]pyrimidin-7-yl)-1,2,4-triazol-3-yl]ethyl]-3,5-bis(trifluoromethyl)benzamide;
(61) N-[1-[2-(3-hydroxyimidazo[1,5-c]pyrimidin-7-yl)-1,2,4-triazol-3-yl]ethyl]-3,5-bis(trifluoromethyl)benzamide;
(62) N-[1-[2-(2-methyl-[1,2,4]triazolo[1,5-c]pyrimidin-7-yl)-1,2,4-triazol-3-yl]ethyl]-3,5-bis(trifluoromethyl)benzamide;
(63) 2-[5-[1-[[3,5-bis(trifluoromethyl)benzoyl]amino]ethyl]-1,2,4-triazol-1-yl]imidazo[1,5-b]pyridazine-4-carboxamide;
(64) N-[1-[2-(2-chloro-[1,2,4]triazolo[1,5-c]pyrimidin-7-yl)-1,2,4-triazol-3-yl]ethyl]-3,5-bis(trifluoromethyl)benzamide;
(65) N-[1-[2-(3-amino-[1,2,4]triazolo[4,3-c]pyrimidin-7-yl)-1,2,4-triazol-3-yl]ethyl]-3,5-bis(trifluoromethyl)benzamide
(66) N-[1-[2-(3-methyl-[1,2,4]triazolo[4,3-c]pyrimidin-7-yl)-1,2,4-triazol-3-yl]ethyl]-3,5-bis(trifluoromethyl)benzamide;
(67) N-[1-[2-(2-aminopyrazolo[1,5-c]pyrimidin-5-yl)-1,2,4-triazol-3-yl]ethyl]-3,5-bis(trifluoromethyl)benzamide; or
the N-oxide, stereoisomer, tautomer, agriculturally or veterinarily acceptable salt of any one of compounds (1) to (67).

In another aspect, the invention is directed to a composition, comprising at least one compound of formula (I) as disclosed herein and any one of a further active substance, an excipient or solvent. The compositions may be agricultural or veterinary compositions.

In still another aspect, the present invention is directed to a method for combating or controlling invertebrate pests, which method comprises contacting said pest or its food supply, habitat or breeding grounds with a pesticidally effective amount of at least one compound or at least one composition according to the invention.

In a still further aspect, the invention relates to a method for protecting growing crops, plants, plant propagation material, growing plants or an animal from attack or infestation by invertebrate pests, which method may comprise contacting a plant, or soil or water wherein the plant is growing, or an animal with a pesticidally effective amount of at least one compound or at least one composition according to the invention or applying such.

In still another aspect, the invention relates to a seed comprising a compound or a composition according to the invention, preferably in an amount of from 0.1 g to 10 kg per 100 kg of seed.

In still another aspect, the invention relates to the use of a compound or of the compositions according to the invention, for protecting growing plants from attack or infestation by invertebrate pests.

In all embodiments and aspects described above, the compounds of formula (I) and/or the N-oxides, stereoisomers, tautomers and agriculturally or veterinarily acceptable salts thereof may be used.

### Detailed description

Unless otherwise indicated, all terms used therein have their common and scientifically accepted meaning.

Unless explicitly indicated otherwise, the term "compound(s) according to the invention" or "compound(s) of the invention" or "compound(s) of formula (I)", refers to the compounds of formula I.

The term "compound(s) according to the invention", or "compounds of formula I" comprises the compound(s) as defined herein as well as a stereoisomer, salt, tautomer or N-oxide thereof. The term "compound(s) of the present invention" is to be understood as equivalent to the term "compound(s) according to the invention", therefore also comprising a stereoisomer, salt, tautomer or N-oxide thereof.

The term "composition(s) according to the invention" or "composition(s) of the present invention" encompasses composition(s) comprising at least one compound of formula I according to the invention as defined above. The compositions of the invention are preferably agricultural or veterinary compositions.

Depending on the substitution pattern, the compounds according to the invention may have one or more centers of chirality, in which case they can be present as mixtures of enantiomers or diastereomers. The invention provides both the single pure enantiomers or pure diastereomers of the compounds according to the invention, and their mixtures and the use according to the invention of the pure enantiomers or pure diastereomers of the compounds according to the invention or their mixtures. Suitable compounds according to the invention also include all possible geometrical stereoisomers (cis/trans isomers) and mixtures thereof. Cis/trans isomers may be present with respect to an alkene, carbon-nitrogen double-bond or amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula I, i.e. to single enantiomers or diastereomers, as well as to mixtures thereof.

The compounds according to the invention may be amorphous or may exist in one or more different crystalline states (polymorphs) which may have different macroscopic properties such as stability or show different biological properties such as activities. The present invention relates to amorphous and crystalline compounds according to the invention, mixtures of different crystalline states of the respective compounds according to the invention, as well as amorphous or crystalline salts thereof.

The term "tautomers" encompasses isomers, which are derived from the compounds of formula I by the shift of an H-atom involving at least one H-atom located at a nitrogen, oxygen or sulphur atom. Examples of tautomeric forms are keto-enol forms, imine-enamine forms, urea-isourea forms, thiourea-isothiourea forms, (thio)amide-(thio)imidate forms etc.

The term "stereoisomers" encompasses both optical isomers, such as enantiomers or diastereomers, the latter existing due to more than one center of chirality in the molecule, as well as geometrical isomers (cis/trans isomers).

Depending on the substitution pattern, the compounds of the formula I may have one or more centers of chirality, in which case they are present as mixtures of enantiomers or diastereomers. The invention provides both the pure enantiomers or diastereomers and their mixtures and the use according to the invention of the pure enantiomers or diastereomers of the compound I or its mixtures. Suitable compounds of the formula I also include all possible geometrical stereoisomers (cis/trans isomers) and mixtures thereof.

The term N-oxides relates to a form of compounds I in which at least one nitrogen atom is present in oxidized form (as NO). To be more precise, it relates to any compound of the present invention which has at least one tertiary nitrogen atom that is oxidized to an N-oxide moiety. N-oxides of compounds I can in particular be prepared by oxidizing e.g. the ring nitrogen atom of an N-heterocycle or an imino-nitrogen with a suitable oxidizing agent, such as peroxo carboxylic acids or other peroxides. The person skilled in the art knows if and in which positions compounds of the present invention may form N-oxides.

Salts of the compounds of the formula I are preferably agriculturally and veterinarily acceptable salts. They can be formed in a customary method, e.g. by reacting the compound with an acid of the anion in question if the compound of formula I has a basic functionality or by reacting an acidic compound of formula I with a suitable base.

Suitable agriculturally or veterinarily acceptable salts are especially the salts of those cations or the acid addition salts of those acids whose cations and anions, which are known and accepted in the art for the formation of salts for agricultural or veterinary use respectively, and do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH⁴⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or -CH₂-phenyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzyl-triethylammonium. Suitable anions include, but are not limited to, phosphonium ions, sulfonium ions, preferably tri(C₁-C₄-alkyl)sulfonium, and sulfoxonium ions, preferably tri(C₁-C₄-alkyl)sulfoxonium. Suitable acid addition salts, e.g. formed by compounds of formula I containing a basic nitrogen atom, e.g. an amino group, include salts with inorganic acids, for example hydrochlorides, sulphates, phosphates, and nitrates and salts of organic acids for example acetic acid, maleic acid, dimaleic acid, fumaric acid, difumaric acid, methane sulfenic acid, methane sulfonic acid, and succinic acid.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting a compound of formulae I with an acid of the corresponding anion, preferably of hydrochloric acid, hydrobromic acid, sulfuric acid, phosphoric acid or nitric acid.

The term "invertebrate pest" as used herein encompasses animal populations, such as insects, arachnids and nematodes, which may attack plants, thereby causing substantial damage to the plants attacked, as well as ectoparasites which may infest animals, in particular warm blooded animals such as e.g. mammals or birds, or other higher animals such as reptiles, amphibians or fish, thereby causing substantial damage to the animals infested.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. The plant propagation materials may be treated prophylactically with a plant protection compound either at or before planting or transplanting. Said young plants may also be protected before transplantation by a total or partial treatment by immersion or pouring.

The term "plants" comprises any types of plants including "modified plants" and in particular "cultivated plants".

The term "modified plants" refers to any wild type species or related species or related genera of a cultivated plant.

The term "cultivated plants" is to be understood as including plants which have been modified by breeding, mutagenesis or genetic engineering including but not limiting to agricultural biotech products on the market or in development (cf. http://www.bio.org/speeches/pubs/er/agri_products.asp). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i.e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CrylA(b), CrylA(c), CrylF, CrylF(a2), CryIIA(b), CrylllA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins.

Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum)* or T4-lysozym (e. g. potato cultivars capable of synthesizing these proteins with increased resistance against bacteria such as *Erwinia amylvora*)*.* The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The organic moieties mentioned in the above definitions of the variables are - like the term halogen - collective terms for individual listings of the individual members. The prefix Cₙ-Cₘ indicates in each case the possible number of carbon atoms in the group.

The term "partially or fully substituted" by a radical means that in general the group is substituted with same or different radicals.

The term "halogen" denotes in each case fluorine, bromine, chlorine, or iodine, in particular fluorine, chlorine, or bromine.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are methyl (Me), ethyl (Et), n-propyl (n-Pr), iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 10 carbon atoms, frequently from 1 to 6 carbon atoms, preferably from 1 to 4 carbon atoms, wherein the hydrogen atoms of this group are partially or totally replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 10 carbon atoms, frequently from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 10, frequently 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 10 carbon atoms, frequently from 1 to 6 carbon atoms, preferably 1 to 4 carbon atoms, wherein the hydrogen atoms of this group are partially or totally replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoromethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoro-ethoxy, 2,2dichloro-2-fluorethoxy, 2,2,2-trichloroethoxy, pentafluoroethoxy and the like.

The term "alkylthio "(alkylsulfanyl: S-alkyl)" as used herein refers to a straight-chain or branched saturated alkyl group having 1 to 10 carbon atoms, preferably 1 to 4 carbon atoms (= C₁-C₄-alkylthio), more preferably 1 to 3 carbon atoms, which is attached via a sulfur atom.

The term "haloalkylthio" as used herein refers to an alkylthio group as mentioned above wherein the hydrogen atoms are partially or fully substituted by fluorine, chlorine, bromine and/or iodine.

The term "alkylsulfinyl" (alkylsulfoxyl: S(=O)-alkyl), as used herein refers to a straight-chain or branched saturated alkyl group (as mentioned above) having 1 to 10 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "haloalkylsulfinyl" as used herein refers to an alkylsulfinyl group as mentioned above wherein the hydrogen atoms are partially or fully substituted by fluorine, chlorine, bromine and/or iodine.

The term "alkylsulfonyl" (S(=O)₂-alkyl) as used herein refers to a straight-chain or branched saturated alkyl group having 1 to 10 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "haloalkylsulfonyl" as used herein refers to an alkylsulfonyl group as mentioned above wherein the hydrogen atoms are partially or fully substituted by fluorine, chlorine, bromine and/or iodine.

The term "alkylcarbonyl" refers to an alkyl group as defined above, which is bonded via the carbon atom of a carbonyl group (C=O) to the remainder of the molecule.

The term "haloalkylcarbonyl" refers to an alkylcarbonyl group as mentioned above, wherein the hydrogen atoms are partially or fully substituted by fluorine, chlorine, bromine and/or iodine.

The term "alkoxycarbonyl" refers to an alkylcarbonyl group as defined above, which is bonded via an oxygen atom to the remainder of the molecule.

The term "haloalkoxycarbonyl" refers to an alkoxycarbonyl group as mentioned above, wherein the hydrogen atoms are partially or fully substituted by fluorine, chlorine, bromine and/or iodine.

The term "alkenyl" as used herein denotes in each case a singly unsaturated hydrocarbon radical having usually 2 to 10, frequently 2 to 6, preferably 2 to 4 carbon atoms, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the hydrogen atoms are partially or totally replaced with halogen atoms.

The term "alkynyl" as used herein denotes in each case a singly unsaturated hydrocarbon radical having usually 2 to 10, frequently 2 to 6, preferably 2 to 4 carbon atoms, e.g. ethynyl, propargyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the hydrogen atoms are partially or totally replaced with halogen atoms.

The term "cycloalkyl" as used herein and in the cycloalkyl moieties of cycloalkoxy and cycloalkylthio denotes in each case a monocyclic cycloaliphatic radical having usually from 3 to 10 or from 3 to 6 carbon atoms, such as cyclopropyl (cC₃H₅), cyclobutyl (cC₄H₇), cyclopentyl (cC₅H₉), cyclohexyl (cC₆H₁₁), cycloheptyl, cyclooctyl, cyclononyl and cyclodecyl or cyclopropyl, cyclobutyl, cyclopentyl and cyclohexyl.

The term "cycloalkylalkyl" refers to a cycloalkyl group as defined above which is bonded via an alkylene group, such as a C₁-C₅-alkyl group or a C₁-C₄-alkyl group, in particular a methylene group CH₂ (=cycloalkylmethyl), to the remainder of the molecule. Generally, the expression "C₃-C₆-cycloalkyl-C₁-C₆-alkyl" refers to a group, where the alkyl group is connected to the rest of the molecule. Similarly, a term such as "C₁-C₆-alkyl-C₃-C₆-cycloalkyl" refers to a group, where the cycloalkyl group is connected to the rest of the molecule.

The term "halocycloalkyl" as used herein and in the halocycloalkyl moieties of halocycloalkoxy and halocycloalkylthio denotes in each case a monocyclic cycloaliphatic radical having usually from 3 to 10 C atoms or 3 to 6 C atoms, wherein at least one, e.g. 1, 2, 3, 4 or 5 of the hydrogen atoms, are replaced by halogen, in particular by fluorine or chlorine. Examples are 1- and 2-fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "halocycloalkenyl" as used herein and in the halocycloalkenyl moieties of halocycloalkenyloxy and halocycloalkenylthio denotes in each case a monocyclic singly unsaturated non-aromatic radical having usually from 3 to 10, e.g. 3 or 4 or from 5 to 10 carbon atoms, preferably from 3- to 8 carbon atoms, wherein at least one, e.g. 1, 2, 3, 4 or 5 of the hydrogen atoms, are replaced by halogen, in particular by fluorine or chlorine. Examples are 3,3-difluorocyclopropen-1-yl and 3,3-dichlorocyclopropen-1-yl.

The term "cycloalkenylalkyl" refers to a cycloalkenyl group as defined above which is bonded via an alkyl group, such as a C₁-C₅-alkyl group or a C₁-C₄-alkyl group, in particular a methylene group (= cycloalkenylmethyl), to the remainder of the molecule.

The term "carbocycle" or "carbocyclyl" includes in general a 3- to 12-membered, preferably a 3- to 8-membered or a 5- to 8-membered, more preferably a 5- or 6-membered mono-cyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above.

The term "heterocycle" or "heterocyclyl" includes in general 3- to 12-membered, preferably 3- to 6-membered, in particular 6-membered monocyclic heterocyclic non-aromatic radicals. The heterocyclic non-aromatic radicals usually comprise 1, 2, 3, 4 or 5, preferably 1, 2 or 3 heteroatoms selected from N, O, and S, wherein S-atoms as ring members may be present as S, SO, or SO₂, and optionally one or two groups C(O) as ring members. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-onyl, and the like.

The term "hetaryl" includes monocyclic 5- or 6-membered heteroaromatic radicals comprising as ring members 1, 2, 3 or 4 heteroatoms selected from N, O, and S. Examples of 5- or 6-membered heteroaromatic radicals include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2- or 5-(1,3,4-thiadiazol)yl, thiadiazolyl, e.g. 2- or 5-(1,3,4-thiadiazol)yl, 4- or 5-(1,2,3-thiadiazol)yl, 3- or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1H- or 2H-tetrazolyl. The term "hetaryl" also includes bicyclic 8 to 10-membered heteroaromatic radicals comprising as ring members 1, 2 or 3 heteroatoms selected from N, O, and S, wherein a 5- or 6-membered heteroaromatic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The terms "heterocyclylalkyl" and "hetarylalkyl" refer to heterocyclyl or hetaryl, respectively, as defined above which are bonded via a C₁-C₅-alkyl group or a C₁-C₄-alkyl group, in particular a methylene group (= heterocyclylmethyl or hetarylmethyl, resp.), to the remainder of the molecule.

The term "arylalkyl" and "phenylalkyl" refer to aryl as defined above and phenyl, respectively, which are bonded via C₁-C₅-alkyl group or a C₁-C₄-alkyl group, in particular a methyl group (= arylmethyl or phenylmethyl), to the remainder of the molecule, examples including benzyl, 1-phenylethyl, 2-phenylethyl, 2-phenoxyethyl etc.

The terms "alkylene", "cycloalkylene", "heterocycloalkylene", "alkenylene", "cycloalkenylene", "heterocycloalkenylene" and "alkynylene" refer to alkyl, cycloalkyl, heterocycloalkyl, alkenyl, cycloalkenyl, heterocycloalkenyl and alkynyl as defined above, resp., which are bonded to the remainder of the molecule, via two atoms, preferably via two carbon atoms, of the respective group, so that they represent a linker between two moieties of the molecule.

The compounds of the invention are compounds of formula (I) 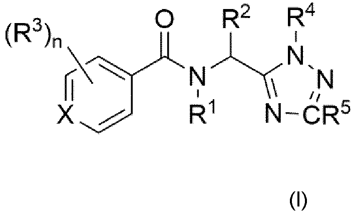 wherein
- R¹: is H, OR¹⁰, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl,, wherein the alkyl, alkoxy, alkenyl, alkynyl, and cycloalkyl moieties are unsubstituted or substituted with R¹¹; C(=N-R¹³)R¹², or C(O)R^{11a};
- R²: is H, C₁-C₃-alkyl, C₂-C₃-alkenyl, C₃-C₆-cycloalkyl, or C₂-C₃-alkynyl, wherein the alkyl, alkenyl, alkynyl and cycloalkyl moieties are unsubstituted or substituted with halogen;
- R³: is halogen, CN, NO₂, SF₅; C₁-C₆-alkyl, C₃-C₆-cycloalkyl, C₁-C₆-alkenyl, C₁-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, heterocyclyl and cycloalkyl moieties are unsubstituted or substituted with R^{a}; OR¹⁰, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a}; or
two R³ bound to two adjacent C-atoms can form a 4-, 5-, or 6-membered ring, which may contain one or two heteroatoms selected from N, O, and S as ring members, wherein the ring is unsubstituted or substituted with halogen, CN, C₁-C₃-alkyl, and/or C₁-C₃-haloalkyl;
- R⁴: is a heterocyclic ring which is selected from the group consisting of 8-, 9- or 10-membered saturated or partially unsaturated bicyclic heterocyclyl, and 8- 9- or 10- membered bicyclic hetaryl rings, wherein the heterocyclyl moieties are unsubstituted or substituted by 1, 2, 3 or 4 substituents and the hetaryl moieties are unsubstituted or substituted by 1, 2 or 3 substituents, wherein the substituents are independently selected from the group consisting of halogen, =O (oxo), -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, S(O)ₘ-R¹⁴, OR¹⁰, NR¹²R¹³, C(O)NR¹²R¹³, C(O)OR¹⁰, C(O)R¹⁴, -C(=NOC₁-C₄-alkyl)H, -C(=NOC₁-C₄-alkyl)-C₁-C₄-alkyl or -C(=NOC₁-C₄-alkyl)-C₃-C₆-cycloalkyl, wherein alkyl, alkenyl, alkynyl, cycloalkyl, and alkoxy are unsubstituted or substituted with R¹¹;
- R⁵: is H, halogen, CN, NH₂, NO₂, CONH₂, C₁-C₆-alkyl, C₁-C₆-haloalkyl, or C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₁-C₆-haloalkoxy, CH(C₁-C₆-alkoxy)₂, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, OR¹⁰, NR¹²R¹³, C(O)NR¹²R¹³, C(O)OR¹⁰, C(O)R¹⁴, S(O)ₘ-R¹⁴, -C(=NOC₁-C₄-alkyl)H, or-C(=NOC₁-C₄-alkyl)-C₁-C₄-alkyl, wherein alkyl, alkenyl, alkynyl, cycloalkyl and alkoxy are unsubstituted or substituted with R¹¹;
- R¹⁰: is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
- R¹¹: is halogen, CN, NO₂, NR¹²R¹³, C(O)NH₂, C(S)NH₂, C(O)OH, OR¹⁰, Si(CH₃)₃; 3- to 6-membered heterocyclyl, 5- or 6-membered hetaryl, or phenyl, wherein the heterocyclyl, hetaryl and phenyl moieties are unsubstituted or substituted with halogen, C₁-C₃-haloalkyl, and/or CN;
- R^{11a}: is NR¹²R¹³, C(O)NH₂, C(S)NH₂, C(O)OH, OR¹⁰, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, wherein the cycloalkyl moiety is unsubstituted or substituted with 1 or 2 halogen; or 3- to 6-membered heterocyclyl, wherein the heterocyclyl moiety is unsubstituted or substituted with halogen, C₁-C₃-haloalkyl, and/or CN;
- R¹², R¹³: are independently from each other H, C₁-C₄-alkyl, C₁-C₄-alkoxy, C₁-C₄-haloalkoxy, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, wherein the phenyl, heterocyclyl and hetaryl moieties are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, wherein the phenyl, heterocyclyl and hetaryl moieties are unsubstituted or substituted with halogen, C₁-C₃-haloalkyl, and/or CN; or
R¹² and R¹³ together with the atom(s) to which they are bound, form a 3-, 4-, 5-, 6-, or 7-membered saturated, partially or fully unsaturated heterocycle, which heterocycle may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R^{12a}, R^{13a}: are independently from each other C₁-C₄-alkyl, C₁-C₄-haloalkyl, C₃-C₆-cycloalkyl, unsubstituted or substituted with halogen, C₁-C₃-haloalkyl, and/or CN; or R^{12a} and R^{13a} together with the atom to which they are bound, form a 3-, 4-, 5-, 6-, or 7-membered saturated, partially or fully unsaturated heterocycle, wherein the heterocycle moiety may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R¹⁴: is H, C₁-C₆-alkyl (such as C₁-C₄-alkyl), C₁-C₄-haloalkyl, C₃-C₆-cycloalkyl, C₃-C₆-halocycloalkyl, wherein the alkyl and cycloalkyl moieties are unsubstituted or partially or fully substituted with R¹¹; or 3- to 6-membered heterocyclyl, 5- or 6-membered hetaryl, or phenyl, wherein the phenyl, heterocyclyl and hetaryl moieties are unsubstituted or substituted with R^{a};
- R^{a}: is halogen, CN, NO₂, OH, C₁-C₄-alkyl, C₁-C₄-alkoxy, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl;
- m: is 0, 1, or 2;
- n: is 0, 1, 2, or 3;
- X: is N, CH, or CR³.

In another aspect, the compounds of the invention are compounds of formula (I) 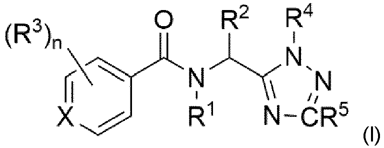 wherein
- R¹: is H, OR^{10a}, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, wherein the alkyl, alkoxy, alkenyl, alkynyl, and cycloalkyl moieties are unsubstituted or substituted with R¹¹; C(=N-R¹³)R¹², or C(O)R^{11a};
- R²: is H, C₁-C₃-alkyl, C₂-C₃-alkenyl, C₃-C₆-cycloalkyl, or C₂-C₃-alkynyl, wherein the alkyl, alkenyl, alkynyl and cycloalkyl moieties are unsubstituted or substituted with halogen;
- R³: is halogen, CN, NO₂, SF₅; C₁-C₆-alkyl, C₁-C₆-alkoxy, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, alkoxy, heterocyclyl and cycloalkyl moieties are unsubstituted or substituted with R¹¹; OR^{10a}, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a}; or
two R³ bound to two adjacent C-atoms can form a 4-, 5-, or 6-membered ring, which may contain one or two heteroatoms selected from N, O, and S as ring members, wherein the ring is unsubstituted or substituted with halogen, CN, C₁-C₃-alkyl, and/or C₁-C₃-haloalkyl;
- R⁴: is a heterocyclic ring which is selected from the group consisting of 8-, 9- or 10-membered saturated or partially unsaturated bicyclic heterocyclyl, and 8- 9- or 10- membered bicyclic hetaryl rings, wherein the heterocyclyl moieties are unsubstituted or substituted by 1, 2, 3 or 4 substituents and the hetaryl moieties are unsubstituted or substituted by 1, 2 or 3 substituents, wherein the substituents are independently selected from the group consisting of halogen, =O (oxo), -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, S(O)ₘ-R¹⁴, OR^{10a}, NR¹²R¹³, C(O)NR¹²R¹³, C(O)OR¹⁰, C(O)R¹⁴, - C(=NOC₁-C₄-alkyl)H, -C(=NOC₁-C₄-alkyl)-C₁-C₄-alkyl or -C(=NOC₁-C₄-alkyl)-C₃-C₆-cycloalkyl, wherein alkyl, alkenyl, alkynyl, cycloalkyl, and alkoxy are unsubstituted or substituted with R¹¹;
- R⁵: is H, halogen, CN, NH₂, NO₂, CONH₂, C₁-C₆-alkyl, or C₃-C₆-cycloalkyl, C₃-C₆-cycloalkyl-C₁-C₆-alkyl, C₁-C₆-alkyl-C₃-C₆-cycloalkyl, C₁-C₆-alkoxy, CH(C₁-C₆-alkoxy)₂, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, OR^{10a}, NR¹²R¹³, C(O)NR¹²R¹³, C(O)OR¹⁰, C(O)R¹⁴, S(O)ₘ-R¹⁴, -C(=NOC₁-C₄-alkyl)H, or -C(=NOC₁-C₄-alkyl)-C₁-C₄-alkyl, wherein alkyl, alkenyl, alkynyl, cycloalkyl and alkoxy are unsubstituted or substituted with R¹¹;
- R¹⁰: is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
- R^{10a}: is H, C₃-C₆-cycloalkyl, C₃-C₆-halocycloalkyl, C₃-C₄-cycloalkyl-C₁-C₂-alkyl, C₃-C₄-halocycloalkyl-C₁-C₂-alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
- R¹¹: is halogen, CN, NO₂, NR¹²R¹³, C(O)NH₂, C(S)NH₂, C(O)OH, OR¹⁰, Si(CH₃)₃; 3- to 6-membered heterocyclyl, 5- or 6-membered hetaryl, or phenyl, wherein the heterocyclyl, hetaryl and phenyl moieties are unsubstituted or substituted with halogen, C₁-C₃-haloalkyl, and/or CN;
- R^{11a}: is NR¹²R¹³, C(O)NH₂, C(S)NH₂, C(O)OH, OR¹⁰, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂-alkyl, wherein the cycloalkyl moiety is unsubstituted or substituted with 1 or 2 halogen; or 3- to 6-membered heterocyclyl, wherein the heterocyclyl moiety is unsubstituted or substituted with halogen, C₁-C₃-haloalkyl, and/or CN;
- R¹², R¹³: are independently from each other H**,** C₁-C₄-alkyl, C₁-C₄-alkoxy, C₁-C₄-haloalkoxy, C₁-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, wherein the phenyl, heterocyclyl and hetaryl moieties are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, wherein the phenyl, heterocyclyl and hetaryl moieties are unsubstituted or substituted with halogen, C₁-C₃-haloalkyl, and/or CN; or
R¹² and R¹³ together with the atom(s) to which they are bound, form a 3-, 4-, 5-, 6-, or 7-membered saturated, partially or fully unsaturated heterocycle, which heterocycle may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R^{12a}, R^{13a}: are independently from each other C₁-C₄-alkyl, C₁-C₄-haloalkyl, C₃-C₆-cycloalkyl, unsubstituted or substituted with halogen, C₁-C₃-haloalkyl, and/or CN; or R^{12a} and R^{13a} together with the atom to which they are bound, form a 3-, 4-, 5-, 6-, or 7-membered saturated, partially or fully unsaturated heterocycle, wherein the heterocycle moiety may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
- R¹⁴: is H, C₁-C₆-alkyl, C₃-C₆-cycloalkyl, wherein the alkyl and cycloalkyl moieties are unsubstituted or partially or fully substituted with R¹¹; or 3- to 6-membered heterocyclyl, 5- or 6-membered hetaryl, or phenyl, wherein the phenyl, heterocyclyl and hetaryl moieties are unsubstituted or substituted with R^{a};
- R^{a}: is halogen, CN, NO₂, OH, C₁-C₄-alkyl, C₁-C₄-alkoxy, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl;
- m: is 0, 1, or 2;
- n: is 0, 1, 2, or 3;
- X: is N, CH, or CR³;
and the N-oxides, stereoisomers, tautomers, and agriculturally or veterinarily acceptable salts thereof.

Also encompassed are the N-oxides, stereoisomers, tautomers, and agriculturally or veterinarily acceptable salts of the compounds of formula (I).

In various embodiments, the compound of formula (I) is or essentially consists of the S isomer

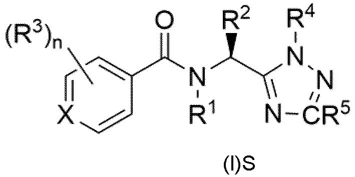

In all embodiments of the compounds of formula (I) described herein, the compound of formula (I) may be the S isomer according to formula (I)S. If a compound of formula (I) is described to "essentially consist of' the S isomer, this means that it is not enantiomerically pure but also comprises the R isomer but that the S isomer is present in amounts of >50%, preferably at least 60, at least 65, at least 70, at least 75, at least 80, at least 85 or at least 90%, relative to the sum of both S and R isomer.

R¹ is H, OR¹⁰, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl,, C(=N-R¹³)R¹², or C(O)R^{11a}. In these groups, alkyl, alkoxy, alkenyl, alkynyl, and cycloalkyl moieties are unsubstituted or substituted with R¹¹. In various embodiments, R¹ is C₁-C₆-alkyl, for example methyl, or C₃-C₆-cycloalkyl-C₁-C₆-alkyl, for example CH₂-cyclopropyl.

In various embodiments, R¹ is H, OR^{10a}, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, wherein the alkyl, alkoxy, alkenyl, alkynyl, and cycloalkyl moieties are unsubstituted or substituted with R¹¹; C(=N-R¹³)R¹², or C(O)R^{11a}. In these groups, alkyl, alkoxy, alkenyl, alkynyl, and cycloalkyl moieties are unsubstituted or substituted with R¹¹. In various embodiments, R¹ is C₁-C₆-alkyl, for example methyl, or C₃-C₆-cycloalkyl-C₁-C₆-alkyl, for example CH₂-cyclopropyl.

R³ is halogen, CN, NO₂, SF₅, C₁-C₆-alkyl, C₃-C₆-cycloalkyl, C₁-C₆-alkenyl, C₁-C₆-alkynyl, C₃-C₆-cycloalkyl-C₁-C₆-alkyl, C₁-C₆-alkyl-C₃-C₆-cycloalkyl, 3- to 6-membered heterocyclyl, OR¹⁰, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a}. In these groups, the alkyl, alkenyl, alkynyl, heterocyclyl and cycloalkyl moieties are unsubstituted or substituted with R^{a}. Alternatively or additionally, two R³ bound to two adjacent C-atoms can form a 4-, 5-, or 6-membered ring, which may contain one or two heteroatoms selected from N, O, and S as ring members, wherein the ring is unsubstituted or substituted with halogen, CN, C₁-C₃-alkyl, and/or C₁-C₃-haloalkyl.

In various embodiments, R³ is halogen, CN, NO₂, SF₅; C₁-C₆-alkyl, C₁-C₆-alkoxy, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, alkoxy, heterocyclyl and cycloalkyl moieties are unsubstituted or substituted with R¹¹; OR^{10a}, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a}; or two R³ bound to two adjacent C-atoms can form a 4-, 5-, or 6-membered ring, which may contain one or two heteroatoms selected from N, O, and S as ring members, wherein the ring is unsubstituted or substituted with halogen, CN, C₁-C₃-alkyl, and/or C₁-C₃-haloalkyl.

R⁴ is a heterocyclic ring which is selected from the group consisting of 8-, 9- or 10-membered saturated or partially unsaturated bicyclic heterocyclyl, and 8- 9- or 10- membered bicyclic hetaryl rings. The heterocyclyl rings may be unsubstituted or substituted by one to four substituents, for example 1, 2, 3, or 4 substituents, such as 1, 2 or 3 substituents. The hetaryl rings may be unsubstituted or substituted by one to three substituents, for example 1, 2, or 3 substituents, such as 1 or 2 substituents. The substituents may be independently selected from the group consisting of halogen, =O (oxo), -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, S(O)ₘ-R¹⁴, OR¹⁰, NR¹²R¹³, C(O)NR¹²R¹³, C(O)OR¹⁰, C(O)R¹⁴, -C(=NOC₁-C₄-alkyl)H, -C(=NOC₁-C₄-alkyl)-C₁-C₄-alkyl or -C(=NOC₁-C₄-alkyl)-C₃-C₆-cycloalkyl, wherein alkyl, alkenyl, alkynyl, cycloalkyl, and alkoxy are unsubstituted or substituted with R¹¹. In various embodiments, the substituents may be independently selected from the group consisting of halogen, =O (oxo), -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₁-C₆-alkylthio, C₁-C₆-alkylsulfinyl, C₁-C₆-alkylsulfonyl, C₁-C₆-alkylsulfonate, C₁-C₆-cycloalkylsulfonate, C₃-C₆-cycloalkylsulfanyl, C₃-C₆-cycloalkylsulfinyl, C₃-C₆-cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl or -C(=NOC₁-C₄-alkyl)-C₃-C₆-cycloalkyl. In these groups, alkyl, cycloalkyl and alkoxy are optionally substituted with halogen and/or CN. Accordingly, said list of possible substituents also comprises groups, such as C₁-C₃-haloalkyl, C₁-C₃-haloalkoxy, C₁-C₆-haloalkylthio, C₁-C₆-haloalkylsulfinyl, C₁-C₆-haloalkylsulfonyl, C₁-C₆-haloalkylsulfonate. Herein, the alkyl groups may additionally be substituted with CN.

In various embodiments, R¹ is H or C₁-C₆-alkyl or C₃-C₆-cycloalkyl-C₁-C₄-alkyl or C₁-C₄-alkyl-C₃-C₆-cycloalkyl, preferably H, methyl or CH₂-cyclopropyl.

In various embodiments, R² is H or C₁-C₃-alkyl, preferably methyl.

In various embodiments, R¹ is H or C₁-C₆-alkyl or C₃-C₆-cycloalkyl-C₁-C₄-alkyl or C₁-C₄-alkyl-C₃-C₆-cycloalkyl, preferably H, methyl or CH₂-cyclopropyl, and R² is H or C₁-C₃-alkyl, preferably methyl.

In various embodiments, X is CR³, preferably CH.

In various embodiments, n is at least 1, preferably 2. The R³ substituents on the phenyl ring are preferably in the meta positions relative to the attachment point, i.e. in the 3- and/or 5-position. Particularly preferred is a di-substituted phenyl ring, in which the two substituents are in the 3- and 5-positions.

The R³ substituents on the phenyl ring may, in various embodiments, be selected from R^{3d}, wherein each R^{3d} is independently C₁-C₆-cyanoalkyl, C₃-C₆-cycloalkyl, C₃-C₆-halocycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, wherein the alkyl, cycloalkyl, alkenyl and alkynyl groups are unsubstituted or substituted with R³³; NR¹²R¹³, C(O)NR¹²R¹³, C(O)OR¹⁰, C(O)R¹⁴, OS(O)₂-R¹⁴, and S(O)ₘ-R¹⁴ wherein m is 1 or 2; and each R^{3a} is independently halogen, CN, NO₂, OH, C₁-C₄-alkyl, C₁-C₄-haloalkyl, C₁-C₄-alkoxy, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl. R^{3d} may thus for example be C(CH₃)₂CN.

In various embodiments, at least one R³ group in (R³)ₙ may be selected from R^{3d}, wherein each R^{3d} is independently C₁-C₆-alkyl, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, wherein the alkyl, cycloalkyl, alkenyl and alkynyl groups are unsubstituted or substituted independently with halogen, CN, NO₂, OH, NR¹²R¹³, C(O)NR¹²R¹³, C(O)OR¹⁰, C(O)R¹⁴, OS(O)₂-R¹⁴, and S(O)ₘ-R¹⁴ wherein m is 1 or 2. C₁-C₆-alkyl may be C₁-C₆-cyanoalkyl. C₃-C₆-cycloalkyl may be C₃-C₆-halocycloalkyl.

In various embodiments, each R³ is independently selected from CF₃, Cl, Br, SO₂-CF₃, O-SO₂-CF₃, SO-CF₃, SO-CH₃, C(CH₃)₂CN, 1-CN-cC₃H₄ (CN-substituted cyclopropyl), OCHF₂, and SO₂-(2-F-phenyl). These R³ substituents may be in the 3- and/or 5-positions. In various embodiments, n is 2 and one R³ is CF₃ and the other is also CF₃, or any one of Cl, Br, SO₂-CF₃, O-SO₂-CF₃, SO-CF₃, SO-CH₃, C(CH₃)₂CN, 1-CN-cC₃H₄ (CN-substituted cyclopropyl), OCHF₂, and SO₂-(2-F-phenyl). In various embodiments, n is 2 and one R³ is SO-CH₃, SO-CF₃, SO₂-CF₃ or SO₂-(2-F-phenyl) and the other R³ is CI or Br. In one embodiment, one R³ is 1-CN-cC₃H₄ (CN-substituted cyclopropyl) and the other is OCHF₂. In one embodiment, one R³ is C(CH₃)₂CN and the other is Br.

In various embodiments, R⁴ is a heterocyclic ring which is selected from the group consisting of 8-, 9- or 10-membered saturated or partially unsaturated bicyclic heterocyclyl, and 8-, 9- or 10- membered bicyclic hetaryl rings, wherein the heterocyclyl and hetaryl moieties are unsubstituted or substituted by one to three substituents, and wherein the bicyclic heterocyclyl and hetaryl moieties comprise at least one N ring atom, preferably at least two N ring atoms. In various embodiments, the bicyclic heterocyclyl and hetaryl moieties comprise only N as the heteroatom in the ring, i.e. consist of C and N ring atoms.

In various embodiments, R⁴ is a heterocyclic ring which is selected from the group consisting of 9- or 10-membered saturated or partially unsaturated bicyclic heterocyclyl, and 9- or 10- membered bicyclic hetaryl rings, wherein the heterocyclyl and hetaryl moieties are unsubstituted or substituted by one to three substituents, and wherein the bicyclic heterocyclyl and hetaryl moieties comprise at least one N ring atom, preferably at least two N ring atoms. In various embodiments, the bicyclic heterocyclyl and hetaryl moieties comprise only N as the heteroatom in the ring, i.e. consist of C and N ring atoms.

In various embodiments, at least one ring of the bicyclic ring structure is an aromatic ring.

In various embodiments, R⁴ is selected from the following structures:

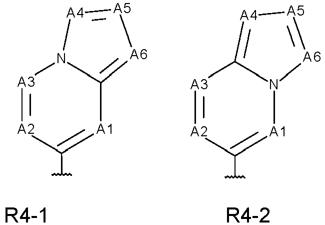

In these structures,
A1, A2 and A3 are independently CR^{4a} or N, provided not more than two of A1, A2 and A3 are N and at least one of A1 and A2 is N;
A4, A5 and A6 are independently CR^{4a} or N, provided not more than two of A4, A5 and A6 are N;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl;
with the provisos that
not more than four of A1, A2, A3, A4, A5 and A6 are N;
if in R4-1 A3 and A4 are N, A2 and A5 are not N;
if in R4-1 A2 and A3 are N, A4 is not N;
if in R4-1 A4 and A5 are N, A3 and A6 are not N;
if in R4-2 A1 and A6 are N, A5 is not N; and
if in R4-2 A5 and A6 are N, A1 and A4 are not N.

In various embodiments, one of A1 and A2 is N and the other is CH or CCH₃.

In various embodiments R⁴ is R4-1, A2 and A5 are N and the remaining A1, A3, A4 and A6 are CR^{4a}.

In various embodiments R⁴ is R4-1, A2, A4 and A6 are N and the remaining A1, A3 and A5 are CR^{4a}.

In various embodiments R⁴ is R4-1, A1 and A5 are N and the remaining A2, A3, A4 and A6 are CR^{4a}.

In various embodiments R⁴ is R4-2, A1, A4 and A5 are N and the remaining A2, A3 and A6 are CR^{4a}.

In various embodiments R⁴ is R4-2, A1 and A4 are N and the remaining A2, A3, A5 and A6 are CR^{4a}.

In various embodiments R⁴ is R4-2, A1, A2 and A4 are N and the remaining A3, A5 and A6 are CR^{4a}.

In various embodiments R⁴ is R4-2, A2, A4 and A5 are N and the remaining A1, A3 and A6 are CR^{4a}.

Alternatively, R⁴ may be any one of the following:

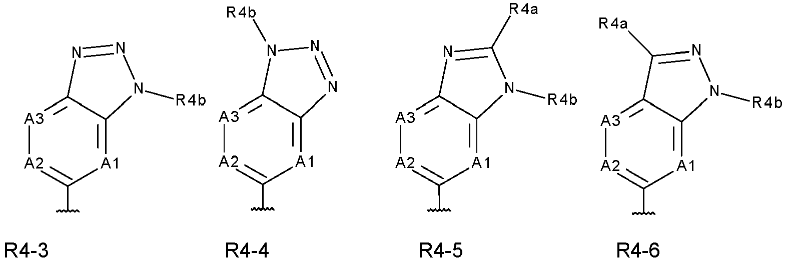

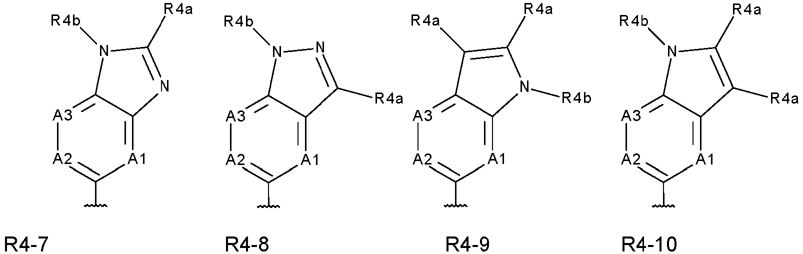

In these structures, A1, A2 and A3 are as defined above;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, - N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; and
R^{4b} is selected from H, halogen, -OH, -CN, -COOH, -CONH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, - CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl.

In various embodiments of these structures:
(1) A1 is N and A2 and A3 are CR^{4a}
(2) A2 is N and A1 and A3 are CR^{4a}, or
(3) A1 and A2 are N and A3 is CR^{4a}.

In various embodiments R⁴ is R4-6, A1 is N and A2 and A3 are CR^{4a}.

In various embodiments R⁴ is R4-6, A2 is N and A1 and A3 are CR^{4a}.

In various embodiments R⁴ is R4-6, A1 and A2 are N and A3 is CR^{4a}.

In still other embodiments, R⁴ is selected from

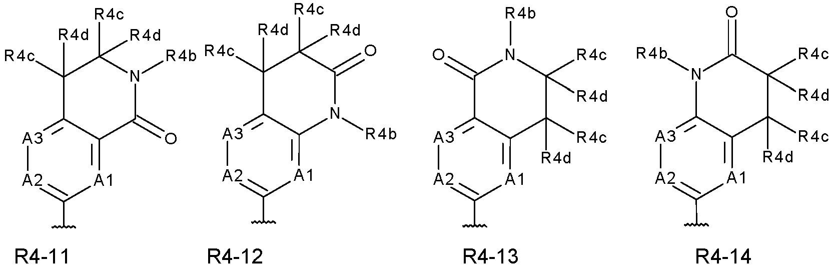

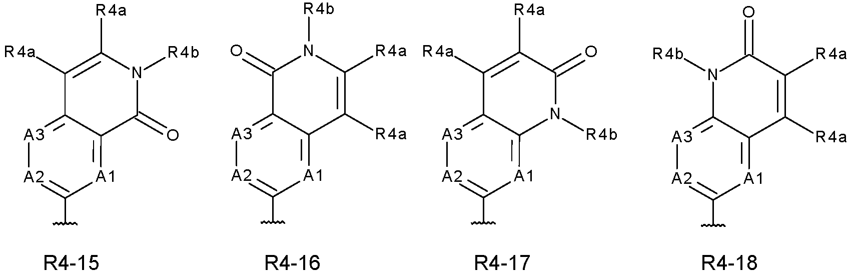

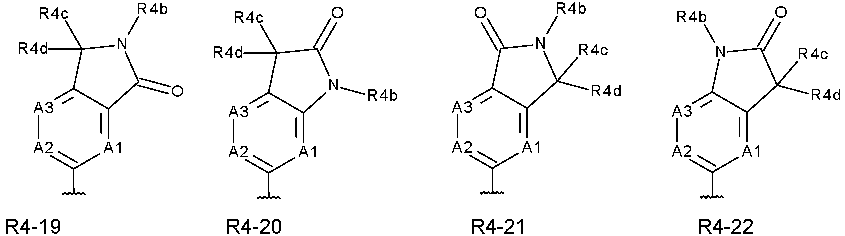

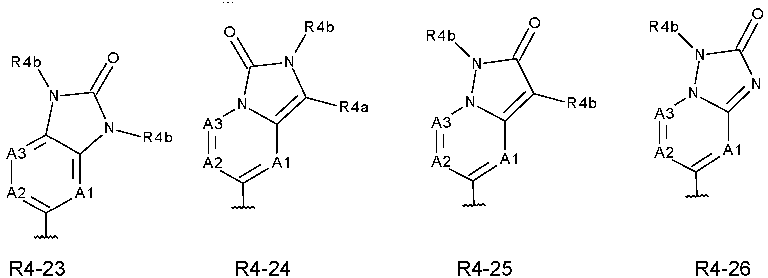

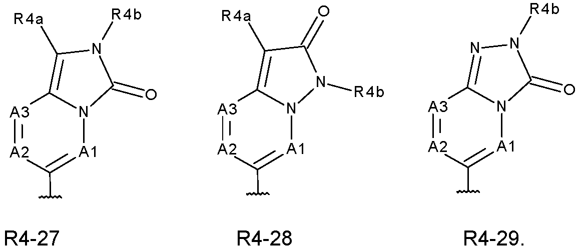

In these structures, A1, A2 and A3 are as defined above;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, - N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; R^{4b} is selected from H, halogen, -OH, -CN, -COOH, -CONH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, - CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; and
R^{4c} and R^{4d} are independently from each other selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₄-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, or
R^{4c} and R^{4d} together with the carbon atom to which they are bound, form a 3-, 4-, 5-, or 6-membered saturated or unsaturated carbocyclic ring.

Since R^{4c} and R^{4d} can combine to form together with the carbon atom to which they are bound a carbocyclic ring, the structures also cover the resulting spiro compounds, where the bicyclic ring shares one common atom with a third ring.

In various embodiments, R⁴ is R4-11 or R4-12 wherein A2 is N and A1 and A3 are CR^{4a}. In these embodiments, A3 may be CH. In these embodiments, R^{4c} and R^{4d} may be H. In these embodiments, R^{4b} may be H.

In various embodiments, R⁴ is R4-19 or R4-20 wherein A2 is N and A1 and A3 are CR^{4a}. In these embodiments, A3 may be CH. In these embodiments, R^{4c} may be H. In these embodiments, R^{4b} may be H.

In various embodiments, R^{4a} is selected from H, -COOH, -CONH₂, -NH₂, C₁-C₆-alkyl, -CO₂C₁-C₄-alkyl, - CONH(C₁-C₄-alkyl), and -CON(C₁-C₄-alkyl)₂, for example H, -COOH, -CONH₂, -NH₂, -CH₃, -CO₂ethyl, - and -CON(CH₃)₂.

In various embodiments, R^{4b} is H or methyl.

In various embodiments, R^{4c} and R^{4d} are H.

In various embodiments, R⁴ is selected from the following structures: 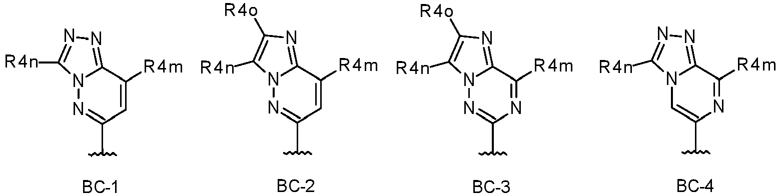 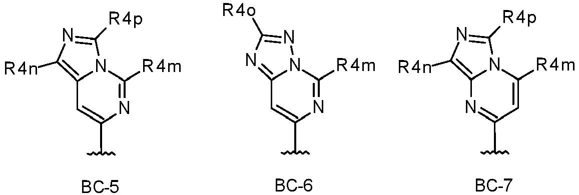 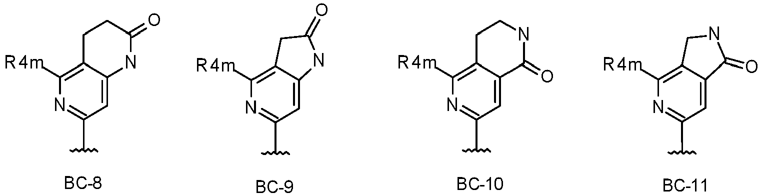 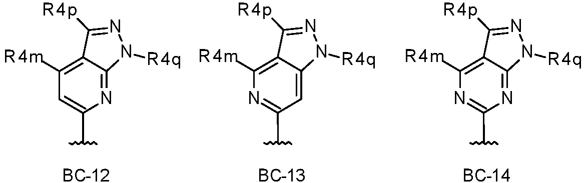 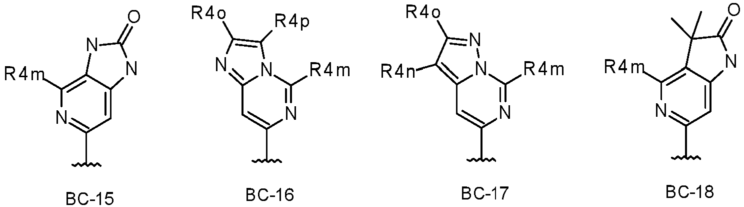 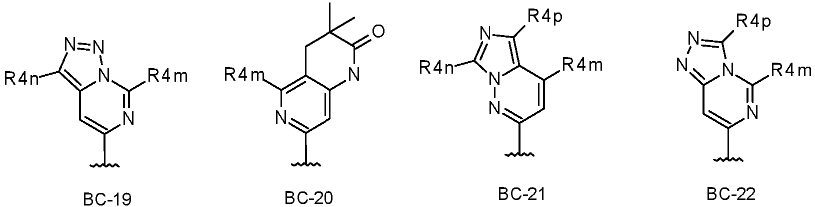 wherein R4m, R4n, R4o, R4p are defined as R^{4a} above and R4q is defined as R^{4b} above.

R⁵ is selected from H, halogen, CN, NH₂, NO₂, CONH₂, C₁-C₆-alkyl, C₁-C₆-haloalkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₁-C₆-haloalkoxy, CH(C₁-C₆-alkoxy)₂, C₂-C₆-alkenyl, C₂-C₆-alkynyl, C₁-C₆-alkoxy-C₁-C₆-alkyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, OR¹⁰ or OR^{10a}, NR¹²R¹³, C(O)NR¹²R¹³, C(O)OR¹⁰, C(O)R¹⁴, S(O)ₘ-R¹⁴, -C(=NOC₁-C₄-alkyl)H, or -C(=NOC₁-C₄-alkyl)-C₁-C₄-alkyl, wherein alkyl, alkenyl, alkynyl, cycloalkyl and alkoxy are unsubstituted or substituted with R¹¹. In various embodiments, R⁵ is H, halogen, CN, NH₂, NO₂, CONH₂, C₁-C₃-alkyl, C₁-C₃-haloalkyl, or C₃-C₆-cycloalkyl, C₁-C₃-alkoxy, C₁-C₃-haloalkoxy, -C(O)C₁-C₃alkoxy, CH(C₁-C₃alkoxy)₂, -CO₂C₁-C₄alkyl, CONH(C₁-C₄alkyl), -CON(C₁-C₄alkyl)₂, NH(C₁-C₄-alkyl), -N(C₁-C₄-alkyl)₂, -NHCO-C₁-C₄alkyl, - N(C₁-C₄alkyl)CO-C₁-C₄alkyl, C(=NOC₁-C₄alkyl)H, or -C(=NOC₁-C₄alkyl)-C₁-C₄alkyl. In various embodiments, R⁵ is H.

Exemplary compounds of formula I are the following:
(1) *N*-[1-[2-([1,2,4]triazolo[4,3-b]pyridazin-6-yl)-1,2,4-triazol-3-yl]ethyl]-3,5-bis(trifluoromethyl)benzamide;
(2) *N*-[1-(2-imidazo[2,1-f][1,2,4]triazin-2-yl-1,2,4-triazol-3-yl)ethyl]-3,5-bis(trifluoromethyl)benzamide;
(3) 3-chloro-*N*-[1-[2-([1,2,4]triazolo[4,3-b]pyridazin-6-yl)-1,2,4-triazol-3-yl]ethyl]-5-(trifluoromethyl)benzamide;
(4) 3-chloro-*N*-[1-(2-imidazo[2,1-f][1,2,4]triazin-2-yl-1,2,4-triazol-3-yl)ethyl]-5-(trifluoromethyl)benzamide;
(5) *N*-[1-[2-(4-methylsulfanylimidazo[2,1-f][1,2,4]triazin-2-yl)-1,2,4-triazol-3-yl]ethyl]-3,5-bis(trifluoromethyl)benzamide;
(6) *N*-[1-(2-imidazo[2,1-f][1,2,4]triazin-2-yl-1,2,4-triazol-3-yl)ethyl]-3,5-bis(trifluoromethyl)benzamide;
(7) 3-chloro-*N*-[1-[2-(8-methyl-[1,2,4]triazolo[4,3-b]pyridazin-6-yl)-1,2,4-triazol-3-yl]ethyl]-5-(trifluoromethyl)benzamide;
(8) *N*-[1-[2-(8-methyl-[1,2,4]triazolo[4,3-b]pyridazin-6-yl)-1,2,4-triazol-3-yl]ethyl]-3,5-bis(trifluoromethyl)benzamide;
(9) 3-chloro-*N*-[1-[2-([1,2,4]triazolo[4,3-a]pyrazin-6-yl)-1,2,4-triazol-3-yl]ethyl]-5-(trifluoromethyl)benzamide;
(10) 3-chloro-*N*-methyl-*N*-[1-[2-([1,2,4]triazolo[4,3-b]pyridazin-6-yl)-1,2,4-triazol-3-yl]ethyl]-5-(trifluoromethyl)benzamide;
(11) *N*-[1-[2-([1,2,4]triazolo[4,3-a]pyrazin-6-yl)-1,2,4-triazol-3-yl]ethyl]-3,5-bis(trifluoromethyl)benzamide;
(12) *N*-methyl-*N*-[1-[2-([1,2,4]triazolo[4,3-b]pyridazin-6-yl)-1,2,4-triazol-3-yl]ethyl]-3,5-bis(trifluoromethyl)benzamide;
(13) 3-chloro-*N*-[1-[2-([1,2,4]triazolo[4,3-b]pyridazin-6-yl)-1,2,4-triazol-3-yl]ethyl]-5-(trifluoromethylsulfonyl)benzamide;
(14) [3-chloro-5-[1-[2-([1,2,4]triazolo[4,3-b]pyridazin-6-yl)-1,2,4-triazol-3-yl]ethylcarbamoyl]phenyl] trifluoromethanesulfonate;
(15) 3-chloro-*N*-[1-[2-([1,2,4]triazolo[4,3-b]pyridazin-6-yl)-1,2,4-triazol-3-yl]ethyl]-5-(trifluoromethylsulfinyl)benzamide;
(16) 3-chloro-5-methylsulfonyl-*N*-[1-[2-([1,2,4]triazolo[4,3-b]pyridazin-6-yl)-1,2,4-triazol-3-yl]ethyl]benzamide;
(17) 3-bromo-5-(1-cyano-1-methyl-ethyl)-*N*-[1-[2-([1,2,4]triazolo[4,3-b]pyridazin-6-yl)-1,2,4-triazol-3-yl]ethyl]benzamide;
(18) 3-(1-cyanocyclopropyl)-5-(difluoromethoxy)-*N*-[1-[2-([1,2,4]triazolo[4,3-b]pyridazin-6-yl)-1,2,4-triazol-3-yl]ethyl]benzamide;
(19) 3-chloro-5-(4-fluorophenyl)sulfonyl-*N*-[1-[2-([1,2,4]triazolo[4,3-b]pyridazin-6-yl)-1,2,4-triazol-3-yl]ethyl]benzamide;
(20) *N*-[1-[2-(2-oxo-3,4-dihydro-1*H*-1,6-naphthyridin-7-yl)-1,2,4-triazol-3-yl]ethyl]-3,5-bis(trifluoromethyl)benzamide;
(21) *N*-[1-[2-(2-oxo-1,3-dihydropyrrolo[3,2-c]pyridin-6-yl)-1,2,4-triazol-3-yl]ethyl]-3,5-bis(trifluoromethyl)benzamide;
(22) *N*-[1-[2-(1-oxo-2,3-dihydropyrrolo[3,4-c]pyridin-6-yl)-1,2,4-triazol-3-yl]ethyl]-3,5-bis(trifluoromethyl)benzamide;
(23) *N*-[1-[2-(5-oxo-7,8-dihydro-6*H*-2,6-naphthyridin-3-yl)-1,2,4-triazol-3-yl]ethyl]-3,5-bis(trifluoromethyl)benzamide;
(24) *N*-methyl-*N*-[1-[2-(1-methylpyrazolo[3,4-b]pyridin-6-yl)-1,2,4-triazol-3-yl]ethyl]-3,5-bis(trifluoromethyl)benzamide;
(25) *N*-methyl-*N*-[1-[2-(1*H*-pyrazolo[3,4-b]pyridin-6-yl)-1,2,4-triazol-3-yl]ethyl]-3,5-bis(trifluoromethyl)benzamide;
(26) *N*-[1-[2-(1*H*-pyrazolo[3,4-b]pyridin-6-yl)-1,2,4-triazol-3-yl]ethyl]-3,5-bis(trifluoromethyl)benzamide;
(27) *N*-[1-(2-imidazo[1,5-c]pyrimidin-7-yl-1,2,4-triazol-3-yl)ethyl]-3,5-bis(trifluoromethyl)benzamide;
(28) *N*-[1-[2-([1,2,4]triazolo[1,5-c]pyrimidin-7-yl)-1,2,4-triazol-3-yl]ethyl]-3,5-bis(trifluoromethyl)benzamide;
(29) *N*-[1-[2-(3-amino-[1,2,4]triazolo[4,3-b]pyridazin-6-yl)-1,2,4-triazol-3-yl]ethyl]-3,5-bis(trifluoromethyl)benzamide;
(30) 3-chloro-*N*-[1-[2-([1,2,4]triazolo[1,5-c]pyrimidin-7-yl)-1,2,4-triazol-3-yl]ethyl]-5-(trifluoromethyl)benzamide;
(31) *N*-[1-[2-(3-amino-[1,2,4]triazolo[4,3-b]pyridazin-6-yl)-1,2,4-triazol-3-yl]ethyl]-*N*-methyl-3,5-bis(trifluoromethyl)benzamide;
(32) *N*-methyl-*N*-[1-[2-(1-methylpyrazolo[4,5-c]pyridin-6-yl)-1,2,4-triazol-3-yl]ethyl]-3,5-bis(trifluoromethyl)benzamide;
(33) *N*-[1-[2-(1*H*-pyrazolo[4,5-c]pyridin-6-yl)-1,2,4-triazol-3-yl]ethyl]-3,5-bis(trifluoromethyl)benzamide;
(34) *N*-[1-(2-imidazo[1,5-a]pyrimidin-2-yl-1,2,4-triazol-3-yl)ethyl]-3,5-bis(trifluoromethyl)benzamide;
(35) *N*-[1-[2-(1*H*-pyrazolo[5,4-d]pyrimidin-6-yl)-1,2,4-triazol-3-yl]ethyl]-3,5-bis(trifluoromethyl)benzamide;
(36) *N*-methyl-*N*-[1-[2-(1*H*-pyrazolo[4,5-c]pyridin-6-yl)-1,2,4-triazol-3-yl]ethyl]-3,5-bis(trifluoromethyl)benzamide;
(37) *N*-[1-[2-(3-amino-[1,2,4]triazolo[4,3-a]pyrazin-6-yl)-1,2,4-triazol-3-yl]ethyl]-3,5-bis(trifluoromethyl)benzamide;
(38) *N*-[1-[2-(3-amino-[1,2,4]triazolo[4,3-a]pyrazin-6-yl)-1,2,4-triazol-3-yl]ethyl]-*N*-methyl-3,5-bis(trifluoromethyl)benzamide;
(39) 6-[5-[(1*S*)-1-[[3,5-bis(trifluoromethyl)benzoyl]amino]ethyl]-1,2,4-triazol-1-yl]-*N*,*N*-dimethyl-[1,2,4]triazolo[4,3-b]pyridazine-8-carboxamide;
(40) 6-[5-[(1*S*)-1-[[3,5-bis(trifluoromethyl)benzoyl]-methyl-amino]ethyl]-1,2,4-triazol-1-yl]-[1,2,4]triazolo[4,3-b]pyridazine-8-carboxylic acid;
(41) ethyl 6-[5-[(1*S*)-1-[[3,5-bis(trifluoromethyl)benzoyl]amino]ethyl]-1,2,4-triazol-1-yl]-[1,2,4]triazolo[4,3-a]pyrazine-3-carboxylate;
(42) 6-[5-[(1*S*)-1-[[3,5-bis(trifluoromethyl)benzoyl]amino]ethyl]-1,2,4-triazol-1-yl]-[1,2,4]triazolo[4,3-b]pyridazine-8-carboxamide;
(43) *N*-methyl-*N*-[(1*S*)-1-[2-(8-methyl-[1,2,4]triazolo[4,3-b]pyridazin-6-yl)-1,2,4-triazol-3-yl]ethyl]-3,5-bis(trifluoromethyl)benzamide;
(44) 6-[5-[(1*S*)-1-[[3,5-bis(trifluoromethyl)benzoyl]-methyl-amino]ethyl]-1,2,4-triazol-1-yl]-[1,2,4]triazolo[4,3-b]pyridazine-8-carboxamide;
(45) 6-[5-[(1*S*)-1-[[3,5-bis(trifluoromethyl)benzoyl]amino]ethyl]-1,2,4-triazol-1-yl]-[1,2,4]triazolo[4,3-a]pyrazine-3-carboxamide;
(46) 3-(1-cyanocyclopropyl)-5-(difluoromethoxy)-N-[1-[2-([1,2,4]triazolo[1,5-c]pyrimidin-7-yl)-1,2,4-triazol-3-yl]ethyl]benzamide;
(47) 3-chloro-N-[1-[2-([1,2,4]triazolo[1,5-c]pyrimidin-7-yl)-1,2,4-triazol-3-yl]ethyl]-5-(trifluoromethylsulfonyl)benzamide;
(48) 6-[5-[(1S)-1-[[3,5-bis(trifluoromethyl)benzoyl]amino]ethyl]-1,2,4-triazol-1-yl]imidazo[1,2-b]pyridazine-8-carboxamide;
(49) N-[1-[2-(2-oxo-1,3,3a,7a-tetrahydroimidazo[4,5-c]pyridin-6-yl)-1,2,4-triazol-3-yl]ethyl]-3,5-bis(trifluoromethyl)benzamide;
(50) N-[1-(2-imidazo[1,2-c]pyrimidin-7-yl-1,2,4-triazol-3-yl)ethyl]-3,5-bis(trifluoromethyl)benzamide;
(51) 3-chloro-N-[1-[2-([1,2,4]triazolo[1,5-c]pyrimidin-7-yl)-1,2,4-triazol-3-yl]ethyl]-5-(trifluoromethylsulfinyl)benzamide;
(52) 3-chloro-N-[1-[2-([1,2,4]triazolo[1,5-c]pyrimidin-7-yl)-1,2,4-triazol-3-yl]ethyl]-5-(trifluoromethoxy)benzamide;
(53) 3-bromo-5-(1-cyano-1-methyl-ethyl)-N-[1-[2-([1,2,4]triazolo[1,5-c]pyrimidin-7-yl)-1,2,4-triazol-3-yl]ethyl]benzamide;
(54) [3-chloro-5-[1-[2-([1,2,4]triazolo[1,5-c]pyrimidin-7-yl)-1,2,4-triazol-3-yl]ethylcarbamoyl]phenyl] trifluoromethanesulfonate;
(55) 3-(1-cyanocyclopropyl)-N-[1-[2-([1,2,4]triazolo[1,5-c]pyrimidin-7-yl)-1,2,4-triazol-3-yl]ethyl]-5-(trifluoromethoxy)benzamide;
(56) N-[1-(2-pyrazolo[1,5-c]pyrimidin-5-yl-1,2,4-triazol-3-yl)ethyl]-3,5-bis(trifluoromethyl)benzamide;
(57) N-[1-[2-(3,3-dimethyl-2-oxo-3a,7a-dihydro-1H-pyrrolo[3,2-c]pyridin-6-yl)-1,2,4-triazol-3-yl]ethyl]-3,5-bis(trifluoromethyl)benzamide;
(58) N-[1-[2-(triazolo[1,5-c]pyrimidin-5-yl)-1,2,4-triazol-3-yl]ethyl]-3,5-bis(trifluoromethyl)benzamide; and
(59) N-[1-[2-(3,3-dimethyl-2-oxo-1,4,4a,8a-tetrahydro-1,6-naphthyridin-7-yl)-1,2,4-triazol-3-yl]ethyl]-3,5-bis(trifluoromethyl)benzamide.
(60) N-[1-[2-(2-ethyl-[1,2,4]triazolo[1,5-c]pyrimidin-7-yl)-1,2,4-triazol-3-yl]ethyl]-3,5-bis(trifluoromethyl)benzamide;
(61) N-[1-[2-(3-hydroxyimidazo[1,5-c]pyrimidin-7-yl)-1,2,4-triazol-3-yl]ethyl]-3,5-bis(trifluoromethyl)benzamide;
(62) N-[1-[2-(2-methyl-[1,2,4]triazolo[1,5-c]pyrimidin-7-yl)-1,2,4-triazol-3-yl]ethyl]-3,5-bis(trifluoromethyl)benzamide;
(63) 2-[5-[1-[[3,5-bis(trifluoromethyl)benzoyl]amino]ethyl]-1,2,4-triazol-1-yl]imidazo[1,5-b]pyridazine-4-carboxamide;
(64) N-[1-[2-(2-chloro-[1,2,4]triazolo[1,5-c]pyrimidin-7-yl)-1,2,4-triazol-3-yl]ethyl]-3,5-bis(trifluoromethyl)benzamide;
(65) N-[1-[2-(3-amino-[1,2,4]triazolo[4,3-c]pyrimidin-7-yl)-1,2,4-triazol-3-yl]ethyl]-3,5-bis(trifluoromethyl)benzamide
(66) N-[1-[2-(3-methyl-[1,2,4]triazolo[4,3-c]pyrimidin-7-yl)-1,2,4-triazol-3-yl]ethyl]-3,5-bis(trifluoromethyl)benzamide;
(67) N-[1-[2-(2-aminopyrazolo[1,5-c]pyrimidin-5-yl)-1,2,4-triazol-3-yl]ethyl]-3,5-bis(trifluoromethyl)benzamide.

All these compounds are specific embodiments of the compounds of formula I of the present invention. Also encompassed are the N-oxides, stereoisomers, tautomers, agriculturally or veterinarily acceptable salts of these compounds.

The compounds disclosed herein can be obtained using synthesis procedures known to those skilled in the art. The starting materials required for preparing the compounds of formula I are commercially available or known from literature. Exemplary synthesis schemes are given in the examples below. It is understood that additional compounds may be synthesized by adapting these synthesis procedures accordingly, which is well within the routine capability of those skilled in the art.

The reaction mixtures obtained can be worked up in a customary manner, for example by mixing with water, extracting with an appropriate organic solvent, separating the phases and, if appropriate, chromatographic purification of the crude products. Some of the intermediates and end products are obtained in the form of colourless or slightly brownish viscous oils which are purified or freed from volatile components under reduced pressure and at moderately elevated temperature. If the intermediates and end products are obtained as solids, purification can also be carried out by recrystallization or digestion.

If individual compounds of formula I cannot be obtained by the routes described herein below, they can be prepared by derivatization of other compounds of formula I. If the synthesis yields mixtures of isomers, a separation is generally not strictly necessarily since in some cases the individual isomers can be interconverted during work-up for use or during application (for example under the action of light, acids or bases). Such conversions may also take place after use, for example in the treatment of plants in the treated plant, or in the pest to be controlled.

### Mixtures

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The following list M of pesticides, grouped according the Mode of Action Classification of the Insecticide Resistance Action Committee (IRAC), together with which the compounds of the invention can be used and with which potential synergistic effects might be produced, illustrates the possible combinations:
M.1 AChE inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifosmethyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds: endosulfan, chlordane; phenylpyrazoles: ethiprole, fipronil, flufiprole, pyrafluprole, pyriprole;
M.3 Sodium channel modulators: pyrethroids: acrinathrin, allethrin, d-cis-trans allethrin, d-trans allethrin, bifenthrin, kappa-bifenthrin, bioallethrin, bioallethrin S-cylclopentenyl, bio-resmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; sodium channel modulators, e.g.: DDT, methoxychlor;
M.4 nAChR agonists: neonicotinoids: acetamiprid, clothianidin, cycloxaprid, dinotefuran, im-idacloprid, nitenpyram, thiacloprid, thiamethoxam; 4,5-dihydro-N-nitro-1-(2-oxiranylmethyl)-1H-imidazol-2-amine, (2E-)-1-[(6-Chloropyridin-3-yl)methyl]-N'-nitro-2-pentylidenehydrazinecarboximidamide; 1-[(6-Chloropyridin-3-yl)methyl]-7-methyl-8-nitro-5-propoxy-1,2,3,5,6,7-hexahydroimidazo[1,2-a]pyridine; nicotine; sulfoxaflor; flupyradifurone; triflumezopyrim, fenmezoditiaz, flupyrimin, 1-[(2-chlorothiazol-5-yl)methyl]-3-(3,5-dimethylisoxazol-4-yl)pyrido[1,2-a]pyrimidine-2,4-dione;
M.5 Nicotinic acetylcholine receptor allosteric activators:spinosyns, e.g. spinosad or spineto-ram;
M.6 Chloride channel activators from the class of avermectins and milbemycins, e.g. abamectin, emamectin benzoate, ivermectin, lepimectin, or milbemectin;
M.7 Juvenile hormone mimics, such as hydroprene, kino-prene, methoprene; fenoxycarb, or pyriproxyfen;
M.8 miscellaneous multi-site inhibitors: CH₃Br, other alkyl halides, chloropicrin, sulfuryl fluoride, borax, tartar emetic;
M.9 Chordotonal organ TRPV channel modulators: afidopyropen, pymetrozine; pyrifluquinazon;
M.10 Mite growth inhibitors: clofentezine, hexythiazox, diflovidazin, etoxazole;
M.11 Microbial disruptors of insect midgut membranes: *bacillus thuringiensis, bacillus sphaericus,* and insecticdal proteins they produce e.g.: *bacillus thuringiensis* subsp. *israelensis, bacillus sphaericus, bacillus thuringiensis* subsp. *aizawai, bacillus thuringiensis* subsp. *kurstaki, bacillus thuringiensis* subsp. *tenebrionis,* Bt crop proteins: Cry1Ab, Cry1Ac, Cry1Fa, Cry2Ab, mCry3A, Cry3Ab, Cry3Bb, Cry34/35Ab1;
M.12 Inhibitors of mitochondrial ATP synthase: diafenthiuron, organotin miticides, e.g.: azocyclotin, cyhexatin, fenbutatin oxide, propargite, tetradifon;
M.13 Uncouplers of oxidative phosphorylation via disruption of the proton gradient: chlorfenapyr, DNOC, sulfluramid;
M.14 nAChR channel blockers: nereistoxin analogues bensultap, cartap hydrochloride, thio-cyclam, thiosultap-sodium;
M.15 Inhibitors of the chitin biosynthesis type 0, e.g.: bistrifluron, chlorfluazuron, difluben-zuron, flucycloxuron, flufenoxuron, hexaflumuron, lufenuron, novaluron, noviflumuron, teflubenzuron, triflumuron;
M.16 Inhibitors of the chitin biosynthesis type 1: buprofezin;
M.17 Moulting disruptors: Dipteran, cyromazine;
M.18 Ecdyson receptor agonists, e.g.: methoxyfenozide, tebufenozide, halofenozide, fufeno-zide, chromafenozide;
M.19 Octopamin receptor agonists: amitraz;
M.20 Mitochondrial complex III electron transport inhibitors: hydramethylnon, acequinocyl, fluacrypyrim; bifenazate;
M.21 METI acaricides and insecticides, e.g.: fenazaquin, fenpyroximate, pyrimidifen, pyrida-ben, tebufenpyrad, tolfenpyrad, rotenone;
M.22 Voltage-dependent sodium channel blockers: indoxacarb, metaflumizone, 2-[2-(4-cyanophenyl)-1-[3-(trifluoromethyl)phenyl]ethylidene]-N-[4-(difluoromethoxy)phenyl]-hydrazine carboxamide, N-(3-chloro-2-methylphenyl)-2-[(4-chlorophenyl)[4-[methyl(methylsulfonyl)amino]phenyl]methylene]-hydrazinecarboxamide, N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide, 2-[2-(4-cyanophenyl)-1-[3-(trifluoromethyl)phenyl]ethylidene]-N-[4-(difluoromethoxy)phenyl]-hydrazinecarboxamide;
M.23 Inhibitors of the of acetyl CoA carboxylase, e.g.: spirodiclofen, spiromesifen, spirotetramat; spiropidion; spirobudifen, 11-(4-chloro-2,6-dimethylphenyl)-12-hydroxy-1,4-dioxa-9-azadispiro[4.2.4.2]tetradec-11-en-10-one, spidoxamat;
M.24 Mitochondrial complex IV electron transport inhibitors: e.g. aluminium phosphide, calcium phosphide, zinc phosphide, cyanide;
M.25 Mitochondrial complex II electron transport inhibitors, e.g.: cyenopyrafen, cyflumetofen, cyetpyrafen, pyflubumide;
M.26 Ryanodine receptor-modulators: chlorantraniliprole, cyantraniliprole, cyclaniliprole, flubendiamide, fluchlordiniliprole, (R)-3-chloro-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide, methyl-2-[3,5-dibromo-2-({[3-bromo-1-(3-chlorpyridin-2-yl)-1H-pyrazol-5-yl]carbonyl}amino)benzoyl]-1,2-dimethylhydrazine-carboxylate; N-[2-(5-amino-1,3,4-thiadiazol-2-yl)-4-chloro-6-methylphenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; tetraniliprole; tiorantraniliprole; N-[4-chloro-2-[[(1,1-dimethylethyl)amino]carbonyl]-6-methyl-phenyl]-1-(3-chloro-2-pyridinyl)-3-(fluoromethoxy)-1H-pyrazole-5-carboxamide; cyhalodiamide; N-[2-(5-amino-1,3,4-thiadiazol-2-yl)-4-chloro-6-methylphenyl]-3-bromo-1-(3-chloro-2-pyridinyl)-1H-pyrazole-5-carboxamide, pixoaniliprole;
M.27: Chordotonal organ Modulators: flonicamid;
M.28: broflanilide; fluxametamide, isocycloseram, piperflanilide;
M.29 acynonapyr;
M.UN. Unknown mode of action: afoxolaner, azadirachtin, amidoflumet, ben-zoximate, bromopropylate, chinomethionat, cryolite, cyproflanilid, dicloromezotiaz, dicofol, dimpropyridaz, flufenerim, flometoquin, fluensulfone, fluhexafon, fluopyram, fluralaner, metaldehyde, metoxadiazone, mivorilaner, modoflaner, piperonyl butoxide, pyridalyl, tioxazafen, trifluenfuronate, umifoxolaner, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 4-cyano-N-[2-cyano-5-[[[2,6-dibromo-4-[1,2,2,3,3,3-hexafluoro-1-(trifluoromethyl)propyl]phenyl]amino]carbonyl]phenyl]-2-methyl-benzamide, 4-cyano-3-[(4-cyano-2-methyl-benzoyl)amino]-N-[2,6-dichloro-4-[1,2,2,3,3,3-hexafluoro-1-(trifluoromethyl)propyl]phenyl]-2-fluoro-benzamide, N-[5-[[[2-chloro-6-cyano-4-[1,2,2,3,3,3-hexafluoro-1-(trifluoromethyl)propyl]phenyl]amino]carbonyl]-2-cyano-phenyl]-4-cyano-2-methyl-benzamide, N-[5-[[[2-bromo-6-chloro-4-[2,2,2-trifluoro-1-hydroxy-1-(trifluoro-methyl)ethyl]phenyl]amino]carbonyl]-2-cyano-phenyl]-4-cyano-2-methyl-benzamide, N-[5-[[[2-bromo-6-chloro-4-[1,2,2,3,3,3-hexafluoro-1-(trifluoromethyl)propyl]phenyl]amino]carbonyl]-2-cyano-phenyl]-4-cyano-2-methyl-benzamide, 4-cyano-N-[2-cyano-5-[[[2,6-dichloro-4-[1,2,2,3,3,3-hexafluoro-1-(trifluoromethyl)propyl]phenyl]amino]carbonyl]phenyl]-2-methyl-benzamide, 1-[2-fluoro-4-methyl-5-[(2,2,2-trifluoro-ethyl)sulfinyl]phenyl]-3-(trifluoromethyl)-1H-1,2,4-triazole-5-amine, N-[5-[[[2-bromo-6-chloro-4-[1,2,2,2-tetrafluoro-1-(trifluoromethyl)ethyl]phenyl]amino]carbonyl]-2-cyano-phenyl]-4-cyano-2-methyl-benzamide, 4-cyano-N-[2-cyano-5-[[[2,6-dichloro-4-[1,2,2,2-tetrafluoro-1-(trifluoromethyl)ethyl]phenyl]amino]carbonyl]phenyl]-2-methyl-benzamide, actives on basis of *bacillus firmus* (Votivo, I-1582); fluazaindolizine; 5-[3-[2,6-dichloro-4-(3,3-dichloroallyloxy)-phenoxy]propoxy]-1H-pyrazole; N-[5-[[2-bromo-6-chloro-4-[1,2,2,3,3,3-hexafluoro-1-(trifluoromethyl)-propyl]phenyl]-carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide;
2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; N-methylsulfonyl-6-[2-(3-pyridyl)thiazol-5-yl]pyridine-2-carboxamide; N-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; N-(3-chloro-2-methylphenyl)-2-[(4-chlorophenyl)[4-[methyl(methylsulfonyl)amino]phenyl]methylene]-hydrazinecarboxamide; 1-[(6-chloro-3-pyridinyl)methyl]-1,2,3,5,6,7-hexahydro-5-methoxy-7-methyl-8-nitro-imidazo[1,2-a]pyridine; 2-(3-pyridinyl)-N-(2-pyrimidinylmethyl )-2H-indazole-5-carboxamide; tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(tri-fluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; N-[4-chloro-3-(cyclopropylcarbamoyl)phenyl]-2-methyl-5-(1,1,2,2,2-pentafluoroethyl)-4-(trifluoromethyl)pyrazole-3-carboxamide, N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; benzpyrimoxan; tigolaner; oxazosulfyl; [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine; N-[[2-fluoro-4-[(2S,3S)-2-hydroxy-3-(3,4,5-trichlorophenyl)-3-(trifluoromethyl)pyrrolidin-1-yl]phenyl]methyl]cyclopropanecarboxamide; sulfiflumin; flupentiofenox, N-[3-chloro-1-(3-pyridyl)pyrazol-4-yl]-2-methylsulfonyl-propanamide, cyclobutrifluram; N-[4-chloro-3-[(1-cyanocyclopropyl)carbamoyl]phenyl]-2-methyl-4-methylsulfonyl-5-(1,1,2,2,2-pentafluoroethyl)pyrazole-3-carboxamide, cyproflanilide, nicofluprole; 1,4-dimethyl-2-[2-(pyridin-3-yl)-2h-indazol-5-yl]-1,2,4-triazolidine-3,5-dione, indazapyroxamet, N-[4-chloro-2-(3-pyridyl)thiazol-5-yl]-N-ethyl-3-methylsulfonyl-propanamide, N-cyclopropyl-5-[(5S)-5-(3,5-dichloro-4-fluoro-phenyl)-5-(trifluoromethyl)-4H-isoxazol-3-yl]isoquinoline-8-carboxamide, 5-[(5S)-5-(3,5-dichloro-4-fluoro-phenyl)-5-(trifluoromethyl)-4H-isoxazol-3-yl]-N-(pyrimidin-2-ylmethyl)isoquinoline-8-carboxamide, N-[1-(2,6-difluorophenyl)pyrazol-3-yl]-2-(trifluoromethyl)benzamide, 5-((1R,3R)-3-(3,5-Bis(trifluoromethyl)phenyl)-2,2-dichlorocyclopropane-1-carboxamido)-2-chloro-N-(3-(2,2-difluoroacetamido)-2,4-difluorophenyl)benzamide, 1-[6-(2,2-difluoro-7-methyl-[1,3]dioxolo[4,5-f]benzimidazol-6-yl)-5-ethylsulfonyl-3-pyridyl]cyclopropanecarbonitrile, 6-(5-cyclopropyl-3-ethylsulfonyl-2-pyridyl)-2,2-difluoro-7-methyl-[1,3]dioxolo[4,5-f]benzimidazole, Ledprona. Flupyroxystrobin, 3,5-bis(trifluoromethyl)-N-[(1S)-1-[1-[6-(trifluoromethyl)-4-pyrimidinyl]-1H-1,2,4-triazol-5-yl]ethyl]-benzamide, 2-(3-ethylsulfonyl-2-pyridyl)-5-(2,2,3,3,3-pentafluoropropoxy)pyrazine, 2-[3-ethylsulfonyl-6-(trifluoromethyl)pyrazolo[1,5-a]pyridin-2-yl]-3-methyl-6-(trifluoromethyl)imidazo[4,5-b]pyridine, 9-(methoxymethyl)-5-(3-pyridyl)-2-oxa-5,6,9,14-tetrazatricyclo[8.4.0.0^{3,7}]tetradeca-1(10),3,6,11,13-pentaen-8-one, bisulfufen, 2-[5-[(E)-2-chloro-3,3,3-trifluoro-prop-1-enyl]-1-methyl-imidazol-2-yl]-5-cyclopropyl-3-ethylsulfonyl-pyridine, 2-(5-cyclopropyl-3-ethylsulfonyl-2-pyridyl)-5-(trifluoromethylsulfinyl)-1,3-benzoxazole, isoflualanam.

The commercially available compounds M listed above may be found in The Pesticide Man-ual, 18th Edition, C. MacBean, British Crop Protection Council (2018), or http://bcpcdata.com/pesticide-manual.html, http://www.alanwood.net/pesticides.

The active compounds described by IUPAC nomenclature are known from CN103814937; WO2013/003977, WO2007/101369, WO2018/177970, CN10171577, CN102126994, WO2007/101540, WO2007/043677, WO2011/085575, WO2008/134969, WO2012/034403, WO2006/089633, WO2008/067911, WO2006/043635, WO2009/124707, WO2013/050317, WO2010/060379, WO2010/127926, WO2010/006713, WO2012/000896, WO2007/101369, WO2012/143317, WO2015/038503, EP2910126, WO2015/059039, WO2015/190316, WO2012/126766, WO2009/102736, WO2013/116053, WO2018/052136, WO2015150252, WO2020055955, WO2021158455, WO2013092350, WO201811111, EP3608311, WO2019236274, WO2013092350, WO 2018052136, WO2009102736, WO2016174049, WO2012126766, CN106554335, WO2017054524, CN105153113, WO2022072650; WO2018071327, WO2022101502, WO2012158396, WO2007079162, WO2020013147, WO2020097414, EP242081, WO2023058748, WO2017065228, EP3428166, WO2021029308, WO2022009058, WO2017067500, WO2020090585, WO2017146226, WO2021043115, WO2023016278.

The following list of fungicides, in conjunction with which the compounds of the invention can be used, illustrates the possible combinations:
A) Respiration (C)
   - complex III at Qₒ site (Qol, C3): azoxystrobin (A.1.1), bifujunzhi (A.1.37), coumethoxystrobin (A.1.2), coumoxystrobin (A.1.3), dimoxystrobin (A.1.4), enestroburin (A.1.5), famoxadone (A.1.21), fenamidone (A.1.21), fenaminstrobin (A.1.6), flufenoxystrobin (A.1.7), fluoxastrobin (A.1.8), kresoxim-methyl (A.1.9), mandestrobin (A.1.10), metominostrobin (A.1.11), metyltetraprole (A.1.25; member of MoA subgroup A), orysastrobin (A.1.12), picoxystrobin (A.1.13), pyraclostrobin (A.1.14), pyrametostrobin (A.1.15), pyraoxystrobin (A.1.16), pyribencarb (A.1.19), pyriminostrobin (A.1.36), triclopyricarb (A.1.20), trifloxystrobin (A.1.17), 2-(2-(3-(2,6-dichlorophenyl)-1-methyl-allylideneaminooxymethyl)-phenyl)-2-methoxyimino-*N*-methyl-acetamide (A.1.18), methyl-*N*-[2-[(1,4-dimethyl-5-phenyl-pyrazol-3-yl)oxylmethyl]phenyl]-*N*-methoxy-carbamate (A.1.22), (Z,2*E*)-5-[1-(2,4-dichlorophenyl)pyrazol-3-yl]-oxy-2-methoxyimino-*N*,3-dimethyl-pent-3-enamide (A.1.34), (*Z,*2*E*)-5-[1-(4-chlorophenyl)pyrazol-3-yl]oxy-2-methoxyimino-*N*,3-dimethyl-pent-3-enamide (A.1.35), 2-(ortho-((2,5-dimethylphenyl-oxymethylen)phenyl)-3-methoxy-acrylic acid methylester (A.1.38), methyl (*Z*)-3-methoxy-2-[2-methyl-5-(3-propylpyrazol-1-yl)phenoxy]prop-2-enoate, methyl (*Z*)-2-[5-(3-isopropylpyrazol-1-yl)-2-methyl-phenoxy]-3-methoxy-prop-2-enoate, methyl (*Z*)-3-methoxy-2-[2-methyl-5-[3-(trifluoromethyl)pyrazol-1-yl]phenoxy]prop-2-enoate, methyl (*Z*)-3-methoxy-2-[2-methyl-5-(4-propyltriazol-2-yl)phenoxy]prop-2-enoate, methyl (*Z*)-3-methoxy-2-[2-methyl-5-[4-(trifluoromethyl)triazol-2-yl]phenoxy]prop-2-enoate, methyl (*Z*)-2-[5-(4-isopropyltriazol-2-yl)-2-methyl-phenoxy]-3-methoxy-prop-2-enoate, methyl (*Z*)-2-(5-cyclobutyl-2-methyl-phenoxy)-3-methoxy-prop-2-enoate, methyl (*Z*)-2-(5-cyclopentyl-2-methyl-phenoxy)-3-methoxy-prop-2-enoate, methyl (*Z*)-2-(5-cyclopropyl-2-methyl-phenoxy)-3-methoxy-prop-2-enoate, methyl (*Z*)-2-(5-cyclohexyl-2-methyl-phenoxy)-3-methoxy-prop-2-enoate, methyl (*E*)-3-methoxy-2-[(2-methyl-5-phenyl-phenyl)methyl]prop-2-enoate, methyl (*E*)-3-methoxy-2-[(2-methyl-5-phenyl-phenyl)methyl]prop-2-enoate; methyl (*E*)-3-methoxy-2-[[5-[(*E*)-*N*-methoxy-*C*-methyl-carbonimidoyl]-2,4-dimethylphenyl]methyl]prop-2-enoate; methyl (*E*)-2-[[5-(2-cyclopropylethynyl)-2,4-dimethyl-phenyl]methyl]-3-methoxy-prop-2-enoate; methyl (*E*)-3-methoxy-2-(2-phenyl-1,3-benzoxazol-4-yl)prop-2-enoate; methyl (*E*)-3-methoxy-2-(2-phenyl-1,3-benzothiazol-4-yl)prop-2-enoate; methyl (*E*)-3-methoxy-2-(2-phenyl-1,3-benzoxazol-7-yl)prop-2-enoate; methyl (*Z*)-2-[6-(2-cyclopropylethynyl)benzimidazol-1-yl]-3-methoxy-prop-2-enoate; methyl (*Z*)-3-methoxy-2-(6-phenylbenzimidazol-1-yl)prop-2-enoate; methyl (*Z*)-2-(3-chloro-6-phenyl-indol-1-yl)-3-methoxy-prop-2-enoate; methyl (*Z*)-2-(2,3-dichloro-6-phenyl-indol-1-yl)-3-methoxy-prop-2-enoate; methyl (*Z*)-3-methoxy-2-[6-[(*E*)-methoxyiminomethyl]indol-1-yl]prop-2-enoate; methyl (*Z*)-3-methoxy-2-[6-[(*E*)-*N*-methoxy-*C*-methyl-carbonimidoyl]indol-1-yl]prop-2-enoate, methyl (*E*)-2-[4-chloro-2-(4-methylpent-1-ynyl)phenyl]-3-methoxy-prop-2-enoate, methyl (*E*)-3-methoxy-2-[4-methoxy-2-(4-methylpent-1-ynyl)phenyl]prop-2-enoate, methyl (*E*)-2-(4-chloro-2-hex-1-ynyl-phenyl)-3-methoxy-prop-2-enoate, methyl (*E*)-2-(2-hex-1-ynyl-4-methoxy-phenyl)-3-methoxy-prop-2-enoate, methyl (*E*)-2-[2-[[1-(4-chlorophenyl)pyrazol-3-yl]oxymethyl]-6-methyl-phenyl]-3-methoxy-prop-2-enoate, methyl (2*E*)-2-[2-[[1-(4-chlorophenyl)pyrazol-3-yl]oxymethyl]-6-methyl-phenyl]-2-methoxyimino-actate, (2*E*)-2-[2-[[1-(4-chlorophenyl)pyrazol-3-yl]oxymethyl]-6-methyl-phenyl]-2-methoxyimino-*N*-methylacetamide, methyl (*E*)-2-[2-[[(*E*)-1-(4-chlorophenyl)ethylideneamino]oxymethyl]-6-methyl-phenyl]-3-methoxy-prop-2-enoate, methyl (*E*)-3-methoxy-2-[2-methyl-6-[[(*E*)-1-[4-(trifluoromethyl)phenyl]ethylideneamino]oxymethyl]phenyl]prop-2-enoate, methyl (*E*)-2-[4-chloro-2-[[(E)-1-(4-chlorophenyl)ethylideneamino]oxymethyl]phenyl]-3-methoxy-prop-2-enoate, methyl (*E*)-2-[2-chloro-6-[[(E)-1-(4-chlorophenyl)ethylideneamino]oxymethyl]phenyl]-3-methoxy-prop-2-enoate, methyl *N*-[[5-[1-(2,6-difluoro-4-isopropyl-phenyl)pyrazol-3-yl]-2-methylphenyl]methyl]carbamate, methyl *N*-[[5-[1-(4-cyclopropyl-2,6-difluoro-phenyl)pyrazol-3-yl]-2-methylphenyl]methyl]carbamate;
   - complex III at Qᵢ site (Qil, C4): cyazofamid (A.2.1), amisulbrom (A.2.2), [(6*S*,7*R*,8*R*)-8-benzyl-3-[(3-hydroxy-4-methoxy-pyridine-2-carbonyl)amino]-6-methyl-4,9-dioxo-1,5-dioxonan-7-yl]-2-methylpropanoate (A.2.3), fenpicoxamid (A.2.4), florylpicoxamid (A.2.5), metarylpicoxamid (A.2.6);
   - complex II (SDHI, C2): benodanil (A.3.1), benzovindiflupyr (A.3.2), bixafen (A.3.3), boscalid (A.3.4), carboxin (A.3.5), cyclobutrifluram (A.3.24), fenfuram (A.3.6), fluopyram (A.3.7), flutolanil (A.3.8), fluxapyroxad (A.3.9), furametpyr (A.3.10), inpyrfluxam (A.3.22), isofetamid (A.3.11), isoflucypram (A.3.31), isopyrazam (A.3.12), mepronil (A.3.13), oxycarboxin (A.3.14), penflufen (A.3.15), penthiopyrad (A.3.16), pydiflumetofen (A.3.17), pyrapropoyne (A.3.23), pyraziflumid (A.3.18), sedaxane (A.3.19), tecloftalam (A.3.20), thifluzamide (A.3.21), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), 2-(difluoromethyl)-*N*-(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*)-3-ethyl-1,1-dimethyl-indan-4-yl]pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]pyridine-3-carboxamide (A.3.37), 2-(difluoromethyl)-*N*-(3-isobutyl-1,1-dimethyl-indan-4-yl)pyridine-3-carboxamide (A.3.38), 2-(difluoromethyl)-*N*-[(3*R*)-3-isobutyl-1,1-dimethyl-indan-4-yl]pyridine-3-carboxamide (A.3.39);
   - complex I NADH oxido-reductase (C1): diflumetorim (A.4.1);
   - uncouplers (C5): binapacryl (A.4.2), dinobuton (A.4.3), dinocap (A.4.4), fluazinam (A.4.5), meptyldinocap (A.4.6), ferimzone (A.4.7);
   - inhibitors of ox. phosphorylation (C6): fentin salts, e.g. fentin-acetate (A.4.8), fentin chloride (A.4.9) or fentin hydroxide (A.4.10);
   - ATP transport: silthiofam (A.4.11);
   - quinone inside and outside inhibitor stigmatellin binding type (QioSI; C8): ametoctradin (A.5.1);
B) Sterol biosynthesis (G)
   - C14 demethylase (DMI, G1): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluoxytioconazole (B.1.33), fluquinconazole (B.1.10), flusilazole (B.1.11), flutriafol (B.1.12), hexaconazole (B.1.13), imibenconazole (B.1.14), ipconazole (B.1.15), ipfentrifluconazole (B.1.37), mefentrifluconazole (B.1.38), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), 2-(chloromethyl)-2-methyl-5-(p-tolylmethyl)-1-(1,2,4-triazol-1-ylmethyl)cyclopentanol (B.1.43), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55), (2*R*)-2-[4-(4-chlorophenoxy)-2-(trifluoromethyl)phenyl]-1-(1,2,4-triazol-1-yl)propan-2-ol, (2*S*)-2-[4-(4-chlorophenoxy)-2-(trifluoromethyl)phenyl]-1-(1,2,4-triazol-1-yl)propan-2-ol, methyl 2-[2-chloro-4-(4-chlorophenoxy)phenyl]-2-hydroxy-3-(1,2,4-triazol-1-yl)propanoate (B.1.56), 2-[2-chloro-4-(4-chlorophenoxy)phenyl]-2-hydroxy-3-(1,2,4-triazol-1-yl)propanoic acid (B.1.57); imidazoles: imazalil (B.1.44), pefurazoate (B.1.45), prochloraz (B.1.46), triflumizole (B.1.47); pyrimidines, pyridines, piperazines: fenarimol (B.1.49), pyrifenox (B.1 .50), triforine (B.1.51), [3-(4-chloro-2-fluoro-phenyl)-5-(2,4-difluorophenyl)isoxazol-4-yl]-(3-pyridyl)methanol (B.1.52);
   - delta14-reductase (G2): aldimorph (B.2.1), dodemorph (B.2.2), dodemorph-acetate (B.2.3), fenpropidin (B.2.6), fenpropimorph (B.2.4), piperalin (B.2.7), spiroxamine (B.2.8), tridemorph (B.2.5);
   - 3-keto reductase (G3): fenhexamid (B.3.1), fenpyrazamine (B.3.2);
   - other: chlorphenomizole (B.4.1);
C) Nucleic acids metabolism (A)
   - RNA polymerase I (A1): benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - adenosine deaminase (A2): bupirimate (C.2.4), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4-amine (C.2.8);
   - DNA/RNA synthesis (A3): 5-fluorocytosine (C.2.5), hymexazole (C.2.1), octhilinone (C.2.2),
   - gyrase (A4): oxolinic acid (C.2.3);
   - dihydroorotate dehydrogenase (DHODH; A5): ipflufenoquin (C.5.1), quinofumelin (C.5.2), fenaptamidoquin (C.5.3);
D) Cytoskeleton and motor protein (B)
   - tubulin polymerization (MBC; B1): benomyl (D.1.1), carbendazim (D.1.2), fuberidazole (D.1.3), pyridachlometyl (D.1.6), thiabendazole (D.1.4), thiophanate-methyl (D.1.5), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluoro-phenyl)-*N*-(2-chloro-6-fluoro-phenyl)-2,5-dimethyl-pyrazol-3-amine (D.1.16), 4-(2-bromo-4-fluorophenyl)-*N*-(2-fluoro-6-nitrophenyl)-1,3-dimethyl-1*H*-pyrazol-5-amine, 4-(2-chloro-4,6-difluorophenyl)-*N*-(2-fluoro-6-nitrophenyl)-1,3-dimethyl-1*H*-pyrazol-5-amine, 4-(2-chloro-4-fluorophenyl)-*N*-(2-fluoro-6-nitrophenyl)-3-ethyl-1-methyl-1*H*-pyrazol-5-amine, 4-(2-chloro-4-fluorophenyl)-*N*-(2-fluoro-4-methyl-6-nitrophenyl)-1,3-dimethyl-1*H*-pyrazol-5-amine, 4-(2-chloro-4-fluorophenyl)-*N*-(2-fluoro-6-nitrophenyl)-1,3-dimethyl-1*H*-pyrazol-5-amine, 4-(2,4-difluorophenyl)-*N*-(2-fluoro-6-nitrophenyl)-1,3-dimethyl-1*H*-pyrazol-5-amine;
   - tubulin polymerization (B2): diethofencarb (D.2.1),
   - tubulin polymersation (B3): ethaboxam (D.2.2), zoxamide (D.2.5);
   - cell division (B4): pencycuron (D.2.3);
   - spectrin-like proteins (B5): fluopicolide (D.2.4), fluopimomide (D.2.9);
   - actin/myosin/fimbrin function (B6): metrafenone (D.2.6), phenamacril (D.2.8), pyriofenone (D.2.7);
E) Amino acids and protein synthesis (D)
   - methionine synthesis (D1): cyprodinil (E.1.1), mepanipyrim (E.1.2), pyrimethanil (E.1.3);
   - ribosome, termination step (D2): blasticidin-S (E.2.1);
   - ribosome initiation step (D3): kasugamycin (E.2.2), kasugamycin hydrochloride-hydrate (E.2.3);
   - ribosome initiation step (D4): streptomycin (E.2.5);
   - ribosome elongation step (D5): mildiomycin (E.2.4), oxytetracyclin (E.2.6);
F) Signal transduction
   - mechanism unknown (E1): proquinazid (F.2.2), quinoxyfen (F.2.1);
   - MAP/histidine kinase os-2 (E2): fludioxonil (F.1.5);
   - MAP/histidine kinase os-1 (E3): iprodione (F.1.2), procymidone (F.1.3), vinclozolin (F.1.4);
G) Lipid synthesis or transport / membrane (F)
   - methyl transferase (F2): edifenphos (G.1.1), iprobenfos (G.1.2), isoprothiolane (G.1.4); pyrazophos (G.1.3);
   - cell peroxidation (F3): biphenyl (G.2.5), chloroneb (G.2.6), dicloran (G.2.1), etridiazole (G.2.7), quintozene (G.2.2), tecnazene (G.2.3), tolclofos-methyl (G.2.4);
   - cell membrane permeability (F4): propamocarb (G.4.1);
   - ergosterol binding (F8): natamycin;
   - oxysterol binding protein (F9): fluoxapiprolin (G.5.3), oxathiapiprolin (G.5.1), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11), (1-(4-(4-(5-(2,6-dichlorophenyl)-4,5-dihydroisoxazol-3-yl)thiazol-2-yl)piperidin-1-yl)-2-((3-trifluoromethyl)pyrazin-2-yl)oxy)ethan-1-one, 1-(4-(4-(5-(2-chloro-6-fluorophenyl)-4,5-dihydroisoxazol-3-yl)thiazol-2-yl)piperidin-1-yl)-2-((3-trifluoromethyl)pyridin-2-yl)oxy)ethan-1-one, *tert*-butyl 4-(4-(5-(2-bromo-6-fluorophenyl)-4,5-dihydroisoxazol-3-yl)thiazol-2-yl)piperidin-1-carboxylate, ((2-(3-(2-(1-(2-(3,5-bis(trifluoromethyl)-1*H*-pyrazol-1-yl)acetyl)piperidin-4-yl)thiazol-4-yl)-4,5-dihydroisoxazo-5-yl)-3-fluorophenyl)imino)dimethyl-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-fluorophenyl)imino)dimethyl-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-chorophenyl)imino)(isopropyl)(methyl)-λ⁶-sulfanone, ((2-(3-(2-(1-(2-(3,5-bis(trifluoromethyl)-1*H*-pyrazol-1-yl)acetyl)piperidin-4-yl)thiazol-4-yl)-4,5-dihydroisoxazo-5-yl)-3-fluorophenyl)imino)(isopropyl)(methyl)-λ⁶-sulfanone, ((2-(3-(2-(1-(2-(3,5-bis(difluoromethyl)-1*H*-pyrazol-1-yl)acetyl)piperidin-4-yl)thiazol-4-yl)-4,5-dihydroisoxazo-5-yl)-3-(trifluoromethyl)phenyl)imino)dimethyl-λ⁶-sulfanone, ((3-fluoro-2-(3-(2-(1-(2-(5-methyl-3-(trifluoromethyl)-1*H*-pyrazol-1-yl)acetyl)piperidin-4-yl)thiazol-4-yl)-4,5-dihydroisoxazo-5-yl)-phenyl)imino)dimethyl-λ⁶-sulfanone;
H) Multi Site Activity (M)
   - inorganics (M01): Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - dithiocarbamates and relatives: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9), zinc thiazole (H.2.10);
   - organochlorine compounds (M04, M05, M06, M08): anilazine (H.3.1), captafol (H.3.3), captan (H.3.4), chlorothalonil (H.3.2), dichlofluanid (H.3.6), dichlorophen (H.3.7), folpet (H.3.5), hexachlorobenzene (H.3.8), pentachlorphenole (H.3.9) and its salts, phthalide (H.3.10), tolylfluanid (H.3.11);
   - guanidines, quinones, quinoxalines, maleimides, thiocarbamates (M07, M09, M10, M11, M12): chinomethionat (H.4.13), dithianon (H.4.9), fluoroimide (H.4.11), guanidine (H.4.1), guazatine (H.4.4), guazatine-acetate (H.4.5), iminoctadine (H.4.6), iminoctadine-triacetate (H.4.7), iminoctadine-tris(albesilate) (H.4.8), methasulfocarb (H.4.12), 2,6-dimethyl-1*H*,5*H*-[1,4]dithiino[2,3-c:5,6-c']dipyrrole-1,3,5,7(2*H*,6*H*)-tetraone (H.4.10),
I) Cell wall biosynthesis (H) and melanin synthesis in cell wall (I)
   - chitin synthase (H4): polyoxin B (I.1.2);
   - cellulose synthase (H5): benthiavalicarb (I.3.5), dimethomorph (I.3.1), flumorph (I.3.2), iprovalicarb (I.3.6), mandipropamid (I.3.3), pyrimorph (I.3.4), valifenalate (I.3.7);
   - reductase in melanin synthesis (MBI-R; I1) pyroquilon (I.2.1), tricyclazole (I.2.2);
   - dehydratase in melanin synthesis (MBI-D, I2); carpropamid (I.2.3), dicyclomet (I.2.4), fenoxanil (I.2.5);
   - polyketide synthase in melanin synthesis (MBI-P, I3): tolprocarb (I.2.6);
J) Plant defence induction (P1 to P8)
   - salicylate-related (P01-P03, P08): acibenzolar-S-methyl (J.1.1), probenazole (J.1.2), isotianil (J.1.3), tiadinil (J.1.4), dichlobentiazox (J.1.13); phosphonates (P07): fosetyl (J.1.6), fosetyl-aluminum (J.1.7), phosphorous acid and its salts (J.1.8), calcium phosphonate (J.1.11), potassium phosphonate (J.1.12); others: potassium or sodium bicarbonate (J.1.9), 4-cyclopropyl-N-(2,4-dimethoxyphenyl)thiadiazole-5-carboxamide (J.1.10);
K) Unknown mode of action (U)
   - aminopyrifen (K.1.54), benziothiazolinone (K.1.48), bromothalonil (K.1.49), bronopol (K.1.1), cyflufenamid (K.1.3), cymoxanil (K.1.4), dazomet (K.1.5), debacarb (K.1.6), diclomezine (K.1.8), difenzoquat (K.1.9), difenzoquat-methylsulfate (K.1.10), diphenylamin (K.1.11), dodine, dodine free base (K.1.18), fenitropan (K.1.12), flufenoxadiazam (K.1.58) [MoA proposed: class II histone deacetylase inhibitor], flumetover (K.1.14), flumetylsulforim (K.1.60), flusulfamide (K.1.15), flutianil (K.1.16), harpin (K.1.17), nitrapyrin (K.1.19), nitrothal-isopropyl (K.1.20), oxine-copper (K.1.22), picarbutrazox (K.1.41), pyrisoxazole (K.1.37), seboctylamine (K.1.61), tebufloquin (K.1.24), tecloftalam (K.1.25), triazoxide (K.1.26), validamycin (K.1.2); *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N*-ethyl-*N*-methyl formamidine (K.1.28), *N*'-[4-[[3-[(4-chlorophenyl)methyl]-1,2,4-thiadiazol-5-yl]-oxy]-2,5-dimethyl-phenyl]-*N*-ethyl-*N*-methyl-formamidine (K.1.29), *N*'-(5-bromo-6-indan-2-yloxy-2-methyl-3-pyridyl)-*N*-ethyl-*N*-methyl-formamidine (K.1.30), *N*'-[5-bromo-6-[1-(3,5-difluorophenyl)ethoxy]-2-methyl-3-pyridyl]-*N*-ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), *N*'-[5-bromo-2-methyl-6-(1-phenylethoxy)-3-pyridyl]-N-ethyl-N-methyl-formamidine (K.1.33), *N*'-(2-methyl-5-trifluoromethyl-4-(3-trimethylsilanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (*Z*)-3-amino-2-cyano-3-phenyl-prop-2-enoate (K.1.40), pentyl *N*-[6-[[(*Z*)-[(1-methyltetrazol-5-yl)-phenylmethylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), 2-(6-benzyl-2-pyridyl)quinazoline (K.1.50), 2-[6-(3-fluoro-4-methoxy-phenyl)-5-methyl-2-pyridyl]-quinazoline (K.1.51), *N*'-(2,5-dimethyl-4-phenoxy-phenyl)-*N*-ethyl-*N*-methyl-formamidine (K.1.53), *N'*-[5-bromo-2-methyl-6-(1-methyl-2-propoxy-ethoxy)-3-pyridyl]-*N*-ethyl-*N*-methyl-formamidine (K.1.56), *N*'-[4-(4,5-dichlorothiazol-2-yl)oxy-2,5-dimethyl-phenyl]-*N*-ethyl-*N*-methyl-formamidine (K.1.57), *N*-methyl-4-[5-(trifluoromethyl)-1,2,4-oxadiazol-3-yl]benzenecarbothioamide (K.1.59), *N*-methoxy-*N*-[[4-[5-(trifluoromethyl)-1,2,4-oxadiazol-3-yl]phenyl]methyl]cyclopropane-carboxamide (K.1.61), *N*-((4-[5-(trifluoromethyl)-1,2,4-oxadiazol-3-yl]phenyl)methyl)propanamide (K.1.62), 3,3,3-trifluoro-*N*-[[3-fluoro-4-[5-(trifluoromethyl)-1,2,4-oxadiazol-3-yl]phenyl]methyl]propanamide (K.1.63), 3,3,3-trifluoro-*N*-[[2-fluoro-4-[5-(trifluoromethyl)-1,2,4-oxadiazol-3-yl]phenyl]methyl] propanamide (K.1.64), *N*-[2,3-difluoro-4-[5-(trifluoromethyl)-1,2,4-oxadiazol-3-yl]benzyl]-butanamide (K.1.65), *N*-[[2,3-difluoro-4-[5-(trifluoromethyl)-1,2,4-oxadiazol-3-yl]phenyl]methyl]-3,3,3-trifluoro-propanamide (K.1.66), 1-methoxy-1-methyl-3-[[4-[5-(trifluoromethyl)-1,2,4-oxadiazol-3-yl]phenyl]methyl]urea (K.1.67), 1,1-diethyl-3-[[4-[5-[trifluoromethyl]-1,2,4-oxadiazol-3-yl]phenyl]methyl]urea (K.1.68), *N*,2-dimethoxy-*N*-[[4-[5-(trifluoromethyl)-1,2,4-oxadiazol-3-yl]phenyl]methyl]propanamide (K.1.69), *N*-ethyl-2-methyl-N-[[4-[5-(trifluoromethyl)-1,2,4-oxadiazol-3-yl]phenyl]methyl]propanamide (K.1.70), 1-methoxy-3-methyl-1-[[4-[5-(trifluoromethyl)-1,2,4-oxadiazol-3-yl]phenyl]methyl]urea (K.1.71), 1-[[4-[5-(trifluoromethyl)-1,2,4-oxadiazol-3-yl]phenyl]methyl]pyrrolidin-2-one (K.1.72), 1-[[4-[5-(trifluoromethyl)-1,2,4-oxadiazol-3-yl]phenyl]methyl]piperidin-2-one (K.1.73), 4-[[4-[5-(trifluoromethyl)-1,2,4-oxadiazol-3-yl]phenyl]methyl]morpholin-3-one (K.1.74), 4,4-dimethyl-2-[[4-[5-(trifluoromethyl)-1,2,4-oxadiazol-3-yl]phenyl]methyl]isoxazolidin-3-one (K.1.75), 2-[[4-[5-(trifluoromethyl)-1,2,4-oxadiazol-3-yl]phenyl]methyl]isoxazolidin-3-one (K.1.76), 5,5-dimethyl-2-[[4-[5-(trifluoromethyl)-1,2,4-oxadiazol-3-yl]phenyl]methyl]isoxazolidin-3-one (K.1.77), 3,3-dimethyl-1-[[4-[5-(trifluoromethyl)-1,2,4-oxadiazol-3-yl]phenyl]methyl]piperidin-2-one (K.1.78), 2-[[4-[5-(trifluoromethyl)-1,2,4-oxadiazol-3-yl]phenyl]methyl]oxazinan-3-one (K.1.79), 1-[[3-fluoro-4-[5-(trifluoromethyl)-1,2,4-oxadiazol-3-yl]phenyl]methyl]azepan-2-one (K.1.80), 4,4-dimethyl-1-[[4-[5-(trifluoromethyl)-1,2,4-oxadiazol-3-yl]phenyl]methyl]pyrrolidin-2-one (K.1.81), 5-methyl-1-[[4-[5-(trifluoromethyl)-1,2,4-oxadiazol-3-yl]phenyl]methyl]pyrrolidin-2-one (K.1.82), ethyl 1-[[4-[5-(trifluoromethyl)-1,2,4-oxadiazol-3-yl]phenyl]methyl]pyrazole-4-carboxylate (K.1.83), *N*-methyl-1-[[4-[5-(trifluoromethyl)-1,2,4-oxadiazol-3-yl]phenyl]methyl]pyrazole-4-carboxamide (K.1.84), *N*,*N*-dimethyl-1-[4-[5-(trifluoromethyl)-1,2,4-oxadiazol-3-yl]benzyl]-1*H*-1,2,4-triazol-3-amine (K.1.85), *N*-methoxy-N-methyl-1-[[4-[5-(trifluoromethyl)-1,2,4-oxadiazol-3-yl]phenyl]methyl]pyrazole-4-carboxamide (K.1.86), propyl-1-[[4-[5-(trifluoromethyl)-1,2,4-oxadiazol-3-yl]phenyl]methyl]pyrazole-4-carboxamide (K.1.87), *N*-methoxy-1-[[4-[5-(trifluoromethyl)-1,2,4-oxadiazol-3-yl]phenyl]methyl]pyrazole-4-carboxamide (K.1.88), *N*-allyl-*N*-[[4-[5-(trifluoromethyl)-1,2,4-oxadiazol-3-yl]phenyl]methyl]propanamide (K.1.89), 3-ethyl-1-methoxy-1-[[4-[5-(trifluoromethyl)-1,2,4-oxadiazol-3-yl]phenyl]methyl]urea (K.1.90), 1,3-dimethoxy-1-[[4-[5-(trifluoromethyl)-1,2,4-oxadiazol-3-yl]phenyl]methyl]urea (K.1.91), *N*-allyl-*N*-[[4-[5-(trifluoromethyl)-1,2,4-oxadiazol-3-yl]phenyl]methyl]acetamide (K.1.92), N-[4-[5-(trifluoromethyl)-1,2,4-oxadiazol-3-yl]benzyl]cyclopropanecarboxamide (K.1.93), 1-methyl-3-[[4-[5-(trifluoromethyl)-1,2,4-oxadiazol-3-yl]phenyl]methyl]urea (K.1.94), *N*'-[2-chloro-4-(2-fluorophenoxy)-5-methyl-phenyl]-*N*-ethyl-*N*-methyl-formamidine (K.1.95), *N*'-[2-chloro-4-[(4-methoxy-phenyl)methyl]-5-methyl-phenyl]-*N*-ethyl-*N*-methyl-formamidine (K.1.96), *N*'-[2-chloro-4-[(4-cyano-phenyl)methyl]-5-methyl-phenyl]-*N*-ethyl-*N*-methyl-formamidine (K.1.97), *N*'-[2,5-dimethyl-4-(o-tolylmethyl)phenyl]-N-ethyl-N-methyl-formamidine (K.1.98), 6-chloro-3-(3-cyclopropyl-2-fluoro-phenoxy)-*N*-[2-(2,4-dimethylphenyl)-2,2-difluoro-ethyl]-5-methylpyridazine-4-carboxamide (K.1.99), 3-(3-bromo-2-fluoro-phenoxy)-6-chloro-N-[2-(2-chloro-4-methylphenyl)-2,2-difluoro-ethyl]-5-methyl-pyridazine-4-carboxamide (K.1.100), 6-chloro-N-[2-(2-chloro-4-methyl-phenyl)-2,2-difluoro-ethyl]-3-(3-cyclopropyl-2-fluoro-phenoxy)-5-methyl-pyridazine-4-carboxamide (K.1.101), 6-chloro-3-(3-cyclopropyl-2-fluoro-phenoxy)-*N*-[2-(3,4-dimethylphenyl)-2,2-difluoro-ethyl]-5-methyl-pyridazine-4-carboxamide (K.1.102), 6-chloro-3-(3-chloro-2-fluoro-phenoxy)-*N*-[2-(2,4-dimethylphenyl)-2,2-difluoro-ethyl]-5-methyl-pyridazine-4-carboxamide (K.1.103), *N*-[2-(2-bromo-4-methyl-phenyl)-2,2-difluoro-ethyl]-6-chloro-3-(3-cyclopropyl-2-fluoro-phenoxy)-5-methyl-pyridazine-4-carboxamide (K.1.104), 2-[cyano-(2,6-difluoro-4-pyridyl)amino]-5-methyl-*N*-spiro[3.4]octan-3-yl-thiazole-4-carboxamide, 2-[acetyl-(2,6-difluoro-4-pyridyl)amino]-N-(2,2-dimethylcyclobutyl)-5-methyl-thiazole-4-carboxamide, 2-[(2,6-difluoro-4-pyridyl)-(2-methoxyacetyl)amino]-*N*-(2,2-dimethylcyclobutyl)-5-methyl-thiazole-4-carboxamide, 2-[cyano-(2,6-difluoro-4-pyridyl)amino]-*N*-(2,2-dimethylcyclobutyl)-5-methyl-thiazole-4-carboxamide, *N*-[1-[[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]methyl]-2-methyl-propyl]-2-methyl-propanamide, *N*-[1-[[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]methyl]-2-methyl-propyl]-2,2-dimethyl-propanamide, N-[2-[3-[2-(5-fluoro-2-methoxy-phenyl)-2-hydroxy-ethyl]-5-[(*Z*)-*N*-isopropoxy-*C*-methyl-carbonimidoyl]-2,6-dioxo-pyrimidin-1-yl]-1-methylethyl]-2-methyl-propanamide, *N*-[2-[3-[2-hydroxy-2-(2-methoxyphenyl)ethyl]-5-[(*Z*)*-N-isopropoxy-C*-methyl-carbonimidoyl]-2,6-dioxo-pyrimidin-1-yl]-1-methyl-ethyl]-2-methyl-propanamide, *N*-[2-[3-[2-(2-cyanoethoxy)-2-(5-fluoro-2-methoxy-phenyl)ethyl]-5-[(*Z*)-*N*-isopropoxy-*C*-methyl-carbonimidoyl]-2,6-dioxo-pyrimidin-1-yl]-1-methyl-ethyl]-2-methyl-propanamide, *rac*-3-[3-(3-chloro-2-fluoro-phenoxy)-6-methyl-pyridazin-4-yl]-5-[(2-chloro-4-methyl-phenyl)methyl]-5,6-dihydro-4H-1,2,4-oxadiazine, (5S)-3-[3-(3-chloro-2-fluoro-phenoxy)-6-methyl-pyridazin-4-yl]-5-[(2-chloro-4-methyl-phenyl)methyl]-5,6-dihydro-4*H*-1,2,4-oxadiazine, (5*R*)-3-[3-(3-chloro-2-fluoro-phenoxy)-6-methyl-pyridazin-4-yl]-5-[(2-chloro-4-methyl-phenyl)methyl]-5,6-dihydro-4*H*-1,2,4-oxadiazine, 2-(4-fluorophenoxy)-1-[4-[5-(trifluoromethyl)-1,2,4-oxadiazol-3-yl]phenyl]ethanone, 2-[(6-fluoro-3-pyridyl)oxy]-1-[4-[5-(trifluoromethyl)-1,2,4-oxadiazol-3-yl]phenyl]ethanone, 2-(4-fluoroanilino)-1-[4-[5-(trifluoromethyl)-1,2,4-oxadiazol-3-yl]phenyl]ethenone, ethyl 1-[[4-[[2-(trifluoromethyl)-1,3-dioxolan-2-yl]methoxy]phenyl]methyl]-1*H*-pyrazole-4-carboxylate, ethyl 1-[[4-[[(1Z)-2-ethoxy-3,3,3-trifluoro-1-propen-1-yl]oxy]phenyl]methyl]-1*H*-pyrazole-4-carboxylate.

The fungicides described by common names, their preparation and their activity e.g. against harmful fungi is known (cf.: http://www.alanwood.net/pesticides/); these substances are commercially available.

The active substances referred to as component 2) or 3) herein, their preparation and their activity e.g. against fungi is known (www.bcpcpesticidecompendium.bcpc.org/); many of them are commercially available. Compounds defined by IUPAC nomenclature, their preparation and pesticidal activity are also known (e.g. Can. J. Plant Sci. 48(6), 587-94, 1968; EP-141 317; EP-152 031; EP-226 917; EP-243 970; EP-256 503; EP-428 941; EP-532 022; EP-1 028 125; EP-1 035 122; EP-1 201 648; EP-1 122 244, JP2002316902; DE19650197; DE10021412; DE102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265, WO 23/99460, WO 21/244950, WO 21/244951, WO 22/130188, WO 22/243810, WO 21/255070, WO 21/176057, WO 21/153754, JP2023064110, JP2022153603, JP2022046550, JP2022031355, WO 22/249074, WO 23/046861, WO 18/177894, WO 20/212513, WO 20/097012, US 2023/0069915. Some compounds are identified by their CAS Registry Number.

### Biopesticides

Suitable mixing partners for the compounds of the invention also include biopesticides.

Biopesticides have been defined as a form of pesticides based on micro-organisms (bacteria, fungi, viruses, nematodes, etc.) or natural products (compounds, e.g. metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (e.g. growth or developmental regulation, attractants, repellents or defence activators (e.g. induced resistance) and are relatively non-toxic to mammals.

The following list of biopesticides, in conjunction with which the compounds of the invention can be used, illustrates the possible combinations:
L) Biopesticides
L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliquefaciens, B. amyloliquefaciens* ssp. *plantarum* (also referred to as B. *velezensis), B. megaterium, B. mojavensis, B. mycoides, B. pumilus, B. simplex, B. solisalsi, B. subtilis, B. subtilis* var. *amyloliquefaciens, B. velezensis, Candida oleophila, C. saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea f. catenulate* (also named *Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus, P. polymyxa, Pantoea vagans, Penicillium bilaiae, Phlebiopsis gigantea, Pseudomonas* sp., *Pseudomonas chloraphis, Pseudozyma flocculosa, Pichia anomala, Pythium oligandrum, Sphaerodes mycoparasitica, Streptomyces griseoviridis, S. Iydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahliae,* zucchini yellow mosaic virus (avirulent strain);
L2) Biochemical pesticides with fungicidal, bactericidal, viricidal and/or plant defense activator activity: harpin protein, plant oils (BM3): tea tree oil, orange oil (L.2.1), eugenol, limonene (L.2.2), geraniol (L.2.3), thymol (L.2.4); Reynoutria sachalinensis extract, aureobasidin (in particular aureobasidin A (L.2.5)), ambruticin (L.2.6), bafilomycin (L.2.7) (in particular bafilomycin A1, B1 and C1), chlorflavonin (L.2.8), cinnamaldehyde (L.2.9), natamycin (L.2.10; F8);
L3) Microbial pesticides with insecticidal, acaricidal, molluscidal and/or nematicidal activity: *Agrobacterium radiobacter, Bacillus cereus, B*. *firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, M. anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans, P. ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus;*
L4) Biochemical pesticides with insecticidal, acaricidal, molluscidal, pheromone and/or nematicidal activity: L-carvone, citral, (E,Z)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl-1-butanol, methyl eugenol, methyl jasmonate, (*E*,*Z*)-2,13-octadecadien-1-ol, (*E*,*Z*)-2,13-octadecadien-1-ol acetate, (*E*,*Z*)-3,13-octadecadien-1-ol, (*R*)-1-octen-3-ol, pentatermanone, (*E*,*Z*,*Z*)-3,8,11-tetradecatrienyl acetate, (*Z*,*E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract;
L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B*. *elkanii, B. japonicum, B. liaoningense, B. lupini, Delftia acidovorans, Glomus intraradices, Mesorhizobium* spp., *Rhizobium leguminosarum* bv. *phaseoli, R. I.* bv. *trifolii, R. I.* bv. *viciae, R. tropici, Sinorhizobium meliloti.*

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices e.g. ATCC or DSM refer to the acronym of the respective culture collection, for details see e.g. here: http://www. wfcc.info/ccinfo/collection/by_acronym/), are referred to in literature, registered and/or are commercially available: mixtures of Aureobasidium pullulans DSM 14940 and DSM 14941 isolated in 1989 in Konstanz, Germany (e.g. blastospores in BlossomProtect^{®} from bio-ferm GmbH, Austria), Azospirillum brasilense Sp245 originally isolated in wheat reagion of South Brazil (Passo Fundo) at least prior to 1980 (BR 11005; e.g. GELFlX^{®} Gramineas from BASF Agricultural Specialties Ltd., Brazil), A. brasilense strains Ab-V5 and Ab-V6 (e.g. in AzoMax from Novozymes BioAg Produtos papra Agricultura Ltda., Quattro Barras, Brazil or Simbiose-Maíz^{®} from Simbiose-Agro, Brazil; Plant Soil 331, 413-425, 2010), Bacillus amyloliquefaciens strain AP-188 (NRRL B-50615 and B-50331; US8,445,255); B. amyloliquefaciens ssp. plantarum strains formerly also sometimes referred to as B. subtilis, recently together with B. methylotrophicus, and B. velezensis classified as B. velezensis (Int. J. Syst. Evol. Microbiol. 66, 1212-1217, 2016): B. a. ssp. plantarum or B. velezensis D747 isolated from air in Kikugawashi, Japan (US 20130236522 A1; FERM BP 8234; e.g. Double Nickel^{™} 55 WDG from Certis LLC, USA), B. a. ssp. plantarum or B. velezensis FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), B. a. ssp. plantarum or B. velezensis FZB42 isolated from soil in Brandenburg, Germany (DSM 23117; J. Plant Dis. Prot. 105, 181-197, 1998; e.g. RhizoVital^{®} 42 from AbiTEP GmbH, Germany), B. a. ssp. plantarum or B. vele-zensis MBI600 isolated from faba bean in Sutton Bonington, Nottinghamshire, U.K. at least before 1988 (also called 1430; NRRL B 50595; US 2012/0149571 A1; e.g. Integral^{®} from BASF Corp., USA), B. a. ssp. plantarum or B. velezensis QST-713 isolated from peach orchard in 1995 in California, U.S.A. (NRRL B 21661; e.g. Serenade^{®} MAX from Bayer Crop Science LP, USA), B. a. ssp. plantarum or B. velezensis TJ1000 isolated in 1992 in South Dakoda, U.S.A. (also called 1BE; ATCC BAA-390; CA 2471555 A1; e.g. QuickRoots^{™} from TJ Technologies, Watertown, SD, USA); B. firmus CNCM I-1582, a variant of parental strain EIP-N1 (CNCM I-1556) isolated from soil of central plain area of Israel (WO 2009/126473, US6,406,690; e.g. Votivo^{®} from Bayer CropScience LP, USA), B. pumilus GHA 180 isolated from apple tree rhizo-sphere in Mexico (IDAC 260707-01; e.g. PRO-MIX^{®} BX from Premier Horticulture, Quebec, Canada), B. pumilus INR-7 otherwise referred to as BU F22 and BU-F33 isolated at least be-fore 1993 from cucumber infested by Erwinia tracheiphila (NRRL B-50185, NRRL B-50153; US 8,445,255), B. pumilus KFP9F isolated from the rhizosphere of grasses in South Africa at least before 2008 (NRRL B-50754; WO 2014/029697; e.g. BAC-UP or FUSION-P from BASF Agricultural Specialities (Pty) Ltd., South Africa), B. pumilus QST 2808 was isolated from soil collected in Pohnpei, Federated States of Micronesia, in 1998 (NRRL B 30087; e.g. Sonata^{®} or Ballad^{®} Plus from Bayer Crop Science LP, USA), B. simplex ABU 288 (NRRL B-50304; US8,445,255), B. subtilis FB17 also called UD 1022 or UD10-22 isolated from red beet roots in North America (ATCC PTA-11857; System. Appl. Microbiol. 27, 372-379, 2004; US2010/0260735; WO 2011/109395); B. thuringiensis ssp. aizawai ABTS-1857 isolated from soil taken from a lawn in Ephraim, Wisconsin, U.S.A., in 1987 (also called ABG 6346; ATCC SD-1372; e.g. XenTari^{®} from BioFa AG, Münsingen, Germany), B. t. ssp. kurstaki ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), B. t. ssp. kurstaki SB4 isolated from E. saccharina larval cadavers (NRRL B-50753; e.g. Beta Pro^{®} from BASF Agricultural Specialities (Pty) Ltd., South Africa), B. t. ssp. tenebrionis NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), Beauveria bassiana GHA (ATCC 74250; e.g. BotaniGard^{®} 22WGP from Laverlam Int. Corp., USA), B. bassiana JW-1 (ATCC 74040; e.g. Naturalis^{®} from CBC (Europe) S.r.l., Italy), B. bassiana PPRI 5339 isolated from the larva of the tortoise beetle Conchyloctenia punctata (NRRL 50757; e.g. BroadBand^{®} from BASF Agricultural Specialities (Pty) Ltd., South Africa), Bradyrhizobium elkanii strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), B. japonicum 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), B. japonicum E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), B. japonicum SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); Burkholderia sp. A396 isolated from soil in Nikko, Japan, in 2008 (NRRL B-50319; WO 2013/032693; Marrone Bio Innovations, Inc., USA), Coniothyrium minitans CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N Tek from Plant Health Care plc, U.K.), Helicoverpa armigera nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), Helicoverpa zea single capsid nucleopolyhedrovirus (HzSNPV) (e.g. Gemstar^{®} from Certis LLC, USA), Helicoverpa zea nucleopolyhedrovirus ABA-NPV-U (e.g. Heligen^{®} from AgBiTech Pty Ltd., Queensland, Australia), Heterorhabditis bacteriophora (e.g. Nemasys^{®} G from BASF Agricultural Specialities Limited, UK), Isaria fumosorosea Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), Metarhizium anisopliae var. anisopliae F52 also called 275 or V275 isolated from codling moth in Austria (DSM 3884, ATCC 90448; e.g. Met52^{®} Novozymes Biologicals BioAg Group, Canada), Metschnikowia fructicola 277 isolated from grapes in the central part of Israel (US 6,994,849; NRRL Y-30752; e.g. formerly Shemer^{®} from Agrogreen, Israel), Paecilomyces ilacinus 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), Paenibacillus alvei NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), Paenibacillus strains isolated from soil samples from a variety of European locations including Germany: P. epiphyticus Lu17015 (WO 2016/020371; DSM 26971), P. polymyxa ssp. plantarum Lu16774 (WO 2016/020371; DSM 26969), P. p. ssp. plantarum strain Lu17007 (WO 2016/020371; DSM 26970); Pasteuria nishizawae Pn1 isolated from a soybean field in the mid-2000s in Illinois, U.S.A. (ATCC SD 5833; Federal Register 76(22), 5808, February 2, 2011; e.g. Clariva^{™} PN from Syngenta Crop Protection, LLC, USA), Penicillium bilaiae (also called P. bilaii) strains ATCC 18309 (= ATCC 74319), ATCC 20851 and/or ATCC 22348 (=ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provide^{®} from Novozymes Biologicals BioAg Group, Canada), Reynoutria sachalinensis extract (EP 0307510 B1; e.g. Regalia^{®} SC from Marrone Biolnnovations, Davis, CA, USA or Milsana^{®} from BioFa AG, Germany), Steinernema carpocapsae (e.g. Millenium^{®} from BASF Agricultural Specialities Limited, UK), S. feltiae (e.g. Nemashield^{®} from BioWorks, Inc., USA; Nemasys^{®} from BASF Agricultural Specialities Limited, UK), Streptomyces microflavus NRRL B-50550 (WO 2014/124369; Bayer CropScience, Germany), Trichoderma asperelloides JM41R isolated in South Africa (NRRL 50759; also referred to as T. fertile; e.g. Trichoplus^{®} from BASF Agricultural Specialities (Pty) Ltd., South Africa), T. harzianum T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to the invention, the solid material (dry matter) of the biopesticides (with the ex-ception of oils e.g. Neem oil) are considered as active components (e.g. to be obtained after drying or evaporation of the extraction or suspension medium in case of liquid formulations of the microbial pesticides).

In accordance with the invention, the weight ratios and percentages used herein for a biological extract e.g. Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1×10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, e.g. Steinernema feltiae.

When mixtures comprising microbial pesticides are employed in crop protection, the application rates range from 1×10⁶ to 5×10¹⁶ (or more) CFU/ha, preferably from 1×10⁸ to 1×10¹³ CFU/ha, and even more preferably from 1×10⁹ to 5×10¹⁵ CFU/ha and in particular from 1×10¹² to 5×10¹⁴ CFU/ha. In the case of nematodes as microbial pesticides (e.g. Steinernema feltiae), the application rates regularly range from 1×10⁵ to 1×10¹² (or more), preferably from 1×10⁸ to 1×10¹¹, more preferably from 5×10⁸ to 1×10¹⁰ individuals (e.g. in the form of eggs, juvenile or any other live stages, preferably in an infetive juvenile stage) per ha.

When mixtures comprising microbial pesticides are employed in seed treatment, the application rates generally range from 1×10⁶ to 1×10¹² (or more) CFU/seed, preferably from 1×10⁶ to 1×10⁹ CFU/seed. Furthermore, the application rates with respect to seed treatment generally range from 1×10⁷ to 1×10¹⁴ (or more) CFU per 100 kg of seed, preferably from 1×10⁹ to 1×10¹² CFU per 100 kg of seed.

### Formulations

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agrochemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)₂SO₄, (NH₄)₃PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and am-photeric surfactants, block polymers, polyelectrolytes, and mixtures thereof. Such surfactants can be used as emusifier, dispersant, solubilizer, wetter, penetration enhancer, protective col-loid, or adjuvant. Examples of surfactants are listed in McCutcheon's, Vol.1: Emulsifiers & Detergents, McCutcheon's Directories, Glen Rock, USA, 2008 (International or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines, or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives e.g. alkylisothiazolinones and benzisothiazolinones.

Suitable anti-freezing agents are ethylene glycol, propylene glycol, urea, and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.
iii) Emulsifiable concentrates (EC) 15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active substance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. di-phenylme-thene-4,4'-diisocyanatae) are dispersed into an aqueous solution of a protective colloid (e.g. polyvinyl alcohol). The addition of a polyamine (e.g. hexamethylenediamine) results in the for-ation of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably be-tween 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

### Application methods

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Further-more, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests.

Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grapefruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gmhra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal pro-teins to plants. Transgenes which have most frequently been used are toxin genes of Bacillus spec. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinII. A further approach uses transgenes in order to produce double stranded RNA in plants to target and downregulate in-sect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, be-ing present in corn event MON87403, or by enhancing photosynthesis using the transgene bbx32, being present in the soybean event MON87712.

Cultivated plants comprising a modified oil content have been created by using the transgenes: gmfad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://cera-gmc.org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be trans-planted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hec-tare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenesis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800 g/l of active ingredient, 1-200 g/l Surfactant, 0 to 200 g/l anti-freezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001 wt% to 15 wt%, desirably from 0.001 wt% to 5 wt% of active compound.

Formulations of the compounds of the invention as aerosols (e.g in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 wt%, preferably from 0.01 to 50 wt% and most preferably from 0.01 to 15 wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

### Digital application

The compounds of the invention and the compositions containing them may be applied in combination with, or by utilizing smart agricultural technologies, such as precision agriculture, remote and proximate imaging and image recognition, or smart agricultural site management programs. These smart agricultural technologies typically include models, e.g. computer pro-grams, that support the user by considering information from a wide variety of sources to increase the quality and yield of harvested material, reduce damage by pests including the prediction of pest pressure and smart application of crop protection products, secure environ-mental protection, support quick and reliable agronomic decision making, reduce usage of fertilizers and crop protection products, reduce product residues in consumables increase spatial and temporal precision of agronomical measures, automate processes, and enable traceability of measures.

Commercially available systems which include agronomic models are e.g. FieldScripts^{™} from The Climate Corporation, Xarvio^{™} from BASF, AGLogic^{™} from John Deere, etc.

Information input for these models include but is not limited to soil data, information on the plants that are currently growing or that may grow at the area of interest including crop plants and/or unwanted vegetation, weather information, information on the location of the area and directly derivable information thereof, information on pest pressure, information on beneficial organisms, and / or historic information of any of the aforementioned.

The information usable for precision agriculture may be based on input by at least one user, be accessible from external data sources and databases, or be based on sensor data. Data sources typically includes proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations and generate control signals.

Typical technologies that are used in smart agricultural technologies include self-steering robots (such as tractors, harvesters, drones), artificial intelligence (e.g. machine learning), imaging technologies (e.g. image segmentation technologies), big data analysis, and model generation, cloud computing, and machine-to-machine communication.

Precision agriculture such as precision farming is characterized by spatially and/or temporally resolved, targeted application of active ingredients like pesticides, plant-growth-regulators, fertilizers, and/or water including the variation of application rates over the agronomic site, zone or spot application, and of the spatially and/or temporally resolved, targeted planting or seeding of desired plant propagation material to a agronomic site. Precision farming typically includes the use of geo-positioning technologies like GPS for gaining information on the location and boundaries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment.

Precision farming may typically include the application of smart spraying equipment, e.g. spot spraying, and precision spraying at a farm, e.g. by irrigation systems, tractors, robots, helicopters, airplanes, unmanned aerial vehicles, such as drones. Such equipment usually includes input sensors (such as e.g. a camera) and a processing unit configured to analyze the input data and configured to provide a recommendation or decision based on the analysis of the input data to apply the compounds of the invention or compositions comprising them to the agronomic site, e.g. the soil, the crop plants, or to control pests in a specific and precise manner. For example, pests may be detected, identified, and/or classified from imagery acquired by a camera. Such identification and/ classification can make use of image processing algorithms, which may utilize artificial intelligence (e.g. machine learning algorithms), or decision trees. In this manner, the compounds or compositions described herein can be applied only at the required location, point in time and dose rate.

### Pests

The compounds of the invention are especially suitable for efficiently combating animal pests e.g. arthropods, gastropods and nematodes including:
insects from the order of Lepidoptera, e.g. Achroia grisella, Acleris spp. e.g. A. fimbriana, A. gloverana, A. variana; Acrolepiopsis assectella, Acronicta major, Adoxophyes spp. e.g. A. cyrtosema, A. orana; Aedia leucomelas, Agrotis spp. e.g. A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia) spp. e.g. A. gemmatalis; Apamea spp., Aproaerema modicella, Archips spp. e.g. A. argyrospila, A. fuscocupreanus, A. rosana, A. xyloseanus; Argyresthia conjugella, Argyroploce spp., Argyrotaenia spp. e.g. A. velutinana; Athetis mindara, Austroasca viridigrisea, Autographa gamma, Autographa nigrisigna, Barathra brassicae, Bedellia spp., Bonagota salubricola, Borbo cinnara, Bucculatrix thurberiella, Bupalus piniarius, Busseola spp., Cacoecia spp. e.g. C. murinana, C. podana; Cactoblastis cactorum, Cadra cautella, Calingo braziliensis, Caloptilis theivora, Capua reticulana, Carposina spp. e.g. C. niponensis, C. sasakii; Cephus spp., Chaetocnema aridula, Cheimatobia brumata, Chilo spp. e.g. C. Indicus, C. suppressalis, C. partellus; Choreutis pariana, Choristoneura spp. e.g. C. conflictana, C. fumiferana, C. longicellana, C. murinana, C. occidentalis, C. rosaceana; Chrysodeixis (=Pseudoplusia) spp., e.g. C. eriosoma, C. includens; Cirphis unipuncta, Clysia ambiguella, Cnaphalocerus spp., Cnaphalocrocis medinalis, Cnephasia spp., Cochylis hospes, Coleophora spp., Colias eurytheme, Conopomorpha spp., Conotrachelus spp., Copitarsia spp., Corcyra cephalonica, Crambus caliginosellus, Crambus teterrellus, Crocidosema (=Epinotia) aporema, Cydalima (=Diaphania) perspectalis, Cydia (=Carpocapsa) spp., e.g. C. pomonella, C. latiferreana; Dalaca noctuides, Datana integerrima, Dasychira pinicola, Dendrolimus spp., e.g. D. pini, D. spectabilis, D. sibiricus; Desmia funeralis, Diaphania spp., e.g. D. nitidalis, D. hyalinata; Diatraea grandiosella, Diatraea saccharalis, Diphthera festiva, Earias spp. e.g. E. insulana, E. vittella; Ecdytolopha aurantianu, Egira (=Xylomyges) curialis, Elasmopalpus lignosellus, Eldana saccharina, Endopiza viteana, Ennomos subsignaria, Eoreuma loftini, Ephestia spp., e.g. E. cautella, E. elutella, E. kuehniella; Epinotia aporema, Epiphyas postvittana, Erannis tiliaria, Erionota thrax, Etiella spp., Eulia spp., Eupoecilia ambiguella, Euproctis chrysorrhoea, Euxoa spp., Evetria bouliana, Faronta albilinea, Feltia spp. e.g. F. subterranean; Galleria mellonella, Gracillaria spp., Grapholita spp. e.g. G. funebrana, G. molesta, G. inopinata; Halysidota spp., Harrisina americana, Hedylepta spp., Helicoverpa spp. e.g. H. armigera (=Heliothis armigera), H. zea (=Heliothis zea); Heliothis spp. e.g. H. assulta, H. subflexa, H. virescens; Hellula spp. e.g. H. undalis, H. rogatalis; Helocoverpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera spp. e.g. L. coffeella, L. scitella; Leuminivora lycinivorella, Lithocolletis blancardella, Lithophane antennata, Llattia octo (=Amyna axis), Lobesia botrana, Lophocampa spp., Loxagrotis albicosta, Loxostege spp. e.g. L. sticticalis, L. cereralis; Lymantria spp., e.g. L. dispar, L. monacha; Lyonetia clerkella, Lyonetia prunifoliella, Malacosoma spp., e.g. M. americanum, M. californicum, M. constrictum, M. neu-stria; Mamestra spp., e.g. M. brassicae, M. configurata; Mamstra brassicae, Manduca spp. e.g. M. quinquemaculata, M. sexta; Marasmia spp, Marmara spp., Maruca testulalis, Megalopyge lanata, Melanchra picta, Melanitis leda, Mocis spp., e.g. M. lapites, M. repanda; Mocis latipes, Monochroa fragariae, Mythimna separata, Nemapogon cloacella, Neoleucinodes elegantalis, Nepytia spp., Nymphula spp., Oiketicus spp., Omiodes indicata, Omphisa anastomosalis, Operophtera brumata, Orgyia pseudotsugata, Oria spp., Orthaga thyrisalis, Ostrinia spp. e.g. O. nubilalis; Oulema oryzae, Paleacrita vernata, Panolis flammea, Parnara spp., Papaipema nebris, Papilio cresphontes, Paramyelois transitella, Paranthrene regalis, Paysandisia archon, Pectinophora spp. e.g. P. gossypiella; Peridroma saucia, Perileucoptera spp., e.g. P. coffeella; Phalera bucephala, Phryganidia californica, Phthorimaea spp. e.g. P. operculella; Phyllocnistis citrella, Phyllonorycter spp. e.g. P. blancardella, P. crataegella, P. issikii, P. ringoniella; Pieris spp. e.g. P. brassicae, P. rapae, P. napi; Pilocrocis tripunctata, Plathypena scabra, Platynota spp. e.g. P. flavedana, P. idaeusalis, P. stultana; Platyptilia carduidactyla, Plebejus argus, Plo-dia interpunctella, Plusia spp, Plutella maculipennis, Plutella xylostella, Pontia protodica, Prays spp., Prodenia spp., Proxenus lepigone, Pseudaletia spp. e.g. P. sequax, P. unipuncta; Pyrausta nubilalis, Rachiplusia nu, Richia albicosta, Rhizobius ventralis, Rhyacionia frustrana, Sabulodes aegrotata, Schizura concinna, Schoenobius spp., Schreckensteinia festaliella, Scirpophaga spp. e.g. S. incertulas, S. innotata; Scotia segetum, Sesamia spp. e.g. S. inferens, Seudyra subflava, Sitotroga cerealella, Sparganothis pilleriana, Spilonota lechriaspis, S. ocellina, Spodoptera (=Lamphygma) spp. e.g. S. cosmoides, S. eridania, S. exigua, S. frugiperda, S. latisfascia, S. littoralis, S. litura, S. omithogalli; Stigmella spp., Stomopteryx subsecivella, Strymon bazochii, Sylepta derogata, Synanthedon spp. e.g. S. exitiosa, Tecia solanivora, Telehin licus, Thaumatopoea pityocampa, Thaumatotibia (=Cryptophlebia) leucotreta, Thaumetopoea pityocampa, Thecla spp., Theresimima ampelophaga, Thyrinteina spp, Tildenia inconspicuella, Tinea spp. e.g. T. cloacella, T. pellionella; Tineola bisselliella, Tortrix spp. e.g. T. viridana; Trichophaga tapetzella, Trichoplusia spp. e.g. T. ni; Tuta (=Scrobipalpula) absoluta, Udea spp. e.g. U. rubigalis, U. rubigalis; Virachola spp., Yponomeuta padella, and Zeiraphera canadensis;
insects from the order of Coleoptera, e.g. Acalymma vittatum, Acanthoscehdes obtectus, Adoretus spp., Agelastica alni, Agrilus spp. e.g. A. anxius, A. planipennis, A. sinuatus; Agriotes spp. e.g. A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora spp. e.g. A. glabripennis; Anthonomus spp. e.g. A. eugenii, A. grandis, A. pomorum; Anthrenus spp., Aphthona euphoridae, Apion spp., Apogonia spp., Athous haemorrhoidalis, Atomaria spp. e.g. A. linearis; Attagenus spp., Aulacophora femoralis, Blastophagus piniperda, Blitophaga undata, Bruchidius obtectus, Bruchus spp. e.g. B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus spp. e.g. C. assimilis, C. napi; Chaetocnema tibialis, Cleonus mendicus, Conoderus spp. e.g. C. vespertinus; Conotrachelus nenuphar, Cosmopolites spp., Costelytra zealandica, Crioceris asparagi, Cryptolestes ferrugineus, Cryptorhynchus lapathi, Ctenicera spp. e.g. C. destructor; Curculio spp., Cylindrocopturus spp., Cyclocephala spp., Dac-tylispa balyi, Dectes texanus, Dermestes spp., Diabrotica spp. e.g. D. undecimpunctata, D. speciosa, D. longicornis, D. semipunctata, D. virgifera; Diaprepes abbreviates, Dichocrocis spp., Dicladispa armigera, Diloboderus abderus, Diocalandra frumenti (Diocalandra stigmaticollis), Enaphalodes rufulus, Epilachna spp. e.g. E. varivestis, E. vigintioctomaculata; Epitrix spp. e.g. E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera spp., e.g. H. brunneipennis, H. postica; Hypomeces squamosus, Hypothenemus spp., Ips typographus, Lachnosterna consanguinea, Lasioderma serricorne, Latheticus oryzae, Lathridius spp., Lema spp. e.g. L. bilineata, L. melanopus; Leptinotarsa spp. e.g. L. decemlineata; Leptispa pygmaea, Limonius californi-cus, Lissorhoptrus oryzophilus, Lixus spp., Luperodes spp., Lyctus spp. e.g. L. bruneus; Liogenys fuscus, Macrodactylus spp. e.g. M. subspinosus; Maladera matrida, Megaplatypus mutates, Megascelis spp., Melanotus communis, Meligethes spp. e.g. M. aeneus; Melolontha spp. e.g. M. hippocastani, M. melolontha; Metamasius hemipterus, Microtheca spp., Migdolus spp. e.g. M. fryanus, Monochamus spp. e.g. M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon spp. e.g. P. brassicae, P. cochleariae; Phoracantha recurva, Phyllobius pyri, Phyllopertha horticola, Phyllophaga spp. e.g. P. helleri; Phyllotreta spp. e.g. P. chrysocephala, P. nemorum, P. striolata, P. vittula; Phyllopertha horticola, Popillia japonica, Premnotrypes spp., Psacothea hilaris, Psylliodes chrysocephala, Prostephanus truncates, Psylliodes spp., Ptinus spp., Pulga saltona, Rhizopertha dominica, Rhynchophorus spp. e.g. R. billineatus, R. ferrugineus, R. palmarum, R. phoenicis, R. vulneratus; Saperda candida, Scolytus schevyrewi, Scyphophorus acupunctatus, Sitona lineatus, Sitophilus spp. e.g. S. granaria, S. oryzae, S. zeamais; Sphenophorus spp. e.g. S. levis; Stegobium paniceum, Sternechus spp. e.g. S. subsignatus; Strophomorphus ctenotus, Symphyletes spp., Tanymecus spp., Tenebrio molitor, Tenebrioides mauretanicus, Tribolium spp. e.g. T. castaneum; Trogoderma spp., Tychius spp., Xylotrechus spp. e.g. X. pyrrhoderus; and, Zabrus spp. e.g. Z. tenebrioides;
insects from the order of Diptera e.g. Aedes spp. e.g. A. aegypti, A. albopictus, A. vexans; Anastrepha ludens, Anopheles spp. e.g. A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia spp. e.g. C. bezziana, C. hominivorax, C. macellaria; Chrysops atlanticus, Chrysops discalis, Chrysops silacea, Cochliomyia spp. e.g. C. hominivorax; Contarinia spp. e.g. C. sorghicola; Cordylobia anthropophaga, Culex spp. e.g. C. nigripalpus, C. pipiens, C. quinquefasciatus, C. tarsalis, C. tritaeniorhynchus; Culicoides furens, Culiseta inornata, Culiseta melanura, Cuterebra spp., Dacus cucurbitae, Dacus oleae, Dasineura brassicae, Dasineura oxycoccana, Delia spp. e.g. D. antique, D. coarctata, D. platura, D. radicum; Dermatobia hominis, Drosophila spp. e.g. D. suzukii, Fannia spp. e.g. F. canicularis; Gastraphilus spp. e.g. G. intestinalis; Geomyza tipunctata, Glossina spp. e.g. G. fuscipes, G. morsitans, G. palpalis, G. tachinoides; Haematobia irritans, Haplodiplosis equestris, Hippelates spp., Hylemyia spp. e.g. H. platura; Hypoderma spp. e.g. H. lineata; Hyppobosca spp., Hydrellia philippina, Leptoconops torrens, Liriomyza spp. e.g. L. sativae, L. trifolii; Lucilia spp. e.g. L. caprina, L. cuprina, L. sericata; Lycoria pectoralis, Mansonia titillanus, Mayetiola spp. e.g. M. destructor; Musca spp. e.g. M. autumnalis, M. domestica; Muscina stabulans, Oestrus spp. e.g. O. ovis; Opomyza florum, Oscinella spp. e.g. O. frit; Orseolia oryzae, Pegomya hysocyami, Phlebotomus argentipes, Phorbia spp. e.g. P. antiqua, P. brassicae, P. coarctata; Phytomyza gymnostoma, Prosimulium mixtum, Psila rosae, Psorophora columbiae, Psorophora discolor, Rhagoletis spp. e.g. R. cerasi, R. cingulate, R. indifferens, R. mendax, R. pomonella; Rivellia quadrifasciata, Sarcophaga spp. e.g. S. haemorrhoidalis; Simulium vittatum, Sitodiplosis mosellana, Stomoxys spp. e.g. S. calcitrans; Tabanus spp. e.g. T. atratus, T. bovinus, T. lineola, T. similis; Tannia spp., Thecodiplo-sis japonensis, Tipula oleracea, Tipula paludosa, Wohlfahrtia spp, and Zaprionus indianus;
insects from the order of Thysanoptera e.g., Baliothrips biformis, Dichromothrips corbetti, Dichromothrips ssp., Echinothrips americanus, Enneothrips flavens, Frankliniella spp. e.g. F. fusca, F. occidentalis, F. tritici; Heliothrips spp., Hercinothrips femoralis, Kakothrips spp., Microcephalothrips abdominalis, Neohydatothrips samayunkur, Pezothrips kellyanus, Rhipiphorothrips cruentatus, Scirtothrips spp. e.g. S. citri, S. dorsalis, S. perseae; Stenchaetothrips spp, Taeniothrips cardamoni, Taeniothrips inconsequens, Thrips spp. e.g. T. imagines, T. hawaiiensis, T. oryzae, T. palmi, T. parvispinus, T. tabaci;
insects from the order of Hemiptera e.g., Acizzia jamatonica, Acrosternum spp., e.g. A. hilare; Acyrthosipon spp., e.g. A. onobrychis, A. pisum; Adelges laricis, Adelges tsugae, Adelphocoris spp., e.g. A. rapidus, A. superbus; Aeneolamia spp., Agonoscena spp., Aulacorthum solani, Aleurocanthus woglumi, Aleurodes spp., Aleurodicus disperses, Aleurolobus barodensis, Aleurothrixus spp., Amrasca spp., Anasa tristis, Antestiopsis spp., Anuraphis cardui, Aonidiella spp., Aphanostigma piri, Aphidula nasturtii, Aphis spp. e.g. A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella spp., Aspidiotus spp., Atanus spp., Aulacaspis yasumatsui, Aulacorthum solani, Bactericera cockerelli (Paratrioza cockerelli), Bemisia spp. e.g. B. argentifolii, B. tabaci (Aleurodes tabaci); Blissus spp. e.g. B. leucopterus; Brachycaudus spp. e.g. B. cardui, B. helichrysi, B. persicae, B. prunicola; Brachycolus spp., Brachycorynella as-paragi, Brevicoryne brassicae, Cacopsylla spp. e.g. C. fulguralis, C. pyricola (Psylla piri); Calligypona marginata, Calocoris spp., Campylomma livida, Capitophorus horni, Carneocephala fulgida, Cavelerius spp., Ceraplastes spp., Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimex spp. e.g. C. hemipterus, C. lectularius; Circulifer tenellus, Coccomytilus halli, Coccus spp. e.g. C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus spp., Dasynus piperis, Dialeurodes spp. e.g. D. citrifolii; Dalbulus maidis, Diaphorina spp. e.g. D. citri; Diaspis spp. e.g. D. bromeliae; Dichelops furcatus, Diconocoris hewetti, Doralis spp., Dreyfusia nordmannianae, Dreyfusia piceae, Drosicha spp., Dysaphis spp. e.g. D. plantaginea, D. pyri, D. radicola; Dysaulacorthum pseudosolani, Dysdercus spp. e.g. D. cingulatus, D. intermedius; Dysmicoccus spp., Edessa spp., Geocoris spp., Empoasca spp. e.g. E. fabae, E. solana; Epidiaspis leperii, Eriosoma spp. e.g. E. lanigerum, E. pyricola; Erythroneura spp., Eurygaster spp. e.g. E. integriceps; Euscelis bilobatus, Euschistus spp. e.g. E. heros, E. impictiventris, E. servus; Fiorinia theae, Geococcus coffeae, Glycaspis brimblecombei, Halyomorpha spp. e.g. H. halys; Heliopeltis spp., Homalodisca vitripennis (=H. coagulata), Horcias nobilellus, Hyalopterus pruni, Hyperomyzus lactucae, Icerya spp. e.g. I. purchase; Idiocerus spp., Idioscopus spp., Laodelphax striatellus, Lecanium spp., Lecanoideus floccissimus, Lepidosaphes spp. e.g. L. ulmi; Leptocorisa spp., Leptoglossus phyllopus, Lipaphis erysimi, Lygus spp. e.g. L. hesperus, L. lineolaris, L. praten-sis; Maconellicoccus hirsutus, Marchalina hellenica, Macropes excavatus, Macrosiphum spp. e.g. M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella spp., Metopolophium dirhodum, Monellia costalis, Mo-nelliopsis pecanis, Myzocallis coryli, Murgantia spp., Myzus spp. e.g. M. ascalonicus, M. cerasi, M. nicotianae, M. persicae, M. varians; Nasonovia ribisnigri, Neotoxoptera formosana, Neomegalotomus spp, Nephotettix spp. e.g. N. malayanus, N. nigropictus, N. parvus, N. vires-cens; Nezara spp. e.g. N. viridula; Nilaparvata lugens, Nysius huttoni, Oebalus spp. e.g. O. pugnax; Oncometopia spp., Orthezia praelonga, Oxycaraenus hyalinipennis, Parabemisia myricae, Parlatoria spp., Parthenolecanium spp. e.g. P. corni, P. persicae; Pemphigus spp. e.g. P. bursarius, P. populivenae; Peregrinus maidis, Perkinsiella saccharicida, Phenacoccus spp. e.g. P. aceris, P. gossypii; Phloeomyzus passerinii, Phorodon humuli, Phylloxera spp. e.g. P. devastatrix, Piesma quadrata, Piezodorus spp. e.g. P. guildinii; Pinnaspis aspidistrae, Planococcus spp. e.g. P. citri, P. ficus; Prosapia bicincta, Protopulvinaria pyriformis, Psallus seriatus, Pseudacysta persea, Pseudaulacaspis pentagona, Pseudococcus spp. e.g. P. comstocki; Psylla spp. e.g. P. mali; Pteromalus spp., Pulvinaria amygdali, Pyrilla spp., Quadraspidiotus spp., e.g. Q. perniciosus; Quesada gigas, Rastrococcus spp., Reduvius senilis, Rhizoecus americanus, Rhodnius spp., Rhopalomyzus ascalonicus, Rhopalosiphum spp. e.g. R. pseudobrassicas, R. insertum, R. maidis, R. padi; Sagatodes spp., Sahlbergella singularis, Saissetia spp., Sappaphis mala, Sappaphis mali, Scaptocoris spp., Scaphoideus titanus, Schizaphis graminum, Schizoneura lanuginosa, Scotinophora spp., Selenaspidus articulatus, Sitobion avenae, Sogata spp., Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta spp. e.g. T. accerra, T. perditor; Tibraca spp., Tomaspis spp., Toxoptera spp. e.g. T. aurantii; Trialeurodes spp. e.g. T. abutilonea, T. ricini, T. vaporariorum; Triatoma spp., Trioza spp., Typhlocyba spp., Unaspis spp. e.g. U. citri, U. yanonensis; and Viteus vitifolii;
insects from the order Hymenoptera e.g. Acanthomyops interjectus, Athalia rosae, Atta spp. e.g. A. capiguara, A. cephalotes, A. cephalotes, A. laevigata, A. robusta, A. sexdens, A. texana, Bombus spp., Brachymyrmex spp., Camponotus spp. e.g. C. floridanus, C. pennsylvanicus, C. modoc; Cardiocondyla nuda, Chalibion sp, Crematogaster spp., Dasymutilla occidentalis, Diprion spp., Dolichovespula maculata, Dorymyrmex spp., Dryocosmus kuriphilus, Formica spp., Hoplocampa spp. e.g. H. minuta, H. testudinea; Iridomyrmex humilis, Lasius spp. e.g. L. niger, Linepithema humile, Liometopum spp., Leptocybe invasa, Monomorium spp. e.g. M. pharaonis, Monomorium, Nylandria fulva, Pachycondyla chinensis, Paratrechina longicornis, Paravespula spp., e.g. P. germanica, P. pennsylvanica, P. vulgaris; Pheidole spp. e.g. P. megacephala; Pogonomyrmex spp. e.g. P. barbatus, P. californicus, Polistes rubiginosa, Prenolepis impairs, Pseudomyrmex gracilis, Schelipron spp., Sirex cyaneus, Solenopsis spp. e.g. S. geminata, S.invicta, S. molesta, S. richteri, S. xyloni, Sphecius speciosus, Sphex spp., Tapinoma spp. e.g. T. melanocephalum, T. sessile; Tetramorium spp., e.g. T. caespitum, T. bicarinatum, Vespa spp., e.g. V. crabro; Vespula spp., e.g. V. squamosal; Wasmannia auropunctata, Xylocopa sp; insects from the order Orthoptera e.g. Acheta domesticus, Calliptamus italicus, Chortoicetes terminifera, Ceuthophilus spp., Diastrammena asynamora, Dociostaurus maroccanus, Gryllotalpa spp. e.g. G. africana, G. gryllotalpa; Gryllus spp., Hieroglyphus daganensis, Kraussaria angulifera, Locusta spp. e.g. L. migratoria, L. pardalina; Melanoplus spp. e.g. M. bivittatus, M. femurrubrum, M. mexicanus, M. sanguinipes, M. spretus; Nomadacris septemfasciata, Oedaleus senegalensis, Scapteriscus spp., Schistocerca spp. e.g. S. americana, S. gregaria, Stemopelmatus spp., Tachycines asynamorus, and Zonozerus variegatus;
pests from the Class Arachnida e.g. Acari,e.g. of the families Argasidae, Ixodidae and Sar-coptidae, e.g. Amblyomma spp. (e.g. A. americanum, A. variegatum, A. maculatum), Argas spp. e.g. A. persicu), Boophilus spp. e.g. B. annulatus, B. decoloratus, B. microplus, Dermacentor spp. e.g. D.silvarum, D. andersoni, D. variabilis, Hyalomma spp. e.g. H. truncatum, Ixodes spp. e.g. I. ricinus, I. rubicundus, I. scapularis, I. holocyclus, I. pacificus, Rhipicephalus sanguineus, Ornithodorus spp. e.g. O. moubata, O. hermsi, O. turicata, Ornithonyssus bacoti, Otobius megnini, Dermanyssus gallinae, Psoroptes spp. e.g. P. ovis, Rhipicephalus spp. e.g. R. sanguineus, R. appendiculatus, Rhipicephalus evertsi, Rhizoglyphus spp., Sarcoptes spp. e.g. S. Scabiei; and Family Eriophyidae including Aceria spp. e.g. A. sheldoni, A. anthocoptes, Acallitus spp., Aculops spp. e.g. A. lycopersici, A. pelekassi; Aculus spp. e.g. A. schlechtendali; Colomerus vitis, Epitrimerus pyri, Phyllocoptruta oleivora; Eriophytes ribis and Eriophyes spp. e.g. Eriophyes sheldoni; Family Tarsonemidae including Hemitarsonemus spp., Phytonemus pallidus and Polyphagotarsonemus latus, Stenotarsonemus spp. Steneotarsonemus spinki; Family Tenuipalpidae including Brevipalpus spp. e.g. B. phoenicis; Family Tetranychidae including Eotetranychus spp., Eutetranychus spp., Oligonychus spp., Petrobia latens, Tetranychus spp. e.g. T. cinnabarinus, T. evansi, T. kanzawai, T, pacificus, T. phaseulus, T. telarius and T. urticae; Bryobia praetiosa; Panonychus spp. e.g. P. ulmi, P. citri; Metatetranychus spp. and Oligonychus spp. e.g. O. pratensis, O. perseae, Vasates lycopersici; Raoiella indica, Family Carpoglyphidae including Carpoglyphus spp.; Penthaleidae spp. e.g. Halotydeus destructor; Family Demodicidae with species e.g. Demodex spp.; Family Trombicidea including Trombicula spp.; Family Macronyssidae including Ornothonyssus spp.; Family Pyemotidae including Pyemotes tritici; Tyrophagus putrescentiae; Family Acaridae includ-ing Acarus siro; Family Araneida including Latrodectus mactans, Eratigena agrestis, Cheiracanthium sp, Lycosa sp Achaearanea tepidariorum and Loxosceles reclusa;
pests from the Phylum Nematoda, e.g. plant parasitic nematodes e.g. root-knot nematodes, Meloidogyne spp. e.g. M. hapla, M. incognita, M. javanica; cyst-forming nematodes, Globodera spp. e.g. G. rostochiensis; Heterodera spp. e.g. H. avenae, H. glycines, H. schachtii, H. trifolii; Seed gall nematodes, Anguina spp.; Stem and foliar nematodes, Aphelenchoides spp. e.g. A. besseyi; Sting nematodes, Belonolaimus spp. e.g. B. longicaudatus; Pine nematodes, Bursaphelenchus spp. e.g. B. lignicolus, B. xylophilus; Ring nematodes, Criconema spp., Criconemella spp. e.g. C. xenoplax and C. ornata; and, Criconemoides spp. e.g. Criconemoides informis; Mesocriconema spp.; Stem and bulb nematodes, Ditylenchus spp. e.g. D. destructor, D. dipsaci; Awl nematodes, Dolichodorus spp.; Spiral nematodes, Heliocotylenchus multicinctus; Sheath and sheathoid nematodes, Hemicycliophora spp. and Hemicriconemoides spp.; Hirshmanniella spp.; Lance nematodes, Hoploaimus spp.; False rootknot nematodes, Nacobbus spp.; Needle nematodes, Longidorus spp. e.g. L. elongatus; Lesion nematodes, Pratylenchus spp. e.g. P. brachyurus, P. neglectus, P. penetrans, P. curvitatus, P. goodeyi; Burrowing nema-todes, Radopholus spp. e.g. R. similis; Rhadopholus spp.; Rhodopholus spp.; Reniform nematodes, Rotylenchus spp. e.g. R. robustus, R. reniformis; Scutellonema spp.; Stubby-root nematode, Trichodorus spp. e.g. T. obtusus, T. primitivus; Paratrichodorus spp. e.g. P. minor; Stunt nematodes, Tylenchorhynchus spp. e.g. T. claytoni, T. dubius; Citrus nematodes, Tylenchulus spp. e.g. T. semipenetrans; Dagger nematodes, Xiphinema spp.; and other plant parasitic nematode species;
insects from the order Blattodea e.g. Macrotermes spp. e.g. M. natalensis; Cornitermes cu-mulans, Procornitermes spp., Globitermes sulfureus, Neocapritermes spp. e.g. N. opacus, N. parvus; Odontotermes spp., Nasutitermes spp. e.g. N. corniger; Coptotermes spp. e.g. C. for-mosanus, C. gestroi, C. acinaciformis; Reticulitermes spp. e.g. R. hesperus, R. tibialis, R. speratus, R. flavipes, R. grassei, R. lucifugus, R. virginicus; Heterotermes spp. e.g. H. aureus, H. longiceps, H. tenuis; Cryptotermes spp. e.g. C. brevis, C. cavifrons; Incisitermes spp. e.g. I. minor, I. snyderi; Marginitermes hubbardi, Kalotermes flavicollis, Neotermes spp. e.g. N. cas-taneus, Zootermopsis spp. e.g. Z. angusticollis, Z. nevadensis, Mastotermes spp. e.g. M. dar-winiensis; Blatta spp. e.g. B. orientalis, B. lateralis; Blattella spp. e.g. B. asahinae, B. germanica; Rhyparobia maderae, Panchlora nivea, Periplaneta spp. e.g. P. americana, P. australasiae, P. brunnea, P. fuliginosa, P. japonica; Supella longipalpa, Parcoblatta pennsylvanica, Eurycotis floridana, Pycnoscelus surinamensis;
insects from the order Siphonoptera e.g. Cediopsylla simples, Ceratophyllus spp., Ctenoce-phalides spp. e.g. C. felis, C. canis, Xenopsylla cheopis, Pulex irritans, Trichodectes canis, Tunga penetrans, and Nosopsyllus fasciatus;
insects from the order Thysanura e.g. Lepisma saccharina, Ctenolepisma urbana, and Thermobia domestica;
pests from the class Chilopoda e.g. Geophilus spp., Scutigera spp. e.g. Scutigera coleoptrata;
pests from the class Diplopoda e.g. Blaniulus guttulatus, Julus spp., Narceus spp.;
pests from the class Symphyla e.g. Scutigerella immaculata;
insects from the order Dermaptera, e.g. Forficula Auricularia;
insects from the order Collembola, e.g. Onychiurus spp., e.g. Onychiurus armatus;
pests from the order Isopoda, e.g. Armadillidium vulgare, Oniscus asellus, Porcellio scaber;
insects from the order Phthiraptera, e.g. Damalinia spp., Pediculus spp. e.g. Pediculus hu-manus capitis, Pediculus humanus corporis, Pediculus humanus humanus; Pthirus pubis, Haematopinus spp. e.g. Haematopinus eurysternus, Haematopinus suis; Linognathus spp. e.g. Linognathus vituli; Bovicola bovis, Menopon gallinae, Menacanthus stramineus and Solenopotes capillatus, Trichodectes spp..

Further pest species which may be controlled by compounds I include: from the Phylum Mollusca, class Bivalvia, e.g., Dreissena spp.; class Gastropoda, e.g., Arion spp., Biomphalaria spp., Bulinus spp., Deroceras spp., Galba spp., Lymnaea spp., Oncomelania spp., Pomacea canaliclata, Succinea spp.; from the class of the helminths, e.g., Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp. e.g. Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuel-leborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

The compounds of the invention are particularly suitable for efficiently combating insects from the sub-order of Auchenorrhyncha, e.g. Amrasca biguttula, Empoasca spp., Nephotettix virescens, Sogatella furcifera, Mahanarva spp., Laodelphax striatellus, Nilapar-vata lugens, Diaphorina citri, Lycorma delicatula, Pentastiridus leporinus;
Lepidoptera, e.g. Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;
True bugs, e.g. Lygus spp., Stink bugs such as Euschistus spp., Halyomorpha halys, Nezara viridula, Piezodorus guildinii, Dichelops furcatus;
Thrips, e.g. Frankliniella spp., Thrips spp., Dichromothrips corbettii;
Aphids, e.g. Acyrthosiphon pisum, Aphis spp., Myzus persicae, Rhopalosiphum spp., Schi-zaphis graminum, Megoura viciae;
Whiteflies, e.g. Trialeurodes vaporariorum, Bemisia spp.;
Coleoptera, e.g. Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;
Flies, e.g. Delia spp., Ceratitis capitate, Bactrocera spp., Liriomyza spp.;
Coccoidea, e.g. Aonidiella aurantia, Ferrisia virgate;
Anthropods of class Arachnida (Mites), e.g. Penthaleus major, Tetranychus spp.;
Nematodes, e.g. Heterodera glycines, Meloidogyne sp., Pratylenchus spp., Caenorhabditis elegans.

### Animal health

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or con-trolling parasites in and on animals, which comprises contacting the parasites with a parasitically effective amount of a compound of the invention.

The invention also relates to the non-therapeutic use of compounds of the invention for con-trolling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. Ctenocephalides felis, C. canis, Xenopsylla cheopis, Pulex irri-tans, Tunga penetrans, and Nosopsyllus fasciatus; cockroaches (Blattaria - Blattodea), e.g. Blattella germanica, B. asahinae, Periplaneta americana, P. japonica, P. brunnea, P. fuligginosa, P. australasiae, and Blatta orientalis; flies, mosquitoes (Diptera), e.g. Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma line-ata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola, and T. similis; lice (Phthiraptera), e.g. Pediculus humanus capitis, P. humanus humanus, Pthirus pubis, Haematopinus eurysternus, H. suis, Linognathus vituli, Bovicola bovis, Menopon gallinae, Menacanthus stramineus, and Solenopotes capillatus; ticks and parasitic mites (Parasitiformes): ticks (Ixodida), e.g. Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata and parasitic mites (Mesostigmata), e.g. Ornithonyssus bacoti, Dermanyssus gallinae; Actinedida (Prostigmata) and Acaridida (Astigmata), e.g. Acarapis spp., Cheyletiella spp., Ornithocheyletia spp., Myobia spp., Psorergates spp., Demo-dex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., and Laminosioptes spp; Bugs (Het-eropterida): Cimex lectularius, C. hemipterus, Reduvius senilis, Triatoma spp., Rhodnius ssp., Panstrongylus ssp., and Arilus critatus; Anoplurida, e.g. Haematopinus spp., Linognathus spp., Pediculus spp., Phtirus spp., and Solenopotes spp.; Mallophagida (suborders Arnblycerina and Ischnocerina), e.g. Trimenopon spp., Menopon spp., Trinoton spp., Bovicola spp., Werneckiella spp., Lepikentron spp., Trichodectes spp., and Felicola spp.; Roundworms Nematoda: Wipeworms and Trichinosis (Trichosyringida), e.g. Trichinellidae (Trichinella spp.), (Trichuridae) Trichuris spp., Capillaria spp.; Rhabditida, e.g. Rhabditis spp., Strongyloides spp., Helicephalobus spp.; Strongylida, e.g. Strongylus spp., Ancylostoma spp., Necator americanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus, and Dioctophyma renale; Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi; Camallanida, e.g. Dracunculus medinensis (guinea worm); Spirurida, e.g. Thelazia spp., Wuchereria spp., Brugia spp., Onchocerca spp., Dirofilari spp.a, Dipetalonema spp., Setaria spp., Elaeophora spp., Spirocerca lupi, and Habronema spp.; Thorny headed worms (Acanthocephala), e.g. Acanthocephalus spp., Macracanthorhynchus hirudinaceus and Oncicola spp.; Planarians (Plathelminthes): Flukes (Trematoda), e.g. Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp., and Nanocyetes spp.; Cercomeromorpha, in particular Cestoda (Tapeworms), e.g. Diphyllo-bothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp., and Hymenolepis spp..

The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and salt-water fish e.g. trout, carp and eels. Particularly preferred are domestic animals, e.g. dogs or cats.

Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be ad-ministered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day.

Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suit-able solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dis-solving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active com-pound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### Examples

### Preparation examples

The compounds were characterized by melting point determination, by NMR spectroscopy or by the mass-to-charge ratio ([m/z]) and retention time (RT; [min.]), as determined by mass spectrometry (MS) coupled with HPLC analysis (HPLC-MS = high performance liquid chromatography-coupled mass spectrometry) or LC analysis (LC-MS = liquid chromatography-coupled mass spectrometry).

Method A: LC-MS: SHIMADZU LCMS-2020 ESI; Column: Diamonsil plus 5 µm 30*3.0mm; Mobile Phase: A: 0.05% TFA; B: ACN; Temperature: 40°C; Flow: 2.0 mL/min; MS: ESI positive; Mass range (m/z): 100-1000.

Method B: Agilent 1200 & G6130A; Column: Diamonsil Plus, C-18, 5 µm, 30*4.6 mm; Mobile Phase: A: 0.05% TFA; B: ACN; Temperature: 40°C; Flow: 2.0 mL/min; MS: ESI positive; Mass range (m/z): 100-1000.

Method C: LC: Shimadzu LC-30AD, ESI; Column: Kinetex EVO C18.5µm 2.1x30mm; Mobile phase: A: water + 0.04% TFA; B: ACN + 0.02% TFA; Temperature: 40°C; Gradient: 5% B to 100% B in 2.5 min; 100% B to 5% B in 0.02min; 5% B for 0.5min; Flow: 0.8mL/min; MS: ESI positive; Mass range: 100-2000.

Method D: LC: Shimadzu LC-30AD, ESI; Column: Xbridge C18, 5µm 4.6*30mm; mobile phase: B (ACN) A (0.05%TFA aq.); gradient (B%), Gradient: 20% B to 95% B in 7 min. MS: ESI positive; Mass range: 100-1000.

### Intermediates:

### Intermediate Int-1: Synthesis of tert-butyl N-[1-[2-([1,2,4]triazolo[4,3-b]pyridazin-6-yl)-1,2,4-triazol-3-yl]ethyl]carbamate (I-1)

Step 1: To a mixture of 6-bromo-[1,2,4]triazolo[4,3-b]pyridazine (240 mg, 1.21 mmol) in EtOH (2 mL) was added dropwise hydrazinium hydroxide (74 mg, 1.45 mmol, 98%). The reaction was stirred at rt for 2 hours. The mixture was concentrated to afford the compound [1,2,4]triazolo[4,3-b]pyridazin-6-ylhydrazine (200 mg, 88% yield) as a white solid. LC-MS (ES) m/z = 151.3 (M+H)⁺.

Step 2: To a mixture of [1,2,4]triazolo[4,3-b]pyridazin-6-ylhydrazine (100 mg, 0.67 mmol) in 1,4-dioxane (1 mL) and AcOH (2 mL) was added tert-butyl N-[2-[(E)-dimethylaminomethyleneamino]-1-methyl-2-oxo-ethyl]carbamate (324 mg, 1.33 mmol) in DCM (1 mL). The reaction was stirred at 25 °C for 16 hours. The LCMS showed intermediate state. The mixture was stirred at 80 °C for 3 hours. The mixture was diluted with H2O (20 mL) and extracted with EA (10 mL * 2). The combined organic phase was dried over Na₂SO₄ and concentrated. The residue was purified by chromatography on C₁₈ (ACN/H2O = 5%-60%) to afford the compound tert-butyl N-[1-[2-([1,2,4]triazolo[4,3-b]pyridazin-6-yl)-1,2,4-triazol-3-yl]ethyl]carbamate (Int-1) (150 mg, 61% yield) as a yellow oil. LC-MS (ES) m/z = 331.4 (M+H)⁺. ¹H NMR (400 MHz, DMSO-*d*₆) δ [ppm] 9.70 (s, 1H), 8.64 (d, *J* = 9.6 Hz, 1H), 8.29 (s, 1H), 7.81 (d, *J* = 10.0 Hz, 1H), 7.55 (d, *J =* 7.6 Hz, 1H), 5.48-5.44 (m, 1H), 1.52 (d, *J* = 6.8 Hz, 3H), 1.22 (s, 9H).

### Final compounds:

### Example 1: Synthesis of N-[1-[2-([1,2,4]triazolo[4,3-b]pyridazin-6-yl)-1,2,4-triazol-3-yl]ethyl]-3,5-bis(trifluoromethyl)benzamide (I-1)

Step 1: A solution of tert-butyl N-[1-[2-([1,2,4]triazolo[4,3-b]pyridazin-6-yl)-1,2,4-triazol-3-yl]ethyl]carbamate (Int-1) (150 mg, 0.45 mmol) in HCl/dioxane (2 mL, 5mol/L) was stirred at room temperature for 2 hours. The mixture was concentrated to afford the compound 1-[2-([1,2,4]triazolo[4,3-b]pyridazin-6-yl)-1,2,4-triazol-3-yl]ethanamine (100 mg, 100% yield) as a white solid. LC-MS (ES) m/z =231.3 (M+H)⁺.

Step 2: To a solution of 1-[2-([1,2,4]triazolo[4,3-b]pyridazin-6-yl)-1,2,4-triazol-3-yl]ethanamine (100 mg, 0.43 mmol), DIEA (168 mg, 1.30 mmol) in DCM (2 mL) stirred under nitrogen at 0 °C was added 3,5-bis(trifluoromethyl)benzoyl chloride (20 mg, 0.56 mmol). The reaction mixture was stirred at rt for 2 hours.

The mixture was concentrated. The residue was purified by Prep-HPLC (5-95 % ACN in water with 0.1 % NH₄HCO₃) to afford the compound N-[1-[2-([1,2,4]triazolo[4,3-b]pyridazin-6-yl)-1,2,4-triazol-3-yl]ethyl]-3,5-bis(trifluoromethyl)benzamide (I-1) (20 mg, 10% yield) as a white solid.

LC-MS (ES) m/z =471.0 (M+H)⁺. ¹H NMR (400 MHz, DMSO-d₆) δ [ppm] 9.63 (s, 1H), 9.59 (d, J = 6.8 Hz, 1H), 8.60 (dd, J = 0.8, 9.6 Hz, 1H), 8.41 (s, 2H), 8.36 (s, 1H), 8.29 (s, 1H), 7.83 (d, J = 10 Hz, 1H), 5.98 - 5.94 (m, 1H), 1.72 (d, J = 7.2 Hz, 3H).

### Example 2: Synthesis of N-[1-(2-imidazo[1,2-b]pyridazin-6-yl-1,2,4-triazol-3-yl)ethyl]-3,5-bis(trifluoromethyl)benzamide (I-2)

Step 1 : To a solution of 6-bromoimidazo[1,2-b]pyridazine (1100 mg, 5.6 mmol) in hydrazine hydrate (10 mL). The reaction mixture was stirred at 100°C for 1 hour. The mixture was cooled to room temperature and filtered. Then the filter cake was concentrated to give the desired compound imidazo[1,2-b]pyridazin-6-ylhydrazine (660 mg, 84% yield) as a white solid. 1H NMR (400 MHz, DMSO-d₆) δ [ppm] 7.94 (s, 1H), 7.81 (s, 1H), 7.69 (dd, J = 0.8, 9.6 Hz, 1H), 7.41-7.40 (m, 1H), 6.66 (d, J = 9.6 Hz, 1H), 4.18 (s, 2H).

Step 2 : To a solution of imidazo[1,2-b]pyridazin-6-ylhydrazine (100 mg, 0.6 mmol) in AcOH (2 mL) and 1,4-dioxane (1 mL) was adde tert-butyl N-[2-[(E)-dimethylaminomethyleneamino]-1-methyl-2-oxoethyl]carbamate (310 mg, 1.27 mmol) in DCM (1 mL). The reaction mixture was stirred at 25 °C for 18 hours. Then the reaction was heated to 80 °C and stirred for 2 hours. The mixture was concentrated. The residue was purified by column chromatography on silical gel (EA) to give the desired compound tert-butyl N-[1-(2-imidazo[1,2-b]pyridazin-6-yl-1,2,4-triazol-3-yl)ethyl]carbamate (150 mg, 60% yield) as a white solid. LC-MS (ES) m/z = 330.3 (M+H)⁺.

Step 3 : To a solution of tert-butyl N-[1-(2-imidazo[1,2-b]pyridazin-6-yl-1,2,4-triazol-3-yl)ethyl]carbamate (150 mg, 0.4 mmol) in 1,4-dioxane (2 mL) was added 1,4-dioxane/HCl (2 mL). The reaction mixture was stirred at room temperature for 2 hours. The mixture was concentrated to afford the desired compound 1-(1-(Imidazo[1,2-b]pyridazin-6-yl)-1H-1,2,4-triazol-5-yl)ethan-1-amine (110 mg, 98% yield) as a yellow solid. LC-MS (ES) m/z = 230.2 (M+H)⁺.

Step 4 : To a solution of 1-(1-(Imidazo[1,2-b]pyridazin-6-yl)-1H-1,2,4-triazol-5-yl)ethan-1-amine (110 mg, 0.5 mmol), TEA (226 mg, 2.2 mmol) in DCM (5 mL) was added 3,5-bis(trifluoromethyl)benzoyl chloride (136 mg, 0.5 mmol). The reaction mixture was stirred at 25°C for 2 hours. The mixture was concentrated. The residue was purified by prep-HPLC (5-95% ACN in water with 0.1 % NH4HCO3) to give the desired N-(1-(1-(imidazo[1,2-b]pyridazin-6-yl)-1H-1,2,4-triazol-5-yl)ethyl)-3,5-bis(trifluoromethyl)benzamide (81 mg, 38% yield) as a white solid. LC-MS (ES) m/z = 470.0 (M+H)⁺. 1H NMR (400 MHz, DMSO-d₆) δ [ppm] 9.55 (d, J = 7.2 Hz, 1H), 8.34-8.31 (m, 4H), 8.26 (d, J = 6.8 Hz, 2H), 7.86 (d, J = 1.2 Hz, 1H), 7.65 (d, J = 9.6 Hz, 1H), 5.95-5.88 (m, 1H), 1.72 (d, J = 6.8 Hz, 3H).

### Example 3 : Synthesis of N-(1-(1-(imidazo[2,1-f][1,2,4]triazin-2-yl)-1H-1,2,4-triazol-5-yl)ethyl)-3,5-bis(trifluoromethyl)benzamide (I-6)

Step 1 : To a solution of 6-bromo-1,2,4-triazin-3-amine (5.00 g, 28.6 mmol), CuCl₂ (5.10 g, 37.2 mmol) in ACN (50 mL) was added tert-butyl nitrite (4.72 g, 45.8 mmol). The reaction mixture was stirred at 70°C for 2 hours. The mixture was concentrated. The residue was purified by chromatography on silica gel (PE/EA=10/1) to afford 6-bromo-3-chloro-1,2,4-triazine (2.00 g, 33% yield) as a yellow solid. 1H NMR (400 MHz, CDCl₃) δ [ppm] 8.48 (s, 1H).

Step 2 : A mixture of 6-bromo-3-chloro-1,2,4-triazine (1.70 g, 8.70 mmol), 2,2-diethoxyethan-1-amine (1.39 g, 10.44 mmol) and DIPEA (1.35 g, 10.44 mmol) in DMF (20 mL) was stirred at 80 °C for 3 hrs. The mixture was diluted with water (20 mL) and extracted with EtOAc (30 mL * 3). The organic layer was washed with brine (30 mL * 3) and the organic layer was concentrated. The residue was purified by column chromatography (PE/EtOAc = 3/1) to afford 3-chloro-N-(2,2-diethoxyethyl)-1,2,4-triazin-6-amine (2.10 g, 98% yield) as a yellow solid. 1H NMR (400 MHz, DMSO-d₆) δ [ppm] 8.43 (s, 1H), 8.00 (br s, 1H), 4.65 (s, 1H), 3.67-3.59 (m, 2H), 3.52-3.44 (m, 2H), 3.40 (s, 2H), 1.13-1.08 (m, 6H).

Step 3 : A mixture of 3-chloro-N-(2,2-diethoxyethyl)-1,2,4-triazin-6-amine (2.00 g, 8.10 mmol) in TFA (10 mL) was stirred at 80 °C for 16 hrs. The mixture was concentrated and the residue was purified by column chromatography (PE/EtOAc = 1/1) to afford 2-chloroimidazo[2,1-f][1,2,4]triazine (810.00 mg, 64% yield) as a yellow solid. LC-MS (ES) m/z (M+H)⁺ = 154.9.

Step 4 : A mixture of 2-chloroimidazo[2,1-f][1,2,4]triazine (800 mg, 5.18 mmol) and N₂H₄*H₂O (1.04 g, 20.70 mmol) in EtOH (10 mL) was stirred at 90°C for 2 hrs. The mixture was cooled to 25 °C and filtered. The filter cake was dried to afford 2-hydrazineylimidazo[2,1-f][1,2,4]triazine (650.0 mg, 84% yield) as a yellow solid. LC-MS (ES) m/z (M+H)⁺ = 151.0.

Step 5 : A mixture of 2-hydrazineylimidazo[2,1-f][1,2,4]triazine (650 mg, 4.33 mmol) and tert-butyl N-[2-[(E)-dimethylaminomethyleneamino]-1-methyl-2-oxo-ethyl]carbamate (2.11 g, 8.66 mmol) in HOAc (7 mL) and dioxane (7 mL) was stirred at 25 °C for 0.5 hr. The mixture was concentrated and the residue was dissolved in HOAc (10 mL). The mixture was stirred at 50°C for 2 hrs. The mixture was concentrated and the residue was diluted with EtOAc (30 mL). The mixture was washed with saturate Na₂CO₃ aqueous to pH=8-9. The organic layer was concentrated and the residue was purified by column chromatography (PE/EtOAc = 1/1) to afford tert-butyl (1-(1-(imidazo[2,1-f][1,2,4]triazin-2-yl)-1H-1,2,4-triazol-5-yl)ethyl)carbamate (1.30 g, 91% yield) as a yellow solid. LC-MS (ES) m/z (M+H)⁺ = 330.9.

Step 6 : A mixture of tert-butyl (1-(1-(imidazo[2,1-f][1,24]triazin-2-yl)-1H-1,2,4-triazol-5-yl)ethyl)carbamate (2.80 g, 2.50 mmol) in HCl/EtOAc (30 mL) was stirred at 30 °C for 1 hr. The reaction mixture was purified by prep-HPLC (5-95% ACN in water) to afford the 1-(1-(imidazo[2,1-f][1,2,4]triazin-2-yl)-1H-1,2,4-triazol-5-yl)ethan-1-amine hydrogen chloride (0.60 g, 88% yield) as a yellow solid. LC-MS (ES) m/z (M+H)⁺ = 230.9.

Step 7 : A mixture of 1-(1-(imidazo[2,1-f][1,2,4]triazin-2-yl)-1H-1,2,4-triazol-5-yl)ethan-1-amine hydrogen chloride (190.0 mg, 0.71 mmol) and TEA (216.0 mg, 2.14 mmol) in DMF (5 mL) was added 3,5-bis(trifluoromethyl)benzoyl chloride (197.0 mg, 0.71 mmol) at 0 °C. The mixture was stirred at 0 °C for 2 hrs. The mixture was diluted with water (10 mL) and extracted with EtOAc (20 mL * 3). The organic layer was washed with brine (20 mL * 3) and concentrated. The residue was purified by column chromatography (PE/EtOAc = 1/2) to afford N-(1-(1-(imidazo[2,1-f][1,2,4]triazin-2-yl)-1H-1,2,4-triazol-5-yl)ethyl)-3,5-bis(trifluoromethyl)benzamide (I-6) (42.90 mg, 13% yield) as a yellow solid. LC-MS (ES) m/z (M+H)⁺ = 471.0. 1H NMR (400 MHz, DMSO-d₆) δ [ppm] 9.57 (d, J = 7.2 Hz, 1H), 9.01 (s, 1H), 8.39 (d, J = 4.8 Hz, 3H), 8.30 (d, J = 5.2 Hz, 2H), 8.09 (s, 1H), 5.91-5.85 (m, 1H), 1.70 (d, J = 6.8 Hz, 3H).

### Example 4 : Synthesis of N-(1-(1-(4-(methylthio)imidazo[2,1-f][1,2,4]triazin-2-yl)-1H-1,2,4-triazol-5-yl)ethyl)-3,5-bis(trifluoromethyl)benzamide (I-5)

Step 1 : To a mixture of 2,4-dichloroimidazo[2,1-f][1,2,4]triazine (2.00 g, 5.30 mmol) in THF (20 mL) was added NaSMe (0.82 g, 11.66 mmol) at 0 °C. The mixture was stirred at 10°C for 16 hrs. The mixture was diluted with H₂O (20 mL), extracted with EtOAc (20 mL * 3). The organic layer was concentrated. The residue was purified by column chromatography on silica gel (PE/EtOAc = 5/1) to afford 2-chloro-4-(methylthio)imidazo[2,1-f][1,2,4]triazine (1.64 g, 77% yield) as a white solid. LC-MS (ES) m/z = 200.9 (M+H)⁺.

Step 2: A mixture of 2-chloro-4-(methylthio)imidazo[2,1-f][1,2,4]triazine (1.00 g, 5.00 mmol), (diphenylmethylidene)hydrazine (1.47 g, 7.50 mmol), Cs2CO3 (3.26 g, 10.00 mmol) and BrettPhos Pd G3 (0.45 g, 0.50 mmol) in dioxane (20 mL) was stirred at 100 °C for 6 hrs. The reaction mixture was diluted with water and extracted with EtOAc (30 mL * 3). The combined organic phase was dried over anhydrous Na₂SO₄, concentrated and the residue was purified by flash chromatography on silica gel (PE/EtOAc = 4/1) to afford 2-(2-(diphenylmethylene)hydrazineyl)-4-(methylthio)imidazo[2,1-f][1,2,4]triazine (1 g, 56% yield) as a yellow solid. LC-MS (ES) m/z = 360.9 (M+H)⁺.

Step 3 : A solution of 2-(2-(diphenylmethylene)hydrazineyl)-4-(methylthio)imidazo[2,1-f][1,2,4]triazine (1.98 g, 5.50 mmol) in 4N HCl (20 mL) and THF (20 mL) was stirred at 50 °C for 1 hr. The reaction was concentrated and basified with 1M NaOH to pH > 7. The aqueous layer was purified by reverse flash (C-18) (5-95% ACN in water) to afford 2-hydrazineyl-4-(methylthio)imidazo[2,1-f][1,2,4]triazine (0.66 g, 62% yield) as a yellow solid. 1H NMR (400 MHz, DMSO-d₆) δ [ppm] 8.16 (s, 1H), 7.96 (s, 1H), 7.49 (s, 1H), 4.15 (s, 2H), 2.58 (s, 3H).

Step 4 : A solution of 2-hydrazineyl-4-(methylthio)imidazo[2,1-f][1,2,4]triazine (550 mg, 2.80 mmol), 4860-3b (1023 mg, 4.20 mmol) in AcOH (15 mL) and dioxane (5 mL) was stirred at 25 °C for 1 hr. Then the reaction was stirred at 50 °C for 18 hrs. The mixture was concentrated and purified by reverse flash (C-18) (5-95% ACN in water) to afford tert-butyl (1-(1-(4-(methylthio)imidazo[2,1-f][1,2,4]triazin-2-yl)-1H-1,2,4-triazol-5-yl)ethyl)carbamate (550 mg, 52% yield) as a yellow solid. LC-MS (ES) m/z = 376.9 (M+H)⁺.

Step 5 : A solution of tert-butyl (1-(1-(4-(methylthio)imidazo[2,1-f][1,2,4]triazin-2-yl)-1H-1,2,4-triazol-5-yl)ethyl)carbamate (500 mg, 1.38 mmol) in HCl/EA(10 mL) was stirred at 30 °C for 1 hr. The reaction mixture was concentrated to afford 1-(1-(4-(methylthio)imidazo[2,1-f][1,2,4]triazin-2-yl)-1H-1,2,4-triazol-5-yl)ethan-1-amine (400 mg, 96% yield) as a yellow solid. LC-MS (ES) m/z = 277.0 (M+H)⁺.

Step 6 : 3,5-Bis(trifluoromethyl)benzoyl chloride (389 mg, 1.41 mmol) was added into a solution of 1-(1-(4-(methylthio)imidazo[2,1-f][1,2,4]triazin-2-yl)-1H-1,2,4-triazol-5-yl)ethan-1-amine (400 mg, 1.28 mmol) and Et3N (388 mg, 3.84 mmol) in DCM (5 mL). The reaction was stirred at 15 °C for 1 hr. The reaction mixture was diluted with water and extracted with EtOAc (10 mL * 3). The combined organic phase was dried over anhydrous Na₂SO₄, concentrated and the residue was purified by flash chromatography on silica gel (PE/EtOAc = 3/1) to afford N-(1-(1-(4-(methylthio)imidazo[2,1-f][1,2,4]triazin-2-yl)-1H-1,2,4-triazol-5-yl)ethyl)-3,5-bis(trifluoromethyl)benzamide (I-5) (300 mg, 45% yield) as a white solid. LC-MS (ES) m/z = 517.0 (M+H)⁺. 1H NMR (400 MHz, DMSO-d₆) δ [ppm] 9.55 (d, J = 7.2 Hz, 1H), 8.44 (s, 1H), 8.30 (s, 2H), 8.26 (d, J = 8.0 Hz, 2H), 7.89 (s, 1H), 5.98 (d, J = 7.2 Hz, 1H), 2.70 (s, 3H), 1.71 (d, J = 6.8 Hz, 3H).

### Example 5 : Synthesis of N-(1-(1-(8-Methyl-[1,2,4]triazolo[4,3-b]pyridazin-6-yl)-1H-1,2,4-triazol-5-yl)ethyl)-3,5-bis(trifluoromethyl)benzamide (I-8)

Step 1 : A solution of 3-chloro-5-methyl-1H-pyridazin-6-one hydrazone (7.33 g, 46.2 mmol) in formic acid (50 mL) was stirred at 110 °C for 1 hr. The reaction mixture was cooled to rt, then poured into water (100 mL) and extracted with DCM (200 mL * 2). The organic layer was washed with brine (100 mL), dried over anhydrous MgSO₄, filtered and concentrated. The residue was purified by column chromatography on silica gel (PE/EtOAc = 3/7) to afford 6-chloro-8-methyl-[1,2,4]triazolo[4,3-b]pyridazine (3.2 g, 41% yield) as a yellow solid. 1H NMR (400 MHz, CDCl₃) δ [ppm] 9.01 (s, 1H), 6.95 (d, J = 1.2 Hz, 1H), 2.75 (d, J = 1.2 Hz, 3H).

Step 2 : A mixture of 6-chloro-8-methyl-[1,2,4]triazolo[4,3-b]pyridazine (1.0 g, 5.9 mmol) and NH₂NH₂* H₂O (900 mg, 17.8 mmol) in EtOH (10 mL) was stirred at 80 °C for 2 hrs. The mixture was cooled to rt. The mixture was filtered. The filter cake was washed with EtOH (2 mL) and H₂O (2 mL), dried to afford 6-hydrazineyl-8-methyl-[1,2,4]triazolo[4,3-b]pyridazine (600 mg, 62% yield) as a white solid. LC-MS (ES) m/z = 165.0 (M+H)⁺.

Step 3 : A mixture of 6-hydrazineyl-8-methyl-[1,2,4]triazolo[4,3-b]pyridazine (600 mg, 3.7 mmol) and tert-butyl N-[2-[(E)-dimethylaminomethyleneamino]-1-methyl-2-oxo-ethyl]carbamate (934 mg, 3.8 mmol) in AcOH (10 mL) was stirred at 20 °C for 2 hrs. Then the mixture was heated to 50 °C for 6 hrs. The mixture was concentrated. The residue was diluted with EtOAc (30 mL), washed with sat. NaHCO₃ (30 mL * 2). The organic layer was concentrated. The residue was purified by column chromatography on silica gel (EtOAc) to afford tert-butyl (1-(1-(8-methyl-[1,2,4]triazolo[4,3-b]pyridazin-6-yl)-1H-1,2,4-triazol-5-yl)ethyl)carbamate (500 mg, 40% yield) as a yellow solid. LC-MS (ES) m/z = 345.0 (M+H)⁺.

Step 4 : A mixture of tert-butyl (1-(1-(8-methyl-[1,2,4]triazolo[4,3-b]pyridazin-6-yl)-1H-1,2,4-triazol-5-yl)ethyl)carbamate (380 mg, 1.1 mmol) in HCl/EtOAc (4 M, 8 mL) was stirred at 20 °C for 4 hrs. The mixture was filtered. The filter cake was dried to afford 1-(1-(8-methyl-[1,2,4]triazolo[4,3-b]pyridazin-6-yl)-1H-1,2,4-triazol-5-yl)ethan-1-amine hydrochloride (250 mg, 81% yield) as a white solid. 1H NMR (400 MHz, DMSO-d₆) δ [ppm] 9.78 (s, 1H), 8.82 (br s, 3H), 8.52 (s, 1H), 7.82 (d, J = 1.2 Hz, 1H), 5.29-5.22 (m, 1H), 2.76 (d, J = 1.2 Hz, 3H), 1.69 (d, J = 6.8 Hz, 3H).

Step 5 : To a mixture of 1-(1-(8-methyl-[1,2,4]triazolo[4,3-b]pyridazin-6-yl)-1H-1,2,4-triazol-5-yl)ethan-1-amine hydrochloride (100 mg, 0.36 mmol) and TEA (108 mg, 1.07 mmol) in THF (2 mL) was added 3,5-bis(trifluoromethyl)benzoyl chloride (118 mg, 0.43 mmol) at 0 °C. The mixture was stirred at 20 °C for 2 hrs. The mixture was diluted with H₂O (5 mL), extracted with EtOAc (10 mL * 2). The organic layer was dried over Na₂SO₄, filtered and concentrated. The residue was purified by reverse flash (C-18) (5-95% ACN in water) to afford N-(1-(1-(8-methyl-[1,2,4]triazolo[4,3-b]pyridazin-6-yl)-1H-1,2,4-triazol-5-yl)ethyl)-3,5-bis(trifluoromethyl)benzamide (I-8) (105 mg, 61% yield) as a white solid. LC-MS (ES) m/z = 485.0 (M+H)⁺. 1H NMR (400 MHz, CDCl₃) δ [ppm] 9.18 (s, 1H), 8.25 (s, 2H), 8.07 (s, 1H), 8.03 (s, 1H), 7.76 (d, J = 1.2 Hz, 1H), 7.31 (d, J = 7.6 Hz, 1H), 6.27-6.20 (m, 1H), 2.88 (d, J = 1.2 Hz, 3H), 1.82 (d, J = 7.2 Hz, 3H).

### Example 6 : Synthesis of N-(1-(1-([1,2,4]triazolo[4,3-a]pyrazin-6-yl)-1H-1,2,4-triazol-5-yl)ethyl)-3,5-bis(trifluoromethyl)benzamide (I-11)

Step 1 : To a solution of 2,5-dibromopyrazine (12.0 g, 50.4 mmol) in IPA (100 mL) was added hydrazine monohydrate (12.75 g, 252.0 mmol). The reaction was stirred at 65 °C for 16 hrs. The mixture was cooled to 0 °C and filtered. The filter cake was purified by triturated with ice-water (20 mL) to afford the compound (Z)-5-bromo-2-hydrazineylidene-1,2-dihydropyrazine (7.37 g, 77% yield) as a yellow solid. 1H NMR (400 MHz, DMSO-d₆) δ [ppm] 8.19 (s, 1H), 8.08 (d, J = 1.2 Hz, 1H), 7.94 (d, J = 1.2 Hz, 1H), 4.39 (s, 2H).

Step 2 : A mixture of (Z)-5-bromo-2-hydrazineylidene-1,2-dihydropyrazine (1.0 g, 5.29 mmol) and tert-butyl N-[2-[(E)-dimethylaminomethyleneamino]-1-methyl-2-oxo-ethyl]carbamate (1.35 g, 5.56 mmol) in AcOH (10 mL) was stirred at 20 °C for 2 hrs. Then the mixture was heated to 50 °C for 6 hrs. The mixture was concentrated. The residue was diluted with EtOAc (50 mL), washed with sat. NaHCO3 (30 mL * 2). The organic layer was concentrated. The residue was purified by column chromatography on silica gel (PE/EtOAc = 2/1) to afford tert-butyl (1-(1-(5-bromopyrazin-2-yl)-1H-1,2,4-triazol-5-yl)ethyl)carbamate (1.1 g, 56% yield) as a yellow solid. LC-MS (ES) m/z = 369.2 (M+H)⁺.

Step 3 : A mixture of tert-butyl (1-(1-(5-bromopyrazin-2-yl)-1H-1,2,4-triazol-5-yl)ethyl)carbamate (834 mg, 2.26 mmol) and hydrazine hydrate (571 mg, 11.29 mmol) in EtOH (15 mL) was stirred at 80 °C for 2 hrs. The mixture was concentrated. The residue was purified by reverse flash (C-18) (5-95% ACN in water with 0.1% NH₄HCO₃) to afford tert-butyl (1-(1-(5-hydrazineylpyrazin-2-yl)-1H-1,2,4-triazol-5-yl)ethyl)carbamate (660 mg, 91% yield) as a yellow solid. LC-MS (ES) m/z = 321.3 (M+H)⁺.

Step 4 : A mixture of tert-butyl (1-(1-(5-hydrazineylpyrazin-2-yl)-1H-1,2,4-triazol-5-yl)ethyl)carbamate (660 mg, 2.06 mmol), Trimethoxymethane (437 mg, 4.12 mmol) and TsOH * H₂O (78 mg, 0.41 mmol) in toluene (10 mL) and 1,4-dioxane (3 mL) was stirred at 120 °C for 2 hrs. The mixture was cooled to rt and diluted with sat. NaHCO₃ (10 mL), extracted with EtOAc (20 mL * 2). The organic layer was concentrated. The residue was purified by column chromatography on silica gel (DCM/MeOH = 20/1) to afford tert-butyl (1-(1-([1,2,4]triazolo[4,3-a]pyrazin-6-yl)-1H-1,2,4-triazol-5-yl)ethyl)carbamate (554 mg, 81% yield) as a yellow solid. 1H NMR (400 MHz, DMSO-d₆) δ [ppm] 9.57 (s, 1H), 9.49 (s, 1H), 9.12 (s, 1H), 8.16 (s, 1H), 7.43 (d, J = 7.6 Hz, 1H), 5.32-5.25 (m, 1H), 1.43 (d, J = 7.2 Hz, 3H), 1.19 (s, 9H).

Step 5 : A mixture of tert-butyl (1-(1-([1,2,4]triazolo[4,3-a]pyrazin-6-yl)-1H-1,2,4-triazol-5-yl)ethyl)carbamate (600 mg, 1.82 mmol) in EtOAc (10 mL) and HCl/EtOAc (2 M, 10 mL) was stirred at 20 °C for 2 hrs. The mixture was filtered. The filter cake was dried to afford 1-(1-([1,2,4]triazolo[4,3-a]pyrazin-6-yl)-1H-1,2,4-triazol-5-yl)ethan-1-amine (470 mg, 97% yield) as a yellow solid. LC-MS (ES) m/z = 231.2 (M+H)⁺.

Step 6 : To a solution of 1-(1-([1 ,2,4]triazolo[4,3-a]pyrazin-6-yl)-1H-1 ,2,4-triazol-5-yl)ethan-1-amine (210 mg, 0.79 mmol) and TEA (239 mg, 2.36 mmol) in THF (4 mL) was added 3,5-bis(trifluoromethyl)benzoyl chloride (218 mg, 0.79 mmol) at 0 °C. The mixture was stirred at 0 °C for 2 hrs. The mixture was diluted with H₂O (5 mL), extracted with EtOAc (10 mL * 2). The organic layer was dried over Na₂SO₄, filtered and concentrated. The residue was purified by column chromatography on silica gel (DCM/MeOH = 20/1) to afford the crude compound. The crude product was purified by triturated with MeOH (3 mL) to yield N-(1-(1-([1,2,4]triazolo[4,3-a]pyrazin-6-yl)-1H-1,2,4-triazol-5-yl)ethyl)-3,5-bis(trifluoromethyl)-benzamide (I-11) (189 mg, 51% yield) as a white solid. LC-MS (ES) m/z = 471.0 (M+H)⁺. 1H NMR (400 MHz, DMSO-d₆) δ [ppm] 9.57-9.55 (m, 2H), 9.45 (s, 1H), 9.19 (s, 1H), 8.38 (s, 2H), 8.29 (s, 1H), 8.24 (s, 1H), 5.89-5.82 (m, 1H), 1.67 (d, J = 6.8 Hz, 3H).

### Example 7 : Synthesis of N-(1-(1-([1,2,4]triazolo[4,3-b]pyridazin-6-yl)-1H-1,2,4-triazol-5-yl)ethyl)-N-methyl-3,5-bis(trifluoromethyl)benzamide (I-12)

Step 1 : To a solution of 2-[tert-butoxycarbonyl(methyl)amino]propanoic acid (5.05 g, 24.8 mmol) in THF (20 mL) was added N-methylmorpholine (2.51 g, 24.8 mmol) and isobutyl carbonochloridate (3.39 g, 24.8 mmol) at -10 °C. The mixture was stirred at -10 °C for 0.5 hr. Then NH₄HCO₃ (9.80 g, 124.0 mmol) was added to the mixture. The mixture was stirred at 20 °C for 3 hrs. The mixture was concentrated. The residue was diluted with H₂O (30 mL), acidified with 10% KHSO4 to adjust pH = 1-2, extracted with EtOAc (100 mL * 3). The organic layer was washed with saturated NaHCO₃ (50 mL) and brine (30 mL), dried over MgSO₄, filtered and concentrated to afford tert-butyl (1-amino-1-oxopropan-2-yl)(methyl)carbamate (4.33 g, 86% yield) as a white solid. 1H NMR (400 MHz, CDCl₃) δ [ppm] 6.11-5.83 (m, 2H), 4.74-4.62 (m, 1H), 2.78 (s, 3H), 1.46 (s, 9H), 1.32 (d, J = 6.8 Hz, 3H).

Step 2 : To a solution of tert-butyl (1-amino-1-oxopropan-2-yl)(methyl)carbamate (4.33 g, 21.4 mmol) in 2-methyltetrahydrofuran (50 mL) was added DMF-DMA (3.06 g, 25.7 mmol). The mixture was stirred at 40 °C for 1 hr. The mixture was concentrated to afford tert-butyl (E)-(1-(((dimethylamino)methylene)amino)-1-oxopropan-2-yl)(methyl)carbamate (5.15 g, 93% yield) as a yellow solid. LC-MS (ES) m/z = 202.1 (M+H-t-Bu)⁺.

Step 3 : A mixture of [1 ,2,4]triazolo[4,3-b]pyridazin-6-ylhydrazine (800 mg, 5.33 mmol) and tert-butyl (E)-(1-(((dimethylamino)methylene)amino)-1-oxopropan-2-yl)(methyl)carbamate (2.06 g, 7.99 mmol) in AcOH (10 ml) was stirred at 20 °C for 2 hrs. The mixture was heater to 70 °C and stirred for 10 hrs. The mixture was concentrated. The residue was diluted with EtOAc (30 mL), washed with sat. NaHCO₃ (10 mL * 2). The organic layer was concentrated. The residue was purified by column chromatography on silica gel (DCM/MeOH = 20/1) to afford tert-butyl (1-(1-([1,2,4]triazolo[4,3-b]pyridazin-6-yl)-1H-1,2,4-triazol-5-yl)ethyl)(methyl)carbamate (1.42 g, 77% yield) as a yellow solid. LC-MS (ES) m/z = 345.3 (M+H)⁺.

Step 4 : A mixture of tert-butyl (1-(1-([1,2,4]triazolo[4,3-b]pyridazin-6-yl)-1H-1,2,4-triazol-5-yl)ethyl)(methyl)carbamate (800 mg, 2.32 mmol) in HCl/EtOAc (10 mL) was stirred at 20 °C for 2 hrs. The mixture was filtered. The filter cake was dried to afford 1-(1-([1,2,4]triazolo[4,3-b]pyridazin-6-yl)-1H-1,2,4-triazol-5-yl)-N-methylethan-1-amine (650 mg, 100% yield) as a white solid. LC-MS (ES) m/z = 245.2 (M+H)⁺.

Step 5 : To a solution of 1-(1-([1,2,4]triazolo[4,3-b]pyridazin-6-yl)-1H-1,2,4-triazol-5-yl)-N-methylethan-1-amine (250 mg, 0.89 mmol) and TEA (270 mg, 2.67 mmol) in THF (5 mL) was added 3,5-bis(trifluoromethyl)benzoyl chloride (246 mg, 0.89 mmol) at 0 °C. The mixture was stirred at 0 °C for 2 hrs. The mixture was diluted with H₂O (10 mL), extracted with EtOAc (20 mL * 2). The organic layer was dried over Na₂SO₄, filtered and concentrated. The residue was purified by column chromatography on silica gel (DCM/MeOH = 20/1) to afford the crude compound. The crude compound was purified by triturated with MeOH (4 mL) to afford N-(1-(1-([1,2,4]triazolo[4,3-b]pyridazin-6-yl)-1H-1,2,4-triazol-5-yl)ethyl)-N-methyl-3,5-bis(trifluoromethyl)benzamide (I-12) (225 mg, 52% yield) as a white solid. LC-MS (ES) m/z = 485.0 (M+H)⁺. 1H NMR (400 MHz, DMSO-d₆) δ [ppm] 9.33-9.20 (m, 1H), 8.68-8.59 (m, 1H), 8.44 (s, 1H), 8.20 (s, 1H), 7.94-7.65 (m, 3H), 6.48-5.80 (m, 1H), 3.32-2.78 (m, 3H), 1.73 (d, J = 7.2 Hz, 3H).

### Amide coupling using ethyl amine building blocks :

### Example 8 : Synthesis of 3-chloro-N-[1-[2-([1,2,4]triazolo[4,3-b]pyridazin-6-yl)-1,2,4-triazol-3-yl]ethyl]-5-(trifluoromethylsulfonyl)benzamide (I-13)

Step 1: A solution of tert-butyl N-[1-[2-([1,2,4]triazolo[4,3-b]pyridazin-6-yl)-1,2,4-triazol-3-yl]ethyl]carbamate (Int-1) (150 mg, 0.45 mmol) in HCl/dioxane (2 mL, 5mol/L) was stirred at room temperature for 2 hours. The mixture was concentrated to afford the compound 1-[2-([1,2,4]triazolo[4,3-b]pyridazin-6-yl)-1,2,4-triazol-3-yl]ethanamine (100 mg, 100% yield) as a white solid. LC-MS (ES) m/z =231.3 (M+H)⁺.

Step 2 : Amide coupling : A mixture of 3-chloro-5-(trifluoromethylsulfonyl)benzoic acid (86.8 mg, 0.30 mmol, 1 eq.), 1-[2-([1,2,4]triazolo[4,3-b]pyridazin-6-yl)-1,2,4-triazol-3-yl]ethylammonium chloride (80 mg, 0.30 mmol, 1 eq.), [dimethylamino(triazolo[4,5-b]pyridin-3-yloxy)methylene]-dimethylammonium;hexafluorophosphate (148 mg, 0.39 mmol, 1.3 eq.) and N-ethyl-N-isopropyl-porpan-2-amine (0.18 mL, 136 mg, 1.05 mmol, 3.5 eq.) in DMF (3 mL) was stirred for 19 h at room temperature. The solvent was removed under reduced pressure, redissolved in ethyl acetate (10 mL), extracted with H₂O, dried with Na₂SO₄, filtered and the solvent was evapurated. The residue was purified by column chromatography on silica gel (eluting with cyclohexane/ethyl acetate) to afford 3-chloro-N-[1-[2-([1,2,4]triazolo[4,3-b]pyridazin-6-yl)-1,2,4-triazol-3-yl]ethyl]-5-(trifluoromethylsulfonyl)benzamide (88 mg, 0.176 mmol, 59%) as a solid. LC-MS (ES) m/z = 500.9 (M+H)⁺. ¹H NMR (400 MHz, CDCl₃) δ [ppm] 9.21 (d, J = 0.8 Hz 1H), 8.38 + 8.36 (d, J = 0.8 Hz, sum 1H), 8.33 - 8.31 m, 1H), 8.26 (t, J = 1.8, 1H), 8.15 - 8.13 (m, 1H), 8.09 - 8.08 (m, 1H), 7.99 + 7.96 (s, sum 1H), 6.24 (p, J = 6.9, 1H), 1.81 (d, J = 6.8, 3H).

### Example 9 : Synthesis of N-methyl-N-(1-(1-(1-methyl-1H-pyrazolo[3,4-b]pyridin-6-yl)-1H-1 ,2,4-triazol-5-yl)ethyl)-3,5-bis(trifluoromethyl)benzamide (I-24)

Step 1 : A mixture of 6-chloro-1-methyl-pyrazolo[3,4-b]pyridine (400 mg, 2.39 mmol) and hydrazine hydrate (1.20 g, 23.87 mmol) in EtOH (4 mL) was stirred at 100 °C for 16 hours in a sealed tube. The reaction mixture was concentrated. The residue was diluted with water (10 mL) and filtered. The filter cake was dried under vacuum to afford 6-hydrazineyl-1-methyl-1H-pyrazolo[3,4-b]pyridine (240 mg, 62% yield) as a white solid. 1H NMR (400 MHz, DMSO-d₆) δ [ppm] 8.06 (s, 1H), 7.73-7.70 (m, 2H), 6.49 (d, J = 8.8 Hz, 1H), 4.29 (br s, 2H), 3.85 (s, 1H).

Step 2 : A mixture of 6-hydrazineyl-1-methyl-1H-pyrazolo[3,4-b]pyridine (240 mg, 1.47 mmol) and tert-butyl tert-butyl N-[2-[(E)-dimethylaminomethyleneamino]-1-methyl-2-oxo-ethyl]-N-methyl-carbamate (946 mg, 3.68 mmol) in AcOH (4 mL) was stirred at 50 °C for 16 hours. The reaction mixture was concentrated. The residue was purified by column chromatography on silica gel (DCM/MeOH=50/1) to afford tert-butyl methyl(1-(1-(1-methyl-1H-pyrazolo[3,4-b]pyridin-6-yl)-1H-1,2,4-triazol-5-yl)ethyl)carbamate (240 mg, 46% yield) as a white solid. LC-MS (ES) m/z (M+H)⁺ = 358.4.

Step 3 : A mixture of tert-butyl methyl(1-(1-(1-methyl-1H-pyrazolo[3,4-b]pyridin-6-yl)-1H-1 ,2,4-triazol-5-yl)ethyl)carbamate (250 mg, 0.70 mmol) in HCl/dioxane (2M, 5 mL) was stirred at 25 °C for 2 hours. The reaction mixture was concentrated to afford N-methyl-1-(1-(1-methyl-1H-pyrazolo[3,4-b]pyridin-6-yl)-1H-1,2,4-triazol-5-yl)ethan-1-amine hydrochloride (200 mg, 97% yield) as a white solid. LC-MS (ES) m/z (M+H)⁺ = 258.1.

Step 4: To a mixture of N-methyl-1-(1-(1-methyl-1H-pyrazolo[3,4-b]pyridin-6-yl)-1H-1,2,4-triazol-5-yl)ethan-1-amine hydrochloride (200 mg, 0.68 mmol) and TEA (207 mg, 2.04 mmol) in DCM (5 mL) was added 3,5-bis(trifluoromethyl)benzoyl chloride (226 mg, 0.82 mmol) at 0 °C. The reaction was stirred at 0 °C for 10 minutes. The reaction mixture was diluted with ice-water (20 mL) and extracted DCM (20 mL* 2). The combined organic phase was concentrated. The residue was purified by column chromatography on silica gel (DCM/MeOH = 20/1) to afford N-methyl-N-(1-(1-(1-methyl-1H-pyrazolo[3,4-b]pyridin-6-yl)-1H-1,2,4-triazol-5-yl)ethyl)-3,5-bis(trifluoromethyl)benzamide (I-24) (150 mg, 44% yield) as a white solid. LC-MS (ES) m/z (M+H)⁺ = 498.0. 1H NMR (400 MHz, DMSO-d₆) δ 8.54-8.35 (m, 1H), 8.28-7.34 (m, 6H), 6.72-6.05 (m, 1H), 4.00-3.71 (m, 3H), 2.55-2.50 (m, 3H), 1.75 (d, J = 6.8 Hz, 3H).

### Example 10 : Synthesis of N-(1-(1-(1H-pyrazolo[3,4-b]pyridin-6-yl)-1H-1 ,2,4-triazol-5-yl)ethyl)-3,5-bis(trifluoromethyl)benzamide (I-26)

Step 1 : A mixture of 1H-pyrazolo[3,4-b]pyridin-6-ylhydrazine (100 mg, 0.67 mmol) and tert-butyl N-[2-[(E)-dimethylaminomethyleneamino]-1-methyl-2-oxo-ethyl]carbamate (245 mg, 1.01 mmol) in AcOH (2 mL) was stirred at 20 °C for 2 hrs. Then the mixture was stirred at 50 °C for 6 hrs. The mixture was concentrated. The residue was diluted with EtOAc (20 mL), washed with sat. NaHCO₃ (10 mL * 2). The organic layer was dried over MgSO₄, filtered and concentrated. The residue was purified by column chromatography on silica gel (PE/EtOAc = 2/1) to afford tert-butyl (1-(1-(1H-pyrazolo[3,4-b]pyridin-6-yl)-1H-1,2,4-triazol-5-yl)ethyl)carbamate (150 mg, 56% yield) as a yellow solid. LC-MS (ES) m/z = 274.3 (M+H-tBu)⁺.

Step 2: A mixture of tert-butyl (1-(1-(1H-pyrazolo[3,4-b]pyridin-6-yl)-1H-1 ,2,4-triazol-5-yl)ethyl)carbamate (150 mg, 0.46 mmol) in EtOAc (2 mL) and HCl/1,4-dioxane (2 mL) was stirred at 25 °C for 1 hr. The mixture was concentrated to afford 1-(1-(1H-pyrazolo[3,4-b]pyridin-6-yl)-1H-1,2,4-triazol-5-yl)ethan-1-amine (120 mg, 99% yield) as a white solid. LC-MS (ES) m/z = 230.2 (M+H)⁺.

Step 3 : To a mixture of 1-(1-(1H-pyrazolo[3,4-b]pyridin-6-yl)-1H-1,2,4-triazol-5-yl)ethan-1-amine (120 mg, 0.45 mmol) and TEA (137 mg, 1.35 mmol) in THF (2 mL) was added 3,5-bis(trifluoromethyl)benzoyl chloride (150 mg, 0.54 mmol) at 0 °C. The mixture was stirred at 0 °C for 2 hrs. The mixture was diluted with H₂O (5 mL), extracted with EtOAc (10 mL * 2). The organic layer was dried over Na₂SO₄, filtered and concentrated. The residue was purified by column chromatography on silica gel (DCM/MeOH = 20/1) to afford the crude compound. The crude compound was purified by reverse flash (5-95% ACN in water) to afford N-(1-(1-(1H-pyrazolo[3,4-b]pyridin-6-yl)-1H-1,2,4-triazol-5-yl)ethyl)-3,5-bis(trifluoromethyl)benzamide (I-26) (117 mg, 55% yield) as a white solid. LC-MS (ES) m/z = 470.0 (M+H)⁺. 1H NMR (400 MHz, DMSO-d₆) δ [ppm] 13.92 (br s, 1H), 9.62 (d, J = 7.2 Hz, 1H), 8.49 (d, J = 8.4 Hz, 1H), 8.42 (s, 2H), 8.30 (s, 1H), 8.26 (s, 1H), 8.21 (s, 1H), 7.70 (d, J = 8.4 Hz, 1H), 6.25-6.18 (m, 1H), 1.74 (d, J = 6.8 Hz, 3H).

### Example 11: Synthesis of N-(1-(1-(1H-pyrazolo[3,4-b]pyridin-6-yl)-1H-1,2,4-triazol-5-yl)ethyl)-N-methyl-3,5-bis(trifluoromethyl)benzamide (I-25)

Step 1 : A mixture of 1H-pyrazolo[3,4-b]pyridin-6-ylhydrazine (100 mg, 0.67 mmol) and tert-butyl N-[2-[(E)-dimethylaminomethyleneamino]-1-methyl-2-oxo-ethyl]-N-methyl-carbamate (259 mg, 1.01 mmol) in AcOH (2 mL) was stirred at 20 °C for 2 hrs. Then the mixture was stirred at 50 °C for 6 hrs. The mixture was concentrated. The residue was diluted with EtOAc (20 mL), washed with sat. NaHCO₃ (10 mL * 2). The organic layer was dried over MgSO₄, filtered and concentrated. The residue was purified by column chromatography on silica gel (PE/EtOAc = 2/1) to afford tert-butyl (1-(1-(1H-pyrazolo[3,4-b]pyridin-6-yl)-1H-1,2,4-triazol-5-yl)ethyl)(methyl)carbamate (128 mg, 56% yield) as a yellow solid. LC-MS (ES) m/z = 288.3 (M+H-tBu)⁺.

Step 2: A mixture of tert-butyl (1-(1-(1H-pyrazolo[3,4-b]pyridin-6-yl)-1H-1,2,4-triazol-5-yl)ethyl)(methyl)carbamate (128 mg, 0.37 mmol) in MeOH (2 mL) and HCl/1,4-dioxane (2 mL) was stirred at 25 °C for 1 h. The mixture was concentrated to afford 1-(1-(1H-pyrazolo[3,4-b]pyridin-6-yl)-1H-1,2,4-triazol-5-yl)-N-methylethan-1-amine (104 mg, 100% yield) as a yellow solid. LC-MS (ES) m/z = 244.2 (M+H)⁺.

Step 3 : To a mixture of 1-(1-(1H-pyrazolo[3,4-b]pyridin-6-yl)-1H-1,2,4-triazol-5-yl)-N-methylethan-1-amine (104 mg, 0.37 mmol) and TEA (113 mg, 1.12 mmol) in THF (3 mL) was added 3,5-bis(trifluoromethyl)benzoyl chloride (123 mg, 0.45 mmol) at 0 °C. The mixture was stirred at 0 °C for 2 hrs. The mixture was diluted with H₂O (5 mL), extracted with EtOAc (10 mL * 2). The organic layer was dried over Na₂SO₄, filtered and concentrated. The residue was purified by column chromatography on silica gel (DCM/MeOH = 20/1) to afford the crude compound. The crude compound was purified by reverse flash (5-95% ACN in water) to afford N-(1-(1-(1H-pyrazolo[3,4-b]pyridin-6-yl)-1H-1 ,2,4-triazol-5-yl)ethyl)-N-methyl-3,5-bis(trifluoromethyl)benzamide (I-25) (42 mg, yield 55%) as a yellow solid. LC-MS (ES) m/z = 484.0 (M+H)⁺. 1H NMR (400 MHz, CDCl₃) δ [ppm] 11.69 (br s, 1H), 8.31 (d, J = 8.8 Hz, 1H), 8.09 (s, 2H), 7.88 (d, J = 8.4 Hz, 1H), 7.82 (s, 1H), 7.43 (s, 2H), 6.92-6.87 (m, 1H), 2.65 (s, 3H), 1.82 (d, J = 6.4 Hz, 3H).

### Example 12 : Synthesis of N-(1-(1-(imidazo[1,5-c]pyrimidin-7-yl)-1H-1,2,4-triazol-5-yl)ethyl)-3,5-bis(trifluoromethyl)benzamide (I-27)

Step 1 : A mixture of tert-butyl N-[1-[2-(6-chloropyrimidin-4-yl)-1,2,4-triazol-3-yl]ethyl]carbamate (3 g, 9.2 mmol), Zn(CN)₂ (2.16 g, 18.4 mmol), Dppf (1.02 g, 1.84 mmol) and Pd₂(dba)₃ (0.84 g, 0.92 mmol) in DMF (30 mL) was stirred at 80 °C under N₂ for 5 hrs. The mixture was filtered and the filtrate was concentrated. The residue was purified by flash chromatography (PE/EtOAc = 2/1) to afford tert-butyl (1-(1-(6-cyanopyrimidin-4-yl)-1H-1 ,2,4-triazol-5-yl)ethyl)carbamate (2 g, 68% yield) as a yellow solid. LC-MS (ES) m/z = 260.3 (M+H)⁺.

Step 2 : A mixture of tert-butyl (1-(1-(6-cyanopyrimidin-4-yl)-1H-1 ,2,4-triazol-5-yl)ethyl)carbamate (2 g, 1.91 mmol), Pd/C (200 mg) and Pd(OH)₂/C (200 mg) in MeOH (200 mL) was stirred at 50 °C under H₂ for 3 hrs. The mixture was filtered and the filtrate was concentrated to afford crude tert-butyl (1-(1-(6-(aminomethyl)pyrimidin-4-yl)-1H-1,2,4-triazol-5-yl)ethyl)carbamate (1.5 g, 75% yield) as a yellow solid, which was used directly for next step without further purification. LC-MS (ES) m/z = 320.3 (M+H)⁺.

Step 3 : A mixture of HCOOH (3 mL) and (Ac)₂O (9 mL) was stirred at 50 °C for 1 hour. The reaction mixture was cooled to 0 °C. The tert-butyl (1-(1-(6-(aminomethyl)pyrimidin-4-yl)-1H-1,2,4-triazol-5-yl)ethyl)carbamate (1.5 g, 4.7 mmol) in THF (5 mL) was added into the reaction mixture at 0 °C. The reaction was stirred at 0 °C for 1 h. The reaction mixture was diluted with ice-water (5 mL) and extracted with EtOAc (5 mL * 2). The combined organic phase was purified by column chromatography on silica gel (DCM/MeOH = 20/1) to afford tert-butyl (1-(1-(6-(formamidomethyl)pyrimidin-4-yl)-1H-1,2,4-triazol-5-yl)ethyl)carbamate (0.8 g, 49% yield) as a yellow solid. LC-MS (ES) m/z = 348.2 (M+H)⁺.

Step 4: To a solution of tert-butyl (1-(1-(6-(formamidomethyl)pyrimidin-4-yl)-1H-1,2,4-triazol-5-yl)ethyl)carbamate (600 mg, 1.73 mmol) and TEA (350 mg, 3.46 mmol) in DCM (12 mL) was slowly added Trifluoromethanesulfonic anhydride (977 mg, 3.46 mmol) at 0 °C and stirred at 0 °C for 1 hour. The reaction mixture was diluted with ice-water (4 mL) and extracted with DCM (4 mL * 2). The combined organic phase was concentrated. The residue was purified by column chromatography on silica gel (DCM/MeOH=20/1) to afford tert-butyl (1-(1-(imidazo[1,5-c]pyrimidin-7-yl)-1H-1,2,4-triazol-5-yl)ethyl)carbamate (300 mg, 53% yield) as a yellow solid. LC-MS (ES) m/z = 330.1 (M+H)⁺. 1H NMR (400 MHz, DMSO-d₆) δ [ppm] 9.36 (s, 1H), 8.67 (s, 1H), 8.08 (s, 1H), 7.85 (s, 1H), 7.57 (s, 1 H), 7.41 (d, J = 7.6 Hz, 1H), 5.40-5.36 (m, 1H), 1.42 (d, J = 6.8 Hz, 3H), 1.24 (s, 9H).

Step 5: To a solution of tert-butyl (1-(1-(imidazo[1,5-c]pyrimidin-7-yl)-1H-1,2,4-triazol-5-yl)ethyl)carbamate (200 mg, 0.61 mmol) in DCM (2 mL) was added TFA (1 mL) at 0 °C and stirred at 0 °C for 1 h. The solvent was removed by pumping by N₂ to afford the crude product. The crude product was purified by reverse chromatography (5-95% CAN in water) to afford 1-(1-(imidazo[1,5-c]pyrimidin-7-yl)-1H-1,2,4-triazol-5-yl)ethan-1-amine (100 mg, 72% yield) as a yellow solid. LC-MS (ES) m/z = 230.3 (M+H)⁺.

Step 6 : To a solution of 1-(1-(imidazo[1,5-c]pyrimidin-7-yl)-1H-1 ,2,4-triazol-5-yl)ethan-1-amine (100 mg, 0.44 mmol) and 3,5-bis(trifluoromethyl)benzoyl chloride (145 mg, 0.52 mmol) in THF (2 mL) was added TEA (89 mg, 0.87 mmol) at 0 °C and stirred at 0 °C for 1 h. The mixture was diluted into H₂O (2 mL), extracted with EtOAc (2 mL * 2). The organic layer was concentrated. The residue was purified by column chromatography on silica gel (DCM/MeOH = 20/1) to afford N-(1-(1-(imidazo[1,5-c]pyrimidin-7-yl)-1H-1,2,4-triazol-5-yl)ethyl)-3,5-bis(trifluoromethyl)benzamide (I-27) (74 mg, 36% yield) as a yellow solid. LC-MS (ES) m/z = 470.0 (M+H)⁺. 1H NMR (400 MHz, DMSO-d₆) δ [ppm] 9.56 (d, J = 6.8 Hz, 1H), 9.35 (s, 1H), 8.63 (s, 1H), 8.42 (s, 2H), 8.29 (s, 1H), 8.16 (s, 1 H), 7.90 (s, 1 H), 7.55 (s, 1 H), 5.95 (t, J = 6.8 Hz, 1H), 1.67 (d, J = 6.8 Hz, 3H).

### Example 13 : Synthesis of N-(1-(1-([1,2,4]triazolo[1,5-c]pyrimidin-7-yl)-1H-1,2,4-triazol-5-yl)ethyl)-3,5-bis(trifluoromethyl)benzamide (I-28) :

Step 1 : A mixture of 4-chloro-6-hydrazineylpyrimidine (5.0 g, 34.59 mmol) and tert-butyl N-[2-[(E)-dimethylaminomethyleneamino]-1-methyl-2-oxo-ethyl]carbamate (10.1 g, 41.50 mmol) in AcOH (50 mL) was stirred at 20 °C for 20 hrs. The mixture was concentrated. The residue was diluted with EtOAc (200 mL), washed with sat. NaHCO₃ (100 mL * 2), H₂O (100 mL). The organic layer was dried over MgSO₄, filtered and concentrated. The residue was purified by column chromatography on silica gel (PE/EtOAc = 5/1) to afford tert-butyl (1-(1-(6-chloropyrimidin-4-yl)-1H-1 ,2,4-triazol-5-yl)ethyl)carbamate (6.9 g, 61% yield) as a yellow solid. 1H NMR (400 MHz, CDCl₃) δ [ppm] 8.93 (s, 1H), 8.01 (s, 1H), 7.98 (s, 1H), 6.06-6.02 (m, 1H), 5.54 (d, J = 6.8 Hz, 1H), 1.57 (d, J = 6.8 Hz, 3H), 1.42 (s, 9H).

Step 2 : A mixture of tert-butyl (1-(1-(6-chloropyrimidin-4-yl)-1H-1,2,4-triazol-5-yl)ethyl)carbamate (2.5 g, 7.70 mmol), diphenylmethanimine (1.67 g, 9.24 mmol), Pd₂(dba)₃ (710 mg, 0.77 mmol), XantPhos (450 mg, 0.77 mmol) and Cs₂CO₃ (5.02 g, 15.40 mmol) in 1,4-dioxane (25 mL) was stirred at 80 °C for 3 hrs under N₂. The mixture was cooled to rt and diluted with H₂O (50 mL), extracted with EtAOc (100 mL * 3). The organic layer was dried over MgSO₄, filtered and concentrated. The residue was purified by column chromatography on silica gel (PE/EtOAc = 5/1) to afford tert-butyl (1-(1-(6-((diphenylmethylene)amino)pyrimidin-4-yl)-1H-1,2,4-triazol-5-yl)ethyl)carbamate (2.13 g, 58% yield) as a yellow solid. LC-MS (ES) m/z = 470.2 (M+H)⁺.

Step 3 : A mixture of tert-butyl (1-(1-(6-((diphenylmethylene)amino)pyrimidin-4-yl)-1H-1,2,4-triazol-5-yl)ethyl)carbamate (2.13 g, 4.50 mmol) in THF (30 mL) and 2 N HCl (6 mL) was stirred at 0 °C for 1 hr. The mixture was basified with sat. NaHCO₃ to adjust pH = 9, extracted with EtOAc (100 mL *2). The organic layer was concentrated. The residue was purified by column chromatography on silica gel (PE/EtOAc = 2/1) to afford tert-butyl (1-(1-(6-aminopyrimidin-4-yl)-1H-1 ,2,4-triazol-5-yl)ethyl)carbamate (1.34 g, 98% yield) as a yellow solid. LC-MS (ES) m/z = 306.2 (M+H)⁺.

Step 4 : To a mixture of tert-butyl (1-(1-(6-aminopyrimidin-4-yl)-1H-1,2,4-triazol-5-yl)ethyl)carbamate (1.34 g, 4.39 mmol) in i-PrOH (25 mL) was added DMF-DMA (1.36 g, 11.41 mmol). The mixture was stirred at 80 °C for 1.5 hrs. The mixture was cooled to rt. The reaction mixture was used directly for the next step without further purification. LC-MS (ES) m/z = 361.4 (M+H)⁺.

Step 5 : A mixture of tert-butyl (Z)-(1-(4-(6-(((dimethylamino)methylene)amino)pyrimidin-4-yl)-4H-imidazol-5-yl)ethyl)carbamate (1.58 g, 4.38 mmol) and NH₂OH * HCl (457 mg, 6.58 mmol) in i-PrOH (30 mL) was stirred at 50 °C for 3 hrs. The mixture was cooled to rt and diluted with sat. NaHCO₃ (30 mL), extracted with EtAOc (50 mL *2). The organic layer was dried over MgSO₄, filtered and concentrated to afford tert-butyl (Z2)-(1-(1-(6-(((hydroxyamino)methylene)amino)pyrimidin-4-yl)-1H-1,2,4-triazol-5-yl)ethyl)carbamate (1.52 g, 100% yield) as a yellow solid. LC-MS (ES) m/z = 349.4 (M+H)⁺.

Step 6 : A mixture of tert-butyl (Z)-(1-(1-(6-(((hydroxyamino)methylene)amino)pyrimidin-4-yl)-1H-1,2,4-triazol-5-yl)ethyl)carbamate (1.4 g, 4.02 mmol) and PPA (57 g, 168.79 mmol) was stirred at 100 °C for 1 hr. The mixture was diluted with H₂O (200 mL). The mixture was purified by reverse flash (C-18) (5-95% ACN in water with 0.1% NH₄HCO₃) to afford the crude 1-(1-([1,2,4]triazolo[1,5-c]pyrimidin-7-yl)-1H-1,2,4-triazol-5-yl)ethan-1-amine (600 mg, 65% yield) as a yellow solid. 1H NMR (400 MHz, CDCl₃) δ [ppm] 9.98 (s, 1H), 8.79 (s, 1H), 8.17-8.16 (m, 2H), 4.83-4.78 (m, 1H), 1.44 (d, J = 6.8 Hz, 3H).

Step 7 : To a mixture of 1-(1-([1,2,4]triazolo[1,5-c]pyrimidin-7-yl)-1H-1,2,4-triazol-5-yl)ethan-1-amine (250 mg, 1.09 mmol) and TEA (330 mg, 3.26 mmol) in THF (5 mL) was added 3,5-bis(trifluoromethyl)benzoyl chloride (300 mg, 1.09 mmol) at 0 °C. The mixture was stirred at 0 °C for 2 hrs. The mixture was diluted with H₂O (5 mL), extracted with EtOAc (10 mL * 2). The organic layer was dried over Na₂SO₄, filtered and concentrated. The residue was purified by column chromatography on silica gel (DCM/MeOH = 20/1) to afford the crude compound. The crude compound was purified by reverse flash (5-95% ACN in water) to afford N-(1-(1-([1,2,4]triazolo[1,5-c]pyrimidin-7-yl)-1H-1,2,4-triazol-5-yl)ethyl)-3,5-bis(trifluoromethyl)benzamide (I-28) (40 mg, 8% yield) as a white solid. LC-MS (ES) m/z = 471.0 (M+H)⁺. 1H NMR (400 MHz, CDCl₃) δ [ppm] 9.51 (s, 1H), 8.54 (s, 1H), 8.33-8.30 (m, 3H), 8.05-8.03 (m, 2H), 7.61 (d, J = 7.6 Hz, 1H), 6.58-6.51 (m, 1H), 1.77 (d, J = 6.8 Hz, 3H).

### Example 14 : Synthesis of N-(1-(1-(3-amino-[1,2,4]triazolo[4,3-b]pyridazin-6-yl)-1H-1,2,4-triazol-5-yl)ethyl)-3,5-bis(trifluoromethyl)benzamide (I-29)

Step 1 : A mixture of 6-chloro-[1,2,4]triazolo[4,3-b]pyridazin-3-amine (1.00 g, 5.90 mmol) and hydrazine hydrate (2.95 g, 59.00 mmol) in EtOH (10 mL) was stirred at 100 °C for 16 hours in a sealed tube. The reaction mixture was filtered. The filter cake washed with water (30 mL). The filter cake was dried under vacuum to afford 6-hydrazineyl-[1,2,4]triazolo[4,3-b]pyridazin-3-amine (0.88 g, 90% yield) as a white solid. LC-MS (ES) m/z (M+H)⁺ = 166.0.

Step 2 : A mixture of 6-hydrazineyl-[1,2,4]triazolo[4,3-b]pyridazin-3-amine (150 mg, 0.91 mmol) and tert-butyl N-[2-[(E)-dimethylaminomethyleneamino]-1-methyl-2-oxo-ethyl]carbamate (331 mg, 1.36 mmol) in AcOH (3 mL) was stirred at 50 °C for 3 hours. The reaction mixture was concentrated. The residue was purified by column chromatography on silica gel (DCM/MeOH = 19/1) to afford tert-butyl (1-(1-(3-amino-[1,2,4]triazolo[4,3-b]pyridazin-6-yl)-1H-1,2,4-triazol-5-yl)ethyl)carbamate (290 mg, 92% yield) as a yellow solid. LC-MS (ES) m/z (M+H)⁺ = 346.3.

Step 3 : A mixture of tert-butyl (1-(1-(3-amino-[1,2,4]triazolo[4,3-b]pyridazin-6-yl)-1H-1,2,4-triazol-5-yl)ethyl)carbamate (330 mg, 0.96 mmol) in HCl/EtOAc (2 M, 6 mL) was stirred at 25 °C for 2 hours. The reaction mixture was concentrated to afford 6-(5-(1-aminoethyl)-1H-1 ,2,4-triazol-1-yl)-[1 ,2,4]triazolo[4,3-b]pyridazin-3-amine dihydrochloride (300 mg, 99% yield) as a white solid. LC-MS (ES) m/z (M+H)⁺ = 246.1.

Step 4 : To a mixture of 6-(5-(1-aminoethyl)-1H-1 ,2,4-triazol-1-yl)-[1 ,2,4]triazolo[4,3-b]pyridazin-3-amine dihydrochloride (300 mg, 0.94 mmol) and TEA (382 mg, 3.77 mmol) in DCM (6 mL) was added 3,5-bis(trifluoromethyl)benzoyl chloride (313 mg, 1.13 mmol) at 0 °C. The reaction was stirred at 0 °C for 10 minutes. The reaction mixture was diluted with ice-water (20 mL) and extracted DCM (20 mL*2). The combined organic phase was concentrated. The residue was purified by column chromatography on silica gel (DCM/MeOH = 20/1) to afford the crude product. The crude product was purified by reverse chromatography (5-95% ACN in water) to afford N-(1-(1-(3-amino-[1,2,4]triazolo[4,3-b]pyridazin-6-yl)-1H-1,2,4-triazol-5-yl)ethyl)-3,5-bis(trifluoromethyl)benzamide (I-29) (180 mg, 39% yield) as a yellow solid. LC-MS (ES) m/z (M+H)⁺ = 486.0. 1H NMR (400 MHz, DMSO-d₆) δ [ppm] 9.64 (d, J = 7.6 Hz, 1H), 8.43 (s, 2H), 8.33 (s, 1H), 8.26 (s, 1H), 8.22 (d, J = 10.0 Hz, 1H), 6.66 (s, 2H), 6.14-6.08 (m, 1H), 1.71(d, J = 6.8 Hz, 3H).

### Example 15 : Synthesis of N-(1-(1-(3-amino-[1,2,4]triazolo[4,3-b]pyridazin-6-yl)-1H-1,2,4-triazol-5-yl)ethyl)-N-methyl-3,5-bis(trifluoromethyl)benzamide (I-31)

Step 1 : A mixture of 6-hydrazino-[1,2,4]triazolo[4,3-b]pyridazin-3-amine (300 mg, 1.82 mmol) and tert-butyl N-[2-[(E)-dimethylaminomethyleneamino]-1-methyl-2-oxo-ethyl]-N-methyl-carbamate (2.34 g, 9.08 mmol) in AcOH (6 mL) was stirred at 50 °C for 8 hours. The reaction mixture was concentrated. The residue was purified by column chromatography on silica gel (DCM/MeOH=19/1) to afford tert-butyl (1-(1-(3-amino-[1,2,4]triazolo[4,3-b]pyridazin-6-yl)-1H-1,2,4-triazol-5-yl)ethyl)(methyl)carbamate (300 mg, 46% yield) as a yellow solid. LC-MS (ES) m/z (M+H)⁺ = 360.1.

Step 2 : A mixture of tert-butyl (1-(1-(3-amino-[1,2,4]triazolo[4,3-b]pyridazin-6-yl)-1H-1,2,4-triazol-5-yl)ethyl)(methyl)carbamate (240 mg, 0.67 mmol) in HCl/EtOAc (2 M, 6 mL) was stirred at 25 °C for 2 hours. The reaction mixture was concentrated to afford 6-(5-(1-(methylamino)ethyl)-1H-1,2,4-triazol-1-yl)-[1,2,4]triazolo[4,3-b]pyridazin-3-amine dihydrochloride (220 mg, 99% yield) as a white solid. LC-MS (ES) m/z (M+H)⁺ = 260.1.

Step 3 : To a mixture of 6-(5-(1-(methylamino)ethyl)-1 H-1 ,2,4-triazol-1-yl)-[1 ,2,4]triazolo[4,3-b]pyridazin-3-amine dihydrochloride (220 mg, 0.66 mmol) and TEA (268 mg, 2.65 mmol) in DCM (2 mL) was added 3,5-bis(trifluoromethyl)benzoyl chloride (220 mg, 0.79 mmol) at 0 °C. The reaction was stirred at 0 °C for 10 minutes. The reaction mixture was diluted with ice-water (20 mL) and extracted DCM (20 mL * 2). The combined organic phase was concentrated. The residue was purified by column chromatography on silica gel (DCM/MeOH = 20/1) to afford the crude product. The crude product was purified by reverse chromatography (5-95% ACN in water) to afford N-(1-(1-(3-amino-[1,2,4]triazolo[4,3-b]pyridazin-6-yl)-1H-1,2,4-triazol-5-yl)ethyl)-N-methyl-3,5-bis(trifluoromethyl)benzamide (I-31) (200 mg, 60% yield) as a yellow solid. LC-MS (ES) m/z (M+H)⁺ = 500.0. 1H NMR (400 MHz, DMSO-d₆) δ [ppm] 8.38 (s, 1H), 8.27 (d, J = 10.0 Hz, 1H), 8.18 (s, 1H), 7.97 (s, 2H), 7.63 (d, J = 10.0 Hz, 1H), 6.66-6.58 (m, 3H), 3.28-2.71 (m, 3H), 1.70(d, J = 6.8 Hz, 3H).

### Example 16 : Synthesis of N-methyl-N-(1-(1-(1-methyl-1H-pyrazolo[4,3-c]pyridin-6-yl)-1H-1,2,4-triazol-5-yl)ethyl)-3,5-bis(trifluoromethyl)benzamide (I-32)

Step 1 : A mixture of 6-chloro-1-methyl-pyrazolo[4,3-c]pyridine (800 mg, 4.77 mmol), di-tert-butyl hydrazine-1,2-dicarboxylate (1663 mg, 7.16 mmol), Cs₂CO₃ (3110 mg, 9.55 mmol) and BrettphosPdG3 (433 mg, 0.47 mmol) in 1,4-dioxane (8 mL) was stirred at 100 °C for 2 hours under N₂. The reaction mixture was diluted with water (50 mL) and extracted with EtOAc (50 mL*2). The combined organic phase was concentrated. The residue was purified by column chromatography on silica gel (DCM/MeOH = 20/1) to afford di-tert-butyl 1-(1-methyl-1H-pyrazolo[4,3-c]pyridin-6-yl)hydrazine-1,2-dicarboxylate (1300 mg, 75% yield) as brown oil. LC-MS (ES) m/z (M+H)⁺ = 364.4.

Step 2 : A mixture of di-tert-butyl 1-(1-methyl-1H-pyrazolo[4,3-c]pyridin-6-yl)hydrazine-1,2-dicarboxylate (1.10 g, 3.00 mmol) in HCl/EtOAc (2M, 10 mL) was stirred at 25 °C for 2 hours. The reaction mixture was concentrated to afford 6-hydrazineyl-1-methyl-1H-pyrazolo[4,3-c]pyridine dihydrochloride (0.65 g, 93% yield) as a white solid. LC-MS (ES) m/z (M+H)⁺ = 164.0.

Step 3 : A mixture of 6-hydrazineyl-1-methyl-1H-pyrazolo[4,3-c]pyridine dihydrochloride (650 mg, 2.75 mmol) and tert-butyl N-[2-[(E)-dimethylaminomethyleneamino]-1-methyl-2-oxo-ethyl]-N-methylcarbamate (3542 mg, 13.77 mmol) in AcOH (10 mL) was stirred at 50 °C for 16 hours. The reaction mixture was concentrated. The residue was purified by column chromatography on silica gel (DCM/MeOH = 19/1) to afford tert-butyl methyl(1-(1-(1-methyl-1H-pyrazolo[4,3-c]pyridin-6-yl)-1H-1,2,4-triazol-5-yl)ethyl)carbamate (240 mg, 24% yield) as brown oil. LC-MS (ES) m/z (M+H)⁺ = 358.4.

Step 4 : A mixture of tert-butyl methyl(1-(1-(1-methyl-1H-pyrazolo[4,3-c]pyridin-6-yl)-1H-1,2,4-triazol-5-yl)ethyl)carbamate (190 mg, 0.14 mmol) in HCl/EtOAc (2 M, 6 mL) was stirred at 25 °C for 2 hours. The reaction mixture was concentrated to afford N-methyl-1-(1-(1-methyl-1H-pyrazolo[4,3-c]pyridin-6-yl)-1H-1,2,4-triazol-5-yl)ethan-1-amine hydrochloride (150 mg, 96% yield) as a white solid. LC-MS (ES) m/z (M+H)⁺ = 258.1.

Step 5 : To a mixture of N-methyl-1-(1-(1-methyl-1H-pyrazolo[4,3-c]pyridin-6-yl)-1H-1,2,4-triazol-5-yl)ethan-1-amine hydrochloride (150 mg, 0.51 mmol) and TEA (207 mg, 2.04 mmol) in DCM (2 mL) was added 3,5-bis(trifluoromethyl)benzoyl chloride (169 mg, 0.61 mmol) at 0 °C. The reaction was stirred at 0 °C for 10 minutes. The reaction mixture was diluted with ice-water (20 mL) and extracted DCM (20 mL * 2). The combined organic phase was concentrated. The residue was purified by column chromatography on silica gel (DCM/MeOH = 20/1) to afford the crude product. The crude product was purified by reverse flash (5-95% ACN in water) to afford N-methyl-N-(1-(1-(1-methyl-1H-pyrazolo[4,3-c]pyridin-6-yl)-1H-1,2,4-triazol-5-yl)ethyl)-3,5-bis(trifluoromethyl)benzamide (I-32) (160 mg, 63% yield) as a white solid. LC-MS (ES) m/z (M+H)⁺ = 498.0. 1H NMR (400 MHz, DMSO-d₆) δ [ppm] 8.98-8.42 (m, 1H), 8.35-8.31 (m, 1H), 8.24 (s, 1H), 8.15-7.75 (m, 2H), 7.71-7.44 (m, 2H), 6.47-6.07 (m, 1H), 4.12-4.07 (m, 3H), 3.22-2.58 (m, 3H), 1.68 (d, J = 7.2 Hz, 3H).

### Example 17: Synthesis of N-(1-(1-(1H-pyrazolo[4,3-c]pyridin-6-yl)-1H-1,2,4-triazol-5-yl)ethyl)-3,5-bis(trifluoromethyl)benzamide (I-33)

Step 1 : To a solution of 6-chloro-1 H-pyrazolo[4,3-c]pyridine (500 mg, 3.26 mmol) and Boc₂O (853 mg, 3.91 mmol) in DCM (5 mL) was added DMAP (40 mg, 0.33 mmol) at 25 °C. The mixture was stirred at 25 °C for 1 hr. The reaction mixture was concentrated. The residue was purified by column chromatography on silica gel (PE/EtOAc = 3/1) to afford tert-butyl 6-chloro-1H-pyrazolo[4,3-c]pyridine-1-carboxylate (750 mg, 91% yield) as a yellow solid. LC-MS (ES) m/z = 254.2 (M+H)⁺.

Step 2 : A mixture of tert-butyl 6-chloro-1H-pyrazolo[4,3-c]pyridine-1-carboxylate (750 mg, 2.96 mmol), di-tert-butyl bicarbamate (1.37 g, 5.91 mmol), Cs₂CO₃ (1.93 g, 5.91 mmol) and BrettPhosPdG3(268 mg, 0.30 mmol) in 1,4-dioxane (8 mL) was stirred at 100 °C for 3 hrs under N₂. The mixture was filtered. The filtrate was concentrated. The residue was purified by column chromatography on silica gel (DCM/MeOH = 20/1) to afford di-tert-butyl 1-(1-(tert-butoxycarbonyl)-1H-pyrazolo[4,3-c]pyridin-6-yl)hydrazine-1,2-dicarboxylate (1.0 g, 75% yield) as a yellow solid. LC-MS (ES) m/z = 450.5 (M+H)⁺.

Step 3 : To a solution of di-tert-butyl 1-(1-(tert-butoxycarbonyl)-1H-pyrazolo[4,3-c]pyridin-6-yl)hydrazine-1,2-dicarboxylate (1.0 g, 2.23 mmol) in HCl/1,4-dioxane (15 mL) was stirred at 25 °C for 1 hr. The mixture was filtered. The filter cake was dried to afford 6-hydrazineyl-1H-pyrazolo[4,3-c]pyridine hydrochloride (373 mg, 90% yield) as a yellow solid which was used directly for next step without further purification. LC-MS (ES) m/z = 150.0 (M+H)⁺. 1H NMR (400 MHz, DMSO-d₆) δ [ppm] 8.97 (s, 1H), 8.31 (s, 1H), 7.05 (s, 1 H), 1.60 (s, 2H).

Step 4 : A mixture of 6-hydrazineyl-1H-pyrazolo[4,3-c]pyridine hydrochloride (310 mg, 1.68 mmol) and tert-butyl N-[2-[(E)-dimethylaminomethyleneamino]-1-methyl-2-oxo-ethyl]carbamate (612 mg, 2.51 mmol) in AcOH (5 mL) was stirred at 25 °C for 3 hours. Then the mixture was warmed to 70 °C and stirred for 12 hours. The mixture was concentrated. The residue was dissolved in EtOAc (10 mL) and adjusted pH = 9-10 with sat. Na₂CO₃ solution. The mixture was extracted with EtOAc (10 mL * 3). The organic layer was concentrated. The residue was purified by column chromatography on silica gel (DCM/MeOH = 20/1) to afford tert-butyl (1-(1-(1H-pyrazolo[4,3-c]pyridin-6-yl)-1H-1,2,4-triazol-5-yl)ethyl)carbamate (220 mg, 40% yield) as a yellow solid. LC-MS (ES) m/z = 330.3 (M+H)⁺.

Step 5 : To a solution of tert-butyl (1-(1-(1H-pyrazolo[4,3-c]pyridin-6-yl)-1H-1,2,4-triazol-5-yl)ethyl)carbamate (220 mg, 0.67 mmol) in HCl/EtOAc (5 mL) was stirred at 25 °C for 1 hour. The reaction mixture was concentrated to afford 1-(1-(1H-pyrazolo[4,3-c]pyridin-6-yl)-1H-1,2,4-triazol-5-yl)ethan-1-amine hydrochloride (151 mg, 85% yield) as a yellow solid which was used directly for next step without further purification. LC-MS (ES) m/z = 230.2 (M+H)⁺.

Step 6: To a solution of 1-(1-(1H-pyrazolo[4,3-c]pyridin-6-yl)-1H-1,2,4-triazol-5-yl)ethan-1-amine hydrochloride (151 mg, 0.57 mmol) and 3,5-bis(trifluoromethyl)benzoyl chloride (188 mg, 0.68 mmol) in THF (5 mL) was added TEA (172 mg, 1.70 mmol) at 0 °C. The mixture was stirred at 0 °C for 0.5 hr. The mixture was diluted with H₂O (2 mL), extracted with EtOAc (2 mL * 2). The organic layer was concentrated. The residue was purified by column chromatography on silica gel (DCM/MeOH = 20/1) to afford N-(1-(1-(1H-pyrazolo[4,3-c]pyridin-6-yl)-1H-1,2,4-triazol-5-yl)ethyl)-3,5-bis(trifluoromethyl)benzamide (I-33) (106 mg, 40% yield) as a yellow solid. LC-MS (ES) m/z = 470.0 (M+H)⁺. 1H NMR (400 MHz, DMSO-d₆) δ [ppm] 13.71 (s, 1H), 9.51 (d, J = 7.2 Hz, 1H), 9.07 (s, 1H), 8.37 (d, J = 12.8 Hz, 3H), 8.27 (s, 1H), 8.16 (s, 1H), 7.86 (s, 1 H), 6.10-6.06 (m, 1H), 1.67 (d, J = 7.2 Hz, 3H).

### Example 18 : Synthesis of N-(1-(1-(imidazo[1,5-a]pyrimidin-2-yl)-1H-1,2,4-triazol-5-yl)ethyl)-3,5-bis(trifluoromethyl)benzamide (I-34)

Step 1 : To a solution of (2-chloropyrimidin-4-yl)hydrazine (1.0 g, 6.9 mmol) in AcOH (10 mL) was added tert-butyl N-[2-[(E)-dimethylaminomethyleneamino]-1-methyl-2-oxo-ethyl]carbamate (2.52 g, 10 mmol) at 30 °C. The reaction mixture was stirred at 30 °C for 12 hours. The mixture was diluted with water (50 mL), then extracted with EtOAc (50 mL*4). The combined organic layers were washed with sat. NaHCO₃ (100 mL*2), brine (100 mL), dried over Na₂SO₄, filtered and concentrated. The residue was purified by a column chromatography on silica gel (PE/EA=5/1-1/1) to afford (1-(1-(2-chloropyrimidin-4-yl)-1H-1,2,4-triazol-5-yl)ethyl)carbamate (1.3 g, 52% yield) as a yellow solid. LC-MS (ES) m/z = 325.3 (M+H)⁺.

Step 2 : To a mixture of (1-(1-(2-chloropyrimidin-4-yl)-1H-1,2,4-triazol-5-yl)ethyl)carbamate (1.3 g, 4 mmol), Zn(CN)₂ (0.94 g, 8 mmol) and dppf (0.89 g, 1.6 mmol) in DMF (15.0 mL) was added Pd₂(dba)₃ (0.73 g, 0.8 mmol). Then the mixture was stirred at 80 °C for 5 hours. The mixture was diluted with water (50 mL), then extracted with EtOAc (50 mL*3). The combined organic layers were washed with brine (100 mL), dried over Na₂SO₄, filtered and concentrated. The residue was purified by a column chromatography on silica gel (PE/EA=5/1-1/1) to afford the (1-(1-(2-cyanopyrimidin-4-yl)-1H-1,2,4-triazol-5-yl)ethyl)carbamate (1 g, 73% yield) as a yellow solid. LC-MS (ES) m/z = 260.2 (M+H-tBu)⁺.

Step 3 : To a solution of (1-(1-(2-cyanopyrimidin-4-yl)-1H-1,2,4-triazol-5-yl)ethyl)carbamate (900.0 mg, 2.85 mmol) in MeOH (10.0 mL) was added Pd/C (91.1 mg, 0.85 mmol) and Pd(OH)₂/C (120.2 mg, 0.85 mmol). Then the mixture was stirred at 30 °C for 6 hours. The reaction was filtered and the filtrate was directly concentrated to give tert-butyl (1-(1-(2-(aminomethyl)pyrimidin-4-yl)-1H-1,2,4-triazol-5-yl)ethyl)carbamate (1.0 g crude, 100% yield) as a yellow oil, which was used directly next step. LC-MS (ES) m/z = 320.3 (M+H)⁺.

Step 4 : To a solution of (Ac)₂O (9 mL) was added HCOOH (3 mL) and the mixture was stirred at 50 °C for 1h. Then tert-butyl (1-(1-(2-(aminomethyl)pyrimidin-4-yl)-1H-1,2,4-triazol-5-yl)ethyl)carbamate (900 mg, 2.82 mmol) in THF (3 mL) was added at 0 °C and the mixture was stirred at 0 °C for 2 hours. The mixture was diluted with water (20 mL), then extracted with EtOAc (20 mL*3). The combined organic layers were washed with brine (50 mL), dried over Na₂SO₄, filtered and concentrated. The residue was purified by a column chromatography on silica gel (DCM/MeOH=60/1-20/1) to afford tert-butyl (1-(1-(2-(formamidomethyl)pyrimidin-4-yl)-1H-1,2,4-triazol-5-yl)ethyl)carbamate (400 mg, 37% yield) as a yellow solid. LC-MS (ES) m/z = 348.3 (M+H)⁺.

Step 5: To a solution of tert-butyl (1-(1-(2-(formamidomethyl)pyrimidin-4-yl)-1H-1,2,4-triazol-5-yl)ethyl)carbamate (200 mg, 0.58 mmol) in DCM(5 mL) was added Tf₂O (812.3 mg, 2.88 mmol) and TEA (116.5 mg, 1.15 mmol) at 0 °C. The reaction mixture was stirred at 0 °C for 2 hours. The mixture was concentrated to afford 1-(1-(imidazo[1,5-a]pyrimidin-2-yl)-1H-1,2,4-triazol-5-yl)ethan-1-amine (150 mg, 91% yield) as a yellow oil, which was used directly next step. LC-MS (ES) m/z = 230.2 (M+H)⁺. Step 6 : To a solution of 1-(1-(imidazo[1,5-a]pyrimidin-2-yl)-1H-1,2,4-triazol-5-yl)ethan-1-amine (150 mg, 0.65 mmol) in THF (2 mL) was added TEA (198.6 mg, 1.96 mmol) and 3,5-bis(trifluoromethyl)benzoyl chloride (199.1 mg, 0.72 mmol) at 0 °C. The reaction mixture was stirred at 0 °C for 2 hours. The reaction solution was concentrated and the residue was purified by a column chromatography on silica gel (DCM/MeOH=30/1-15/1) to afford N-(1-(1-(imidazo[1,5-a]pyrimidin-2-yl)-1H-1,2,4-triazol-5-yl)ethyl)-3,5-bis(trifluoromethyl)benzamide (I-34) (93.7 mg, 30% yield) as a yellow solid. LC-MS (ES) m/z = 470.0 (M+H)⁺. 1H NMR (400 MHz, DMSO-d₆) δ [ppm] 9.62 (d, J = 7.2 Hz, 1H), 8.95 (dd, J = 0.4 Hz, 7.6 Hz, 1H), 8.47 (s, 1H), 8.43 (s, 1H), 8.31 (s, 1H), 8.24 (s, 1H), 7.47 (s, 1H), 7.34 (d, J = 7.2 Hz, 1H), 6.16-6.13 (m, 1H), 1.71 (d, J = 6.8 Hz, 3H).

### Example 19 : Synthesis of N-(1-(1-(5-oxo-5,6,7,8-tetrahydro-2,6-naphthyridin-3-yl)-1H-1,2,4-triazol-5-yl)ethyl)-3,5-bis(trifluoromethyl)benzamide (I-23) :

Step 1 : A solution of methyl 5-bromo-2-chloro-pyridine-4-carboxylate (5 g, 0.02 mol), methylboronic acid (1.8 g, 0.03 mol), K₃PO₄ (14.86g, 0.07 mol), Pcy₃ (1.12 g, 0.004 mol) and Pd(OAc)₂ (0.45 g, 0.002 mol) in toluene (95 mL) and H₂O (5 mL) was stirred under N₂ at 100°C for 4 hours. The mixture was diluted with water (50 mL), extracted with EtOAc (35 mL * 2), washed with brine (30 mL). The organic layer was concentrated. The residue was purified by chromatography on silica gel (PE/EtOAc = 20/1) to afford the crude product methyl 2-chloro-5-methylisonicotinate (1.8 g, 41% yield) as a yellow oil. LC-MS (ES) m/z = 187.9 (M+H)⁺.

Step 2: A solution of methyl 2-chloro-5-methylisonicotinate (1.0 g, 5.39 mmol), NBS (959 mg, 5.39 mmol) and AIBN (88 mg, 0.54 mmol) in CCl4 (10 mL) under nitrogen was stirred at 90°C for 3 hours. The mixture was diluted with water (20 mL), extracted with DCM (15 mL * 2), washed with brine (20 mL). The organic layer was concentrated. The residue was purified by chromatography on silica gel (PE/EtOAc = 20/1) to afford the product methyl 5-(bromomethyl)-2-chloroisonicotinate (1.0 g, 60% yield) as a yellow oil. LC-MS (ES) m/z = 263.9 (M+H)⁺.

Step 3: To a solution of methyl 5-(bromomethyl)-2-chloroisonicotinate (1.0 g, 3.78 mmol), TMSCN (450 mg, 4.54 mmol) in CAN (10 mL) stirred under nitrogen at 0°C was added tetrabutylammonium fluoride (1.5 g, 5.67 mmol) in THF (5.7 mL) dropwise. The reaction mixture was stirred at 0-RT for 16 hours. The mixture was diluted with water (50 mL), extracted with EtOAc (35 mL * 2), washed with brine (30 mL). The organic layer was concentrated. The residue was purified by chromatography on silica gel (PE/EtOAc = 10/1) to afford methyl 2-chloro-5-(cyanomethyl)isonicotinate (600 mg, 53% yield) as a white solid. LC-MS (ES) m/z = 211.1 (M+H)⁺.

Step 4: A solution of methyl 2-chloro-5-(cyanomethyl)isonicotinate (1.0 g, 4.75 mmol), (tert-butoxy)-N'-[(tert-butoxy)carbonyl]carbohydrazide (1.0 g, 5.22 mmol), Cs₂CO₃ (3.09 g, 9.50 mmol) and BrettphosPdG3 (430 mg, 0.47 mmol) in 1,4 dioxane(10 mL) was stirred under nitrogen at 100°C for 4 hours. The mixture was diluted with water (20 mL), extracted with EtOAc (20 mL * 2), washed with brine (20 mL). The organic layer was concentrated. The residue was purified by chromatography on silica gel (PE/EtOAc = 10/1) to afford di-tert-butyl 1-(5-(cyanomethyl)-4-(methoxycarbonyl)pyridin-2-yl)hydrazine-1,2-dicarboxylate (800 mg, 36% yield) as a yellow oil. LC-MS (ES) m/z = 407.4 (M+H)⁺.

Step 5: A solution of di-tert-butyl 1-(5-(cyanomethyl)-4-(methoxycarbonyl)pyridin-2-yl)hydrazine-1,2-dicarboxylate (800 mg, 1.96 mmol) and PtO₂ (89 mg, 0.39 mmol) in MeOH (10 mL) was stirred under H2 at RT for 15 hours. The mixture was filtered and the filtrate was concentrated. The residue was purified by chromatography column on silica gel (DCM/MeOH=30/1) to afford di-tert-butyl 1-(5-oxo-5,6,7,8-tetrahydro-2,6-naphthyridin-3-yl)hydrazine-1,2-dicarboxylate (300 mg, 36% yield) as a white solid. LC-MS (ES) m/z = 379.4 (M+H)⁺.

Step 6: A solution of di-tert-butyl 1-(5-oxo-5,6,7,8-tetrahydro-2,6-naphthyridin-3-yl)hydrazine-1,2-dicarboxylate (300 mg, 0.79 mmol) in HCl/EtOAc (3 mL) was stirred at RT for 4 hours. The mixture was concentrated to afford 7-hydrazineyl-3,4-dihydro-2,6-naphthyridin-1(2H)-one (160 mg crude, 100% yield) as a yellow solid. LC-MS (ES) m/z = 179.2 (M+H)⁺

Step 7: A solution of 7-hydrazineyl-3,4-dihydro-2,6-naphthyridin-1(2H)-one (160 mg, 0.89 mmol), tert-butyl N-[2-[(E)-dimethylaminomethyleneamino]-1-methyl-2-oxo-ethyl]carbamate (262 mg, 1.08 mmol) in AcOH (3 mL) was stirred at RT for 2 hours. Then the mixture was stirred at 60 °C for 4 hours. The mixture was diluted with water (10 mL), extracted with EA (10 mL * 2), washed with brine (10 mL). The organic layer was concentrated. The residue was purified by chromatography on silica gel (PE/EtOAc = 5/1) to afford tert-butyl (1-(1-(5-oxo-5,6,7,8-tetrahydro-2,6-naphthyridin-3-yl)-1H-1,2,4-triazol-5-yl)ethyl)carbamate (150 mg, 43% yield) as a white solid. LC-MS (ES) m/z = 359.1 (M+H)⁺.

Step 8: A solution of tert-butyl (1-(1-(5-oxo-5,6,7,8-tetrahydro-2,6-naphthyridin-3-yl)-1H-1,2,4-triazol-5-yl)ethyl)carbamate (150 mg, 0.42 mmol mmol) in HCl/EtOAc (2 mL) was stirred at RT for 4 hours. The mixture was concentrated to afford 7-(5-(1-aminoethyl)-1H-1,2,4-triazol-1-yl)-3,4-dihydro-2,6-naphthyridin-1(2H)-one (100 mg, 88% yield) as a white solid. LC-MS (ES) m/z = 259.3 (M+H)⁺.

Step 9: To a solution of 7-(5-(1-aminoethyl)-1H-1,2,4-triazol-1-yl)-3,4-dihydro-2,6-naphthyridin-1(2H)-one (100 mg, 0.39 mmol), 3,5-bis(trifluoromethyl)benzoyl chloride (107 mg, 0.39 mmol) in DCM (2 mL) stirred under nitrogen was added TEA (117 mg, 1.16 mmol) dropwise. The reaction mixture was stirred at RT for 1 hour. The mixture was diluted with water (5 mL), extracted with DCM (5 mL * 2), washed with brine (10 mL). The organic layer was concentrated. The residue was purified by Prep-HPLC (5-95% ACN in water with 0.1 % NH₄HCO₃) to afford N-(1-(1-(5-oxo-5,6,7,8-tetrahydro-2,6-naphthyridin-3-yl)-1H-1,2,4-triazol-5-yl)ethyl)-3,5-bis(trifluoromethyl)benzamide (I-23) (35 mg, 18% yield) as a white solid. LC-MS (ES) m/z = 499.0 (M+H)⁺. 1H NMR (400 MHz, DMSO-d₆) δ [ppm] 9.54 (d, J = 6.8 Hz, 1H), 8.59 (s, 1H),8.46-8.41 (m, 3H), 8.31 (s, 1H), 8.18 (s, 1H), 8.08 (s, 1H), 6.06-6.02 (m, 1H), 3.43-3.46 (m, 2H), 2.99-2.94 (m,2H), 1.66 (d, J = 6.8 Hz, 3H).

### Example 20 : Synthesis of N-(1-(1-(1-oxo-2,3-dihydro-1H-pyrrolo[3,4-c]pyridin-6-yl)-1H-1,2,4-triazol-5-yl)ethyl)-3,5-bis(trifluoromethyl)benzamide (I-22) :

Step 1 : A solution of Methyl 5-bromo-2-chloropyridine-4-carboxylate (5.0 g, 19.96 mmol), CuCN (1.88 g, 20.96 mmol) in DMA (50 mL) under nitrogen was stirred at 170 °C for 2 hours. The mixture was diluted with water (250 mL), extracted with EtOAc (50 mL * 2), washed with brine (50 mL). The organic layer was concentrated. The residue was purified by chromatography on silica gel (PE/EtOAc = 5/1) to afford the product methyl 2-chloro-5-cyanoisonicotinate (1.8 g, 98% yield) as a colorless oil. LC-MS (ES) m/z = 197.1 (M+H)⁺.

Step 2: A solution of methyl 2-chloro-5-cyanoisonicotinate (1000 mg, 5.09 mmol), (*tert*-butoxy)-*N'*-[(*tert-*butoxy)carbonyl]carbohydrazide (1430 mg, 6.10 mmol), Cs₂CO₃ (3315 mg, 10.17 mmol) and BrettphosPdG3 (231 mg, 0.25 mmol) in 1,4-dioxane(20 mL) was stirred under nitrogen at 100 °C for 4 hours. The mixture was diluted with water (20 mL), extracted with EtOAc (20 mL * 2), washed with brine (20 mL). The organic layer was concentrated. The residue was purified by chromatography on silica gel (PE/EtOAc = 10/1) to afford the product di-tert-butyl 1-(5-cyano-4-(methoxycarbonyl)pyridin-2-yl)hydrazine-1,2-dicarboxylate (800 mg, 37% yield) as yellow solid. LC-MS (ES) m/z = 293.3 (M-Boc)⁺.

Step 3: A solution of di-tert-butyl 1-(5-cyano-4-(methoxycarbonyl)pyridin-2-yl)hydrazine-1,2-dicarboxylate (500 mg, 1.27 mmol), PtO₂ (29 mg, 0.13 mmol) in MeOH (5 mL). The reaction mixture was stirred at rt for 16 hours. The mixture was filtered and the filtrate was concentration to afford di-tert-butyl 1-(1-oxo-2,3-dihydro-1H-pyrrolo[3,4-c]pyridin-6-yl)hydrazine-1,2-dicarboxylate (480 mg, 100% yield) as a yellow oil. LC-MS (ES) m/z = 365.3 (M+H)⁺.

Step 4. A solution of di-tert-butyl 1-(1-oxo-2,3-dihydro-1H-pyrrolo[3,4-c]pyridin-6-yl)hydrazine-1,2-dicarboxylate (500 mg, 1.36 mmol) in HCI/EA (5 mL) was stirred at RT for 4 hours. The mixture was concentrated to afford 6-hydrazineyl-2,3-dihydro-1H-pyrrolo[3,4-c]pyridin-1-one (300 mg, 114% yield) as a white solid. LC-MS (ES) m/z = 165.0 (M+H)⁺.

Step 5: A solution of 6-hydrazineyl-2,3-dihydro-1H-pyrrolo[3,4-c]pyridin-1-one (300 mg, 1.83 mmol), tert-butyl (E)-(1-(((dimethylamino)methylene)amino)-1-oxopropan-2-yl)carbamate (534 mg, 2.19288 mmol) in HOAc(5 mL) was stirred at RT for 2 hours. Then the mixture was stirred at 60 °C for 4 hours. The mixture was diluted with water (10 mL), extracted with EA (10 mL * 2), washed with brine (10 mL). The organic layer was concentrated. The residue was purified by chromatography on silica gel (PE/EtOAc = 5/1) to afford tert-butyl (1-(1-(1-oxo-2,3-dihydro-1H-pyrrolo[3,4-c]pyridin-6-yl)-1H-1,2,4-triazol-5-yl)ethyl)carbamate (200 mg, 29% yield) as a white solid. LC-MS (ES) m/z = 345.3 (M+H)⁺.

Step 6: A solution of tert-butyl (1-(1-(1-oxo-2,3-dihydro-1H-pyrrolo[3,4-c]pyridin-6-yl)-1H-1 ,2,4-triazol-5-yl)ethyl)carbamate (200 mg, 0.58 mmol) in HCI/EA (2 mL) was stirred at RT for 4 hours. The mixture was concentrated to afford 6-(5-(1-aminoethyl)-1*H*-1,2,4-triazol-1-yl)-2,3-dihydro-1H-pyrrolo[3,4-c]pyridin-1-one (150 mg, 29% yield) as a white solid. LC-MS (ES) m/z = 245.0 (M+H)⁺.

Step 7: To a solution of 6-(5-(1-aminoethyl)-1H-1,2,4-triazol-1-yl)-2,3-dihydro-1H-pyrrolo[3,4-c]pyridin-1-one (150 mg, 0.61 mmol), 3,5-bis(trifluoromethyl)benzoyl chloride (188 mg, 0.68 mmol) in DCM (5 mL) stirred under nitrogen at RT was added TEA (187 mg, 1.8423 mmol) dropwise. The reaction mixture was stirred at RT for 1 hour. The mixture was diluted with water (5 mL), extracted with DCM (5 mL * 2), washed with brine (10 mL). The organic layer was concentrated. The residue was purified by Prep-HPLC (5-95 % ACN in water with 0.1 % NH₄HCO₃) to afford *N*-(1-(1-(1-oxo-2,3-dihydro-1H-pyrrolo[3,4-c]pyridin-6-yl)-1H-1 ,2,4-triazol-5-yl)ethyl)-3,5-bis(trifluoromethyl)benzamide (I-22) (40 mg, 10% yield) as a white solid. LC-MS (ES) m/z = 485.0 (M+H)⁺. ¹H NMR (400 MHz, DMSO-*d*₆) δ [ppm] 9.56 (d, *J = 6.8* Hz, 1H), 9.18 (s, 1H), 8.85 (s, 1H), 8.41 (s, 2H), 8.31 (s, 1H), 8.21 (s, 1H), 7.96 (s, 1H), 6.10-6.07(m, 1H), 4.53 (s, 2H), 1.68 (d, *J* = 6.8 Hz, 3H).

### Example 21 : Synthesis of N-(1-(1-(2-oxo-2,3-dihydro-1H-pyrrolo[3,2-c]pyridin-6-yl)-1H-1,2,4-triazol-5-yl)ethyl)-3,5-bis(trifluoromethyl)benzamide (I-21) :

Step 1 : To a solution of 6-chloro-1H-pyrrolo[3,2-c]pyridine (1.00 g, 6.60 mmol) in DMF (10 mL) was added NaH (0.53 g, 13.2 mmol, 60% in oil) at 0 °C. The mixture was stirred at 0 °C for 0.5h. Then benzenesulfonyl chloride (1.28 g, 7.26 mmol) was added to the mixture at 0 °C. The mixture was stirred at 25 °C for 3 hrs. The mixture was quenched with water (30 mL) and the mixture was filtered. The filter cake was washed with water (10 mL * 3) and dried to afford 6-chloro-1-(phenylsulfonyl)-1H-pyrrolo[3,2-c]pyridine (1.34 g, 70% yield) as a white solid. LC-MS (ES) m/z (M+H)⁺ = 293.3.

Step 2: A mixture of 6-chloro-1-(phenylsulfonyl)-1H-pyrrolo[3,2-c]pyridine (1.32 g, 4.50 mmol), tert-butyl carbamate (1.05 g, 9.00 mmol), Pd(OAc)₂ (0.10 g, 0.45 mmol), XPhos (0.21 g, 0.45 mmol) and Cs₂CO₃ (2.93 g, 9.00 mmol) in 1,4-dioxane (15 mL) was stirred at 100 °C for 4 hrs. The mixture was filtered and the filtrate was concentrated. The residue was purified by column chromatography (DCM/EtOAc = 4/1) to afford tert-butyl (1-(phenylsulfonyl)-1H-pyrrolo[3,2-c]pyridin-6-yl)carbamate (1.16 g, 69% yield) as a yellow solid. LC-MS (ES) m/z (M+H)⁺ = 374.0.

Step 3: To a solution of tert-butyl (1-(phenylsulfonyl)-1H-pyrrolo[3,2-c]pyridin-6-yl)carbamate (1.11 g, 3.00 mmol) in DMF (10 mL) was added NaH (0.24 g, 6.00 mmol, 60% in oil) at 0 °C. The mixture was stirred at 0 °C for 0.5 h. (aminooxy)diphenylphosphine oxide (1.06 g, 4.50 mmol) was added to the mixture at 0 °C. The mixture was stirred at 25 °C for 3 hrs. The mixture was quenched with water (30 mL) and extracted with EtOAc (50 mL * 3). The organic layer was washed with brine (50 mL * 3). The organic layer was concentrated. The residue was purified by column chromatography (DCM) to afford tert-butyl 1-(1-(phenylsulfonyl)-1H-pyrrolo[3,2-c]pyridin-6-yl)hydrazine-1-carboxylate (1.05 g, 90% yield) as a yellow solid. LC-MS (ES) m/z (M+H)⁺ = 389.1.

Step 4: To a solution of tert-butyl 1-(1-(phenylsulfonyl)-1H-pyrrolo[3,2-c]pyridin-6-yl)hydrazine-1-carboxylate (950 mg, 2.46 mmol) in 1,4-dioxane (10 mL) was added dioxane/HCl (4M, 20 mL) at 0 °C. The mixture was stirred at 30 °C for 2 hrs. The mixture was filtered and the filter cake was dried to afford 6-hydrazineyl-1-(phenylsulfonyl)-1H-pyrrolo[3,2-c]pyridine hydrogen chloride (597 mg, 75% yield) as a white solid. LC-MS (ES) m/z (M+H)⁺ = 289.0.

Step 5: A mixture of 6-hydrazineyl-1-(phenylsulfonyl)-1H-pyrrolo[3,2-c]pyridine hydrogen chloride (597 mg, 1.84 mmol) and tert-butyl N-[2-[(E)-dimethylaminomethyleneamino]-1-methyl-2-oxoethyl]carbamate (894 mg, 3.68 mmol) in HOAc (15 mL) was stirred at 25 °C for 3 hrs. The mixture was warmed to 70 °C and stirred for 4 hrs. The mixture was concentrated. The residue was dissolved in EtOAc (20 mL) and adjusted PH = 9-10 with sat.Na₂CO₃. The mixture was extracted with EtOAc (30 mL * 3) and the organic layer was concentrated. The residue was purified by column chromatography (DCM/ EtOAc = 1/1) to afford tert-butyl (1-(1-(1-(phenylsulfonyl)-1H-pyrrolo[3,2-c]pyridin-6-yl)-1H-1,2,4-triazol-5-yl)ethyl)carbamate (850 mg, 99% yield) as a yellow solid. LC-MS (ES) m/z (M+H)⁺ = 469.4.

Step 6: A mixture of tert-butyl (1-(1-(1-(phenylsulfonyl)-1*H*-pyrrolo[3,2-c]pyridin-6-yl)-1*H*-1,2,4-triazol-5-yl)ethyl)carbamate (900 mg, 1.92 mmol) in MeOH (19.2 mL) and NaOH (2M in water, 19.2 mL) was stirred at 25 °C for 2 hrs. The mixture was dissolved with water (20 mL) and extracted with EtOAc (20 mL * 3). The organic layer was concentrated. The residue was purified by column chromatography (DCM/EtOAc = 1/2) to afford *tert*-butyl (1-(1-(1*H*-pyrrolo[3,2-c]pyridin-6-yl)-1H-1,2,4-triazol-5-yl)ethyl)carbamate (425 mg, 67% yield) as a white solid. LC-MS (ES) m/z (M+H)⁺ = 329.3.

Step 7: To a solution of te*r*t-butyl (1-(1-(1*H*-pyrrolo[3,2-c]pyridin-6-yl)-1*H*-1 ,2,4-triazol-5-yl)ethyl)carbamate (425 mg, 1.29 mmol) in tBuOH (10 mL) was added pyridinium tribromide (1243 mg, 3.88 mmol) in portion. The mixture was stirred at 25 °C for 16 hrs. The mixture was diluted with EtOAc (30 mL) and water (30 mL). The mixture was extracted with EtOAc (30 mL * 3) and the organic layer was concentrated to afford *tert*-butyl (1-(1-(3,3-dibromo-2-oxo-2,3-dihydro-1*H*-pyrrolo[3,2-c]pyridin-6-yl)-1*H*-1 ,2,4-triazol-5-yl)ethyl)carbamate (600 mg, 92% yield) as a yellow solid. LC-MS (ES) m/z (M+H)⁺ = 501.2.

Step 8: A mixture of *tert*-butyl (1-(1-(3,3-dibromo-2-oxo-2,3-dihydro-1*H*-pyrrolo[3,2-c]pyridin-6-yl)-1*H-*1,2,4-triazol-5-yl)ethyl)carbamate (600 mg, 1.19 mmol) in HOAc (10 mL) was added Zn powder (782 mg, 11.95 mmol) and the mixture was stirred at 25 °C for 1.5 hrs. The mixture was diluted with MeOH and filtered. The filtrate was concentrated and the residue was diluted with EtOAc (30 mL). The mixture was adjusted to PH = 9-10 with sat.Na₂CO₃. The mixture was extracted with EtOAc (30 mL * 3) and the organic layer was concentrated. The residue was purified by column chromatography (DCM/EtOAc = 1/1) to afford tert-butyl (1-(1-(2-oxo-2,3-dihydro-1H-pyrrolo[3,2-c]pyridin-6-yl)-1H-1,2,4-triazol-5-yl)ethyl)carbamate (380 mg, 92% yield) as a white solid. LC-MS (ES) m/z (M+H)+ = 345.1. ¹H NMR (400 MHz, CDCl₃) δ [ppm] 8.36 (s, 1H), 8.20 (s, 1H), 7.91 (s, 1H), 7.41 (s, 1H), 5.94-5.92 (m, 1H), 5.68 (d, J = 8.0 Hz, 1H), 3.60 (s, 2H), 1.56 (d, J = 6.8 Hz, 3H), 1.41 (s, 9H).

Step 9: To a solution of tert-butyl (1-(1-(2-oxo-2,3-dihydro-1H-pyrrolo[3,2-c]pyridin-6-yl)-1H-1,2,4-triazol-5-yl)ethyl)carbamate (230 mg, 0.67 mmol) in 1,4-dioxane (4 mL) was added 1,4-dioxane/HCl (4M, 5 mL) at 0 °C. The mixture was stirred at 25 °C for 2 hrs. The mixture was filtered and the filter cake was dried to afford 6-(5-(1-aminoethyl)-1H-1,2,4-triazol-1-yl)-1,3-dihydro-2H-pyrrolo[3,2-c]pyridin-2-one hydrogen chloride (180 mg, 96% yield) as a yellow solid. LC-MS (ES) m/z (M+H)⁺ = 245.3.

Step 10: To a solution of 6-(5-(1-aminoethyl)-1H-1 ,2,4-triazol-1-yl)-1 ,3-dihydro-2H-pyrrolo[3,2-c]pyridin-2-one hydrogen chloride (180 mg, 0.64 mmol) in DCM (4 mL) was added TEA (195 mg, 1.92 mmol) at 0 °C. 3,5-bis(trifluoromethyl)benzoyl chloride (89 mg, 0.32 mmol) was added to the mixture and the mixture was stirred at 0 °C for 30 mins. The mixture was quenched with water (10 mL) and extracted with DCM (20 mL * 3). The organic layer was concentrated. The residue was purified by column chromatography (CH₂Cl₂/EtOAc = 2/1) to afford 160 mg crude. The crude was purified by reverse phase column (ACN/water = 3/1) to afford *N*-(1-(1-(2-oxo-2,3-dihydro-1*H*-pyrrolo[3,2-c]pyridin-6-yl)-1*H*-1,2,4-triazol-5-yl)ethyl)-3,5-bis(trifluoromethyl)benzamide (I-21) (92.2 mg, 30% yield) as a white solid. LC-MS (ES) m/z = 485.0 (M+H)⁺. ¹H NMR (400 MHz, DMSO-*d*₆) δ [ppm] 11.07 (s, 1H), 9.55 (d, J = 6.8 Hz, 1H), 8.45 (s, 2H), 8.31 (s, 1H), 8.21 (s, 1H), 8.13 (s, 1H), 7.24 (s, 1H), 6.13-6.06 (m, 1H), 3.61 (s, 2H), 1.65 (d, J = 6.8 Hz, 3H).

### Example 22 : Synthesis of N-(1-(1-(2-oxo-1,2,3,4-tetrahydro-1,6-naphthyridin-7-yl)-1H-1,2,4-triazol-5-yl)ethyl)-3,5-bis(trifluoromethyl)benzamide (I-20) :

Step 1 : A mixture of 4-amino-6-chloro-pyridine-3-carbaldehyde (5.00 g, 31.90 mmol) and ethyl triphenylphosphorany lideneacetate (13.34 g, 38.28 mmol) in THF (60 mL) was stirred at 85 °C for 16 hours. The reaction was poured into water (100 mL) and extracted with EtOAc (100 mL). The organic phases was concentrated. The residue was purified by flash chromatography on silica gel (PE/EtOAc = 2/1) to afford ethyl (Z)-3-(4-amino-6-chloropyridin-3-yl)acrylate (5.50 g, 76% yield) as a yellow solid. LC-MS (ES) m/z (M+H)⁺ = 227.1.

Step 2: A mixture of ethyl (Z)-3-(4-amino-6-chloropyridin-3-yl)acrylate (5.00 g, 22.10 mmol) and PtO₂ (0.50 g, 2.21 mmol) in MeOH (50 mL) was stirred at 30 °C for 2 hours under H₂ (1 ATM). The reaction mixture was filtered through celite. The filter cake was washed with MeOH (50 mL). The filtrate was concentrated to afford ethyl 3-(4-amino-6-chloropyridin-3-yl)propanoate (5.00 g, 99% yield) as brown oil. LC-MS (ES) m/z (M+H)⁺ = 229.0.

Step 3: A mixture of ethyl 3-(4-amino-6-chloropyridin-3-yl)propanoate (5.00 g, 21.90 mmol) and MeONa (2.37 g, 43.80 mmol) in MeOH (50 mL) was stirred at 80 °C for 1 hour. The reaction mixture was diluted with ice-water (100 mL) and EtOAc (100 mL*5). The combined organic phase was concentrated to afford 7-chloro-3,4-dihydro-1 ,6-naphthyridin-2(1H)-one (3.90 g, 98% yield) as a white solid. ¹H NMR (400 MHz, DMSO-*d*₆) δ [ppm] 10.72 (br s, 1H), 8.10 (s, 1H), 6.84 (s, 1H), 2.89 (t, *J = 7.6* Hz, 2H), 2.53 (t, *J = 7.6* Hz, 2H).

Step 4 : A mixture of 7-chloro-3,4-dihydro-1,6-naphthyridin-2(1H)-one (1.00 g, 5.50 mmol), di-tert-butyl hydrazine-1,2-dicarboxylate (2.56 g, 11.00 mmol), Cs₂CO₃ (3.58 g, 11.00 mmol) and BrettphosPdG3 (0.50 g, 0.55 mmol) in 1,4-dioxane (15 mL) was stirred at 100 °C for 16 hours under N₂. The reaction mixture was diluted with water (50 mL) and extracted with EtOAc (50 mL * 2). The combined organic phase was concentrated. The residue was purified by column chromatography on silica gel (DCM/MeOH = 20/1) to afford di-tert-butyl 1-(2-oxo-1,2,3,4-tetrahydro-1,6-naphthyridin-7-yl)hydrazine-1,2-dicarboxylate (1.20 g, 58% yield) as a white solid. LC-MS (ES) m/z (M+H)⁺ = 379.2.

Step 5: A mixture of di-tert-butyl 1-(2-oxo-1,2,3,4-tetrahydro-1,6-naphthyridin-7-yl)hydrazine-1,2-dicarboxylate (1.20 g, 3.20 mmol) in HCl/dioxane (2M, 20 mL) was stirred at 30 °C for 2 hours. The reaction mixture was concentrated to afford 7-hydrazineyl-3,4-dihydro-1,6-naphthyridin-2(1*H*)-one dihydrochloride (0.80 g, 100% yield) as a white solid. LC-MS (ES) m/z (M+H)⁺ = 179.0.

Step 6: A mixture of 7-hydrazineyl-3,4-dihydro-1,6-naphthyridin-2(1*H*)-one dihydrochloride (800 mg, 3.19 mmol) and tert-butyl N-[2-[(E)-dimethylaminomethyleneamino]-1-methyl-2-oxo-ethyl]carbamate (1008 mg, 4.14 mmol) in AcOH (20 mL) was stirred at 50 °C for 24 hours. The reaction mixture was concentrated. The residue was purified by column chromatography on silica gel (DCM/MeOH = 20/1) to afford *tert*-butyl (1-(1-(2-oxo-1,2,3,4-tetrahydro-1,6-naphthyridin-7-yl)-1H-1,2,4-triazol-5-yl)ethyl)carbamate (500 mg, 44% yield) as a white solid. LC-MS (ES) m/z (M+H)⁺ = 359.1. ¹H NMR (400 MHz, DMSO-*d*₆) δ [ppm] 10.72 (s, 1H), 8.24 (s, 1H), 8.06 (s, 1H), 7.41 (d, *J* = 7.2 Hz, 0.8H), 7.27 (s, 1H), 7.11 (s, 0.2H), 5.62-5.45 (m, 1H), 2.96 (t, *J =* 7.2 Hz, 2H), 2.57 (t, *J =* 7.2 Hz, 2H), 1.40 (d, *J* = 6.4 Hz, 3H), 1.30-1.05 (m, 9H).

Step 7: A mixture of Tert-butyl N-{1-[2-(2-oxo-3,4-dihydro-1H-1,6-naphthyridin-7-yl)-1,2,4-triazol-3-yl]ethyl}carbamate (200 mg, 0.56 mmol) in HCl/dioxane (2M, 4 mL) was stirred at 15 °C for 2 hours. The reaction mixture was concentrated to afford 7-(5-(1-aminoethyl)-1*H*-1,2,4-triazol-1-yl)-3,4-dihydro-1,6-naphthyridin-2(1*H*)-one hydrochloride (160 mg, 97% yield) as a white solid. LC-MS (ES) m/z (M+H)⁺ = 259.1

Step 8 : To a mixture of 7-(5-(1-aminoethyl)-1*H*-1,2,4-triazol-1-yl)-3,4-dihydro-1,6-naphthyridin-2(1*H*)-one hydrochloride (160 mg, 0.54 mmol) and TEA (165 mg, 1.63 mmol) in DCM (5 mL) was added 3,5-Bis(trifluoromethyl)benzoyl chloride (180 mg, 0.65 mmol) at 0 °C. The reaction was stirred at 0 °C for 10 minutes. The reaction mixture was diluted with ice-water (10 mL) and extracted DCM (10 mL * 2). The combined organic phase was concentrated. The residue was purified by column chromatography on silica gel (DCM/MeOH = 20/1) to afford *N*-(1-(1-(2-oxo-1,2,3,4-tetrahydro-1,6-naphthyridin-7-yl)-1H-1,2,4-triazol-5-yl)ethyl)-3,5-bis(trifluoromethyl)benzamide (I-20) (200 mg, 74% yield) as a white solid. LC-MS (ES) m/z (M+H)⁺ = 499.0. ¹H NMR (400 MHz, DMSO-*d*₆) δ [ppm] 10.72 (s, 1H), 9.53 (d, *J* = 6.8 Hz, 1H), 8.44 (s, 2H), 8.28 (s, 1H), 8.23 (s, 1H), 8.10 (s, 1H), 7.28 (s, 1H), 6.07-6.00 (m, 1H), 2.97-2.88 (m, 2H), 2.61-2.51 (m, 2H), 1.63 (d, *J =* 6.8 Hz, 3H).

### Example 23 : Synthesis of N-(1-(1-(1H-pyrazolo[3,4-d]pyrimidin-6-yl)-1H-1,2,4-triazol-5-yl)ethyl)-3,5-bis(trifluoromethyl)benzamide (I-35) :

Step 1 : A mixture of 6-chloro-1H-pyrazolo[5,4-d]pyrimidine (890 mg, 5.76 mmol), (Boc)₂O (3.77 g, 17.28 mmol) and DMAP (70 mg, 0.58 mmol) in DCM (10 mL) was stirred at 25 °C for 2 hrs. The mixture was concentrated and purified by column chromatography (PE/EtOAc = 3/1) to afford *tert*-butyl 6-chloro-1*H-*pyrazolo[3,4-*d*]pyrimidine-1-carboxylate (1.33 g, 91% yield) as a yellow solid. ¹H NMR (400 MHz, DMSO-*d*₆) δ 9.36 (s, 1H), 8.64 (s, 1H), 1.64 (s, 9H).

Step 2: A mixture of tert-butyl 6-chloro-1H-pyrazolo[3,4-d]pyrimidine-1-carboxylate (1.23 g, 4.80 mmol), di-*tert*-butyl hydrazine-1,2-dicarboxylate (2.25 g, 9.60 mmol), Cs₂CO₃ (3.13 g, 9.60 mmol) and BrettPhosPdG3 (440 mg, 0.48 mmol) in dioxane (25 mL) was stirred at 100 °C for 3 hrs under N₂. The mixture was filtered and the filtrate was concentrated. The residue was purified by column chromatography (EtOAc) to afford t*ert*-butyl 6-(2-(*tert*-butoxycarbonyl)hydrazineyl)-1*H*-pyrazolo[3,4-*d*]pyrimidine-1-carboxylate (887 mg, 52% yield) as a yellow solid. LC-MS (ES) m/z (M+H)⁺ = 351.3.

Step 3: A mixture of t*ert*-butyl 6-(2-(*tert*-butoxycarbonyl)hydrazineyl)-1*H*-pyrazolo[3,4-d]pyrimidine-1-carboxylate (787 mg, 2.23 mmol) in EtOAc (5 mL) was added HCl/ EtOAc (10 mL, 4 M) at 0 °C. The mixture was stirred at 25 °C for 5 hrs. The mixture was filtered and the filter cake was dried to afford 6-hydrazineyl-1*H*-pyrazolo[3,4-*d*]pyrimidine hydrogen chloride (399 mg, 96% yield) as a yellow solid. LC-MS (ES) m/z (M+H)⁺ = 151.0.

Step 4: A mixture of 6-hydrazineyl-1*H*-pyrazolo[3,4-*d*]pyrimidine hydrogen chloride (400 mg, 2.14 mmol) and *tert*-butyl *N*-[2-[(*E*)-dimethylaminomethyleneamino]-1-methyl-2-oxo-ethyl]carbamate (782 mg, 3.22 mmol) in HOAc (10 mL) was stirred at 25 °C for 2 hrs. The mixture was warmed to 70 °C and stirred for 3 hrs. The mixture was concentrated and the residue was diluted with EtOAc (30 mL). The mixture was adjusted to pH = 9-10 with *sat*. Na₂CO₃. The mixture was extracted with EtOAc (30 mL * 3). The organic layer was concentrated. The residue was purified by column chromatography (DCM/ EtOAc = 1/1) to afford *tert*-butyl (1-(1-(1*H*-pyrazolo[3,4-*d*]pyrimidin-6-yl)-1*H*-1,2,4-triazol-5-yl)ethyl)carbamate (180 mg, 25% yield) as a yellow solid. LC-MS (ES) m/z (M+H)⁺ = 331.1.

Step 5: To a mixture of tert-butyl (1-(1-(1*H*-pyrazolo[3,4-*d*]pyrimidin-6-yl)-1*H*-1,2,4-triazol-5-yl)ethyl)carbamate (160 mg, 0.48 mmol) in EtOAc (5 mL) was added HCl/ EtOAc (5 mL, 4 M) at 0 °C. The mixture was stirred at 25 °C for 4 hrs. The mixture was filtered. The filter cake was dried to afford 1-(1-(1*H*-pyrazolo[3,4-d]pyrimidin-6-yl)-1H-1,2,4-triazol-5-yl)ethan-1-amine (100 mg, 78% yield) as a yellow solid. LC-MS (ES) m/z (M+H)⁺ = 231.0.

Step 6: To a mixture of 1-(1-(1*H*-pyrazolo[3,4-*d*]pyrimidin-6-yl)-1*H*-1,2,4-triazol-5-yl)ethan-1-amine (80 mg, 0.30 mmol) in DCM (2 mL) was added TEA (91 mg, 0.90 mmol) at 0 °C. Then 3,5-bis(trifluoromethyl)benzoyl chloride (83 mg, 0.30 mmol) was added to the mixture at 0 °C. The mixture was stirred at 0 °C for 0.5 hr. The mixture was quenched with water (20 mL), extracted with EtOAc (20 mL * 3). The organic layer was washed with brine (20 mL * 3) and concentrated. The residue was purified by reverse flash (5-95% ACN in water) to afford *N*-(1-(1-(1*H*-pyrazolo[3,4-d]pyrimidin-6-yl)-1*H*-1,2,4-triazol-5-yl)ethyl)-3,5-bis(trifluoromethyl)benzamide (I-35) (42.3 mg, 30% yield) as a white solid. LC-MS (ES) m/z (M+H)⁺ = 471.0. ¹H NMR (400 MHz, DMSO-*d*₆) δ [ppm] 14.41 (s, 1H), 9.57 (d, J = 6.8 Hz, 1H), 9.45 (s, 1H), 8.46 (s, 1H), 8.35 (s, 2H), 8.28 (s, 1H), 8.20 (s, 1H), 6.17 - 6.10 (m, 1H), 1.70 (d, J = 6.8 Hz, 3H).

### Example 24 : Synthesis of N-(1-(1-(3-Amino-[1,2,4]triazolo[4,3-a]pyrazin-6-yl)-1H-1,2,4-triazol-5-yl)ethyl)-N-methyl-3,5-bis(trifluoromethyl)benzamide (I-38) :

Step 1 : A solution of 5-bromo-1H-pyrazin-2-one hydrazone (600 mg, 3.17 mmol) and 5101-13c (1.6 g, 6.35 mmol) in AcOH (9 mL) was stirred at 25 °C for 2 hrs. The solvent was removed in vacuo. The residue was dissolved in EtOAc (10 mL) and washed with NaHCO₃ (10 mL * 2). The organic layer was dried over Na₂SO₄ and concentrated under reduced pressure. The residue was purified by chromatography on silica gel (PE/EtOAc = 10/1) to afford *tert*-butyl (1-(1-(5-bromopyrazin-2-yl)-1*H-*1,2,4-triazol-5-yl)ethyl)carbamate (890 mg, 73% yield) as a yellow solid. LC-MS (ES) m/z = 327.0 (M+H-tBu)⁺.

Step 2 : A solution of *tert*-butyl (1-(1-(5-bromopyrazin-2-yl)-1*H*-1,2,4-triazol-5-yl)ethyl)carbamate (890 mg, 2.32 mmol) and N₂H₄.H₂O (372 mg, 11.61 mmol, 98%) in EtOH (9 mL) was stirred at 80 °C for 2 hrs. The solvent was removed in vacuo. The residue was poured into water (2 mL) and filtered. The filter cake was dried to afford tert-butyl (*Z*)-(1-(1-(5-hydrazineylidene-4,5-dihydropyrazin-2-yl)-1*H*-1,2,4-triazol-5-yl)ethyl)carbamate (530 mg, 68% yield) as a yellow solid. LC-MS (ES) m/z = 335.2 (M+H)⁺.

Step 3: To a solution of tert-butyl (Z)-(1-(1-(5-hydrazineylidene-4,5-dihydropyrazin-2-yl)-1H-1,2,4-triazol-5-yl)ethyl)carbamate (480 mg, 1.44 mmol) and NaOAc (294 mg, 3.59 mmol) in MeOH (10 mL) was added cyanic bromide (152 mg, 1.44 mmol). The reaction was stirred at 25 °C for 4 hrs. The solvent was removed in vacuo. The residue was purified by chromatography on silica gel (DCM/MeOH = 10/1) to afford tert-butyl (1-(1-(3-amino-[1,2,4]triazolo[4,3-a]pyrazin-6-yl)-1H-1,2,4-triazol-5-yl)ethyl)carbamate (482 mg, 93% yield) as a yellow solid. LC-MS (ES) m/z = 360.2 (M+H)⁺.

Step 4 : To a solution of tert-butyl (1-(1-(3-amino-[1,2,4]triazolo[4,3-a]pyrazin-6-yl)-1H-1,2,4-triazol-5-yl)ethyl)carbamate (480 mg, 1.34 mmol) in EtOAc (5 mL) was added HCl/EtOAc (10 mL). The reaction was stirred at 30 °C for 2 hrs. The solvent was removed in vacuo to afford 6-(5-(1-(methylamino)ethyl)-1H-1,2,4-triazol-1-yl)-[1,2,4]triazolo[4,3-a]pyrazin-3-amine (457 mg, 92% yield) as a yellow solid. LC-MS (ES) m/z = 260.2 (M+H)⁺.

Step 5 : To a solution of 6-(5-(1-(methylamino)ethyl)-1H-1,2,4-triazol-1-yl)-[1,2,4]triazolo[4,3-a]pyrazin-3-amine (450 mg, 1.22 mmol) and TEA (741 mg, 7.32 mmol) in DCM (5 mL) was added 3,5-bis(trifluoromethyl)benzoyl chloride (405 mg, 1.46 mmol) at 0 °C. The reaction was stirred at 0 °C for 1 hr. The reaction was quenched with ice-water (10 mL), extracted with EtOAc (20 mL * 3). The organic layer was washed with brine (20 mL), dried over Na₂SO₄, filtered and concentrated under reduced pressure. The residue was purified by chromatography on silica gel (DCM/MeOH = 10/1) to afford N-(1-(1-(3-amino-[1,2,4]triazolo[4,3-a]pyrazin-6-yl)-1H-1,2,4-triazol-5-yl)ethyl)-N-methyl-3,5-bis(trifluoromethyl)benzamide (I-38) (230 mg, 37% yield) as a yellow solid. LC-MS (ES) m/z = 500.0 (M+H)⁺. ¹H NMR (400 MHz, DMSO-*d*₆) δ [ppm] 9.03-8.42 (m, 2H), 8.26 (s, 1H), 8.20-7.94 (m, 1H), 7.84 (s, 2H), 7.06-7.01 (m, 2H), 6.24-5.86 (m, 1H), 3.21-2.75 (m, 3H), 1.66 (d, J = 6.8 Hz, 3H).

With appropriate modification of the starting materials, the procedures given in the synthesis descriptions were used to obtain further compounds I. The compounds obtained in this manner are listed in Table I below, together with physical data.

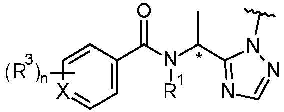

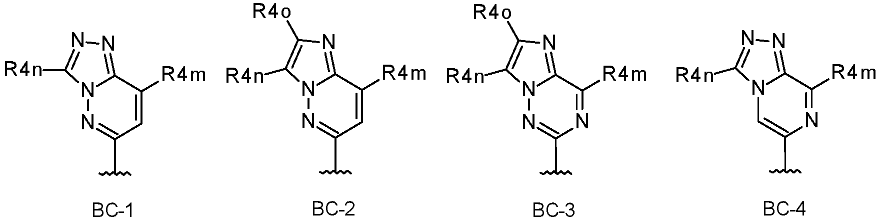

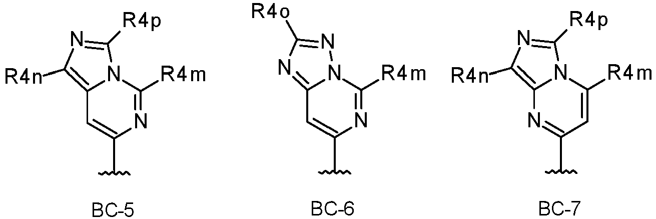

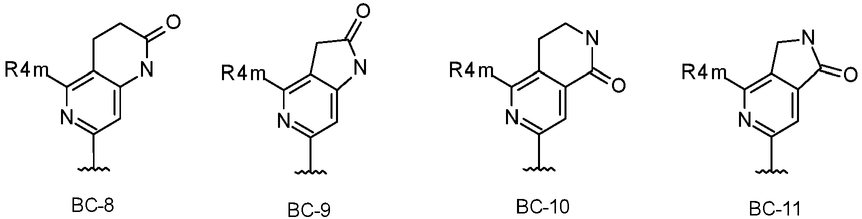

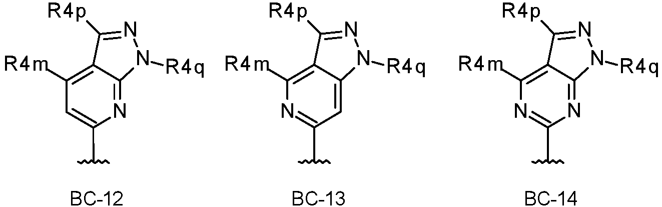

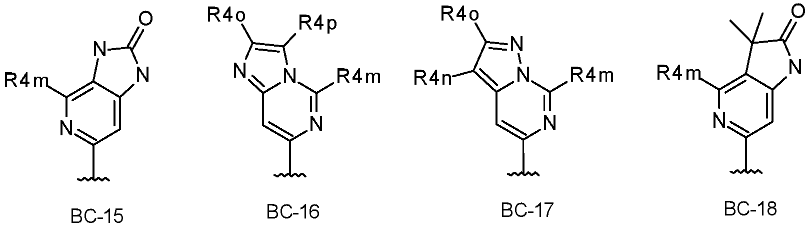

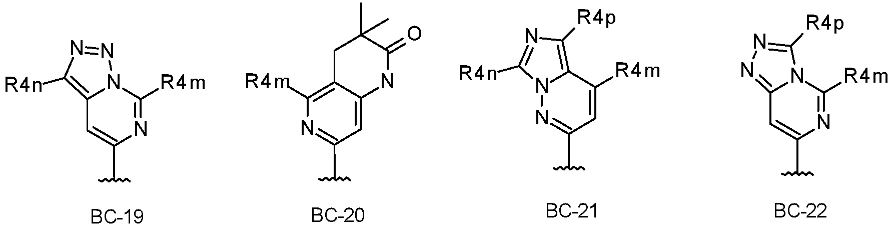

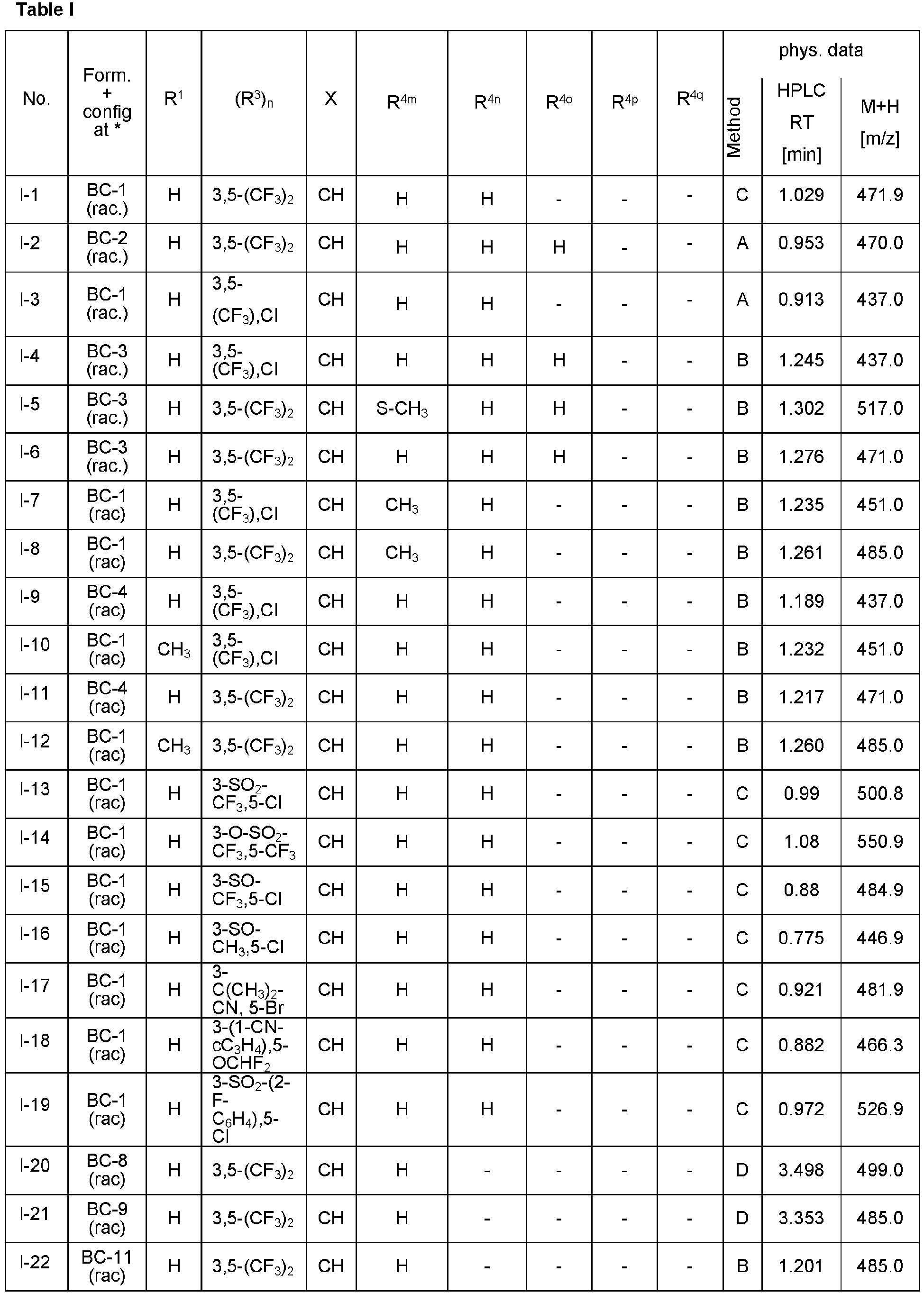

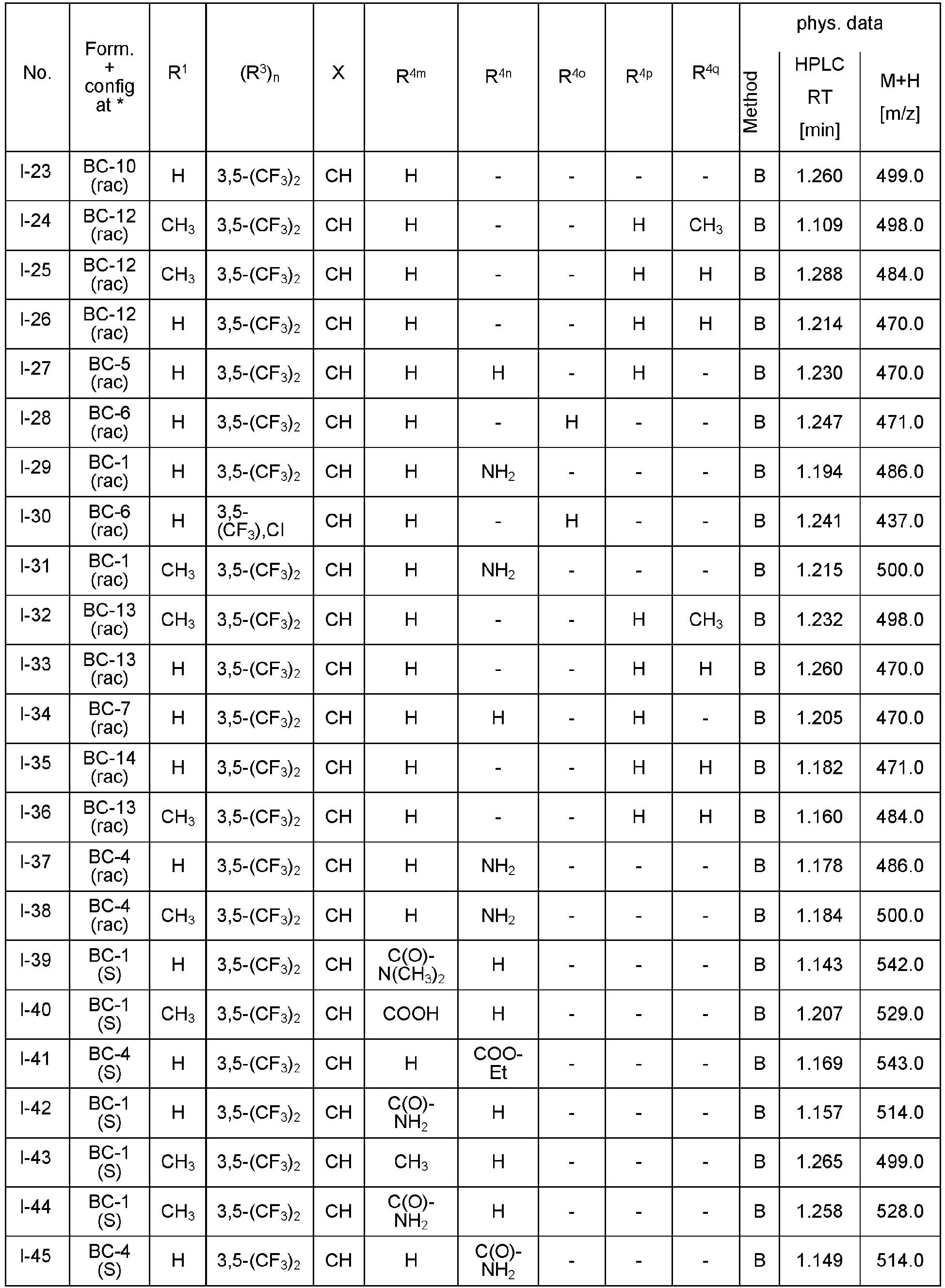

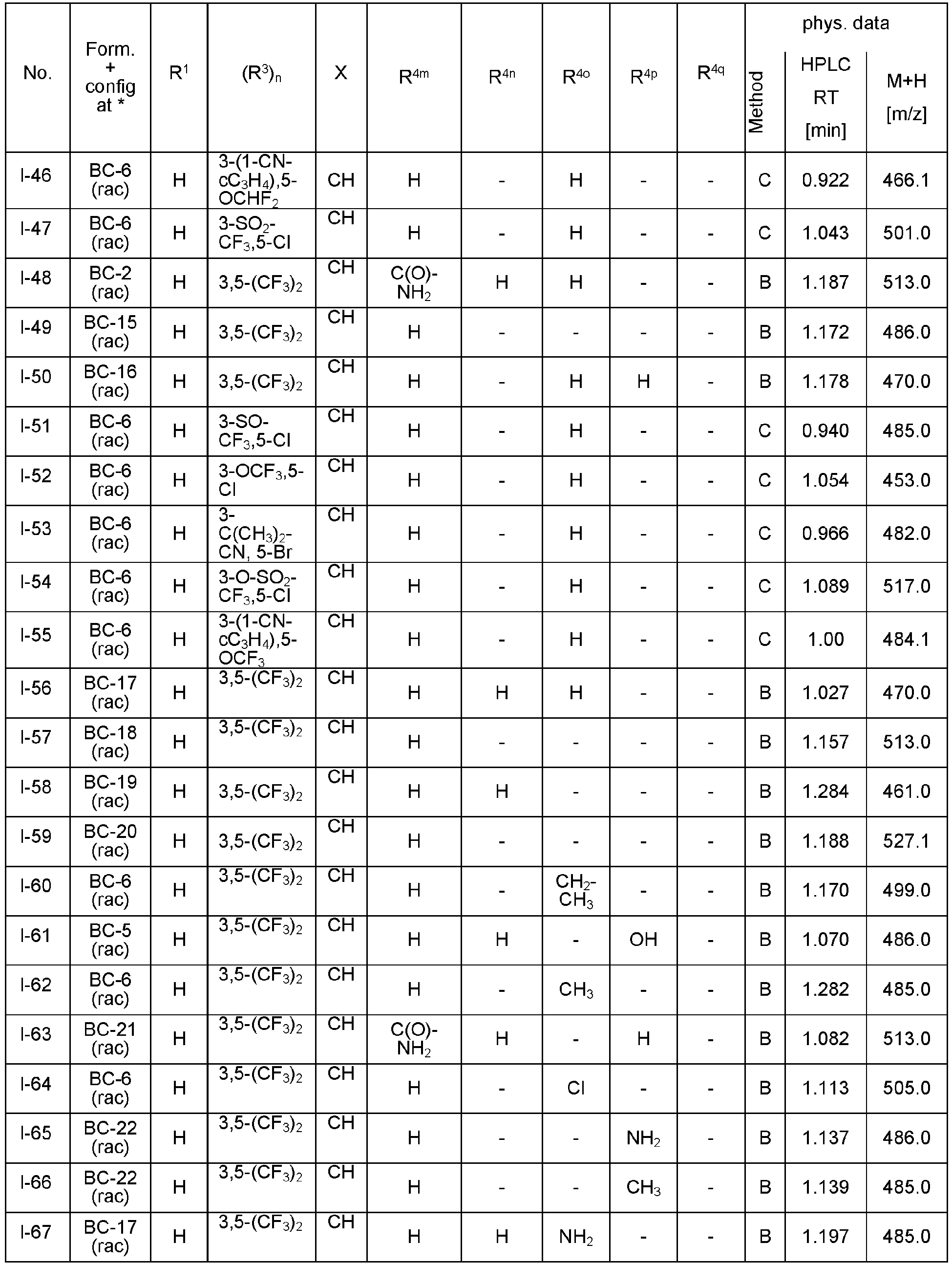

### General experimental procedures to synthesize analogues comprised by Formula I

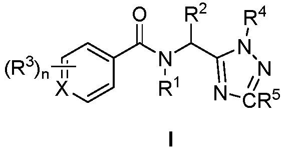

Different R¹ groups could be introduced as it has been shown in the literature, e.g., WO2019201835.

Different R² groups could be introduced as it has been shown in the literature, e.g., WO2019/202077.

Different R³ groups could be introduced as it has been shown in the literature, e.g., WO2021122645.

Different R⁵ groups could be introduced as it has been shown in the literature, e.g., WO2020/094363,

WO2020/188014 and WO2021/13719.

The introduction of R⁴ is described in the following general synthetic routes.

The amin precursors I.A can be prepared as described in the following general synthetic routes. I.A

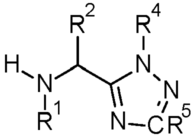

The triazole containing compound I.A can be synthesized starting from a commerically available precursor I.A.1, with an protecting group PG, e.g. a boc-protecting group. As described in the literature, the primary amide can react with, e.g., N,N-dimethylformamide dimethyl acetal (DMF-DMA) to obtain an amidine containing precursor I.A.2. This transformation is usually carried out at temperatures of from 0°C to 100°C, preferably from 0°C to 80 °C, in an inert solvent. Suitable solvents are ethers such as diethylether, diisopropylether, tert.-butylmethylether (MTBE), dioxane, anisole, and tetrahydrofurane (THF), preferably THF. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts or in excess. I.A.2 can undergo a cyclization reaction with the hydrazino-group containing compound R⁴-NH-NH₂ as reported in the literature, e.g., WO2020002563, WO2019197468. This transformation is usually carried out at temperatures of from 0°C to 100 °C, preferably from 25°C to 80°C, in an inert solvent, in the presence of an acid. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH and DMF. It is also possible to use mixtures of the solvents mentioned. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid. Particular preference is given to acetic acid. The acids are generally employed in catalytic amounts; however, they can also be used in equimolar amounts, in excess or, if appropriate, as solvent. Optionally, es described in the literature (e.g.,WO2020/094363, WO2020/188014 and WO2021/13719), an R⁵ group can be introduced to the triazol core to yield I.A.3. As the last step, I.A.3 can undergo a deprotection reaction, e.g., an acid-catalyzed boc-deprotection reaction to yield I.A. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), preferably dioxane. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts, in excess or, if appropriate, as solvent.

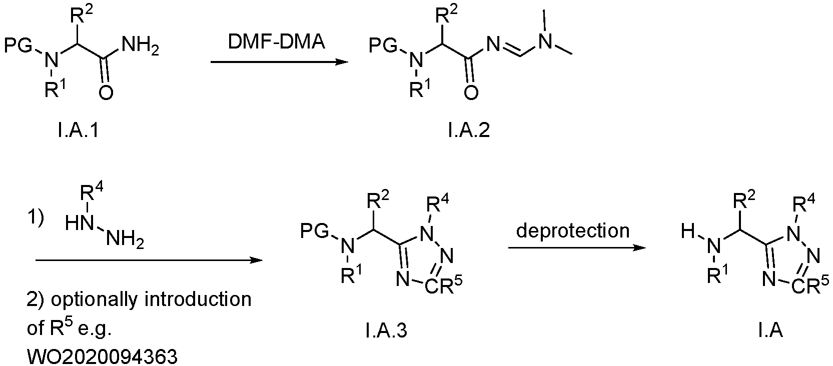

### Dependent on R⁴ the hydrazino-group containing compound (R⁴-NH-NH₂) could be synthesized in the following way.

R⁴ having the BC1 core structure can be synthesized starting from the halogen-precursor Int 1.1. Int 1.1 can undergo a nucleophilic substitution reaction with hyrazine hydrate to obtain Int. 1.2. This transformation is usually carried out at temperatures of from 0°C to 100 °C, preferably from 25°C to 80 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably H₂O, MeOH or DMF.

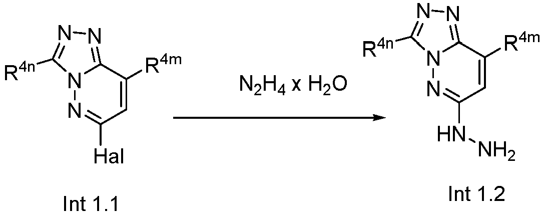

R⁴ having the BC2 core structure can be synthesized starting from the halogen-precursor Int 2.1. Int 2.1 can undergo a nucleophilic substitution reaction with hyradzine hydrate to obtain Int 2.2. This transformation is usually carried out at temperatures of from 0°C to 100 °C, preferably from 25°C to 80 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably H₂O, MeOH or DMF.

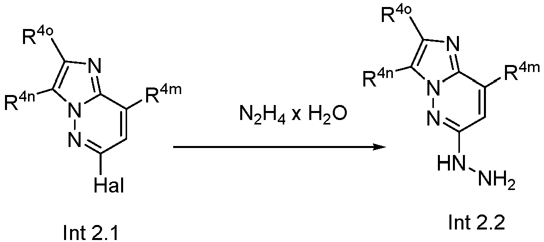

R⁴ having the BC3 core structure can be synthesized starting from the bromo-amino-precursor Int 3.1. The amine-group of Int 3.1 can undergo a chlorination reaction with, e.g., ^{t}BuONO and CuCl as regents of the substitution reaction to obtain Int 3.2. This transformation is usually carried out at temperatures of from -100 °C to 200 °C, preferably from 0°C to 100 °C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF and DMF. Subsequently, the bromo-group of Int 3.2 can undergo a nucleophilc substitution reaction. This transformation is usually carried out at temperatures of from 0°C to 100°C, preferably from 25°C to 80°C, in an inert solvent, in the presence of a base. Suitable solvents are dimethyl sulphoxide (DMSO), dimethyl formamide (DMF), and dimethylacetamide (DMA), preferably DMF. Suitable bases are, in general, organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to DIPEA. The bases are generally employed in catalytic amounts; however, they can also be used in equimolar amounts, in excess or, if appropriate, as solvent. Int 3.3 can undergo an acid-catalyzed intramolecular cyclization reaction to yield Int 3.4. This transformation is usually carried out at temperatures of from 0°C to 100°C, preferably from 25°C to 60°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF and DMF. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid. Particular preference is given to TFA. The acids are generally employed in catalytic amounts; however, they can also be used in equimolar amounts, in excess or, if appropriate, as solvent. Int 3.5, which incorperates R⁴ with the BC3 core structure, can be synthesized from Int 3.4. Int 3.4 can undergo a nucleophilic substitution reaction with hyradzine hydrate. This transformation is usually carried out at temperatures of from 0°C to 100 °C, preferably from 25°C to 80 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably H₂O, MeOH, EtOH or DMF.

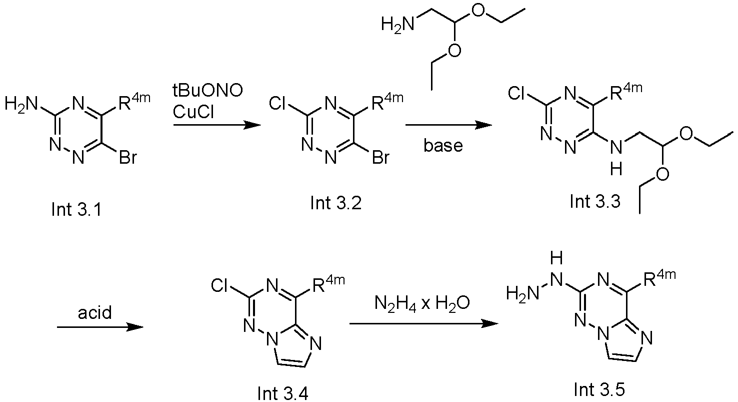

R⁴ having the BC8 core structure can be synthesized starting from the chloro-amino-precursor Int 8.1. Int 8.1 can undergo an olefination reaction with, e.g., ethyl 2-(triphenyl-λ⁵-phosphanylidene)acetate to obtain Int 8.2. This transformation is usually carried out at temperatures of from 0°C to 150°C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF. Subsequently Int 8.2 can undergo a reduction reaction in the presence of a hydrogen donor, such as H₂ and PtO₂ as a catalyst, to obtain Int 8.3. This transformation is usually carried out at temperatures of from 0°C to 100°C, preferably at 25.°C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH. The cyclization reaction to obtain Int 8.4 can be conducted in the presence of a base. This transformation is usually carried out at temperatures of from 0°C to 100 °C, preferably from 25°C to 80°C, in an inert solvent. Suitable solvents are alcohols such as methanol (MeOH), ethanol (EtOH), n-propanol, isopropanol, n-butanol, and tert.-butanol, preferably MeOH. It is also possible to use mixtures of the solvents mentioned. Suitable bases are, in general, inorganic compounds, such as alkali metal and alkaline earth metal hydroxides, such as lithium hydroxide, sodium hydroxide, potassium hydroxide and calcium hydroxide, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to MeONa. The bases are generally employed in catalytic amounts; however, they can also be used in equimolar amounts, in excess or, if appropriate, as solvent. Int 8.4 can undergo a palladium-catalyzed cross-coupling reaction with, e.g., BrettPhosPdG3 and a base, with a protected hyradzine derivative, e.g. the boc-protected hydrazine derivative, to obtain Int 8.5. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably 1,4-dioxane or DMF. Suitable bases are, alkali metal and alkaline earth metal carbonates, such as lithium carbonate, potassium carbonate and calcium carbonate, and also alkali metal bicarbonates, such as sodium bicarbonate, preferably CsCO₃. In the last step the hydrazino compound Int 8.5. can be deprotected, e.g. acid-catalyzed to yield Int 8.6. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), preferably THF. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid. Particular preference is given to aq. HCl. The acids are generally employed in catalytic amounts; however, they can also be used in equimolar amounts, in excess or, if appropriate, as solvent.

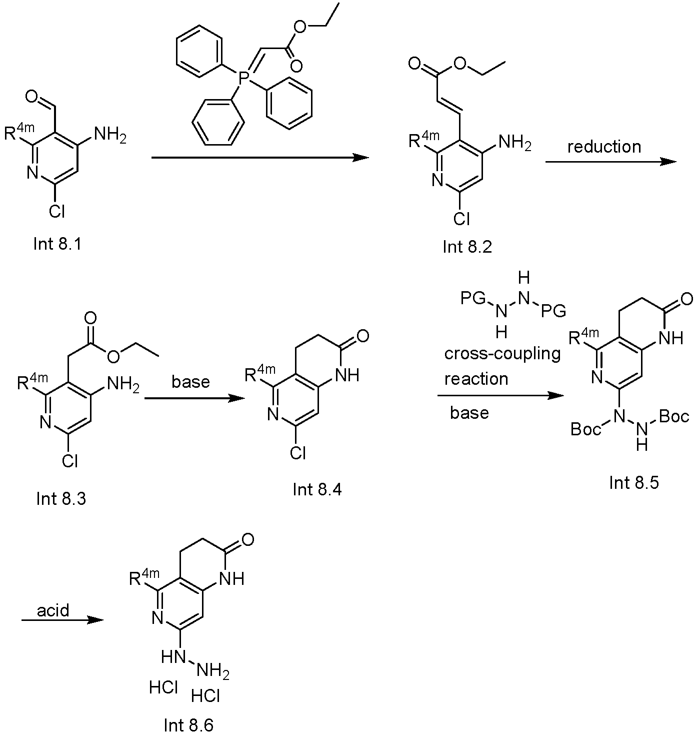

R⁴ having the BC9 core structure can be synthesized starting from the chloro-precursor Int 9.1. Int 9.1 can undergo a nucleophilic substitution reaction with benzenesulfonyl chloride in the presence of a base. This transformation is usually carried out at temperatures of from 0°C to 150°C, preferably from 25°C to 80°C, in an inert solvent. Suitable solvents are dimethyl sulphoxide (DMSO), dimethyl formamide (DMF), and dimethylacetamide (DMA), preferably DMF. Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal hydrides, such as lithium hydride, sodium hydride, potassium hydride and calcium hydride. Particular preference is given to sodium hydride. The bases are generally employed in catalytic amounts; however, they can also be used in equimolar amounts or in excess. Int 9.2 can undergo a metal-based cross-coupling reaction with e.g., Pd(OAc)₂ as a catalyst, a phosphine ligand, such as XPhos and e.g. NH₂-Boc as the coupling couterpart, in the presence of a base, to yield Int 9.3. This transformation is usually carried out at temperatures of from 0°C to 150°C, preferably from 25°C to 120°C, in an inert solvent. Suitable solvents are ethers such as diethylether, diisopropylether, tert.-butylmethylether (MTBE), dioxane, anisole, and tetrahydrofurane (THF), dimethyl sulphoxide (DMSO), dimethyl formamide (DMF), and dimethylacetamide (DMA), preferably dioxane. Suitable bases are, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to CsCO₃. Int 9.3 can undergo a amination reaction with e.g. O-diphenylphosphorylhydroxylamine, as the amine donor in the presence of a base. This transformation is usually carried out at temperatures of from -100°C to 200 °C, preferably from °C to 100 °C, in an inert solvent. Suitable solvents are dimethyl sulphoxide (DMSO), dimethyl formamide (DMF), and dimethylacetamide (DMA), preferably DMF. Suitable bases are, in general, inorganic compounds, such as alkali metal and alkaline earth metal hydrides, such as lithium hydride, sodium hydride, potassium hydride and calcium hydride, preferably sodium hydride. Int 9.4 can undergo a, e.g. acid-catalyzed, deprotection reaction to yield the free hydrazino derivative Int 9.5. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), preferably dioxane. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts, in excess or, if appropriate, as solvent. Int 9.5 can undergo a second deprotection reaction with contrary conditions to yield the hydrazino compound Int 9.6. This transformation is usually carried out at temperatures of from 0°C to 100°C, preferably from 25°C to 100°C, in an inert solvent, in the presence of a base. Suitable solvents are alcohols such as methanol (MeOH), ethanol (EtOH), n-propanol, isopropanol, n-butanol, and tert.-butanol, preferably MeOH. Suitable bases are, in general, inorganic compounds, such as alkali metal and alkaline earth metal hydroxides, such as lithium hydroxide, sodium hydroxide, potassium hydroxide and calcium hydroxide, preferably sodium hydroxide. Int 9.6 can undergo a bromination reaction with, e.g., pyridinium perbromide to yield Int 9.7. This transformation is usually carried out at temperatures of from -100°C to 200 °C, preferably from 0°C to 100°C, in an inert solvent. Suitable solvents are alcohols such as methanol (MeOH), ethanol (EtOH), n-propanol, isopropanol, n-butanol, and tert.-butanol, preferably tert.-butanol. It is also possible to use mixtures of the solvents mentioned. Int 9.7 can undergo dehalogenation reaction catalyzed by e.g. Zn in the presence of an acid to yield Int 9.8. This transformation is usually carried out at temperatures of from 25°C to 100°C, preferably from 25°C to 80°C, in an inert solvent. Suitable solvents are alcohols such as methanol (MeOH), ethanol (EtOH), n-propanol, isopropanol, n-butanol, and tert.-butanol, preferably MeOH. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid, preferably acetic acid.

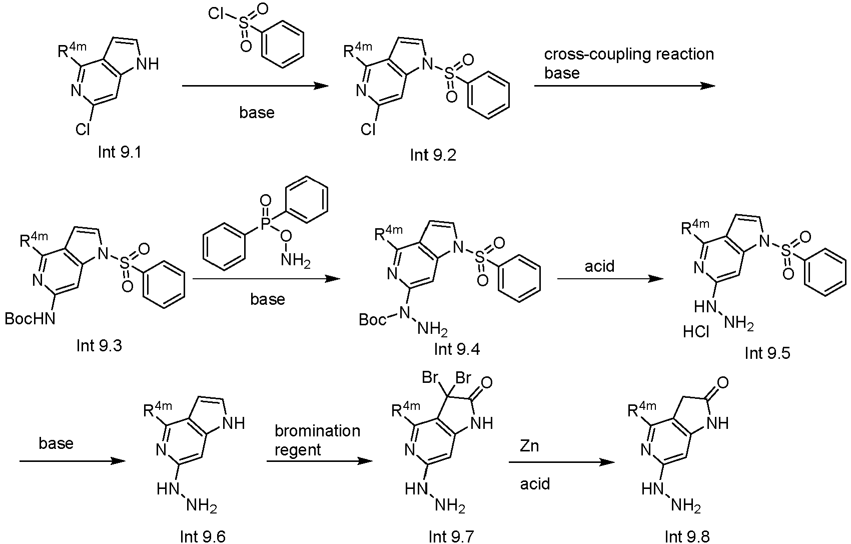

R⁴ having the BC10 core structure can be synthesized starting from the chloro-bromo-precursor Int 10.1. Int 10.1 can undergo a cross-coupling reaction with methylboronic acid, with a palladium catalyst, e.g., palladium acetate, a ligand, e.g. a phosphine ligand such as PCy₃ and a base, to obtain Int 10.2. This transformation is usually carried out at temperatures of from 0°C to 100°C, preferably from 25°C to 100 °C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF, dioxane and DMF. Suitable bases are alkali metal and alkaline earth metal carbonates, such as lithium carbonate, potassium carbonate and calcium carbonate, and also alkali metal bicarbonates, such as sodium bicarbonate, preferably potassium phosphate. The benzylic position of Int 10.2 can be halogenated, with a bromination regent such as N-bromosuccinimide (NBS) and a radical initiator such as AIBN. This transformation is usually carried out at temperatures of from -100°C to 100°C, preferably from 0°C to 50°C, in an inert solvent. Suitable solvents are halogenated hydrocarbons such as methylene chloride, chloroform, and chlorobenzene, preferably methylene chloride. Int 10.3 can undergo a nucleophilic substitution reaction with a nucleophilic CN-source, e.g. TMSCN and an amine catalyst, such as TBAF, to obtain Int 10.4. This transformation is usually carried out at temperatures of from -50°C to 100 °C, preferably from 0°C to 80 °C, in an inert solvent. Suitable solvents are halogenated hydrocarbons such as methylene chloride, chloroform, and chlorobenzene, ethers such as diethylether, diisopropylether, tert.-butylmethylether (MTBE), dioxane, anisole, and tetrahydrofurane (THF), preferably methylene chloride. It is also possible to use mixtures of the solvents mentioned. Int 10.4 can undergo a cross-coupling reaction with, e.g., BrettPhosPdG3 and a base, with a protected hyradzine derivative, e.g. the boc-protected hydrazine derivative, to obtain Int 10.5. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably 1,4-dioxane or DMF. Suitable bases are, alkali metal and alkaline earth metal carbonates, such as lithium carbonate, potassium carbonate and calcium carbonate, and also alkali metal bicarbonates, such as sodium bicarbonate, preferably CsCO₃. Int 10.5 can undergo a reduction reaction in the presence of a hydrogen donor such as H₂, PtO₂ as a catalyst and subsequently cyclize to yield Int 10.6. This transformation is usually carried out at temperatures of from 0°C to 100 °C, preferably from 25°C to 80°C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH and DMF. As the last step Int 10.6 can undergo an acid-catalyzed deprotection reaction, e.g. a boc-deprotection, to yield Int 10.7. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF. Suitable acids and acidic catalysts are in general anorganic acids such as hydrofluoric acid, hydrochloric acid, hydrobromic acid, sulphuric acid und perchloric acid, preferably hydrochloric acid.

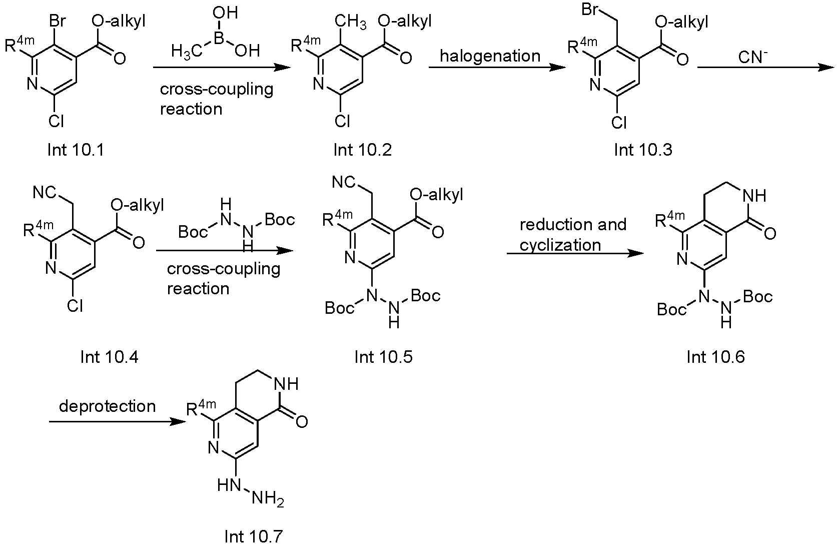

R⁴ having the BC11 core structure can be synthesized starting from the chloro-bromo precursor Int 11.1. Int 11.1 can undergo a nucleophilic substitution reaction with a CN-source, such as CuCN, to yield Int 11.2. This transformation is usually carried out at temperatures of from 0°C to 100°C, preferably from 25°C to 80°C, in an inert solvent. Suitable solvents are dimethyl sulphoxide (DMSO), dimethyl formamide (DMF), and dimethylacetamide (DMA), preferably DMA. It is also possible to use mixtures of the solvents mentioned. Int 11.2 can undergo a cross-coupling reaction with, e.g., BrettPhosPdG3 and a base, with a protected hyradzine derivative, e.g. the boc-protected hydrazine derivative, to obtain Int 11.3. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably 1,4-dioxane or DMF. Suitable bases are, alkali metal and alkaline earth metal carbonates, such as lithium carbonate, potassium carbonate and calcium carbonate, and also alkali metal bicarbonates, such as sodium bicarbonate, preferably CsCO₃. Int 11.3 can undergo a reduction in the presence of an hydrogen donor such as H₂, PtO₂ as a catalyst and subsequently cyclize to yield Int 11.4. This transformation is usually carried out at temperatures of from 0°C to 100 °C, preferably from 25°C to 80°C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH and DMF. As the last step Int 11.4 can undergo an acid-catalyzed deprotection reaction, e.g. a boc-deprotection, to yield Int 11.5. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF. Suitable acids and acidic catalysts are in general anorganic acids such as hydrofluoric acid, hydrochloric acid, hydrobromic acid, sulphuric acid und perchloric acid, preferably hydrochloric acid.

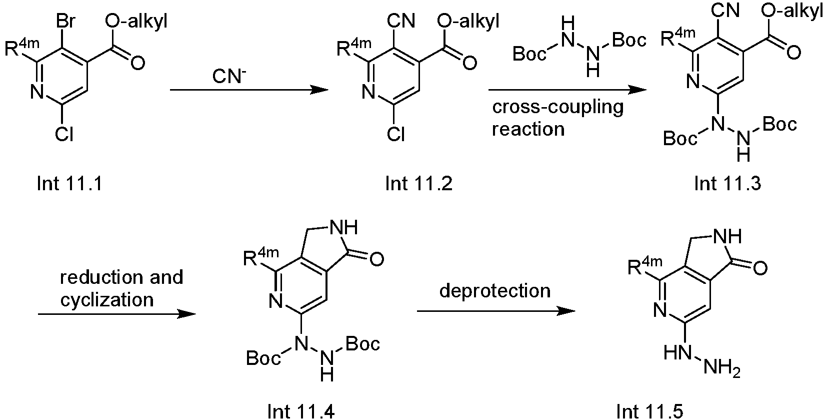

R⁴ having the BC12 core structure can be synthesized starting from the commercially available chloro-compound Int 12.1. (CAS: 63725-51-9). Int 12.1 can undergo a nucleophilic substitution reaction with hydrazine hydrate to obtain Int 12.2. This transformation is usually carried out at temperatures of from 0°C to 100 °C, preferably from 25°C to 80 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably H₂O, MeOH or DMF.

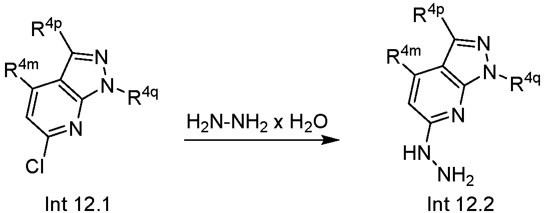

R⁴ having the BC13 core structure can be synthesized starting from the commercially available chloro-precursor Int 13.1 (CAS: 1206979-33-0). Int 13.1 can undergo a nucleophilic substitution reaction with Boc₂O to introduce a protecting group at the secondary nitrogen to yield Int 13.2. This transformation is usually carried out at temperatures of from 0°C to 100 °C, preferably from 25°C to 80 °C, in an inert solvent, in the presence of a base. Suitable solvents are halogenated hydrocarbons such as methylene chloride, chloroform, and chlorobenzene, preferably methylene chloride. Suitable bases are organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to 4-dimethylaminopyridine. The bases are generally employed in catalytic amounts; however, they can also be used in equimolar amounts, in excess or, if appropriate, as solvent. Int 13.2 can undergo a cross-coupling reaction with, e.g., BrettPhosPdG3 and a base, with a protected hyradzine derivative, e.g. the boc-protected hydrazine derivative, to obtain Int 13.3. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably 1,4-dioxane or DMF. Suitable bases are, alkali metal and alkaline earth metal carbonates, such as lithium carbonate, potassium carbonate and calcium carbonate, and also alkali metal bicarbonates, such as sodium bicarbonate, preferably CsCO₃. As the last step Int 13.3 can undergo an acid-catalyzed deprotection reaction, e.g. a boc-deprotection, to yield Int 13.4. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF and dioxane. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts, in excess or, if appropriate, as solvent.

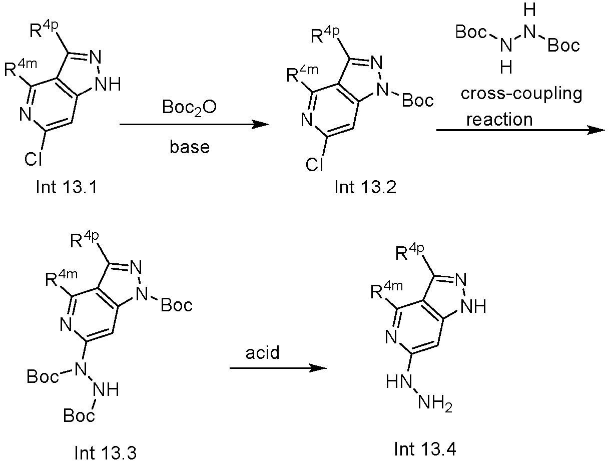

R⁴ having the BC14 core structure can be synthesized starting from the commercially available chloro-precursor Int 14.1 (CAS: 23002-51-9). Int 14.1 can undergo a nucleophilic substitution reaction with Boc₂O to introduce a protecting group at the secondary nitrogen to yield Int 14.2. This transformation is usually carried out at temperatures of from 0°C to 100 °C, preferably from 25°C to 80 °C, in an inert solvent, in the presence of a base. Suitable solvents are halogenated hydrocarbons such as methylene chloride, chloroform, and chlorobenzene, preferably methylene chloride. Suitable bases are organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to 4-dimethylaminopyridine. The bases are generally employed in catalytic amounts; however, they can also be used in equimolar amounts, in excess or, if appropriate, as solvent. Int 13.2 can undergo a cross-coupling reaction with, e.g., BrettPhosPdG3 and a base, with a protected hyradzine derivative, e.g. the boc-protected hydrazine derivative, to obtain Int 13.3. This transformation is usually carried out at temperatures of from 0°C to 100 °C, preferably from 25°C to 80 °C, in an inert solvent. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably 1,4-dioxane or DMF. Suitable bases are, alkali metal and alkaline earth metal carbonates, such as lithium carbonate, potassium carbonate and calcium carbonate, and also alkali metal bicarbonates, such as sodium bicarbonate, preferably CsCO₃. As the last step Int 13.3 can undergo an acid-catalyzed deprotection reaction, e.g. a boc-deprotection, to yield Int 13.4. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF and dioxane. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts, in excess or, if appropriate, as solvent.

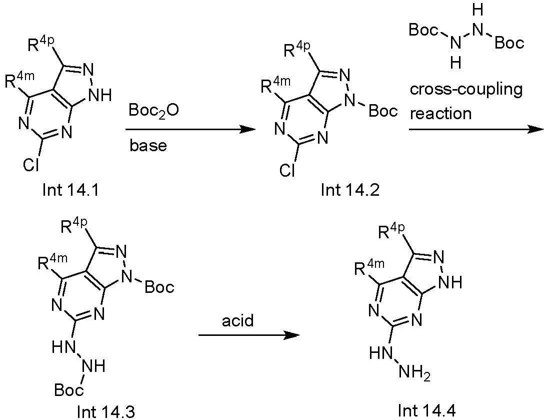

Dependent on R⁴, the hydrazino compound could also be synthesized at an earlier step of the synthetic route and could be cyclized to a triazol as described in e.g., WO2020002563, WO2019197468.

R⁴ having the BC4 core structure can be synthesized starting from the commercially available bromo-pyrazin precursor Int 4.1. Int 4.1 can undergo a nucleophilic substitution reaction with hydrazine hydrate to obtain Int 4.2. This transformation is usually carried out at temperatures of from 0°C to 100 °C, preferably from 25°C to 80 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably H₂O, MeOH or DMF. Int 4.2 can undergo a cyclization reaction with an amidine precursor as reported in the literature, e.g., WO2020002563, WO2019197468, to yield Int 4.3. This transformation is usually carried out at temperatures of from 0°C to 100 °C, preferably from 25°C to 80°C, in an inert solvent, in the presence of an acid. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH and DMF. It is also possible to use mixtures of the solvents mentioned. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid. Particular preference is given to acetic acid. The acids are generally employed in catalytic amounts; however, they can also be used in equimolar amounts, in excess or, if appropriate, as solvent. Int 4.3 can undergo a second nucleophilic substitution reaction with hydrazine hydrate to yield Int 4.4. This transformation is usually carried out at temperatures of from 0°C to 100 °C, preferably from 25°C to 80 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH, EtOH or DMF. Int 4.4 can undergo a cyclization reaction with diethoxymethoxyethane to yield the bicyclic compound Int 4.5. This transformation is usually carried out at temperatures of from 0°C to 150 °C, preferably from 25°C to 100 °C, in an inert solvent. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH or DMF. As the last step Int 4.5 can undergo an acid-catalyzed deprotection reaction, e.g. a boc-deprotection, to yield Int 4.6. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF and dioxane. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts, in excess or, if appropriate, as solvent.

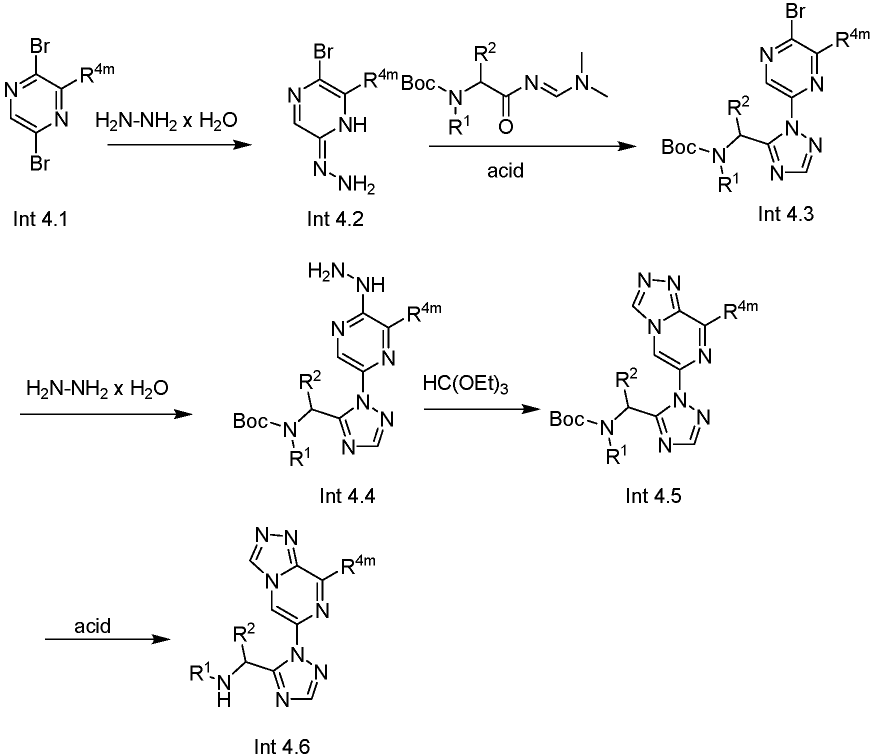

R⁴ having the BC5 core structure can be synthesized starting from Int 5.1. Int 5.1 can undergo a cross-coupling reaction to introduce the cyano group with, e.g., Zn(CN)₂ as a regent to yield Int 5.2. the cross-coupling reaction can be catalyzed with a metal catalyst, such as palladium catalyst, e.g. Pd₂(dba)₃, in the presence of a phosphine ligand, such as dppf. This transformation is usually carried out at temperatures of from 25°C to 200 °C, preferably from 60°C to 150 °C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably DMF. Suitable bases are alkali metal and alkaline earth metal carbonates, such as lithium carbonate, potassium carbonate and calcium carbonate, and also alkali metal bicarbonates, such as sodium bicarbonate, preferably potassium carbonate. Int 5.2 can undergo a reduction reaction in the presence of a palladium catalyst, such as Pd/C or Pd(OH)₂/C and MeOH. This transformation is usually carried out at temperatures of from 0°C to 100 °C, preferably from 25°C to 80°C, in an inert solvent. Suitable solvents are alcohols such as methanol (MeOH), ethanol (EtOH), n-propanol, isopropanol, n-butanol, and tert.-butanol, preferably MeOH. Int 5.3 can undergo a condensation reaction with formic acid and Ac₂O to yield Int 5.4. This transformation is usually carried out at temperatures of from -50°C to 100°C, preferably from 0°C to 25°C, in an inert solvent. Suitable solvents are ethers such as diethylether, diisopropylether, tert.-butylmethylether (MTBE), dioxane, anisole, and tetrahydrofurane (THF), preferably THF. Int 5.4 can undergo a base-catalyzed cyclization reaction in the presence of Tf₂O. This transformation is usually carried out at temperatures of from 0°C to 100°C, preferably from 0°C to 80°C, in an inert solvent. Suitable solvents are halogenated hydrocarbons such as methylene chloride, chloroform, and chlorobenzene, preferably methylene chloride. As the last step Int 5.5 can undergo an acid-catalyzed deprotection reaction, e.g. a Boc-deprotection, to yield Int 5.6. This transformation is usually carried out at temperatures of from 0°C to 100 °C, preferably from 25°C to 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably THF and dioxane. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts, in excess or, if appropriate, as solvent.

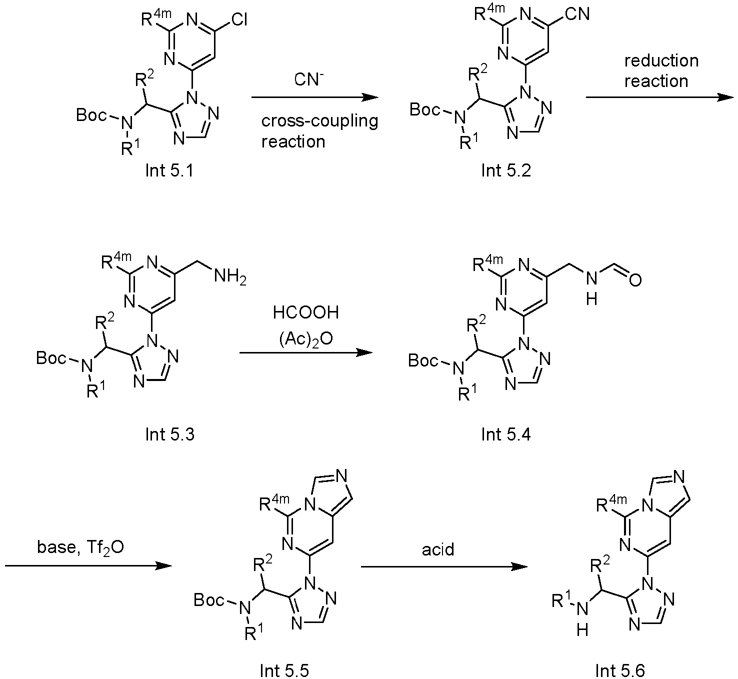

R⁴ having the BC6 core structure can be synthesized starting from Int 6.1. Int 6.1 can undergo a cyclization reaction with an amidine precursor as reported in the literature, e.g., WO2020002563, WO2019197468, to yield Int 6.2. This transformation is usually carried out at temperatures of from 0°C to 100 °C, preferably from 25°C to 80°C, in an inert solvent, in the presence of an acid. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH and DMF. It is also possible to use mixtures of the solvents mentioned. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid. Particular preference is given to acetic acid. The acids are generally employed in catalytic amounts; however, they can also be used in equimolar amounts, in excess or, if appropriate, as solvent. Int 6.2 can undergo cross coupling reaction with diarylmethanimine, e.g. diphenylmethanimine, with a metal catalyst, such as a palladium catalyst, e.g. Pd₂(dba)₃, and a phosphine ligand, such as Xantphos, in the presence of a base. This transformation is usually carried out at temperatures of from 0°C to 200°C, preferably from 25°C to 150°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably dioxane or DMF. Suitable bases are alkali metal and alkaline earth metal carbonates, such as lithium carbonate, potassium carbonate and calcium carbonate, and also alkali metal bicarbonates, such as sodium bicarbonate, Particular preference is given to potassium carbonate. Int 6.3 can undergo an acid-catalyzed reaction to yield the free-amino compound Int 6.4. This transformation is usually carried out at temperatures of from 0°C to 100°C, preferably from 0°C to 80 °C, in an inert solvent. Suitable solvents are ethers such as diethylether, diisopropylether, tert.-butylmethylether (MTBE), dioxane, anisole, and tetrahydrofurane (THF), preferably THF. Suitable acids and acidic catalysts are in general anorganic acids such as hydrofluoric acid, hydrochloric acid, hydrobromic acid, sulphuric acid und perchloric acid, preferably hydrochloric acid. The acids are generally employed in catalytic amounts; however, they can also be used in equimolar amounts or in excess. Int 6.4 can undergo a condensation reaction with DMF-DMA to yield Int 6.5. This transformation is usually carried out at temperatures of from 0°C to 80°C, preferably at 80°C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA) or DMF, alcohols such as methanol (MeOH), ethanol (EtOH), n-propanol, isopropanol, n-butanol, and tert.-butanol, , preferably THF or i-PrOH. Int 6.5 can undergo a subsequent reaction with hydroxyl amine. to yield Int 6.6. This transformation is usually carried out at temperatures of from 0°C to 100°C, preferably at 50 °C, in an inert solvent. Suitable solvents are alcohols such as methanol (MeOH), ethanol (EtOH), n-propanol, isopropanol, n-butanol, and tert.-butanol, preferably i-PrOH. Int 6.6 can undergo an acid-catalysed cyclization reaction to yield the Int 6.7. This transformation is usually carried out at temperatures of from 0°C to 200°C, preferably at 100 °C, in an inert solvent. Suitable solvents are alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably H₂O. Suitable acids and acidic catalysts are in general anorganic acids such as hydrofluoric acid, hydrochloric acid, hydrobromic acid, sulphuric acid und perchloric acid, preferably polyphosphoric acid. The acids are generally employed in catalytic amounts; however, they can also be used in equimolar amounts, in excess or, if appropriate, as solvent.

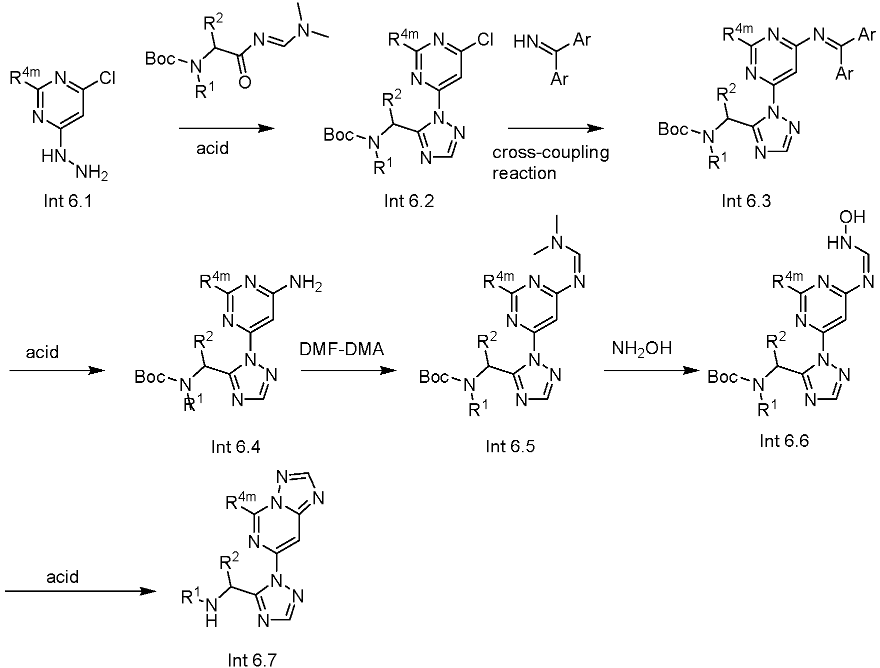

R⁴ having the BC7 core structure can be synthesized starting from Int 7.1. Int 7.1 can undergo a cyclization reaction with an amidine precursor as reported in the literature, e.g., WO2020002563, WO2019197468, to yield Int 7.2. This transformation is usually carried out at temperatures of from 0°C to 100 °C, preferably from 25°C to 80°C, in an inert solvent, in the presence of an acid. Suitable solvents are ethers such as diethylether, diisopropylether, tert.-butylmethylether (MTBE), dioxane, anisole, and tetrahydrofurane (THF), alcohols such as methanol (MeOH), ethanol (EtOH), n-propanol, isopropanol, n-butanol, and tert.-butanol, moreover dimethyl sulphoxide (DMSO), dimethyl formamide (DMF), and dimethylacetamide (DMA), preferably MeOH and DMF. It is also possible to use mixtures of the solvents mentioned. Suitable acids and acidic catalysts are in general anorganic acids such as hydrofluoric acid, hydrochloric acid, hydrobromic acid, sulphuric acid und perchloric acid, moreover organic acids such as formic acid, acetic acid, propionic acid, oxalic acid, toluene sulphonic acid, benzene sulphonic acid, camphor sulphonic acid, citric acid, and trifluoro acetic acid. Particular preference is given to acetic acid. The acids are generally employed in catalytic amounts; however, they can also be used in equimolar amounts, in excess or, if appropriate, as solvent. Int 7.2 can undergo a cross-coupling reaction to introduce the cyano group with, e.g., Zn(CN)₂ as a regent to yield Int 7.3. the cross-coupling reaction can be catalyzed with a metal catalyst, such as palladium catalyst, e.g. Pd₂(dba)₃, in the presence of a phosphine ligand, such as dppf. This transformation is usually carried out at temperatures of from 25°C to 200 °C, preferably from 60°C to 150 °C, in an inert solvent. Suitable solvents are ethers such as diethylether, diisopropylether, tert.-butylmethylether (MTBE), dioxane, anisole, and tetrahydrofurane (THF), moreover dimethyl sulphoxide (DMSO), dimethyl formamide (DMF), and dimethylacetamide (DMA), preferably DMF. Suitable bases are alkali metal and alkaline earth metal carbonates, such as lithium carbonate, potassium carbonate and calcium carbonate, and also alkali metal bicarbonates, such as sodium bicarbonate, preferably potassium carbonate. Int 7.3 can undergo a reduction reaction in the presence of a palladium catalyst, such as Pd/C or Pd(OH)₂/C and MeOH to yield 7.4. This transformation is usually carried out at temperatures of from 0°C to 100 °C, preferably from 25°C to 80°C, in an inert solvent. Suitable solvents are alcohols such as methanol (MeOH), ethanol (EtOH), n-propanol, isopropanol, n-butanol, and tert.-butanol, preferably MeOH. Int 7.4 can undergo a condensation reaction with formic acid and Ac₂O to yield Int 7.5. This transformation is usually carried out at temperatures of from -50°C to 100°C, preferably from 0°C to 25°C, in an inert solvent. Suitable solvents are ethers such as diethylether, diisopropylether, tert.-butylmethylether (MTBE), dioxane, anisole, and tetrahydrofurane (THF), preferably THF. Int 7.5 can undergo a base-catalyzed cyclization reaction in the presence of Tf₂O to yield 7.6. This transformation is usually carried out at temperatures of from 0°C to 100°C, preferably from 0°C to 80°C, in an inert solvent. Suitable solvents are halogenated hydrocarbons such as methylene chloride, chloroform, and chlorobenzene, preferably methylene chloride.

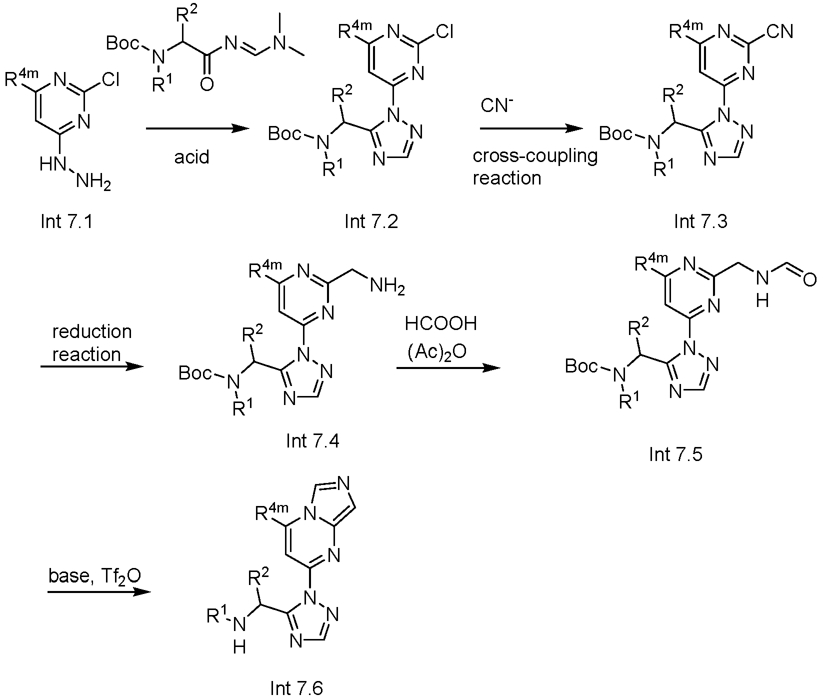

To obtain the final product I, an amine building block Int 1 - Int 14 and a carboxylic acid (commercially availabe or known in the literature, e.g. WO2021122645) can undergo an amide-coupling reaction, e.g. catalyzed by HATU (1-[bis(dimethylamino)methylene]-1H-1,2,3-triazolo[4,5-b]pyridinium 3-oxide hexafluorophosphate) or T3P (2,4,6-tripropyl-1,3,5,2,4,6-trioxatriphosphorinan-2,4,6-trioxid), as reported in, e.g. WO2019197468.

Alternatively, the amine building block Int 1 - Int 14 and a carboxylic acid chlorid (commercially availabe or known in the literature, e.g. WO2020053365) can undergo a nucleophilic substitution reaction with an carboxylic acid chlorid, as reported in WO2020053365.

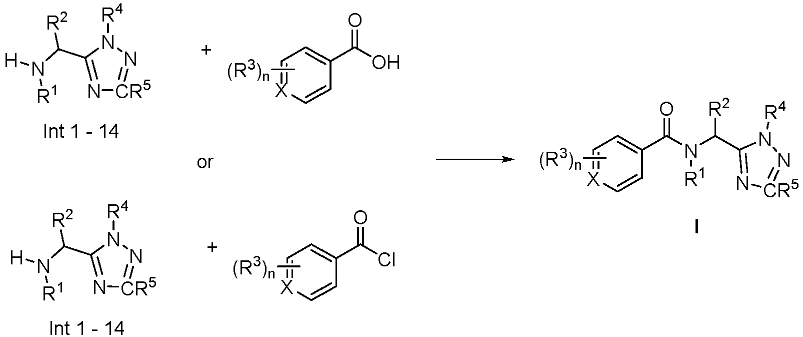

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

If individual compounds I cannot be obtained by the routes described above, they can be prepared by derivatization of other compounds I.

However, if the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus / invertebrate pest to be controlled.

### Biological examples

If not otherwise specified, the test solutions are prepared as follow:
The active compound is dissolved at the desired concentration in a mixture of 1:1 (vol : vol) distilled water : acetone. The test solution is prepared on the day of use.

The activity of the compounds of formula I of the present invention can be demonstrated and evaluated by the following biological tests.

### B.1 Tobacco budworm (Heliothis virescens)

For evaluating control of tobacco budworm (Heliothis virescens) the test unit consists of 96-well-microtiter plates containing an insect diet and 15-25 H. virescens eggs.

The compounds are formulated using a solution containing 75% v/v water and 25% v/v DMSO. Different concentrations of formulated compounds are sprayed onto the insect diet at 10µl, using a custom-built micro atomizer, at two replications.

After application, microtiter plates are incubated at about 28 ± 1°C and about 80 ± 5 % relative humidity for 5 days. Egg and larval mortality is then visually assessed.

In this test, compounds I-1, I-2, I-3, I-4, I-6, I-8, I-9, I-10, I-11, I-12, I-14, I-15, I-17, I-19, I-20, I-21, I-22, I-24, I-26, I-27, I-28, I-30, I-32, I-33, I-35, I-36, I-37, I-38, I-41, I-43, I-46, I-47, I-48, I-51, I-52, I-53, I-54, I-55, I-57, I-59, I-60, I-62, I-64, I-65 and I-66, resp., at 800 ppm showed at least 75% mortality in comparison with untreated controls.

### B.2 Boll weevil (Anthonomus grandis)

For evaluating control of boll weevil (Anthonomus grandis) the test unit consists of 96-well-microtiter plates containing an insect diet and 5-10 A. grandis eggs.

The compounds are formulated using a solution containing 75% v/v water and 25% v/v DMSO. Different concentrations of formulated compounds are sprayed onto the insect diet at 5µl, using a custom-built micro atomizer, at two replications.

After application, microtiter plates are incubated at about 25 ± 1°C and about 75 ± 5 % relative humidity for 5 days. Egg and larval mortality is then visually assessed.

In this test, compounds I-2, I-3, I-4, I-6, I-7, I-8, I-9, I-10, I-11, I-12, I-13, I-14, I-15, I-17, I-18, I-19, I-20, I-21, I-22, I-23, I-24, I-25, I-26, I-27, I-28, I-29, I-30, I-31, I-32, I-33, I-34, I-35, I-36, I-37, I-38, I-39, I-40, I-41, I-42, I-43, I-44, I-45, I-46, I-47, I-48, I-49, I-51, I-52, I-53, I-54, I-55, I-57, I-59, I-60, I-62, I-63, I-64, I-65 and I-66, resp., at 800 ppm showed at least 75% mortality in comparison with untreated controls.

### B.3 Diamond back moth (Plutella xylostella)

The active compound was dissolved at the desired concentration in a mixture of 1:1 (vol:vol) distilled water : acetone. Surfactant (Kinetic HV) was added at a rate of 0.01% (vol/vol). The test solution was prepared on the day of use.

Leaves of cabbage were dipped in test solution and air-dried. Treated leaves were placed in petri dishes lined with moist filter paper and inoculated with ten 3rd instar larvae. Mortality was recorded 72 hours after treatment. Feeding damages were also recorded using a scale of 0-100%.

In this test, compounds I-1, I-2, I-3, I-4, I-6, I-8, I-9, I-10, I-11, I-12, I-13, I-14, I-15, I-17, I-18, I-19, I-20, I-21, I-22, I-23, I-25, I-26, I-27, I-28, I-29, I-30, I-32, I-33, I-35, I-36, I-38, I-39, I-41, I-42, I-43, I-46, I-47, I-48, I-49, I-51, I-52, I-53, I-54, I-55, I-57, I-60, I-62, I-63, I-64, I-65 and I-66, resp., at 800 ppm showed at least 75% mortality in comparison with untreated controls.

### B.4 Yellow fever mosquito (Aedes aegypti)

For evaluating control of yellow fever mosquito (Aedes aegypti) the test unit consists of 96-well-microtiter plates containing 200µl of tap water per well and 5-15 freshly hatched A. aegypti larvae.

The active compounds are formulated using a solution containing 75% (v/v) water and 25% (v/v) DMSO. Different concentrations of formulated compounds or mixtures are sprayed onto the insect diet at 2.5µl, using a custom-built micro atomizer, at two replications.

After application, microtiter plates are incubated at 28±1°C, 80±5 % RH for 2 days. Larval mortality is then visually assessed.

In this test, compounds I-1, I-2, I-3, I-4, I-6, I-7, I-8, I-9, I-10, I-11, I-12, I-13, I-14, I-15, I-17, I-18, I-19, I-20, I-21, I-22, I-23, I-24, I-25, I-26, I-27, I-28, I-29, I-30, I-31, I-33, I-34, I-35, I-36, I-37, I-38, I-39, I-40, I-41, I-42, I-43, I-44, I-45, I-46, I-47, I-48, I-49, I-51, I-52, I-53, I-54, I-55, I-57, I-59, I-60, I-62, I-63, I-64, I-65 and I-66, resp., at 800 ppm showed at least 75% mortality in comparison with untreated controls.

### B.5 Green peach aphid (Myzus persicae)

For evaluating control of green peach aphid (Myzus persicae) through systemic means, the test unit consisted of 96-well-microtiter plates containing liquid artificial diet under an artificial membrane.

The compounds were formulated using a solution containing 75% v/v water and 25% v/v DMSO. Different concentrations of formulated compounds were pipetted into the aphid diet, using a custom built pipetter, at two replications.

After application, 5 - 8 adult aphids were placed on the artificial membrane inside the microtiter plate wells. The aphids were then allowed to suck on the treated aphid diet and incubated at about 23 ± 1°C and about 50 ± 5 % relative humidity for 3 days. Aphid mortality and fecundity was then visually assessed.

In this test, compounds I-1, I-2, I-3, I-7, I-8, I-9, I-10, I-11, I-12, I-13, I-14, I-15, I-17, I-18, I-19, I-20, I-21, I-22, I-23, I-24, I-25, I-26, I-27, I-28, I-30, I-31, I-32, I-33, I-35, I-36, I-37, I-38, I-39, I-40, I-41, I-42, I-43, I-44, I-45, I-46, I-47, I-48, I-49, I-51, I-52, I-53, I-54, I-55, I-57, I-59, I-60, I-62, I-63, I-64, I-65 and I-66, resp., at 800 ppm showed at least 75% mortality in comparison with untreated controls.

## Claims

1. Compound of formula I 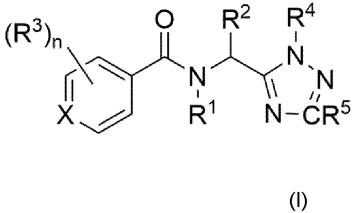 wherein
R¹ is H, OR^{10a}, NR¹²R¹³, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, wherein the alkyl, alkoxy, alkenyl, alkynyl, and cycloalkyl moieties are unsubstituted or substituted with R¹¹; C(=N-R¹³)R¹², or C(O)R^{11a};
R² is H, C₁-C₃-alkyl, C₂-C₃-alkenyl, C₃-C₆-cycloalkyl, or C₂-C₃-alkynyl, wherein the alkyl, alkenyl, alkynyl and cycloalkyl moieties are unsubstituted or substituted with halogen;
R³ is halogen, CN, NO₂, SF₅; C₁-C₆-alkyl, C₁-C₆-alkoxy, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, 3- to 6-membered heterocyclyl, wherein the alkyl, alkenyl, alkynyl, alkoxy,heterocyclyl and cycloalkyl moieties are unsubstituted or substituted with R¹¹; OR^{10a}, NR¹²R¹³, C(O)NR¹²R¹³, C(O)OR¹⁰, C(O)R¹⁴, OS(O)₂-R¹⁴, S(O)ₘ-R¹⁴, -N=S(O)R^{12a}R^{13a}; or
two R³ bound to two adjacent C-atoms can form a 4-, 5-, or 6-membered ring, which may contain one or two heteroatoms selected from N, O, and S as ring members, wherein the ring is unsubstituted or substituted with halogen, CN, C₁-C₃-alkyl, and/or C₁-C₃-haloalkyl;
R⁴ is a heterocyclic ring which is selected from the group consisting of 8-, 9- or 10-membered saturated or partially unsaturated bicyclic heterocyclyl, and 8- 9- or 10- membered bicyclic hetaryl rings, wherein the heterocyclyl moieties are unsubstituted or substituted by 1, 2, 3 or 4 substituents and the hetaryl moieties are unsubstituted or substituted by 1, 2 or 3 substituents, wherein the substituents are independently selected from the group consisting of halogen, =O (oxo), -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₆-alkoxy, C₂-C₆-alkenyl, C₂-C₆-alkynyl, C₁-C₆-alkyl-C₁-C₆-alkoxy, C₁-C₆-al koxy-C₃-C₆-cycloal kyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, S(O)ₘ-R¹⁴, OR^{10a}, NR¹²R¹³, C(O)NR¹²R¹³, C(O)OR¹⁰, C(O)R¹⁴, - C(=NOC₁-C₄-alkyl)H, -C(=NOC₁-C₄-alkyl)-C₁-C₄-alkyl or -C(=NOC₁-C₄-alkyl)-C₃-C₆-cycloalkyl, wherein alkyl, alkenyl, alkynyl, cycloalkyl, and alkoxy are unsubstituted or substituted with R¹¹;
R⁵ is H, halogen, CN, NH₂, NO₂, CONH₂, C₁-C₆-alkyl, or C₃-C₆-cycloalkyl, C₃-C₆-cycloalkyl-C₁-C₆-alkyl, C₁-C₆-alkyl-C₃-C₆-cycloalkyl, C₁-C₆-alkoxyCH(C₁-C₆-alkoxy)₂, C₂-C₆-alkenyl, C₂-C₆-alkynyl, C₁-C₆-alkyl-C₁-C₆-alkoxy, C₁-C₆-alkoxy-C₃-C₆-cycloalkyl, C₃-C₆-cycloalkyl-C₁-C₆-alkoxy, C₂-C₆-alkenyl-C₃-C₆-cycloalkyl, C₂-C₆-alkynyl-C₃-C₆-cycloalkyl, C₃-C₆-cycloalkyl-C₂-C₆-alkenyl, C₃-C₆-cycloalkyl-C₂-C₆-alkynyl, OR^{10a}, NR¹²R¹³, C(O)NR¹²R¹³, C(O)OR¹⁰, C(O)R¹⁴, S(O)ₘ-R¹⁴, - C(=NOC₁-C₄-alkyl)H, or -C(=NOC₁-C₄-alkyl)-C₁-C₄-alkyl, wherein alkyl, alkenyl, alkynyl, cycloalkyl and alkoxy are unsubstituted or substituted with R¹¹;
R¹⁰ is H, C₁-C₄-alkyl, C₁-C₄-haloalkyl, C₃-C₆-cycloalkyl, C₃-C₆-halocycloalkyl, C₃-C₄-cycloalkyl-C₁-C₂-alkyl, C₃-C₄-halocycloalkyl-C₁-C₂alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};
R^{10a} is H, C₃-C₆-cycloalkyl, C₃-C₆-halocycloalkyl, C₃-C₄-cycloalkyl-C₁-C₂alkyl, C₃-C₄-halocycloalkyl-C₁-C₂alkyl, C(O)-C₁-C₄-alkyl, C(O)-C₁-C₄-haloalkyl, C(O)-C₃-C₄-cycloalkyl, C(O)-C₃-C₄-halocycloalkyl, SOₘ-C₁-C₄-alkyl, SOₘ-C₁-C₄-haloalkyl, SOₘ-C₃-C₆-cycloalkyl, or phenyl which is unsubstituted or partially or fully substituted with R^{a};R¹¹ is halogen, CN, NO₂, NR¹²R¹³, C(O)NH₂, C(S)NH₂, C(O)OH, OR¹⁰, Si(CH₃)₃; 3- to 6-membered heterocyclyl, 5- or 6-membered hetaryl, or phenyl, wherein the heterocyclyl, hetaryl and phenyl moieties are unsubstituted or substituted with halogen, C₁-C₃-haloalkyl, and/or CN;
R^{11a} is NR¹²R¹³, C(O)NH₂, C(S)NH₂, C(O)OH, OR¹⁰, Si(CH₃)₃; C₁-C₆-haloalkyl; C₂-C₆-alkenyl; C₂-C₆-haloalkenyl; C₂-C₆-alkynyl; C₂-C₆-haloalkynyl; C₃-C₄-cycloalkyl-C₁-C₂alkyl, wherein the cycloalkyl moiety is unsubstituted or substituted with 1 or 2 halogen; or 3- to 6-membered heterocyclyl, wherein the heterocyclyl moiety is unsubstituted or substituted with halogen, C₁-C₃-haloalkyl, and/or CN;
R¹², R¹³ are independently from each other H, C₁-C₄-alkyl, C₁-C₄-alkoxy, C₁-C₄-haloalkoxy, C₄-C₄-haloalkyl, C₃-C₆-cycloalkyl, C(O)-C₁-C₄-alkyl, C(O)-C₁-C₄-haloalkyl, C(O)-C₃-C₄-cycloalkyl, C(O)-C₃-C₄-halocycloalkyl, C(O)NH-C₁-C₄-alkyl, C(O)NH-C₁-C₄-haloalkyl, C(O)N(C₁-C₄-alkyl)-C₁-C₄-alkyl, C(O)N(C₁-C₄-haloalkyl)-C₁-C₄-alkyl, C(O)N(C₁-C₄-haloalkyl)-C₁-C₄-haloalkyl, C(O)NH-C₁-C₄-alkoxy, C(O)NH-C₁-C₄-haloalkoxy, C(O)NH-C₁-C₄-alkoxy-C₁-C₄-alkyl, C(O)NH-C₁-C₄-alkoxy-C₁-C₄-haloalkyl; C(O)NH-phenyl, C(O)NH-3-6-membered heterocyclyl or 5- or 6-membered hetaryl, C(O)NH-C₁-C₄-alkyl-phenyl, C(O)NH-C₁-C₄-alkyl-3-6-membered heterocyclyl or 5- or 6-membered hetaryl, wherein the phenyl, heterocyclyl and hetaryl moieties are unsubstituted or substituted with halogen, C₁-C₃-haloalkyl, and/or CN; S(O)ₘ-C₁-C₄-haloalkyl, S(O)ₘ-C₃-C₄-cycloalkyl, S(O)ₘ-C₃-C₄-halocycloalkyl; 3- to 6-membered heterocyclyl, 5- or 6-membered hetaryl, or phenyl, wherein the phenyl, heterocyclyl and hetaryl moieties are unsubstituted or substituted with halogen, C₁-C₃-haloalkyl, and/or CN; or
R¹² and R¹³ together with the atom(s) to which they are bound, form a 3-, 4-, 5-, 6-, or 7-membered saturated, partially or fully unsaturated heterocycle, which heterocycle may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
R^{12a}, R^{13a} are independently from each other C₁-C₄-alkyl, C₁-C₄-haloalkyl, C₃-C₆-cycloalkyl, unsubstituted or substituted with halogen, C₁-C₃-haloalkyl, and/or CN; or R^{12a} and R^{13a} together with the atom to which they are bound, form a 3-, 4-, 5-, 6-, or 7-membered saturated, partially or fully unsaturated heterocycle, wherein the heterocycle moiety may additionally contain 1 or 2 heteroatoms or heteroatom-containing groups selected from N, O, S(O)ₘ, and optionally one or two groups C(O) as ring members, and wherein the heterocycle moiety is unsubstituted or substituted with one or more R^{a};
R¹⁴ is H, C₁-C₆-alkyl, C₃-C₆-cycloalkyl, wherein the alkyl and cycloalkyl moieties are unsubstituted or partially or fully substituted with R¹¹; or 3- to 6-membered heterocyclyl, 5- or 6-membered hetaryl, or phenyl, wherein the phenyl, heterocyclyl and hetaryl moieties are unsubstituted or substituted with R^{a};
R^{a} is halogen, CN, NO₂, OH, C₁-C₄-alkyl, C₁-C₄-alkoxy, C₁-C₄-haloalkyl, C₁-C₄-alkoxy-C₁-C₄-alkyl, C₁-C₄-haloalkoxy, C₃-C₄-cycloalkyl, C₃-C₄-halocycloalkyl, S(O)ₘ-C₁-C₄-alkyl, S(O)ₘ-C₁-C₄-haloalkyl, S(O)ₘ-C₃-C₄-cycloalkyl, or S(O)ₘ-C₃-C₄-halocycloalkyl;
m is 0, 1, or 2;
n is 0, 1, 2, or 3;
X is N, CH, or CR³;
and the N-oxides, stereoisomers, tautomers, and agriculturally or veterinarily acceptable salts thereof.

2. The compound of claim 1, wherein R¹ is H, methyl or CH₂-cyclopropyl.

3. The compound of claim 1 or 2, wherein R² is H or C₁-C₃-alkyl, preferably methyl.

4. The compound of any one of claims 1 to 3, wherein X is CR³, preferably CH.

5. The compound of any one of claims 1 to 4, wherein n is 2 and R³ is in positions 3 and 5.

6. The compound of any one of claims 1 to 5, wherein at least one R³ group in (R³)ₙ is selected from R^{3d} , wherein
each R^{3d} is independently C₁-C₆-alkyl, C₃-C₆-cycloalkyl, C₂-C₆-alkenyl, C₂-C₆-alkynyl, C₃-C₆-cycloalkyl-C₁-C₆-alkyl, C₁-C₆-alkyl-C₃-C₆-cycloalkyl, wherein alkyl, alkenyl, alkynyl, and cycloalkyl are unsubstituted or substituted independently with halogen, CN, NO₂, OH, NR¹²R¹³, C(O)NR¹²R¹³, C(O)OR¹⁰, C(O)R¹⁴, OS(O)₂-R¹⁴ and S(O)ₘ-R¹⁴ wherein m is 1 or 2.

7. The compound of any one of claims 1 to 6, wherein R⁴ is selected from any one of: 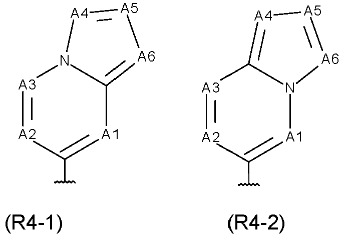 wherein
A1, A2 and A3 are independently CR^{4a} or N, provided not more than two of A1, A2 and A3 are N and at least one of A1 and A2 is N;
A4, A5 and A6 are independently CR^{4a} or N, provided not more than two of A4, A5 and A6 are N; each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl;
with the provisos that
not more than four of A1, A2, A3, A4, A5 and A6 are N;
if in R4-1 A3 and A4 are N, A2 and A5 are not N;
if in R4-1 A2 and A3 are N, A4 is not N;
if in R4-1 A4 and A5 are N, A3 and A6 are not N;
if in R4-2 A1 and A6 are N, A5 is not N;
if in R4-2 A5 and A6 are N, A1 and A4 are not N,
wherein preferably one of A1 and A2 is N and the other is CH or CCH₃.

8. The compound of claim 1 to 7, wherein
(1) R⁴ is selected from any one of the following groups 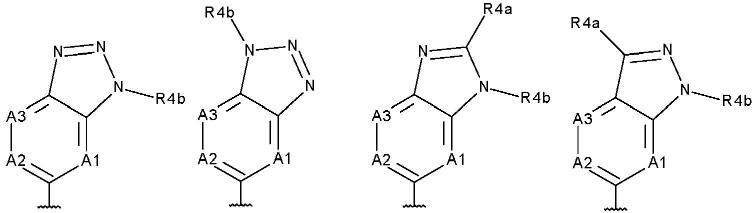 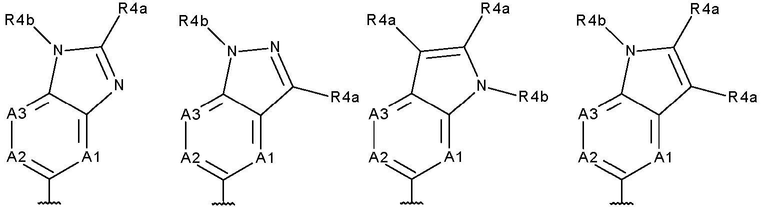 wherein
A1, A2 and A3 are independently CR^{4a} or N, provided not more than two of A1, A2 and A3 are N and at least one of A1 and A2 is N;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, - C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; and
R^{4b} is selected from **H,** halogen, -OH, -CN, -COOH, -CONH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl;
or
(2) R⁴ is selected from any one of the following 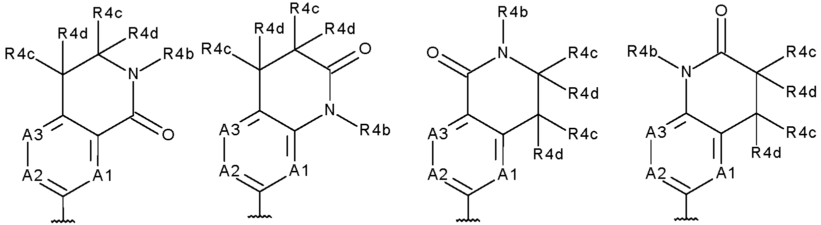 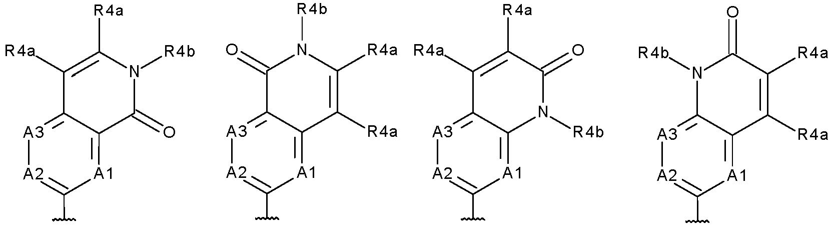 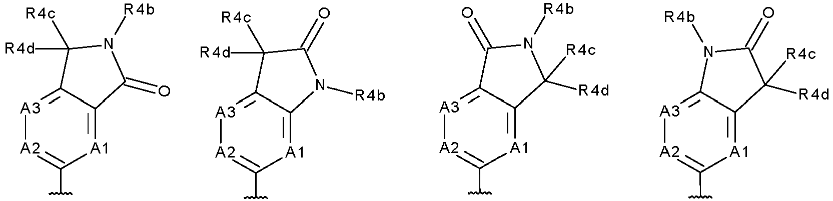 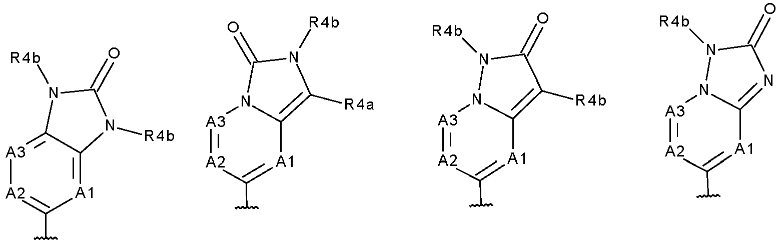 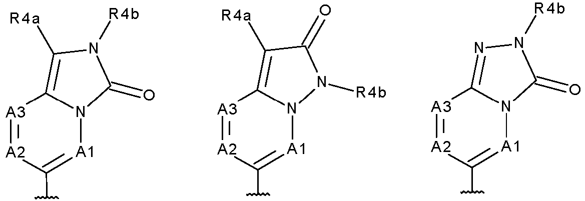 wherein
A1, A2 and A3 are independently CR^{4a} or N, provided not more than two of A1, A2 and A3 are N and at least one of A1 and A2 is N;
each R^{4a} is independently selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆cycloalkylsulfanyl, C₃-C₆cycloalkylsulfinyl, C₃-C₆cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl;
R^{4b} is selected from H, halogen, -OH, -CN, -COOH, -CONH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, and -C(=NOC₁-C₄-alkyl)-C₃-C₆-cycloalkyl; and
R^{4c} and R^{4d} are independently from each other selected from H, halogen, -OH, -CN, -COOH, -CONH₂, -NO₂, -SF₅, -NH₂, C₁-C₆-alkyl, C₃-C₆-cycloalkyl, C₃-C₆-cycloalkyl-C₁-C₆-alkyl, C₁-C₆-alkyl-C₃-C₆-cycloalkyl, C₁-C₃-haloalkyl, C₁-C₆-alkoxy, C₁-C₃-haloalkoxy, C₁-C₆-alkylthio, C₁-C₆-alkylsulfinyl, C₁-C₆-alkylsulfonyl, C₁-C₆-haloalkylthio, C₁-C₆-haloalkylsulfinyl, C₁-C₆-haloalkylsulfonyl, C₃-C₆-cycloalkylsulfanyl, C₃-C₆-cycloalkylsulfinyl, C₃-C₆-cycloalkylsulfonyl, NH(C₁-C₄-alkyl), -N(C₁-C₄-alkyl)₂, NHCO-C₁-C₄-alkyl, -N(C₁-C₄-alkyl)CO-C₁-C₄-alkyl, -CO₂C₁-C₄-alkyl, -CONH(C₁-C₄-alkyl), -CON(C₁-C₄-alkyl)₂, -C(=NOC₁-C₄-alkyl)H, -C(=NOC₁-C₄-alkyl)-C₁-C₄-alkyl, or
R^{4c} and R^{4d} together with the carbon atom to which they are bound, form a 3-, 4-, 5-, or 6-membered saturated or unsaturated carbocyclic ring;
or
(3) R⁴ is selected from any one of the following groups:

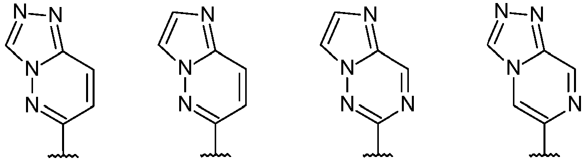

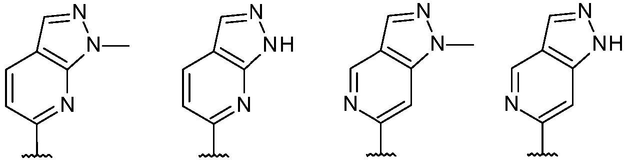

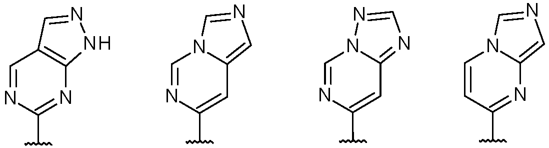

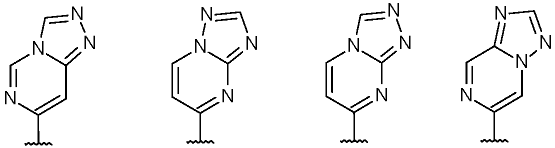

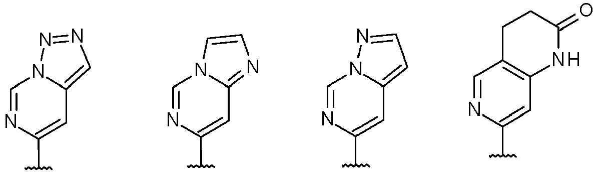

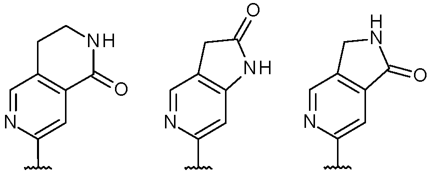

9. The compound of any one of claims 1 to 8, wherein the compound is selected from the group consisting of:
(1) *N*-[1-[2-([1,2,4]triazolo[4,3-b]pyridazin-6-yl)-1,2,4-triazol-3-yl]ethyl]-3,5-bis(trifluoromethyl)benzamide;
(2) *N*-[1-(2-imidazo[2,1-f][1,2,4]triazin-2-yl-1,2,4-triazol-3-yl)ethyl]-3,5-bis(trifluoromethyl)benzamide;
(3) 3-chloro-*N*-[1-[2-([1,2,4]triazolo[4,3-b]pyridazin-6-yl)-1,2,4-triazol-3-yl]ethyl]-5-(trifluoromethyl)benzamide;
(4) 3-chloro-*N*-[1-(2-imidazo[2,1-f][1,2,4]triazin-2-yl-1,2,4-triazol-3-yl)ethyl]-5-(trifluoromethyl)benzamide;
(5) *N*-[1-[2-(4-methylsulfanylimidazo[2,1-f][1,2,4]triazin-2-yl)-1,2,4-triazol-3-yl]ethyl]-3,5-bis(trifluoromethyl)benzamide;
(6) *N*-[1-(2-imidazo[2,1-f][1,2,4]triazin-2-yl-1,2,4-triazol-3-yl)ethyl]-3,5-bis(trifluoromethyl)benzamide;
(7) 3-chloro-*N*-[1-[2-(8-methyl-[1,2,4]triazolo[4,3-b]pyridazin-6-yl)-1,2,4-triazol-3-yl]ethyl]-5-(trifluoromethyl)benzamide;
(8) *N*-[1-[2-(8-methyl-[1,2,4]triazolo[4,3-b]pyridazin-6-yl)-1,2,4-triazol-3-yl]ethyl]-3,5-bis(trifluoromethyl)benzamide;
(9) 3-chloro-*N*-[1-[2-([1,2,4]triazolo[4,3-a]pyrazin-6-yl)-1,2,4-triazol-3-yl]ethyl]-5-(trifluoromethyl)benzamide;
(10) 3-chloro-*N*-methyl-*N*-[1-[2-([1,2,4]triazolo[4,3-b]pyridazin-6-yl)-1,2,4-triazol-3-yl]ethyl]-5-(trifluoromethyl)benzamide;
(11) *N*-[1-[2-([1,2,4]triazolo[4,3-a]pyrazin-6-yl)-1,2,4-triazol-3-yl]ethyl]-3,5-bis(trifluoromethyl)benzamide;
(12) *N*-methyl-N-[1-[2-([1,2,4]triazolo[4,3-b]pyridazin-6-yl)-1,2,4-triazol-3-yl]ethyl]-3,5-bis(trifluoromethyl)benzamide;
(13) 3-chloro-*N*-[1-[2-([1,2,4]triazolo[4,3-b]pyridazin-6-yl)-1,2,4-triazol-3-yl]ethyl]-5-(trifluoromethylsulfonyl)benzamide;
(14) [3-chloro-5-[1-[2-([1,2,4]triazolo[4,3-b]pyridazin-6-yl)-1,2,4-triazol-3-yl]ethylcarbamoyl]phenyl] trifluoromethanesulfonate;
(15) 3-chloro-*N*-[1-[2-([1,2,4]triazolo[4,3-b]pyridazin-6-yl)-1,2,4-triazol-3-yl]ethyl]-5-(trifluoromethylsulfinyl)benzamide;
(16) 3-chloro-5-methylsulfonyl-*N*-[1-[2-([1,2,4]triazolo[4,3-b]pyridazin-6-yl)-1,2,4-triazol-3-yl]ethyl]benzamide;
(17) 3-bromo-5-(1-cyano-1-methyl-ethyl)-*N*-[1-[2-([1,2,4]triazolo[4,3-b]pyridazin-6-yl)-1,2,4-triazol-3-yl]ethyl]benzamide;
(18) 3-(1-cyanocyclopropyl)-5-(difluoromethoxy)-*N*-[1-[2-([1,2,4]triazolo[4,3-b]pyridazin-6-yl)-1,2,4-triazol-3-yl]ethyl]benzamide;
(19) 3-chloro-5-(4-fluorophenyl)sulfonyl-*N*-[1-[2-([1,2,4]triazolo[4,3-b]pyridazin-6-yl)-1,2,4-triazol-3-yl]ethyl] benzamide;
(20) *N*-[1-[2-(2-oxo-3,4-dihydro-1*H*-1,6-naphthyridin-7-yl)-1,2,4-triazol-3-yl]ethyl]-3,5-bis(trifluoromethyl)benzamide;
(21) *N*-[1-[2-(2-oxo-1,3-dihydropyrrolo[3,2-c]pyridin-6-yl)-1,2,4-triazol-3-yl]ethyl]-3,5-bis(trifluoromethyl)benzamide;
(22) *N*-[1-[2-(1-oxo-2,3-dihydropyrrolo[3,4-c]pyridin-6-yl)-1,2,4-triazol-3-yl]ethyl]-3,5-bis(trifluoromethyl)benzamide;
(23) *N*-[1-[2-(5-oxo-7,8-dihydro-6*H*-2,6-naphthyridin-3-yl)-1,2,4-triazol-3-yl]ethyl]-3,5-bis(trifluoromethyl)benzamide;
(24) *N*-methyl-*N*-[1-[2-(1-methylpyrazolo[3,4-b]pyridin-6-yl)-1,2,4-triazol-3-yl]ethyl]-3,5-bis(trifluoromethyl)benzamide;
(25) N-methyl-*N*-[1-[2-(1*H*-pyrazolo[3,4-b]pyridin-6-yl)-1 ,2,4-triazol-3-yl]ethyl]-3,5-bis(trifluoromethyl)benzamide;
(26) *N*-[1-[2-(1*H*-pyrazolo[3,4-b]pyridin-6-yl)-1,2,4-triazol-3-yl]ethyl]-3,5-bis(trifluoromethyl)benzamide;
(27) *N*-[1-(2-imidazo[1,5-c]pyrimidin-7-yl-1,2,4-triazol-3-yl)ethyl]-3,5-bis(trifluoromethyl)benzamide;
(28) *N*-[1-[2-([1,2,4]triazolo[1,5-c]pyrimidin-7-yl)-1,2,4-triazol-3-yl]ethyl]-3,5-bis(trifluoromethyl)benzamide;
(29) *N*-[1-[2-(3-amino-[1,2,4]triazolo[4,3-b]pyridazin-6-yl)-1,2,4-triazol-3-yl]ethyl]-3,5-bis(trifluoromethyl)benzamide;
(30) 3-chloro-*N*-[1-[2-([1,2,4]triazolo[1,5-c]pyrimidin-7-yl)-1,2,4-triazol-3-yl]ethyl]-5-(trifluoromethyl)benzamide;
(31) *N*-[1-[2-(3-amino-[1,2,4]triazolo[4,3-b]pyridazin-6-yl)-1,2,4-triazol-3-yl]ethyl]-*N*-methyl-3,5-bis(trifluoromethyl)benzamide;
(32) *N*-methyl-*N*-[1-[2-(1-methylpyrazolo[4,5-c]pyridin-6-yl)-1,2,4-triazol-3-yl]ethyl]-3,5-bis(trifluoromethyl)benzamide;
(33) *N*-[1-[2-(1*H*-pyrazolo[4,5-c]pyridin-6-yl)-1,2,4-triazol-3-yl]ethyl]-3,5-bis(trifluoromethyl)benzamide;
(34) *N*-[1-(2-imidazo[1,5-a]pyrimidin-2-yl-1,2,4-triazol-3-yl)ethyl]-3,5-bis(trifluoromethyl)benzamide;
(35) *N*-[1-[2-(1*H*-pyrazolo[5,4-d]pyrimidin-6-yl)-1,2,4-triazol-3-yl]ethyl]-3,5-bis(trifluoromethyl)benzamide;
(36) *N*-methyl-*N*-[1-[2-(1*H*-pyrazolo[4,5-c]pyridin-6-yl)-1 ,2,4-triazol-3-yl]ethyl]-3,5-bis(trifluoromethyl)benzamide;
(37) *N*-[1-[2-(3-amino-[1,2,4]triazolo[4,3-a]pyrazin-6-yl)-1,2,4-triazol-3-yl]ethyl]-3,5-bis(trifluoromethyl)benzamide;
(38) *N*-[1-[2-(3-amino-[1,2,4]triazolo[4,3-a]pyrazin-6-yl)-1,2,4-triazol-3-yl]ethyl]-N-methyl-3,5-bis(trifluoromethyl)benzamide;
(39) 6-[5-[(1*S*)-1-[[3,5-bis(trifluoromethyl)benzoyl]amino]ethyl]-1,2,4-triazol-1-yl]-*N,N-*dimethyl-[1,2,4]triazolo[4,3-b]pyridazine-8-carboxamide;
(40) 6-[5-[(1*S*)-1-[[3,5-bis(trifluoromethyl)benzoyl]-methyl-amino]ethyl]-1,2,4-triazol-1-yl]-[1,2,4]triazolo[4,3-b]pyridazine-8-carboxylic acid;
(41) ethyl 6-[5-[(1*S*)-1-[[3,5-bis(trifluoromethyl)benzoyl]amino]ethyl]-1,2,4-triazol-1-yl]-[1,2,4]triazolo[4,3-a]pyrazine-3-carboxylate;
(42) 6-[5-[(1*S*)-1-[[3,5-bis(trifluoromethyl)benzoyl]amino]ethyl]-1,2,4-triazol-1-yl]-[1,2,4]triazolo[4,3-b]pyridazine-8-carboxamide;
(43) *N*-methyl-*N*-[(1*S*)-1-[2-(8-methyl-[1,2,4]triazolo[4,3-b]pyridazin-6-yl)-1,2,4-triazol-3-yl]ethyl]-3,5-bis(trifluoromethyl)benzamide;
(44) 6-[5-[(1*S*)-1-[[3,5-bis(trifluoromethyl)benzoyl]-methyl-amino]ethyl]-1,2,4-triazol-1-yl]-[1,2,4]triazolo[4,3-b]pyridazine-8-carboxamide;
(45) 6-[5-[(1*S*)-1-[[3,5-bis(trifluoromethyl)benzoyl]amino]ethyl]-1,2,4-triazol-1-yl]-[1,2,4]triazolo[4,3-a]pyrazine-3-carboxamide;
(46) 3-(1-cyanocyclopropyl)-5-(difluoromethoxy)-*N*-[1-[2-([1,2,4]triazolo[1,5-*c*]pyrimidin-7-yl)-1,2,4-triazol-3-yl]ethyl]benzamide;
(47) 3-chloro-N-[1-[2-([1,2,4]triazolo[1,5-c]pyrimidin-7-yl)-1,2,4-triazol-3-yl]ethyl]-5-(trifluoromethylsulfonyl)benzamide;
(48) 6-[5-[(1S)-1-[[3,5-bis(trifluoromethyl)benzoyl]amino]ethyl]-1,2,4-triazol-1-yl]imidazo[1,2-b]pyridazine-8-carboxamide;
(49) N-[1-[2-(2-oxo-1,3,3a,7a-tetrahydroimidazo[4,5-c]pyridin-6-yl)-1,2,4-triazol-3-yl]ethyl]-3,5-bis(trifluoromethyl)benzamide;
(50) N-[1-(2-imidazo[1,2-c]pyrimidin-7-yl-1,2,4-triazol-3-yl)ethyl]-3,5-bis(trifluoromethyl)benzamide;
(51) 3-chloro-N-[1-[2-([1,2,4]triazolo[1,5-c]pyrimidin-7-yl)-1,2,4-triazol-3-yl]ethyl]-5-(trifluoromethylsulfinyl)benzamide;
(52) 3-chloro-N-[1-[2-([1,2,4]triazolo[1,5-c]pyrimidin-7-yl)-1,2,4-triazol-3-yl]ethyl]-5-(trifluoromethoxy)benzamide;
(53) 3-bromo-5-(1-cyano-1-methyl-ethyl)-N-[1-[2-([1,2,4]triazolo[1,5-c]pyrimidin-7-yl)-1,2,4-triazol-3-yl]ethyl]benzamide;
(54) [3-chloro-5-[1-[2-([1,2,4]triazolo[1,5-c]pyrimidin-7-yl)-1,2,4-triazol-3-yl]ethylcarbamoyl]phenyl] trifluoromethanesulfonate;
(55) 3-(1-cyanocyclopropyl)-N-[1-[2-([1,2,4]triazolo[1,5-c]pyrimidin-7-yl)-1,2,4-triazol-3-yl]ethyl]-5-(trifluoromethoxy)benzamide;
(56) N-[1-(2-pyrazolo[1,5-c]pyrimidin-5-yl-1,2,4-triazol-3-yl)ethyl]-3,5-bis(trifluoromethyl)benzamide;
(57) N-[1-[2-(3,3-dimethyl-2-oxo-3a,7a-dihydro-1H-pyrrolo[3,2-c]pyridin-6-yl)-1,2,4-triazol-3-yl]ethyl]-3,5-bis(trifluoromethyl)benzamide;
(58) N-[1-[2-(triazolo[1,5-c]pyrimidin-5-yl)-1,2,4-triazol-3-yl]ethyl]-3,5-bis(trifluoromethyl)benzamide;
(59) N-[1-[2-(3,3-dimethyl-2-oxo-1,4,4a,8a-tetrahydro-1,6-naphthyridin-7-yl)-1,2,4-triazol-3-yl]ethyl]-3,5-bis(trifluoromethyl)benzamide;
(60) *N*-[1-[2-(2-ethyl-[1,2,4]triazolo[1,5-c]pyrimidin-7-yl)-1,2,4-triazol-3-yl]ethyl]-3,5-bis(trifluoromethyl)benzamide;
(61) *N*-[1-[2-(3-hydroxyimidazo[1,5-c]pyrimidin-7-yl)-1,2,4-triazol-3-yl]ethyl]-3,5-bis(trifluoromethyl)benzamide;
(62) N-[1-[2-(2-methyl-[1,2,4]triazolo[1,5-c]pyrimidin-7-yl)-1,2,4-triazol-3-yl]ethyl]-3,5-bis(trifluoromethyl)benzamide;
(63) 2-[5-[1-[[3,5-bis(trifluoromethyl)benzoyl]amino]ethyl]-1,2,4-triazol-1-yl]imidazo[1,5-b]pyridazine-4-carboxamide;
(64) N-[1-[2-(2-chloro-[1,2,4]triazolo[1,5-c]pyrimidin-7-yl)-1,2,4-triazol-3-yl]ethyl]-3,5-bis(trifluoromethyl)benzamide;
(65) N-[1-[2-(3-amino-[1,2,4]triazolo[4,3-c]pyrimidin-7-yl)-1,2,4-triazol-3-yl]ethyl]-3,5-bis(trifluoromethyl)benzamide;
(66) N-[1-[2-(3-methyl-[1,2,4]triazolo[4,3-c]pyrimidin-7-yl)-1,2,4-triazol-3-yl]ethyl]-3,5-bis(trifluoromethyl)benzamide;
(67) N-[1-[2-(2-aminopyrazolo[1,5-c]pyrimidin-5-yl)-1,2,4-triazol-3-yl]ethyl]-3,5-bis(trifluoromethyl)benzamide; or
the N-oxide, stereoisomer, tautomer, agriculturally or veterinarily acceptable salt of any one of compounds (1) to (67).

10. A composition, comprising at least one compound of formula I according to any of claims 1 to 9 and any one of a further active substance, an excipient or solvent.

11. A method for combating or controlling invertebrate pests, which method comprises contacting said pest or its food supply, habitat or breeding grounds with a pesticidally effective amount of at least one compound according to any of claims 1 to 9 or the composition according to claim 10.

12. A method for protecting growing plants or an animal from attack or infestation by invertebrate pests, which method comprises contacting a plant, or soil or water wherein the plant is growing, or an animal with a pesticidally effective amount of at least one compound according to any of claims 1 to 9 or the composition according to claim 10.

13. Seed comprising a compound according to any of claims 1 to 9 or a composition according to claim 10, in an amount of from 0.1 g to 10 kg per 100 kg of seed.

14. Use of a compound of the formula I according to any of claims 1 to 9 or of the compositions according to claim 10, for protecting growing plants from attack or infestation by invertebrate pests.
